# EUROPEAN PATENT APPLICATION

(11) **EP 3 772 513 A1**
(43) Date of publication of application: **10.02.2021**
(21) Application number: 19190957.1
(22) Date of filing: 09.08.2019
(51) Int. Cl.: C07D 401/14, C07D 403/04, C07D 403/14, C07D 407/14, C07D 409/14, C07D 413/14, A61P 35/00, A61K 31/4545

(54) **SHP2 INHIBITORS**

(71) Applicant: C.N.C.C.S. S.c.a.r.l. Collezione Nazionale Dei Composti Chimici e Centro Screening, 00071 Pomezia (RM) (IT); IRBM S.P.A., 00071 Pomezia (RM) (IT)
(72) Inventor: The designation of the inventor has not yet been filed
(74) Representative: Capasso, Olga

(57) **Abstract**

The present invention relates to new compounds capable of inhibiting the activity of SHP2 phosphatase, having the general Formula (I) indicated below:

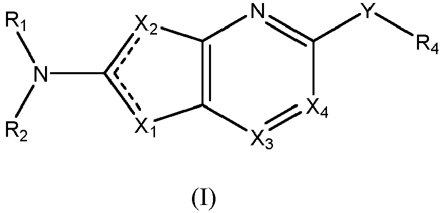

## Description

### FIELD OF THE INVENTION

The present invention relates to new compounds capable of inhibiting the activity of SHP2 phosphatase. Compounds of the invention can be used for the treatment of disorders associated with SHP2 deregulation. The present invention also relates to pharmaceutical compositions containing said compounds and to their method of manufacture.

### BACKGROUND OF THE INVENTION

Src homology phosphotyrosine phosphatase 2 (SHP2) encoded by *PTPN11* is a non-receptor protein tyrosine phosphatase (PTP) composed of a C-terminal domain, a PTP domain, and two N-terminal Src homology (N-SH2) domains, that contributes to multiple cellular functions including proliferation, differentiation, cell cycle maintenance and migration. SHP2 is a positive regulator of signalling downstream of several receptor tyrosine kynases through the Ras-mitogen-activated protein kinase, the JAK-STAT or the phosphoinositol-3-kinase-AKT pathways. The protein exists in an inactive, self-inhibited conformation, stabilized by a binding network involving residues from both the N-SH2 domains and the catalytic PTP domain. Recruitment of SHP2 to an activated receptor releases the self-inhibitory conformation and leads to catalytic activation of its phosphatase domain. In addition to its function as a phosphatase, SHP2 also serves as a docking protein to recruit other signalling intermediates through its two amino terminus N-SH2 domains. Since SHP2 is a positive regulator of cellular signalling leading to proliferation, differentiation, and survival, its constitutive activation is associated with oncogenesis.

SHP2 emerged as an attractive target for therapeutic targeting in the treatment of various diseases, such as Noonan Syndrome, Leopard Syndrome, juvenile myelocytic leukemias, neuroblastoma, melanoma, acute myeloid leukemia and cancers of the breast, lung and colon.

Both academic institutions and pharmaceutical companies have disclosed drug discovery programs exploiting SHP2 inhibitors based on different heterocyclic scaffolds.

WO2015/107493, WO2015/107494 and WO2015/107495 from Novartis disclose compounds of general formula (A) as indicated below:

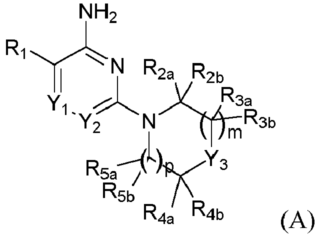

Still from Novartis the compounds of general structures (B), (C), (D) and (E) indicated below are disclosed in, respectively, WO2016/203404, WO2016/203405 and WO2017/216706:

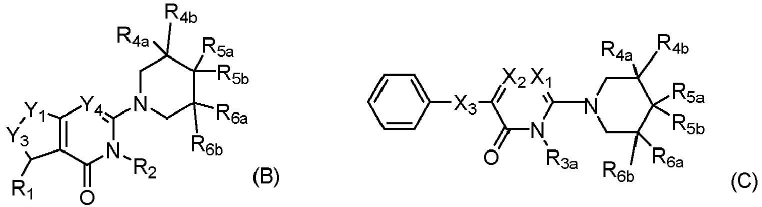

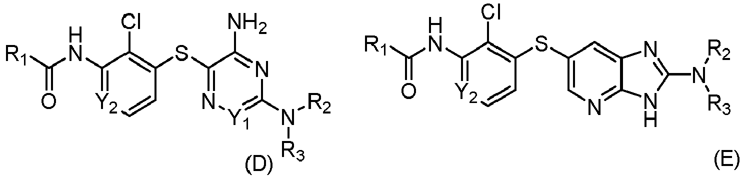

Jacobio Pharmaceuticals disclosed in WO2017/211303 and in WO2018/172984 pyrazine derivatives of structures (F) and (G) as indicated below:

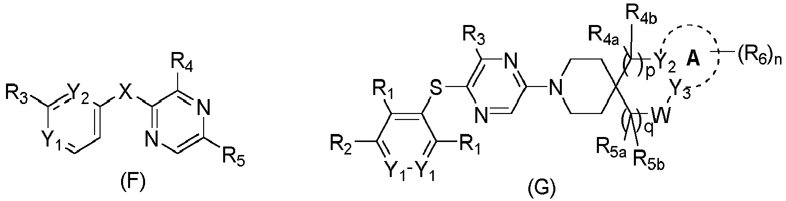

Futher pyridine, pyrazine and triazine compounds as allosteric SHP2 inhibitors have been recently disclosed by Revolution Medicines in WO2018/013597, WO2018/136264 and WO2019/075265.

WO2018/136265 and WO2019/118909 both relate to bicyclic heteroaromatic scaffolds comprising imidazopyrazines, triazolopyrazines, pyrazolopyridine, imidazopyrimidines of general structure (H):

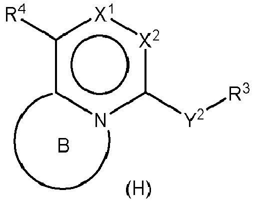

Pyrazolopyrazines and ring-fused pyrimidin-4-ones have been disclosed by the Board of Regents, University of Texas System, in WO2017/210134 and in WO2017/156397, respectively.

Pyrazolopyrazines were further disclosed by Relay Therapeutics in WO2018/081091, WO2018/218133, WO2018/057884 and WO2019/067843.

SHP2 therefore represents a highly attractive target for the development of novel therapies for the treatment of various diseases where it is involved. Therefore, there is the need to develop novel therapeutic agents that act as SHP2 inhibitors. The compounds of the present invention fulfill such need since they are small molecules capable of inhibiting the activity of SHP2.

### DESCRIPTION OF THE INVENTION

The present invention relates to heterocyclic compounds useful as SHP2 inhibitors and for the treatment of conditions mediated by SHP2.

The inventors have found that compounds having a specific general formula surprisingly act as potent SHP2 inhibitors, as evidenced by both enzymatic and cellular IC₅₀ values for SHP2 in the low nanomolar or micromolar range.

Therefore, it is an object of the present invention a compound of Formula (I):

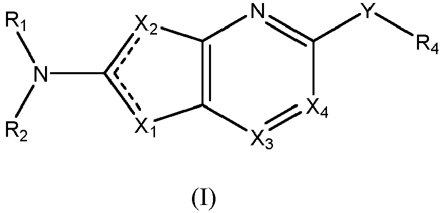

Wherein:
-̅-̅-̅-̅-̅ represents a single bond or a double bond;
X₁ is N, S, O or NR₃;
X₂ is N or NR₃;
if X₁ is N then X₂ is NR₃;
if X₁ is S, O or NR₃ then X₂ is N;
X₃ is N or CRₓ₃ and Rₓ₃ is H, halogen or C₁₋₃alkyl;
X₄ is N or CR₅;
Y is S, O, NR₆, CH₂, CHF, CF₂, CHOH, C(O), SO, SO₂ or a single bond;
R₁ and R₂ are each independently selected from:
   - hydrogen;
   - linear or branched C₁₋₁₂alkyl optionally substituted with one or more substituents independently selected from the group consisting of: OH, halogen, N(R₇)₂, aryl, heteroaryl, partially unsaturated heteroaryl, C₃₋₉cycloalkyl, C₃₋₉heterocycloalkyl and spiro-C₃₋₈cycloalkyl ring optionally containing one heteroatom selected from the group consisting of O, N and S, wherein each of said aryl, heteroaryl, partially unsaturated heteroaryl, C₃₋₉cycloalkyl, C₃₋₉heterocycloalkyl and spiro-C₃₋₈cycloalkyl ring is optionally further substituted with one or more groups independently selected from the group consisting of: C(O)CH₃, C(O)OCH₃, heteroaryl, aryl, OH, halogen, NH₂, C₁₋₆alkyl optionally substituted with N(R₇)₂, C₁₋₃alkylaryl, C₁₋₃alkylheteroaryl, haloC₁₋₆alkyl, hydroxyC₁₋₆alkyl, CN, haloC₁₋₆alkoxy, C₁₋₆alkoxy, C₅₋₇heterocycloalkoxy and a cyclic amine selected from the group consisting of: pyrrolidine, piperidine, morpholine, thiomorpholine, piperazine, N-methylpyperazine and pyrrolidin-3-yloxy; and
   - a cyclic structure selected from the group consisting of: C₃₋₇cycloalkyl, C₃₋₉heterocycloalkyl, aryl, heteroaryl and partially unsaturated heteroaryl, each of said cyclic structure being optionally substituted with one ore more substituents independently selected from the group consisting of: halogen, C₁₋₃alkyl optionally substituted with N(R₇)₂, C₁₋₃alkylaryl, C₁₋₃alkylheteroaryl, C(O)OC₁₋₃alkyl, C(O)C₁₋₃alkyl, N(R₇)₂, aryl and heteroaryl, each of said aryl or heteroaryl being optionally substituted with one or more substituents independently selected from the group consisting of: halogen, hydroxyl, cyano, C₁₋₃alkoxy and C₁₋₃haloalkyl;
or R₁ and R₂ form together with the nitrogen atom to which they are attached a cyclic amine of formula (II)

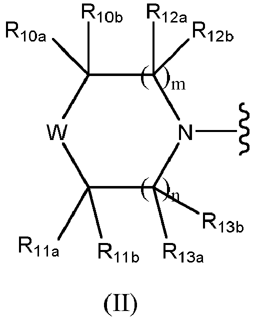

Wherein:
m and n are each independently selected from 0, 1 and 2;
W is absent, O, CR₈R₉, NR₁₀, S, SO or SO₂;
R₁₀ₐ, R1_{0b}, R₁₁ₐ, R_{11b}, R₁₂ₐ, R_{12b}, R₁₃ₐ, R_{13b} are each independently selected from the group consisting of: H, C₁₋₆alkyl, aminoC₁₋₆alkyl, C₁₋₆alkoxy, halogen, NH₂, CN, OH, C(O)NH₂, heteroaryl, optionally substituted aryl, hydroxy-C₁₋₆alkyl, halo-C₁₋₆alkyl, C₁₋₆alkoxy and C₃₋₉heterocycloalkyl;
or any two of R₁₀ₐ, R_{10b}, R₁₁ₐ, R_{11b}, R₁₂ₐ, R_{12b}, R₁₃ₐ, R_{13b} which are not germinal groups, taken together represent a single bond, a C₁₋₄alkanediyl or a C₂₋₄alkenediyl, each of said C₁₋₄alkanediyl or C₂₋₄alkenediyl being independently optionally substituted with one or more of C₁₋₄alkyl and/or halogen; said single bond or said optionally substituted C₁₋₄alkanediyl or C₂₋₄alkenediyl forming together with the bridging atoms to which they are respectively linked a 4-10 membered saturated or partially unsaturated ring;
or any of R₁₀ₐ and R_{10b}, R₁₁ₐ and R_{11b}, R₁₂ₐ and R_{12b} or R₁₃ₐ and R_{13b} taken together with the carbon atom to which they are attached form a 3-7 membered saturated ring optionally containing one or more of O, S, N and/or C(O), the 3-7 membered saturated ring optionally being substituted with one or more substituents each independently selected from the group consisting of halogen, OH, haloC₁₋₆alkyl, CN, C₁₋₆alkyl, C₁₋₆alkoxy, haloC₁₋₆alkoxy, C(O)OC₁₋₃alkyl and C(O)C₁₋₃alkyl;
R₈ and R₉ are each independently selected from the group consisting of: H, OH, NH₂, aryl, heteroaryl, C₁-₃alkylheteroaryl, C(O)NH₂, C₁-₆alkyl, aminoC₁-₆alkyl, NHCH₃ and NHSO₂CH₃;
or R₈ and R₉ taken together with the carbon atom to which they are bound form a spiro-C₃₋₈cycloalkyl ring of formula (III) as indicated below: 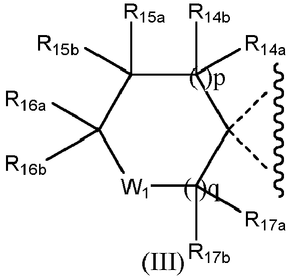 wherein:
   p and q are each independently selected from 0, 1 and 2;
   W₁ is absent, O, S, SO₂, CHF, CF₂ or NR_{w} and R_{w} is H, C(O)C₁₋₆alkyl, C(O)OC₁₋₆alkyl or C₁₋₆alkyl optionally substituted with one or more substituents each independently selected from the group consisting of: aryl, heteroaryl, OH, C₁₋₃alkoxy and halogen;
   R₁₄ₐ, R_{14b}, R₁₅ₐ, R_{15b}, R₁₆ₐ, R_{16b}, R₁₇ₐ, R_{17b} are each independently selected from the group consisting of: H, NH₂, NHSOC₁₋₆alkyl, NHSO₂C₁₋₆alkyl, NHC₁₋₆alkyl, N(C₁₋₆alkyl)₂, NHC(O)C₁₋₆alkyl, NHC(O)OC₁₋₆alkyl, halogen, OH, CN, C₁₋₃alkoxy, C₁₋₆alkyl optionally substituted with NH₂;
   or R₁₅ₐ and R_{15b} taken together with the carbon atom to which they are bound form a spiro-C₃₋₆cycloalkyl ring optionally containing an heteroatom selected from the group consisting of: N, O and S;
   or R₁₅ₐ and R₁₆ₐ are absent and R_{15b} and R_{16b} are joined together to form an aryl or heteroaryl ring, each being optionally substituted with one or more substituents each independently selected from the group consisting of: halogen, NH₂, NHC₁₋₆alkyl, N(C₁₋₆alkyl)₂, NHC(O)C₁₋₆alkyl, NHC(O)OC₁₋₆alkyl, NHSOC₁₋₆alkyl, NHSO₂C₁₋₆alkyl, CN, OH, C₁₋₆alkyl, C₁₋₆alkoxy, haloC₁₋₆alkyl and haloC₁₋₆alkoxy;
   or R_{15b} and R_{16b} are joined together to form a C₅₋₇cycloalkyl or C₅₋₇heterocycloalkyl ring, each being independently optionally substituted with one or more substituents each independently selected from the group consisting of: halogen, NH₂, NHC₁₋₆alkyl, N(C₁₋₆alkyl)₂, NHC(O)C₁₋₆alkyl, NHC(O)OC₁₋₆alkyl, NHSOC₁₋₆alkyl, NHSO₂C₁₋₆alkyl, CN, OH, =O, C₁₋₆alkyl, C₁₋₆alkoxy, haloC₁₋₆alkyl, haloC₁₋₆alkoxy and SO₂CH₃;
   R₁₀ is H, SO₂C₁₋₆alkyl, C(O)C₁₋₆alkyl, C(O)OC₁₋₆alkyl or C₁₋₆alkyl optionally substituted with one or more groups each independently selected from the group consisting of: OH, NH₂, NHC(O)C₁₋₆alkyl, C₁₋₆alkoxy, halogen, cyano, aryl and heteroaryl;
   or R₁₀ and R₁₀ₐ are joined together to form a C₃₋₇heterocycloalkyl ring optionally containing another heteroatom selected from the group consisting of: N, S and O and optionally substituted with one or more groups each independently selected from the group consisting of: C₁₋₆alkyl, halogen, OH and CN;
   or R₁ and R₂ form together with the nitrogen atom to which they are attached a monocyclic or polycyclic heteroaryl or partially unsaturated heteroaryl ring, each of said ring is optionally substituted with one or more groups each independently selected from the group consisting of: halogen, C₁-₆alkyl, C₁-₆alkoxy, haloC₁₋₆alkyl, haloC₁₋₆alkoxy, OH, CN, SO₂C₁₋₆alkyl, NH₂ and C(O)NH₂;
   R₃ is H, C₁-₆alkyl, C₁-₆alkyl-cycloalkyl or C₁-₆alkyl-heterocycloalkyl, each of said groups being optionally substituted with one or more groups independently selected from the group consisting of: C₁-₆alkoxy, hydroxyl, cyano, C(O)OC₁-₆alkyl, C(O)NH₂, C(O)NHC₁-₆alkyl; C(O)N(C₁-₆alkyl)₂, SO₂C₁-₆alkyl, SOC₁-₆alkyl, SO₂NHC₁-₆alkyl and SO₂N(C₁-₆alkyl)₂;
   R₄ is a ring selected from the group consisting of: aryl, heteroaryl, partially unsaturated aryl, partially unsaturated heteroaryl, cycloalkyl, heterocycloalkyl, C₁-₆alkylaryl, C₁-₆alkylheteroaryl and C₁-₆alkylC₃-₇cycloalkyl, each of said ring being optionally substituted with one or more groups each independently selected from the group consisting of: halogen, hydroxy, cyano, C₁-₆alkyl, C₁-₇alkenyl, C₁-₆alkoxy, haloC₁-₆alkyl, haloC₁-₆alkoxy, C₃-₇cycloalkyl, cyano-C₃-₇cycloalkyl, -SF₅, C₅₋₇heterocycloalkoxy, optionally substituted aryl or heteroaryl, partially unsaturated heteroaryl, optionally substituted aryloxy, C(O)OH, C(O)OC₁-₆alkyl, C(O)C₁-₆alkyl, C₁-₆alkylCOOH, SO₂C₁-₆alkyl and N(R₇)₂;
   R₅ is H, halogen, C₁-₆alkyl, C₁-₆alkoxy, haloC₁-₆alkyl, haloC₁-₆alkoxy, OH, C₃-₆cycloalkyl, C₃-₆cycloalkoxy, C₃₋₆heterocycloalkyl or NRₓ₁Rₓ₂ wherein Rₓ₁ and Rₓ₂ are each independently H or C₁-₆alkyl, or Rₓ₁ and Rₓ₂ taken together with the nitrogen atom to which they are attached form a 3-7 membered saturated ring optionally containing one or more heteroatoms each independently selected from the group consisting of O, S and N;
   R₆ is H or C₁-₆alkyl;
   or R₄ and R₆ form together with the nitrogen atom to which they are attached a C₃₋₉heterocycloalkyl, a heteroaryl or a partially unsaturated heteroaryl ring, each being optionally substituted with one or more groups independently selected from the group consisting of: OH, halogen, CN, C₁-₆alkyl, C₁-₆alkoxy, haloC₁-₆alkyl, haloC₁-₆alkoxy, C₃-₇cycloalkyl, C₃-₇cycloalkoxy, aryl and heteroaryl;
   each R₇ is independently selected from the group consisting of: H, C₁-₆alkyl, SO₂C₁-₆alkyl, SOC₁-₆alkyl, C(O)OC₁-₆alkyl, C(O)C₁-₆alkyl, C₃-₇cycloalkyl, aryl, heteroaryl, C₁-₆alkylaryl and C₁-₆alkylheteroaryl;
   or two R₇ taken together with the nitrogen atom to which they are bound form a 3 to 7 membered cyclic amine optionally containing one additional heteroatom selected from the group consisting of S, N and O, said 3 to 7 membered cyclic amine being optionally substituted with one or more groups each independently selected from the group consisting of: OH, halogen, CN, C₁-₆alkyl, C₁-₆alkoxy, haloC₁-₆alkyl, haloC₁-₆alkoxy, C₃-₇cycloalkyl and C₃-₇cycloalkoxy;
   or a pharmaceutically acceptable salt, tautomer, solvate, or stereoisomer thereof.

As used herein, any reference to "the compound(s) of the invention" includes a reference also to any pharmaceutically acceptable salt, tautomer, solvate, or stereoisomer thereof.

Preferably, the compound of Formula (I) is of Formula (I') or (I"):

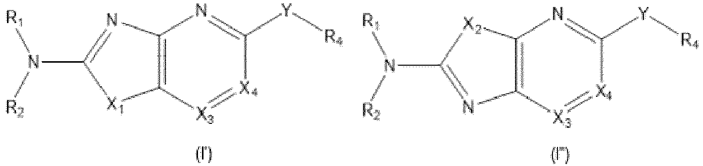

All the following preferred embodiments may refer to all of Formulas (I), (I') and (I") and may be combined amongst themselves in any possible way that would give rise to a chemically-feasible formula.

Preferably, in the compound of Formula (I), X₁ is N and X₂ is NR₃ or X₁ is NR₃ and X₂ is N.

Preferably, R₁ and R₂ are each independently selected from:
- hydrogen;
- linear or branched C₁₋₁₂alkyl optionally substituted with one or more substituents independently selected from the group consisting of: OH, halogen, N(R₇)₂, aryl, heteroaryl, partially unsaturated heteroaryl, C₃₋₉cycloalkyl, C₃₋₉heterocycloalkyl and spiro-C₃₋₈cycloalkyl ring optionally containing one heteroatom selected from the group consisting of O, N and S, wherein each of said aryl, heteroaryl, partially unsaturated heteroaryl, C₃₋₉cycloalkyl, C₃₋₉heterocycloalkyl and spiro-C₃₋₈cycloalkyl ring is optionally further substituted with one or more groups independently selected from the group consisting of: C(O)CH₃, C(O)OCH₃, heteroaryl, aryl, OH, halogen, NH₂, C₁₋₆alkyl optionally substituted with N(R₇)₂, C₁₋₃alkylaryl, C₁₋₃alkylheteroaryl, haloC₁₋₆alkyl, hydroxyC₁₋₆alkyl, CN, haloC₁₋₆alkoxy, C₁₋₆alkoxy, C₅₋₇heterocycloalkoxy and a cyclic amine selected from the group consisting of: pyrrolidine, piperidine, morpholine, thiomorpholine, piperazine, N-methylpyperazine and pyrrolidin-3-yloxy; and
- a C₃₋₇cycloalkyl, C₃₋₉heterocycloalkyl or a partially unsaturated heteroaryl selected from bicyclo[1.1.1]pentane, pirrolidine, piperidine, morpholine, piperazine, 2-azaspiro[3.3]heptane, azepan-2-one, 3-azaspiro[5.5]undecane, 2-azaspiro[4.5]decane, 3-azabicyclo[3.3.1]nonane, 3-oxa-7-azabicyclo[3.3.1]nonane, 6',7'-dihydrospiro[azetidine-3,5'-pyrrolo[1,2-a]imidazole], 5-azaspiro[3.5]nonane, 1-thia-7-azaspiro[3.5]nonane 1,1-dioxide, 3-azabicyclo[3.2.0]heptane, 2-azabicyclo[2.1.1]hexane, 6-azabicyclo[3.2.1]octane, octahydroindole, octahydro-1H-isoindole, 5-oxa-2-azaspiro[3.4]octane and 1,2,3,4-tetrahydroquinoline, each of said groups being optionally substituted with one ore more substituents independently selected from the group consisting of: halogen, C₁₋₃alkyl optionally substituted with N(R₇)₂, C₁₋₃alkylaryl, C₁₋₃alkylheteroaryl, C(O)OC₁₋₃alkyl, C(O)C₁₋₃alkyl, N(R₇)₂, aryl and heteroaryl, each of said aryl or heteroaryl being optionally substituted with one or more substituents independently selected from the group consisting of: halogen, hydroxyl, cyano, C₁₋₃alkoxy and C₁₋₃haloalkyl;
or R₁ and R₂ form together with the nitrogen atom to which they are attached a cyclic amine of formula (II)

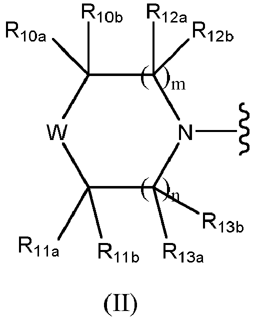

Wherein:
m and n are each independently selected from 0, 1 and 2;
W is absent, O, CR₈R₉, NR₁₀, S, SO or SO₂;
R₁₀ₐ, R_{10b}, R₁₁ₐ, R_{11b}, R₁₂ₐ, R_{12b}, R₁₃ₐ, R_{13b} are each independently selected from the group consisting of: H, C₁₋₆alkyl, aminoC₁₋₆alkyl, C₁₋₆alkoxy, halogen, NH₂, CN, OH, C(O)NH₂, heteroaryl, optionally substituted aryl, hydroxy-C₁₋₆alkyl, halo-C₁₋₆alkyl, C₁₋₆alkoxy and C₃₋₉heterocycloalkyl;
or any two of R₁₀ₐ, R_{10b}, R₁₁ₐ, R_{11b}, R₁₂ₐ, R_{12b}, R₁₃ₐ, R_{13b} which are not germinal groups, taken together represent a single bond, a C₁₋₄alkanediyl or a C₂₋₄alkenediyl, each of said C₁₋₄alkanediyl or C₂₋₄alkenediyl being independently optionally substituted with one or more of C₁₋₄alkyl and/or halogen; said single bond or said optionally substituted C₁₋₄alkanediyl or C₂₋₄alkenediyl forming together with the bridging atoms to which they are respectively linked a 4-10 membered saturated or partially unsaturated ring;
or any of R₁₀ₐ and R_{10b}, R₁₁ₐ and R_{11b}, R₁₂ₐ and R_{12b} or R₁₃ₐ and R_{13b} taken together with the carbon atom to which they are attached form a 3-7 membered saturated ring optionally containing one or more of O, S, N and/or C(O), the 3-7 membered saturated ring optionally being substituted with one or more substituents each independently selected from the group consisting of halogen, OH, haloC₁₋₆alkyl, CN, C₁₋₆alkyl, C₁₋₆alkoxy, haloC₁₋₆alkoxy, C(O)OC₁₋₃alkyl and C(O)C₁₋₃alkyl;
R₈ and R₉ are each independently selected from the group consisting of: H, OH, NH₂, aryl, heteroaryl, C₁-₃alkylheteroaryl, C(O)NH₂, C₁-₆alkyl, aminoC₁-₆alkyl, NHCH₃ and NHSO₂CH₃;
or R₈ and R₉ taken together with the carbon atom to which they are bound form a spiro-C₃₋₈cycloalkyl ring of formula (III) as indicated below: 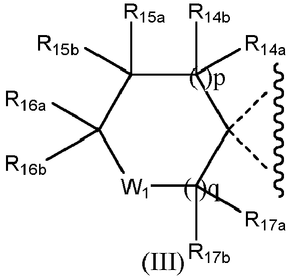 wherein:
   p and q are each independently selected from 0, 1 and 2;
   W₁ is absent, O, S, SO₂, CHF, CF₂ or NR_{w} and R_{w} is H, C(O)C₁₋₆alkyl, C(O)OC₁₋₆alkyl or C₁₋₆alkyl optionally substituted with one or more substituents each independently selected from the group consisting of: aryl, heteroaryl, OH, C₁₋₃alkoxy and halogen;
   R₁₄ₐ, R_{14b}, R₁₅ₐ, R_{15b}, R₁₆ₐ, R_{16b}, R₁₇ₐ, R_{17b} are each independently selected from the group consisting of: H, NH₂, NHSOC₁₋₆alkyl, NHSO₂C₁₋₆alkyl, NHC₁₋₆alkyl, N(C₁₋₆alkyl)₂, NHC(O)C₁₋₆alkyl, NHC(O)OC₁₋₆alkyl, halogen, OH, CN, C₁₋₃alkoxy, C₁₋₆alkyl optionally substituted with NH₂;
   or R₁₅ₐ and R_{15b} taken together with the carbon atom to which they are bound form a spiro-C₃₋₆cycloalkyl ring optionally containing an heteroatom selected from the group consisting of: N, O and S;
   or R₁₅ₐ and R₁₆ₐ are absent and R_{15b} and R_{16b} are joined together to form an aryl or heteroaryl ring, each being optionally substituted with one or more substituents each independently selected from the group consisting of: halogen, NH₂, NHSOC₁₋₆alkyl, NHSO₂C₁₋₆alkyl, NHC₁₋₆alkyl, N(C₁₋₆alkyl)₂, NHC(O)C₁₋₆alkyl, NHC(O)OC₁₋₆alkyl, CN, OH, C₁₋₆alkyl, C₁₋₆alkoxy, haloC₁₋₆alkyl and haloC₁₋₆alkoxy;
   or R_{15b} and R_{16b} are joined together to form a C₅-₇cycloalkyl or C₅-₇heterocycloalkyl ring, each being independently optionally substituted with one or more substituents each independently selected from the group consisting of: halogen, NH₂, NHSOC₁₋₆alkyl, NHSO₂C₁₋₆alkyl, NHC₁₋₆alkyl, N(C₁₋₆alkyl)₂, NHC(O)C₁₋₆alkyl, NHC(O)OC₁₋₆alkyl, CN, OH, =O, C₁₋₆alkyl, C₁₋₆alkoxy, haloC₁₋₆alkyl, haloC₁₋₆alkoxy, SO₂CH₃;
   R₁₀ is H, SO₂C₁₋₆alkyl, C(O)C₁₋₆alkyl, C(O)OC₁₋₆alkyl or C₁₋₆alkyl optionally substituted with one or more groups each independently selected from the group consisting of: OH, NH₂, NHC(O)C₁₋₆alkyl, C₁₋₆alkoxy, halogen, cyano, aryl and heteroaryl;
   or R₁₀ and R₁₀ₐ are joined together to form a C₃₋₇heterocycloalkyl ring optionally containing another heteroatom selected from the group consisting of: N, S and O and optionally substituted with one or more groups each independently selected from the group consisting of: C₁₋₆alkyl, halogen, OH and CN;
   or R₁ and R₂ form together with the nitrogen atom to which they are attached a monocyclic or polycyclic heteroaryl or partially unsaturated heteroaryl ring selected from the group consisting of: pyrrole, pyrazole, indole, benzimidazole, 2H-pyrazolo[3,4-b]pyridine, indazole, 2H-pyrazolo[3,4-c]pyridine, 6H-pyrrolo[3,4-b]pyridine, 6H-pyrrolo[3,4-b]pyrazine, 6H-pyrrolo[3,4-d]pyrimidine, 2H-pyrazolo[3,4-d]pyrimidine and 1,2,3,4-tetrahydroquinoline, and each of said ring is optionally substituted with one or more groups each independently selected from the group consisting of: halogen, C₁-₆alkyl, C₁-₆alkoxy, haloC₁₋₆alkyl, haloC₁₋₆alkoxy, OH, CN, SO₂C₁-₆alkyl, NH₂ and C(O)NH₂;
   R₄ is a ring selected from the group consisting of: phenyl, pyridine, pyrimidine, pyrazine, pyridazine, 1,2,4-triazine, quinoline, indazole, benzothiophene, isoquinoline, thiophene, imidazopyridine, naphthyridine, quinazoline, benzimidazole, indoline, isoindoline, 1,3-dihydroisobenzofuran, 1,2,3,4-tetrahydroquinoline, 3,4-dihydro-2H-benzo[b][1,4]oxazine, 2,3,4,5-tetrahydrobenzo[f][1,4]oxazepine, quinazolin-4(3H)-one, indolin-2-one and 2,3-dihydro-1H-inden-1-one, each of said ring being optionally substituted with one or more groups each independently selected from the group consisting of: halogen, hydroxy, cyano, C₁₋₆alkyl, C₁-₇alkenyl, C₁-₆alkoxy, haloC₁-₆alkyl, haloC₁-₆alkoxy, C₃-₇cycloalkyl, cyano-C₃-₇cycloalkyl, -SF₅, C₅₋₇heterocycloalkoxy, optionally substituted aryl or heteroaryl, partially unsaturated heteroaryl, optionally substituted aryloxy, C(O)OH, C(O)OC₁-₆alkyl, C(O)C₁-₆alkyl, C₁-₆alkylCOOH, SO₂C₁-₆alkyl and N(R₇)₂.

Preferably, the compound of Formula (I) as defined herein above has general Formula (IA) or (IB): 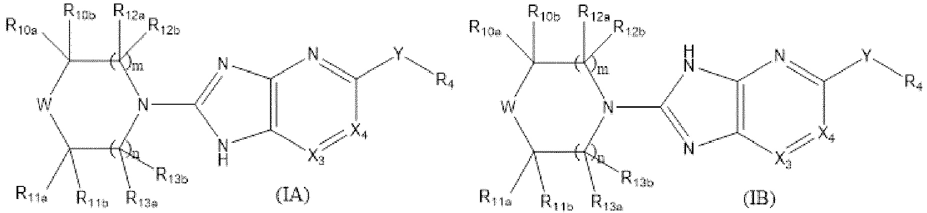 wherein: X₃, X₄, Y, R₄, m, n, W, R₁₀ₐ, R_{10b}, R₁₁ₐ, R_{11b}, R₁₂ₐ, R_{12b}, R₁₃ₐ and R_{13b} are as defined above.

Also preferably, R₁ and R₂ together with the nitrogen atom to which they are attached form a a cyclic amine selected from the group consisting of: aziridine, azetidine, pyrrolidine, piperidine, azepane, morpholine, thiomorpholine, piperazine, 1,4-diazepane, 1,5-diazocane, 8-azaspiro[4.5]decane, 1,7-diazaspiro[3.5]nonane, 2,6-diazaspiro[3.5]nonane, 4,5,6,7-tetrahydro-1H-pyrazolo[4,3-c]pyridine, 5,6,7,8-tetrahydro-1,7-naphthyridine, 4,5,6,7-tetrahydro-1H-imidazo[4,5-c]pyridine, 1,7-diazaspiro[3.5]nonane, 1-oxa-3,7-diazaspiro[4.5]decan-2-one, (1S,4S)-2,5-diazabicyclo[2.2.2]octane, 1-oxa-8-azaspiro[4.5]decane, 2-oxa-8-azaspiro[4.5]decane, 1,3-dihydrospiro[indene-2,4'-piperidine], 5,7-dihydrospiro[cyclopenta[b]pyridine-6,4'-piperidine], 5,7-dihydrospiro[cyclopenta[c]pyridine-6,4'-piperidine], 5,6,7,8-tetrahydro-1,6-naphthyridine, octahydropyrrolo[3,2-b]pyrrole, octahydropyrrolo[3,4-b]pyrrole, 3,9-diazabicyclo[4.2.1]nonane, 3,8-diazabicyclo[3.2.1]octane, 3-azabicyclo[3.1.0]hexane, 2,6-diazaspiro[3.4]octane, 3-azabicyclo[3.2.1]octane, 6-azabicyclo[3.2.1]octane, 5-oxa-2,8-diazaspiro[3.5]nonane, 3,9-diazabicyclo[3.3.1]nonane, 1,2,3,4-tetrahydroisoquinoline, 1-oxa-4,8-diazaspiro[5.5]undecane, hexahydro-1H-thieno[3,4-c]pyrrole 2,2-dioxide, 2-azaspiro[3.4]octane, 5-azaspiro[3.4]octane, 2,7-diazaspiro[4.6]undecane, 4,5,6,7-tetrahydrothiazolo[4,5-c]pyridine, 1-oxa-9-azaspiro[5.5]undecane, 6,8-diazaspiro[3.5]nonane, 6-azaspiro[3.5]nonane, 8-azaspiro[4.5]decane, tetrahydro-1H,4H-3a,6a-(methanoiminomethano)cyclopenta[c]pyrrole, 8-oxa-2-azaspiro[4.5]decane, 6-oxa-2,9-diazaspiro[4.5]decane, 1-oxa-4-azaspiro[5.5]undecane, 8-thia-2-azaspiro[4.5]decane 8,8-dioxide, 3',4'-dihydro-2'H-spiro[azetidine-3,1'-pyrrolo[1,2-a]pyrazine], 3,9-diazabicyclo[4.2.1]nonane, 2,6-diazabicyclo[3.2.2]nonane, 2,7-diazaspiro[4.4]nonane, 3H-spiro[benzofuran-2,4'-piperidine]; each of said cyclic amine being optionally substituted with one or more substituents each independently selected from the group consisting of: C₁₋₆alkyl, aminoC₁₋₆alkyl, C₁₋₆alkoxy, halogen, NH₂, CN, OH, C(O)NH₂, heteroaryl, optionally substituted aryl, hydroxy-C₁₋₆alkyl, halo-C₁₋₆alkyl, C₁₋₆alkoxy, C₃₋₉heterocycloalkyl; or a pharmaceutically acceptable salt, tautomer, solvate, or stereoisomer thereof.

Preferably, Y is S.

Preferably, R₄ is an aryl or heteroaryl ring selected from the group consisting of: phenyl, pyridine, pyrimidine, pyrazine, pyridazine, 1,2,4-triazine, quinoline, isoquinoline, indolin-2-one, indoline and isoindoline and each of said aryl or heteroaryl ring is optionally independently substituted with one or more groups each independently selected from the group consisting of: halogen, cyano, NH₂, CF₃, NHCH₃, NHCOCH₃, cyclopropanamine, cyclobutanamine, azetidine and pyrrolidine, each of said cyclopropanamine, cyclobutanamine, azetidine or pyrrolidine being optionally substituted with one or more groups independently selected from the group consisting of: OH, halogen, cyano and methyl; or a pharmaceutically acceptable salt, tautomer, solvate, or stereoisomer thereof.

Preferably, in any of the compounds of Formula (I), pharmaceutically acceptable salts, tautomers, solvates or stereoisomers thereof as defined above, R₄ is pyridone, pyrimidine, pyrazine or pyridine, preferably 3-pyridine or even more preferably 4-pyridine.

Still preferably, R₄ is pyridine, a pyrazine or a pyridone further substituted as indicated below:

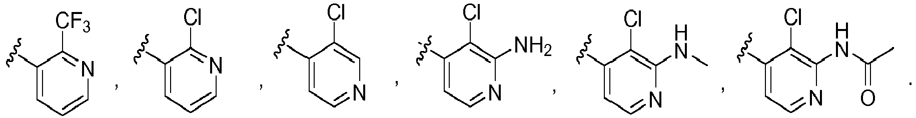

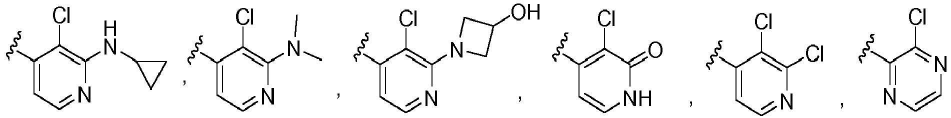

Preferably, in any of the compounds of Formula (I), pharmaceutically acceptable salts, tautomers, solvates or stereoisomers thereof as defined above, X₁ is NR₃, X₂ is N and X₃ is CH.

Preferably, in any of the compounds of Formula (I), or in the pharmaceutically acceptable salts, tautomers, solvates or stereoisomers thereof as defined above, X₁ is NH, X₂ is CH and X₃ is N.

Preferably, in any of the compounds of Formula (I), or in the pharmaceutically acceptable salts, tautomers, solvates or stereoisomers thereof as defined above, X₁ is S, X₂ is N and X₃ is CH.

Preferably, in any of the compounds of Formula (I), (I-A) or (I-B), or in the pharmaceutically acceptable salts, tautomers, solvates or stereoisomers thereof as defined above, X is O or S.

In some embodiments of Formula I, (I-A) or (I-B), R₁ and R₂ form together with the nitrogen atom to which they are attached a monocyclic or polycyclic ring system selected among those indicated below: 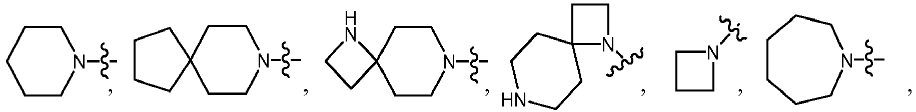 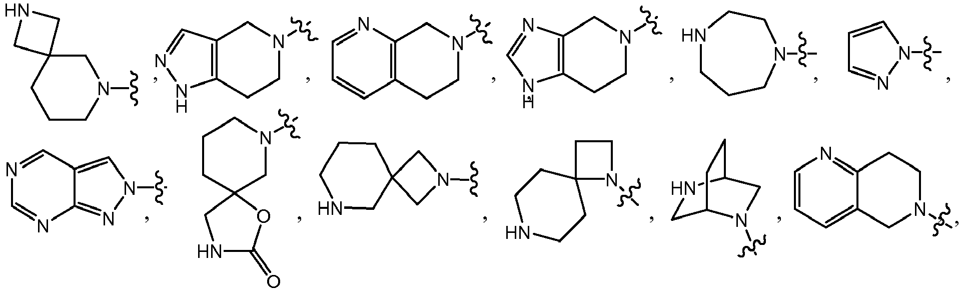 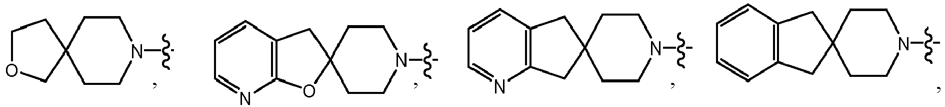 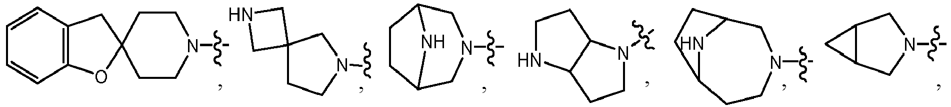 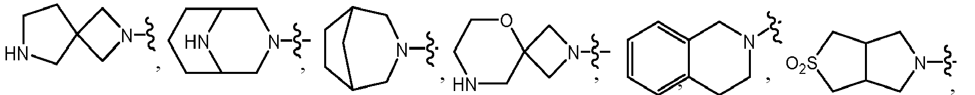 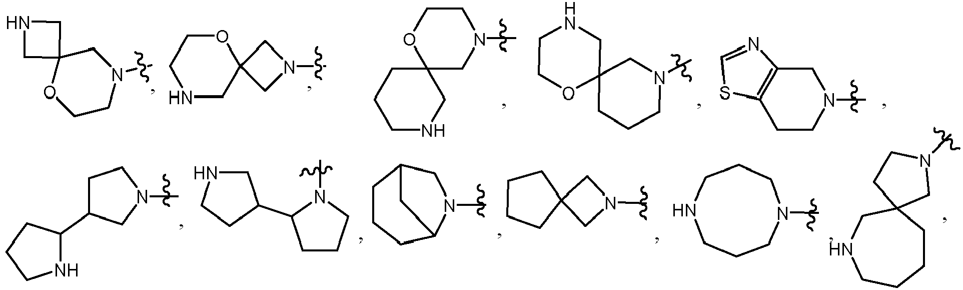 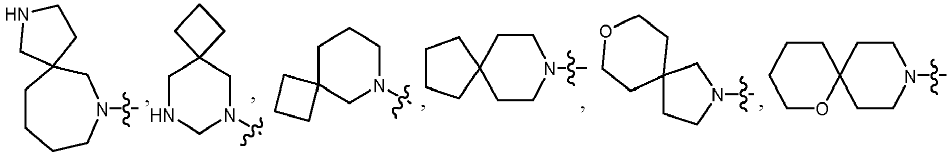 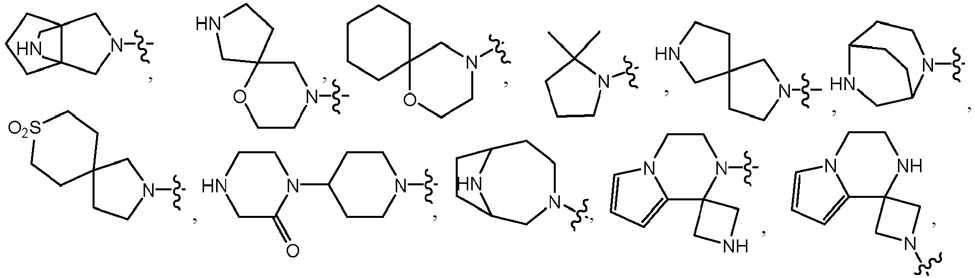 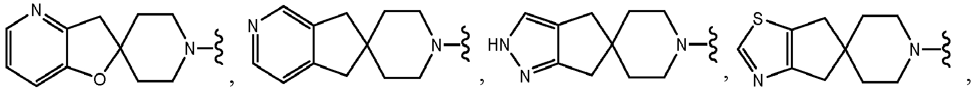 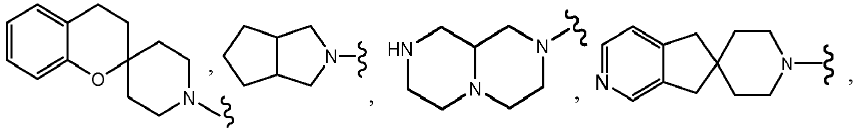 wherein each of the above rings can be further substituted. Preferred substituents are methyl, amino, aminomethyl, F, Cl, Br.

In some embodiments of Formula I, (I-A) or (I-B), R₁ is H and R₂ is selected from: 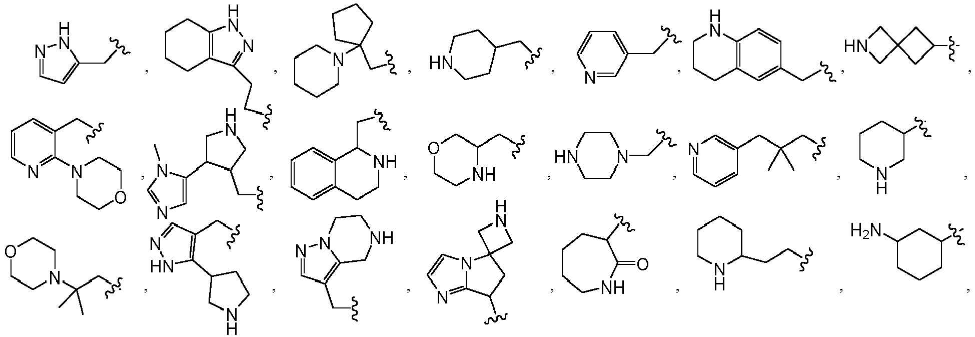 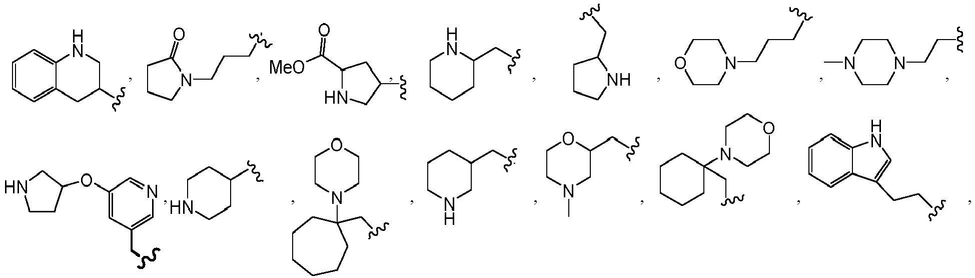 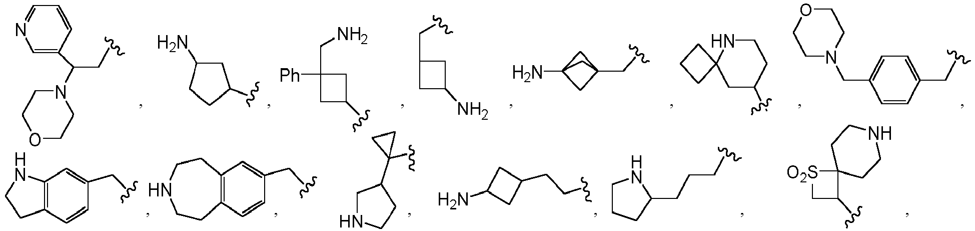 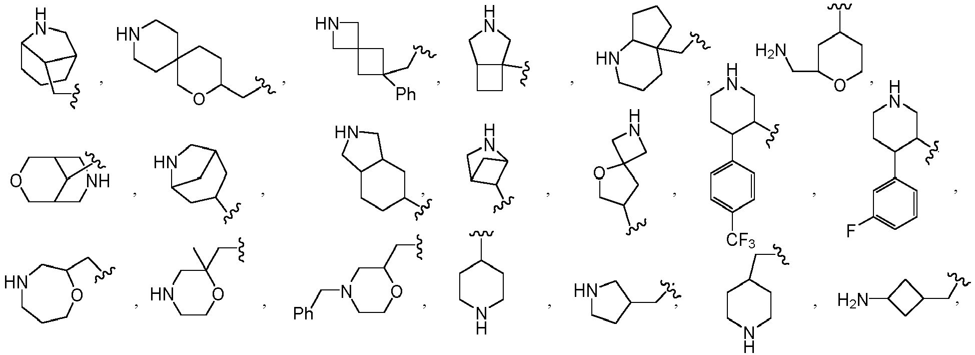 wherein each of the above group can be further substituted. Preferred substituents are methyl, amino, aminomethyl, F, Cl, Br, phenyl and benzyl.

In a preferred embodiment the present invention provides a compound of Formula (I)

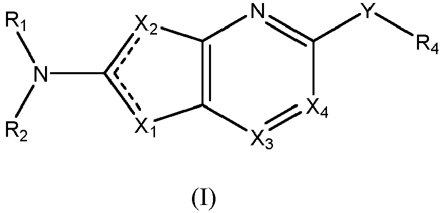

Wherein:
-̅-̅-̅-̅-̅ represents a single bond or a double bond;
X₁ is N, S, O or NR₃;
X₂ is N or NR₃;
if X₁ is N then X₂ is NR₃;
if X₁ is S, O or NR₃ then X₂ is N;
X₃ is N or CRₓ₃ and Rₓ₃ is H, halogen or C₁₋₃alkyl;
X₄ is N or CR₅;
Y is S, O, NR₆, CH₂, CHF, CF₂, CHOH, C(O), SO, SO₂ or a single bond;
R₁ and R₂ are each independently selected from:
   - hydrogen;
   - linear or branched C₁₋₁₂alkyl optionally substituted with one or more substituents independently selected from the group consisting of: OH, halogen, N(R₇)₂, aryl, heteroaryl, partially unsaturated heteroaryl, C₃₋₉cycloalkyl, C₃₋₉heterocycloalkyl and spiro-C₃₋₈cycloalkyl ring optionally containing one heteroatom selected from the group consisting of O, N and S, wherein each of said aryl, heteroaryl, partially unsaturated heteroaryl, C₃₋₉cycloalkyl, C₃₋₉heterocycloalkyl and spiro-C₃₋₈cycloalkyl ring is optionally further substituted with one or more groups independently selected from the group consisting of: C(O)CH₃, C(O)OCH₃, heteroaryl, aryl, OH, halogen, NH₂, C₁₋₆alkyl optionally substituted with N(R₇)₂, C₁₋₃alkylaryl, C₁₋₃alkylheteroaryl, haloC₁₋₆alkyl, hydroxyC₁₋₆alkyl, CN, haloC₁₋₆alkoxy, C₁₋₆alkoxy, C₅₋₇heterocycloalkoxy and a cyclic amine selected from the group consisting of: pyrrolidine, piperidine, morpholine, thiomorpholine, piperazine, N-methylpyperazine and pyrrolidin-3-yloxy; and
   - a C₃₋₇cycloalkyl, C₃₋₉heterocycloalkyl or a partially unsaturated heteroaryl selected from bicyclo[1.1.1]pentane, pirrolidine, piperidine, morpholine, piperazine, 2-azaspiro[3.3]heptane, azepan-2-one, 3-azaspiro[5.5]undecane, 2-azaspiro[4.5]decane, 3-azabicyclo[3.3.1]nonane, 3-oxa-7-azabicyclo[3.3.1]nonane, 6',7'-dihydrospiro[azetidine-3,5'-pyrrolo[1,2-a]imidazole], 5-azaspiro[3.5]nonane, 1-thia-7-azaspiro[3.5]nonane 1,1-dioxide, 3-azabicyclo[3.2.0]heptane, 2-azabicyclo[2.1.1]hexane, 6-azabicyclo[3.2.1]octane, octahydroindole, octahydro-1H-isoindole, 5-oxa-2-azaspiro[3.4]octane and 1,2,3,4-tetrahydroquinoline, each of said groups being optionally substituted with one ore more substituents independently selected from the group consisting of: halogen, C₁₋₃alkyl optionally substituted with N(R₇)₂, C₁₋₃alkylaryl, C₁₋₃alkylheteroaryl, C(O)OC₁₋₃alkyl, C(O)C₁₋₃alkyl, N(R₇)₂, aryl and heteroaryl, each of said aryl or heteroaryl being optionally substituted with one or more substituents independently selected from the group consisting of: halogen, hydroxyl, cyano, C₁₋₃alkoxy and C₁₋₃haloalkyl;
or R₁ and R₂ together with the nitrogen atom to which they are attached form a a cyclic amine selected from the group consisting of: aziridine, azetidine, pyrrolidine, piperidine, azepane, morpholine, thiomorpholine, piperazine, 1,4-diazepane, 1,5-diazocane, 8-azaspiro[4.5]decane, 1,7-diazaspiro[3.5]nonane, 2,6-diazaspiro[3.5]nonane, 4,5,6,7-tetrahydro-1H-pyrazolo[4,3-c]pyridine, 5,6,7,8-tetrahydro-1,7-naphthyridine, 4,5,6,7-tetrahydro-1H-imidazo[4,5-c]pyridine, 1,7-diazaspiro[3.5]nonane, 1-oxa-3,7-diazaspiro[4.5]decan-2-one, (1S,4S)-2,5-diazabicyclo[2.2.2]octane, 1-oxa-8-azaspiro[4.5]decane, 2-oxa-8-azaspiro[4.5]decane, 1,3-dihydrospiro[indene-2,4'-piperidine], 5,7-dihydrospiro[cyclopenta[b]pyridine-6,4'-piperidine], 5,7-dihydrospiro[cyclopenta[c]pyridine-6,4'-piperidine], 5,6,7,8-tetrahydro-1,6-naphthyridine, octahydropyrrolo[3,2-b]pyrrole, octahydropyrrolo[3,4-b]pyrrole, 3,9-diazabicyclo[4.2.1]nonane, 3,8-diazabicyclo[3.2.1]octane, 3-azabicyclo[3.1.0]hexane, 2,6-diazaspiro[3.4]octane, 3-azabicyclo[3.2.1]octane, 6-azabicyclo[3.2.1]octane, 5-oxa-2,8-diazaspiro[3.5]nonane, 3,9-diazabicyclo[3.3.1]nonane, 1,2,3,4-tetrahydroisoquinoline, 1-oxa-4,8-diazaspiro[5.5]undecane, hexahydro-1H-thieno[3,4-c]pyrrole 2,2-dioxide, 2-azaspiro[3.4]octane, 5-azaspiro[3.4]octane, , 2,7-diazaspiro[4.6]undecane, 4,5,6,7-tetrahydrothiazolo[4,5-c]pyridine,,, 1-oxa-9-azaspiro[5.5]undecane, 6,8-diazaspiro[3.5]nonane, 6-azaspiro[3.5]nonane, 8-azaspiro[4.5]decane, tetrahydro-1H,4H-3a,6a-(methanoimmomethano)cyclopenta[c]pyrrole, 8-oxa-2-azaspiro[4.5]decane, 6-oxa-2,9-diazaspiro[4.5]decane, 1-oxa-4-azaspiro[5.5]undecane,, 8-thia-2-azaspiro[4.5]decane 8,8-dioxide, 3',4'-dihydro-2'H-spiro[azetidine-3,1'-pyrrolo[1,2-a]pyrazine], 3,9-diazabicyclo[4.2.1]nonane, 2,6-diazabicyclo[3.2.2]nonane, 2,7-diazaspiro[4.4]nonane, 3H-spiro[benzofuran-2,4'-piperidine];
each of said cyclic amine being optionally substituted with one or more substituents each independently selected from the group consisting of: C₁₋₆alkyl, aminoC₁₋₆alkyl, C₁₋₆alkoxy, halogen, NH₂, CN, OH, C(O)NH₂, heteroaryl, optionally substituted aryl, hydroxy-C₁₋₆alkyl, halo-C₁₋₆alkyl, C₁₋₆alkoxy, C₃₋₉heterocycloalkyl;
or R₁ and R₂ form together with the nitrogen atom to which they are attached a monocyclic or polycyclic heteroaryl or partially unsaturated heteroaryl ring selected from the group consisting of: pyrrole, pyrazole, indole, benzimidazole, 2H-pyrazolo[3,4-b]pyridine, indazole, 2H-pyrazolo[3,4-c]pyridine, 6H-pyrrolo[3,4-b]pyridine, 6H-pyrrolo[3,4-b]pyrazine, 6H-pyrrolo[3,4-d]pyrimidine, 2H-pyrazolo[3,4-d]pyrimidine and 1,2,3,4-tetrahydroquinoline, and each of said ring is optionally substituted with one or more groups each independently selected from the group consisting of: halogen, C₁-₆alkyl, C₁-₆alkoxy, haloC₁₋₆alkyl, haloC₁₋₆alkoxy, OH, CN, SO₂C₁-₆alkyl, NH₂ and C(O)NH₂;
R₃ is H, C₁-₆alkyl, C₁-₆alkyl-cycloalkyl or C₁-₆alkyl-heterocycloalkyl, each of said groups being optionally substituted with one or more groups independently selected from the group consisting of: C₁-₆alkoxy, hydroxyl, cyano, C(O)OC₁-₆alkyl, C(O)NH₂, C(O)NHC₁-₆alkyl; C(O)N(C₁-₆alkyl)₂, SO₂C₁-₆alkyl, SOC₁-₆alkyl, SO₂NHC₁-₆alkyl and SO₂N(C₁-₆alkyl)₂;
R₄ is a ring selected from the group consisting of: phenyl, pyridine, pyrimidine, pyrazine, pyridazine, 1,2,4-triazine, quinoline, indazole, benzothiophene, isoquinoline, thiophene, imidazopyridine, naphthyridine, quinazoline, benzimidazole, indoline, isoindoline, 1,3-dihydroisobenzofuran, 1,2,3,4-tetrahydroquinoline, 3,4-dihydro-2H-benzo[b][1,4]oxazine, 2,3,4,5-tetrahydrobenzo[f][1,4]oxazepine, quinazolin-4(3H)-one, indolin-2-one, and 2,3-dihydro-1H-inden-1-one, each of said ring being optionally substituted with one or more groups each independently selected from the group consisting of: halogen, hydroxy, cyano, C₁₋₆alkyl, C₁-₇alkenyl, C₁-₆alkoxy, haloC₁-₆alkyl, haloC₁-₆alkoxy, C₃-₇cycloalkyl, cyano-C₃-₇cycloalkyl, -SF₅, C₅₋₇heterocycloalkoxy, optionally substituted aryl or heteroaryl, partially unsaturated heteroaryl, optionally substituted aryloxy, C(O)OH, C(O)OC₁-₆alkyl, C(O)C₁-₆alkyl, C₁-₆alkylCOOH, SO₂C₁-₆alkyl and N(R₇)₂;
R₅ is H, halogen, C₁-₆alkyl, C₁-₆alkoxy, haloC₁-₆alkyl, haloC₁-₆alkoxy, OH, C₃-₆cycloalkyl, C₃-₆cycloalkoxy, C₃₋₆heterocycloalkyl or NRₓ₁Rₓ₂ wherein Rₓ₁ and Rₓ₂ are each independently H or C₁-₆alkyl, or Rₓ₁ and Rₓ₂ taken together with the nitrogen atom to which they are attached form a 3-7 membered saturated ring optionally containing one or more heteroatoms each independently selected from the group consisting of O, S and N;
R₆ is H or C₁-₆alkyl;
or R₄ and R₆ form together with the nitrogen atom to which they are attached a C₃₋₉heterocycloalkyl, a heteroaryl or a partially unsaturated heteroaryl ring, each being optionally substituted with one or more groups independently selected from the group consisting of: OH, halogen, CN, C₁-₆alkyl, C₁-₆alkoxy, haloC₁-₆alkyl, haloC₁-₆alkoxy, C₃-₇cycloalkyl, C₃-₇cycloalkoxy, aryl and heteroaryl;
each R₇ is independently selected from the group consisting of: H, C₁-₆alkyl, SO₂C₁-₆alkyl, SOC₁-₆alkyl, C(O)OC₁-₆alkyl, C(O)C₁-₆alkyl, C₃-₇cycloalkyl, aryl, heteroaryl, C₁-₆alkylaryl and C₁-₆alkylheteroaryl;
or two R₇ taken together with the nitrogen atom to which they are bound form a 3 to 7 membered cyclic amine optionally containing one additional heteroatom selected from the group consisting of S, N and O, said 3 to 7 membered cyclic amine being optionally substituted with one or more groups each independently selected from the group consisting of: OH, halogen, CN, C₁-₆alkyl, C₁-₆alkoxy, haloC₁-₆alkyl, haloC₁-₆alkoxy, C₃-₇cycloalkyl and C₃-₇cycloalkoxy;
or a pharmaceutically acceptable salt, tautomer, solvate, or stereoisomer thereof.

In a preferred embodiment of the invention, the compounds are selected from the following list:
- 4-methyl-1-(6-((2-(trifluoromethyl)pyridin-3-yl)thio)-1H-imidazo[4,5-b]pyrazin-2-yl)piperidin-4-amine
- (8-(6-((2-(trifluoromethyl)pyridin-3-yl)thio)-1H-imidazo[4,5-b]pyrazin-2-yl)-8-azaspiro[4.5]decan-1-yl)methanamine
- (R)-8-(6-((2,3-dichlorophenyl)thio)-1H-imidazo[4,5-b]pyrazin-2-yl)-8-azaspiro[4.5]decan-1-amine
- 2-(1,7-diazaspiro[3.5]nonan-7-yl)-5-((2-(trifluoromethyl)pyridin-3-yl)thio)-1H-imidazo[4,5-b]pyrazine
- 1-(5-((2-(trifluoromethyl)pyridin-3-yl)thio)-1H-imidazo[4,5-b]pyrazin-2-yl)piperidin-4-amine
- 2-(4-((2-(4-amino-4-methylpiperidin-1-yl)-1H-imidazo[4,5-b]pyrazin-6-yl)thio)phenyl)-2,4-dihydro-3H-1,2,4-triazol-3-one
- 4-methyl-1-(6-(phenylthio)-1H-imidazo[4,5-b]pyrazin-2-yl)piperidin-4-amine
- 4-methyl-1-(5-((2-(trifluoromethyl)phenyl)thio)-1H-imidazo[4,5-b]pyrazin-2-yl)piperidin-4-amine
- 4-((2-(4-amino-4-methylpiperidin-1-yl)-1H-imidazo[4,5-b]pyrazin-5-yl)thio)-3-(trifluoromethyl)benzonitrile
- 1-(5-((2,4-difluorophenyl)thio)-1H-imidazo[4,5-b]pyrazin-2-yl)-4-methylpiperidin-4-amine
- 1-(5-((2,3-difluorophenyl)thio)-1H-imidazo[4,5-b]pyrazin-2-yl)-4-methylpiperidin-4-amine
- 1-(5-((2,3-dichlorophenyl)thio)-1H-imidazo[4,5-b]pyrazin-2-yl)-4-methylpiperidin-4-amine
- 2-((2-(4-amino-4-methylpiperidin-1-yl)-1H-imidazo[4,5-b]pyrazin-5-yl)thio)benzonitrile
- 1-(5-((2-methoxyphenyl)thio)-1H-imidazo[4,5-b]pyrazin-2-yl)-4-methylpiperidin-4-amine
- 1-(5-((3-chloropyridin-4-yl)thio)-1H-imidazo[4,5-b]pyrazin-2-yl)-4-methylpiperidin-4-amine
- 1-(5-((2-(trifluoromethyl)pyridin-3-yl)thio)-1H-imidazo[4,5-b]pyrazin-2-yl)piperidin-3-amine
- N-(piperidin-4-yl)-5-((2-(trifluoromethyl)pyridin-3-yl)thio)-1H-imidazo[4,5-b]pyrazin-2-amine
- 1-(5-((2-bromophenyl)thio)-1H-imidazo[4,5-b]pyrazin-2-yl)-4-methylpiperidin-4-amine
- 1-(5-((4-chloro-2-methylphenyl)thio)-1H-imidazo[4,5-b]pyrazin-2-yl)-4-methylpiperidin-4-amine
- 1-(5-((2,3-dimethylphenyl)thio)-1H-imidazo[4,5-b]pyrazin-2-yl)-4-methylpiperidin-4-amine
- 2-(1,7-diazaspiro[3.5]nonan-1-yl)-5-((2-(trifluoromethyl)pyridin-3-yl)thio)-1H-imidazo[4,5-b]pyrazine
- 4-methyl-1-(5-((6-(trifluoromethyl)pyridin-2-yl)thio)-1H-imidazo[4,5-b]pyrazin-2-yl)piperidin-4-amine
- 4-methyl-1-(5-((3-(2-methylthiazol-4-yl)phenyl)thio)-1H-imidazo[4,5-b]pyrazin-2-yl)piperidin-4-amine
- 4-methyl-1-(6-(quinolin-5-ylthio)-1H-imidazo[4,5-b]pyrazin-2-yl)piperidin-4-amine
- 4-methyl-1-(1-methyl-5-((2-(trifluoromethyl)pyridin-3-yl)thio)-1H-imidazo[4,5-b]pyrazin-2-yl)piperidin-4-amine
- 1-(5-([1,1'-biphenyl]-3-ylthio)-1H-imidazo[4,5-b]pyrazin-2-yl)-4-methylpiperidin-4-amine
- 4-methyl-1-(5-(naphthalen-1-ylthio)-1H-imidazo[4,5-b]pyrazin-2-yl)piperidin-4-amine
- 4-methyl-1-(5-(quinolin-4-ylthio)-1H-imidazo[4,5-b]pyrazin-2-yl)piperidin-4-amine
- 1-(5-((1,5-naphthyridin-4-yl)thio)-1H-imidazo[4,5-b]pyrazin-2-yl)-4-methylpiperidin-4-amine
- 1-(5-((2-bromopyridin-4-yl)thio)-1H-imidazo[4,5-b]pyrazin-2-yl)-4-methylpiperidin-4-amine
- 1-(5-(isoquinolin-5-ylthio)-1H-imidazo[4,5-b]pyrazin-2-yl)-4-methylpiperidin-4-amine
- 4-methyl-1-(5-(quinoxalin-5-ylthio)-1H-imidazo[4,5-b]pyrazin-2-yl)piperidin-4-amine
- 1-(5-((2-chlorothiophen-3-yl)thio)-1H-imidazo[4,5-b]pyrazin-2-yl)-4-methylpiperidin-4-amine
- 4-methyl-1-(5-((2-(pentafluoro-l6-sulfaneyl)phenyl)thio)-1H-imidazo[4,5-b]pyrazin-2-yl)piperidin-4-amine
- 1-(5-((1,3-dihydroisobenzofuran-4-yl)thio)-1H-imidazo[4,5-b]pyrazin-2-yl)-4-methylpiperidin-4-amine
- 4-methyl-1-(5-(naphthalen-2-ylthio)-1H-imidazo[4,5-b]pyrazin-2-yl)piperidin-4-amine
- 4-methyl-1-(5-(quinolin-8-ylthio)-1H-imidazo[4,5-b]pyrazin-2-yl)piperidin-4-amine
- 1-(5-(isoquinolin-8-ylthio)-1H-imidazo[4,5-b]pyrazin-2-yl)-4-methylpiperidin-4-amine
- 4-methyl-1-(5-((3-(pentafluoro-l6-sulfaneyl)phenyl)thio)-)-1H-imidazo[4,5-b]pyrazin-2-yl)piperidin-4-amine
- 3-((2-(4-amino-4-methylpiperidin-1-yl)-1H-imidazo[4,5-b]pyrazin-5-yl)thio)benzoic acid
- 4-((2-(4-amino-4-methylpiperidin-1-yl)-1H-imidazo[4,5-b]pyrazin-5-yl)thio)benzoic acid
- (3S,4S)-3-methyl-8-(5-((2-(trifluoromethyl)pyridin-3-yl)thio)-1H-imidazo[4,5-b]pyrazin-2-yl)-2-oxa-8-azaspiro[4.5]decan-4-amine
- 4-methyl-1-(5-((8-(trifluoromethyl)quinolin-4-yl)thio)-1H-imidazo[4,5-b]pyrazin-2-yl)piperidin-4-amine
- 1-(5-((2-chlorophenyl)thio)-1H-imidazo[4,5-b]pyrazin-2-yl)-4-methylpiperidin-4-amine
- 4-((2-(4-amino-4-methylpiperidin-1-yl)-1H-imidazo[4,5-b]pyrazin-5-yl)thio)-3-chlorobenzoic acid
- 2-(3-((2-(4-amino-4-methylpiperidin-1-yl)-1H-imidazo[4,5-b]pyrazin-5-yl)thio)phenyl)acetic acid
- 1-(5-((1,8-naphthyridin-4-yl)thio)-1H-imidazo[4,5-b]pyrazin-2-yl)-4-methylpiperidin-4-amine
- (S)-1'-(5-((2-(trifluoromethyl)pyridin-3-yl)thio)-1H-imidazo[4,5-b]pyrazin-2-yl)-1,3-dihydrospiro[indene-2,4'-piperidin]-1-amine
- 1-(5-((3-chloro-2-methylphenyl)thio)-1H-imidazo[4,5-b]pyrazin-2-yl)-4-methylpiperidin-4-amine
- 1-(5-((2-isopropylphenyl)thio)-1H-imidazo[4,5-b]pyrazin-2-yl)-4-methylpiperidin-4-amine
- (S)-1'-(6-chloro-5-((2-(trifluoromethyl)pyridin-3-yl)thio)-1H-imidazo[4,5-b]pyrazin-2-yl)-1,3-dihydrospiro[indene-2,4'-piperidin]-]-1-amine
- 2-(9,9-dimethyl-3,7-diazabicyclo[3.3.1]nonan-3-yl)-5-((2-(trifluoromethyl)pyridin-3-yl)thio)-1H-imidazo[4,5-b]pyrazine
- 2-(3,9-diazabicyclo[4.2.1]nonan-3-yl)-5-((2-(trifluoromethyl)pyridin-3-yl)thio)-1H-imidazo[4,5-b]pyrazine
- N-(5-azaspiro[3.5]nonan-8-yl)-6-((2-(trifluoromethyl)pyridin-3-yl)thio)-1H-imidazo[4,5-b]pyrazin-2-amine
- (8-(6-((2-(trifluoromethyl)pyridin-3-yl)thio)-1H-imidazo[4,5-b]pyrazin-2-yl)-8-azaspiro[4.5]decan-1-yl)methanamine
- N-((5-phenylpyrrolidin-3-yl)methyl)-5-((2-(trifluoromethyl)pyridin-3-yl)thio)-1H-imidazo[4,5-b]pyrazin-2-amine
- (S)-1'-(6-((3-chloro-2-(methylamino)pyridin-4-yl)thio)-1H-imidazo[4,5-b]pyrazin-2-yl)-1,3-dihydrospiro[indene-2,4'-piperidin]-1-amine
- (S)-1'-(5-((2-amino-3-chloropyridin-4-yl)thio)-1H-imidazo[4,5-b]pyrazin-2-yl)-1,3-dihydrospiro[indene-2,4'-piperidin]-1-amine
- (S)-1'-(5-((2,3-dichlorophenyl)thio)-1H-imidazo[4,5-b]pyrazin-2-yl)-1,3-dihydrospiro[indene-2,4'-piperidin]-1-amine
- (S)-4-((2-(1-amino-1,3-dihydrospiro[indene-2,4'-piperidin]-11H-imidazo[4,5-b]pyrazin-5-yl)thio)-3,3-difluoro-1-methylindolin-2-one
- (S)-1-(4-((2-(1-amino-1,3-dihydrospiro[indene-2,4'-piperidin]-1'-yl)-1H-imidazo[4,5-b]pyrazin-5-yl)thio)-3,3-difluoroindolin-1-yl)ethan-1-one
- (S)-1'-(5-((2,2-difluorobenzo[d][1,3]dioxol-4-yl)thio)-1H-imidazo[4,5-b]pyrazin-2-yl)-1,3-dihydrospiro[indene-2,4'-piperidin]-1-amine
- (S)-1'-(5-((2-(trifluoromethyl)pyridin-3-yl)thio)-1H-imidazo[4,5-b]pyrazin-2-yl)-5,7-dihydrospiro[cyclopenta[b]pyridine-6,4'-piperidin]-5-amine
- (S)-3-((2-(1-amino-1,3-dihydrospiro[indene-2,4'-piperidin]-1'-yl)-1H-imidazo[4,5-b]pyrazin-5-yl)thio)-2-(trifluoromethyl)pyridine 1-oxide
- (R)-1'-(5-((2-(trifluoromethyl)pyridin-3-yl)thio)-1H-imidazo[4,5-b]pyrazin-2-yl)-3H-spiro[benzofuran-2,4'-piperidin]-3-amine
- 1-(2-((2,3-dichlorophenyl)thio)-7H-purin-8-yl)-4-methylpiperidin-4-amine
- (S)-1'-(5-((2-amino-3-chloropyridin-4-yl)thio)-1H-imidazo[4,5-b]pyrazin-2-yl)-5,7-dihydrospiro[cyclopenta[b]pyridine-6,4'-piperidin]-5-amine
- (S)-1'-(5-((3-chloro-2-(methylamino)pyridin-4-yl)thio)-1H-imidazo[4,5-b]pyrazin-2-yl)-5,7-dihydrospiro[cyclopenta[b]pyridine-6,4'-piperidin]-5-amine
- (S)-N-(4-((2-(1-amino-1,3-dihydrospiro[indene-2,4'-piperidin]-1'-yl)-1H-imidazo[4,5-b]pyrazin-5-yl)thio)-3-chloropyridin-2-yl)acetamide
- (S)-1'-(5-((3-chloropyridin-4-yl)thio)-1H-imidazo[4,5-b]pyrazin-2-yl)-1,3-dihydrospiro[indene-2,4'-piperidin]-1-amine
- (R)-1'-(5-((2-(trifluoromethyl)pyridin-3-yl)thio)-1H-imidazo[4,5-b]pyrazin-2-yl)-1,3-dihydrospiro[indene-2,4'-piperidin]-1-amine
- (R)-1'-(5-((2-amino-3-chloropyridin-4-yl)thio)-1H-imidazo[4,5-b]pyrazin-2-yl)-3H-spiro[benzofuran-2,4'-piperidin]-3-amine
   - (S)-1'-(5-((3-chloropyridin-4-yl)thio)-1H-imidazo[4,5-b]pyrazin-2-yl)-5,7-dihydrospiro[cyclopenta[b]pyridine-6,4'-piperidin]-5-amine
   - (S)-1'-(5-((3-chloro-2-methoxypyridin-4-yl)thio)-1H-imidazo[4,5-b]pyrazin-2-yl)-5,7-dihydrospiro[cyclopenta[b]pyridine-6,4'-piperidin]-5-amine
   - (S)-4-((2-(1-amino-1,3-dihydrospiro[indene-2,4'-piperidin]-1'-yl)-1H-imidazo[4,5-b]pyrazin-5-yl)thio)-3-chloropyridin-2-ol
   - (S)-1'-(5-((3-chloro-2-(dimethylamino)pyridin-4-yl)thio)-1H-imidazo[4,5-b]pyrazin-2-yl)-5,7-dihydrospiro[cyclopenta[b]pyridine-6,4'-piperidin]-5-amine
   - (R)-1'-(5-((3-chloropyridin-4-yl)thio)-1H-imidazo[4,5-b]pyrazin-2-yl)-3H-spiro[benzofuran-2,4'-piperidin]-3-amine
   - (S)-1'-(5-((4-(trifluoromethyl)pyrimidin-5-yl)thio)-1H-imidazo[4,5-b]pyrazin-2-yl)-1,3-dihydrospiro[indene-2,4'-piperidin]-1-amine
   - (S)-8-((2-(1-amino-1,3-dihydrospiro[indene-2,4'-piperidin]-1'-yl)-1H-imidazo[4,5-b]pyrazin-5-yl)thio)-2H-benzo[b][1,4]oxazin-3(4H)-one
   - (S)-1'-(5-((3-chloropyrazin-2-yl)thio)-1H-imidazo[4,5-b]pyrazin-2-yl)-1 ,3-dihydrospiro[indene-2,4'-piperidin]-1-amine
   - (R)-1'-(5-((2-amino-3-chloropyridin-4-yl)thio)-1H-imidazo[4,5-b]pyrazin-2-yl)-3H-spiro[benzofuran-2,4'-piperidin]-3-amine
   - (S)-1'-(5-((3-chloro-2-(cyclopropylamino)pyridin-4-yl)thio)-1H-imidazo[4,5-b]pyrazin-2-yl)-1,3-dihydrospiro[indene-2,4'-piperidin]-1-amine
   - (S)-1'-(6-((3-chloro-2-(cyclopropylamino)pyridin-4-yl)thio)-1H-imidazo[4,5-b]pyrazin-2-yl)-5,7-dihydrospiro[cyclopenta[b]pyridine-6,4'-piperidin]-5-amine
   - (S)-4-((2-(1-amino-1,3-dihydrospiro[indene-2,4'-piperidin]-1'-yl)-1H-imidazo[4,5-b]pyrazin-5-yl)thio)-3,3-difluoroindolin-2-one
   - (R)-1'-(5-((3-chloropyridin-4-yl)thio)-1H-imidazo[4,5-b]pyrazin-2-yl)-3H-spiro[furo[3,2-b]pyridine-2,4'-piperidin]-3-amine
   - (S)-1'-(6-((1,5-naphthyridin-4-yl)thio)-1H-imidazo[4,5-b]pyrazin-2-yl)-1,3-dihydrospiro[indene-2,4'-piperidin]-1-amine
   - (S)-1-(4-((2-(1-amino-1,3-dihydrospiro[indene-2,4'-piperidin]-1'-yl)-1H-imidazo[4,5-b]pyrazin-5-yl)thio)-3-chloropyridin-2-yl)azetidin-3-ol
   - (S)-4-((2-(1-amino-1,3-dihydrospiro[indene-2,4'-piperidin]-1'-yl)-1H-imidazo[4,5-b]pyrazin-6-yl)thio)-3-chloro-1-methylpyridin-2(1H)-one
   - (S)-1'-(6-((2,3-dihydrobenzo[b][1,4]dioxin-5-yl)thio)-1H-imidazo[4,5-b]pyrazin-2-yl)-1,3-dihydrospiro[indene-2,4'-piperidin]-1-amine
   - (S)-6-((2-(5-amino-5,7-dihydrospiro[cyclopenta[b]pyridine-6,4'-piperidin]-1'-yl)-1H-imidazo[4,5-b]pyrazin-6-yl)thio)-4-chlorobenzo[d]oxazol-2(3H)-one
   - (S)-6-((2-(5-amino-5,7-dihydrospiro[cyclopenta[b]pyridine-6,4'-piperidin]-1'-yl)-1H-imidazo[4,5-b]pyrazin-6-yl)thio)-5-chloro-2H-benzo[b][1,4]oxazin-3(4H)-one
   or a pharmaceutically acceptable salt, tautomer, solvate, or stereoisomer thereof.

The invention further provides a process for the preparation of compounds of the invention. In particular, it is a further object of the invention a process for the synthesis of the compound of Formula (I) or the pharmaceutically acceptable salt, tautomer, solvate or stereoisomer thereof as defined above, said process comprising at least one of the following steps:
a) reacting a compound of formula (A) with a compound of formula R₄YH in the presence of a transition metal catalyst or under photochemical conditions, wherein said transition metal catalyst is preferably a palladium or copper catalyst, such as Pd₂(dba)₃, Pd(PPh₃)₄ and CuI: 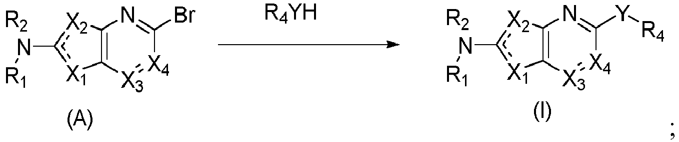 or
b) when in said compound of Formula (I) Y is S, reacting in a first step a compound of formula (A) with 2-ethylhexyl 3-mercaptopropanoate in the presence of a palladium catalyst, and further reacting in a second step the product from the first step with a compound of formula R₄X, wherein X is bromide, chloride, iodide or triflate, in the presence of a palladium catalyst, wherein said first and second steps are carried out in the presence of a tertiary amine, preferably DIPEA or TEA, and wherein the palladium catalyst in said first and/or second step is preferably Pd₂(dba)₃ or Pd(PPh₃)₄: 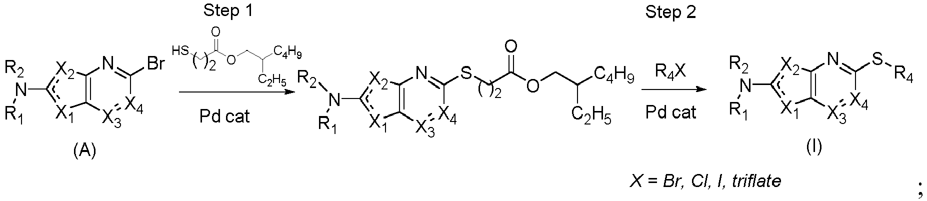 or
c) when in said compound of Formula (I) Y is a bond, reacting a compound of formula (A) with R₄-boronic acid in the presence of a palladium catalyst, preferably Pd(PPh₃)₄, and a base: 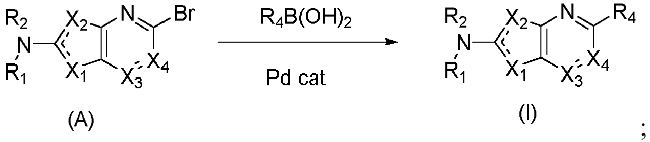 or
d) reacting a compound of formula (B), wherein Lg is a leaving group selected from the group consisting of halogen, SO₂Me and SOMe, with an amine of formula R₁R₂NH at temperature range of about 100°C to about 120°C: 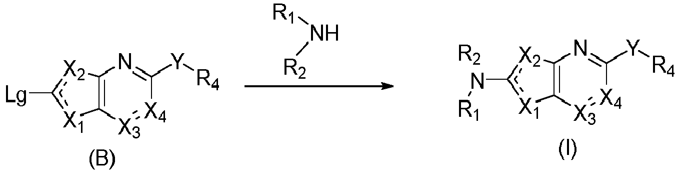 wherein in each of said a), b) c) or d) steps, if X₁ or X₂ is NH, it can be optionally protected for example as a trimethylsilylethoxymethyl (SEM) derivative.

In a preferred embodiment, compounds of Formula (I') and (I") may respectively be prepared according to one of the following synthetic schemes: 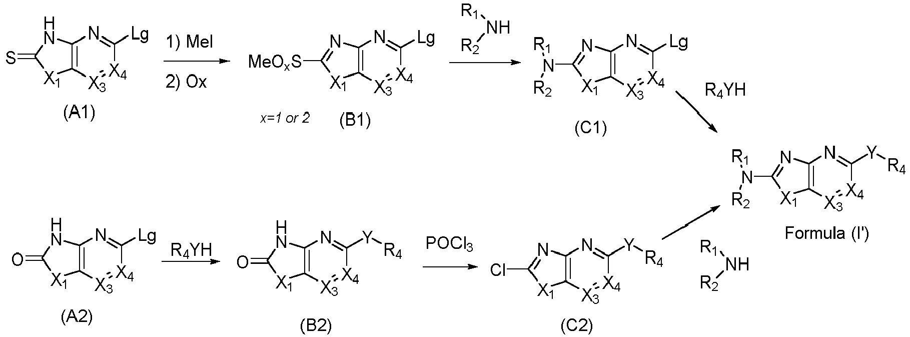 or

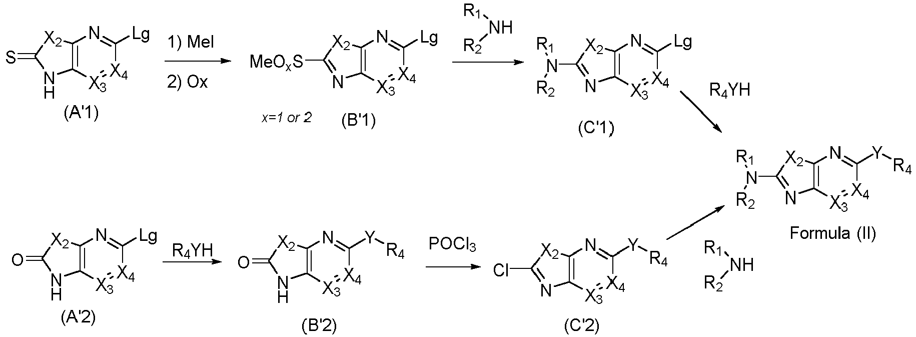

Compounds of Formula (I') may be prepared from compounds of formula (C1) through cross coupling with the appropriate compound of formula R₄YH. Same procedure applies to the synthesis of a compound of Formula (I") from a compound of formula (C'1). The compound of formula R₄YH may be an aryl or heteroaryl derivative such as aryl bromide, aryl chloride, aryl iodide or other suitable activating groups (e.g. triflates, mesylates, tosylates, nonaflates), aryl alcohol, arylthio, aryl-boronate, aryl-stannate, heteroaryl-amine, heteroaryl bromide, heteroaryl chloride, heteroaryl iodide or other suitable activating groups (e.g. triflates, mesylates, tosylates, nonaflates), heteroaryl alcohol, heteroaryl-thio, heteroaryl-boronate, heteroaryl-stannate, heteroaryl-amine. This reaction may be conducted under suitable acid or base conditions, in the presence or absence of a transition metal such as palladium, under different temperature conditions. Alternatively, a compound of Formula (I') can be prepared by reacting compound of formula (C2) with an amine R₁R₂NH under appropriate conditions, in the presence or absence of a base such as diisopropylethylamine. Similarly, the compounds of Formula (I") can be prepared from compounds of formula (C'2).

Compounds of the invention may be used in the form of prodrugs. A prodrug may be a pharmacologically inactive derivative of a biologically active substance (the "parent drug" or "parent molecule", i.e. the compound of the invention) that requires transformation within the body in order to release the active drug, and that has improved delivery properties over the parent drug molecule. The transformation *in vivo* may be, for example, as the result of some metabolic process, such as chemical or enzymatic hydrolysis of a carboxylic, phosphoric or sulphate ester, or reduction or oxidation of a susceptible functionality.

The invention also includes all suitable isotopic variations of a compound of the invention. Examples of isotopes that can be incorporated into compounds of the disclosure include isotopes such as ²H, ³H, ¹³C, ¹⁴C, ¹⁵N, ¹⁷O, ¹⁸O, ³¹P, ³²P, ³⁵S, ¹⁸F and ³⁶Cl, respectively. Certain isotopic variations of the disclosure, for example, those in which a radioactive isotope such as ³H or ¹⁴C is incorporated, are useful in drug and/or substrate tissue distribution studies. Further, substitution with isotopes such as deuterium ²H, may afford certain therapeutic advantages resulting from greater metabolic stability. Isotopic variations of the compounds of the invention can generally be prepared by conventional procedures such as by the illustrative methods and the preparations described in the Descriptions and in the Examples hereafter using appropriate isotopic variations of suitable reagents.

The present invention includes within its scope solvates of the compounds of Formula (I), (IA) and (IB) or of the relative salts, for example, hydrates, alcoholates and the like.

In addition, the compounds disclosed herein may exist as tautomers and all tautomeric forms are intended to be encompassed by the scope of the invention, even though only one tautomeric structure is depicted. For example, a reference to 2-hydroxypyridine also includes pyridin-2-one as its tautomeric form and a reference to 4-hydroxypyridine also includes pyridin-4-one as its tautomeric form. Similarly, a reference to a hydroxypyrimidine derivative also includes the corresponding pyrimidinone tautomer, a reference to 2,4-dihydro-3H-1,2,4-triazol-3-one also includes the corresponding 1H-1,2,4-triazol-5-ol, and so on. In particular, Formula (I) encompasses compounds of structures indicated below: 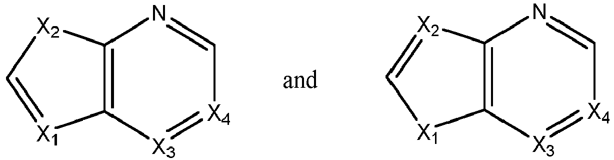 wherein X₁, X₂, X₃ and X₄ are as defined hereinabove.

The compounds disclosed herein may exist in different isomeric forms, all of which are encompassed by the present invention. In particular, any reference to the compound of the present invention is intended to include all its possible resonance forms.

The compounds of the present invention may have asymmetric centers, chiral axes, and chiral planes (as described in: E.L. Eliel and S.H. Wilen, Stereochemistry of Carbon Compounds, John Wiley & Sons, New York, 1994, pages 1119-1190), and occur as racemates, racemic mixtures, and as individual diastereomers, with all possible isomers and mixtures thereof, including optical isomers, all such stereoisomers being included in the present invention.

Pure stereoisomeric forms of the compounds and intermediates of this invention may be obtained by the application of art-known procedures and are intended to be encompassed by the scope of the invention. In particular, "pure stereoisomeric form" or "stereoisomerically pure" indicate a compound having stereoisomeric excess of at least 80%, preferably of at least 85%. For instance, enantiomers may be separated from each other by the selective crystallization of their diastereomeric salts or by chromatographic techniques using chiral stationary phases. Pure stereochemically isomeric forms may also be derived from the corresponding pure stereochemically isomeric forms of the appropriate starting materials, provided that the reaction occurs stereospecifically. The term "enantiomerically pure" shall be interpreted in a similar way, having regard to the enantiomeric ratio.

When any variable (e.g. R₁ and R₂, etc.) occurs more than one time in any constituent, its definition on each occurrence is independent at every other occurrence. Also, combinations of substituents and variables are permissible only if such combinations result in stable compounds. Lines drawn into the ring systems from substituents represent that the indicated bond may be attached to any of the substitutable ring atoms. If the ring system is polycyclic, it is intended that the bond be attached to any of the suitable carbon atoms on the proximal ring only.

It is understood that substituents and substitution patterns on the compounds of the instant invention can be selected by one of ordinary skill in the art to provide compounds that are chemically stable and that can be readily synthesized by techniques known in the art, as well as those methods set forth below, from readily available starting materials. If a substituent is itself substituted with more than one group, it is understood that these multiple groups may be on the same carbon or on different carbons, so long as a stable structure results. The phrase "optionally substituted" should be taken to be equivalent to the phrase "unsubstituted or substituted with one or more substituents" and in such cases the preferred embodiment will have from zero to three substituents. More particularly, there are zero to two substituents.

According to the invention, "-̅-̅-̅-̅-̅" represent a single or a double bond. It will be understood by the skilled person that the two bonds indicated as "-̅-̅-̅-̅-̅" in Formula (I) cannot both be double bonds. The two bonds indicated as "-̅-̅-̅-̅-̅" in Formula (I) may be two single bonds or one single bond and one double bond. In particular, general Formula (I) encompasses the two structures indicated below: 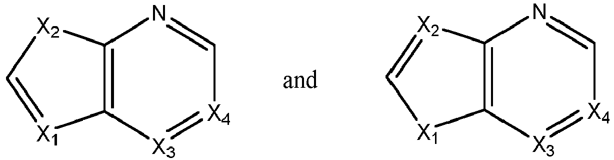 wherein X₁, X₂, X₃ and X₄ are as defined above.

The expressions "one or more substituents" and "one or more groups" refer to in particular to 1, 2, 3, 4 or more substituents, in particular to 1, 2, 3 or 4 substituents, more in particular 1, 2 or 3 substituents.

As used herein "Y is a single bond" indicates that, in the general Formula (I) or Formula (II), R₄ is directly linked via a single bond to the heteroaromatic scaffold.

As used herein, if two residues taken together represent a single bond, this means that the two atoms to which they are attached are connected by a single bond or by an additional bond thus forming a multiple bond.

As used herein, "alkyl" is intended to include both branched and straight-chain saturated aliphatic hydrocarbon groups having the specified number of carbon atoms. For example, "C₁-₁₂alkyl" is defined to include groups having 1, 2, 3, 4, 5, 6, 7, 8, 9, 10, 11 or 12 carbons in a linear or branched arrangement and specifically includes methyl, ethyl, *n*-propyl, *i*-propyl, *n*-butyl, *t*-butyl, *i*-butyl, pentyl, hexyl, and so on. As another example, "C₁-₆alkyl" is defined to include groups having 1, 2, 3, 4, 5 or 6 carbons in a linear or branched arrangement and specifically includes methyl, ethyl, *n*-propyl, *i*-propyl, *n*-butyl, *t*-butyl, *i*-butyl, pentyl, hexyl, and so on. Preferably, "C₁-₁₂alkyl" and "C₁-₆alkyl" refer to "C₁-₄alkyl" or "C₁-₃alkyl". "C₁-₄alkyl" is defined to include groups having 1, 2, 3 or 4 carbons in a linear or branched arrangement. For example, "C₁-₄ alkyl" specifically includes methyl, ethyl, *n*-propyl, *i*-propyl, *n*-butyl, *t*-butyl, *i*-butyl, and so on. "C₁₋₃alkyl" is defined to include groups having 1, 2, or 3 carbons in a linear or branched arrangement. For example, "C₁-₃ alkyl" specifically includes methyl, ethyl, *n*-propyl, *i*-propyl, and so on. Preferred alkyl groups are methyl, ethyl, *i*-propyl, *t*-butyl or *i*-butyl.

The term "alkenyl" as used herein refers to a straight or branched chain hydrocarbon which include one or more double bonds in the normal chain. For example, C₁₋₇alkenyl refers to a straight or branched chain hydrocarbon containing from 1 to 6 carbon atoms which include 1 to 3 double bonds in the normal chain. Representative examples of alkenyl include, but are not limited to, vinyl, 2-propenyl, 2-methyl-propenyl, 3-butenyl, 2-butenyl, 4-pentenyl, 3-pentenyl, 2-hexenyl, 3-hexenyl, 2,4-heptadiene, and the like.

As used herein, "alkoxy" represents an alkyl group of indicated number of carbon atoms attached through an oxygen bridge. "Alkoxy" therefore encompasses the definitions of alkyl above. C₁-₆ alkoxy group is preferably a linear or branched C₁-₄ alkoxy group, more preferably a C₁-₃alkoxy group, still more preferably a C₁-₂ alkoxy group. Examples of suitable alkoxy groups include, but are not limited to methoxy, ethoxy, *n*-propoxy, *i*-propoxy, *n*-butoxy, *s*-butoxy or *t*-butoxy. Preferred alkoxy groups include methoxy, ethoxy and *t*-butoxy.

As used herein, the terms "haloC₁₋₆alkyl" and "haloC₁₋₆alkoxy" mean a C₁₋₆alkyl or C₁₋₆alkoxy group in which one or more (in particular, 1 to 3) hydrogen atoms have been replaced by halogen atoms, especially fluorine or chlorine atoms. HaloC₁-₆alkoxy group is preferably a linear or branched haloC₁-₄alkoxy group, more preferably a haloC₁-₃alkoxy group, still more preferably a haloC₁-₂alkoxy group, for example OCF₃, OCHF₂, OCH₂F, OCH₂CH₂F, OCH₂CHF₂ or OCH₂CF₃, and most especially OCF₃ or OCHF₂. HaloC₁-₆alkyl group is preferably a linear or branched haloC₁-₃alkyl group, more preferably a haloC₁-₂alkyl group for example, CF₃, CHF₂, CH₂F, CH₂CH₂F, CH₂CHF₂, CH₂CF₃ or CH(CH₃)CF₃, and most especially CF₃, CHF₂ or CH(CH₃)CF₃.

As used herein, the term "hydroxyC₁₋₆alkyl" means a C₁₋₆alkyl group in which one or more (in particular, 1 to 3) hydrogen atoms have been replaced by hydroxy groups. Similarly, the term "hydroxyC₁₋₄alkyl" means a C₁₋₄alkyl group in which one or more (in particular, 1 to 2) hydrogen atoms have been replaced by hydroxy groups. Illustrative examples include, but are not limited to CH₂OH, CH₂CH₂OH, CH(CH₃)OH and CHOHCH₂OH.

As used herein, the term "aminoC₁₋₆alkyl" means a C₁₋₆alkyl group in which one or more (in particular, 1 to 3) hydrogen atoms have been replaced by small amino groups, such as NH2, NHCH₃, N(CH₃)₂ and the like.

As used herein, the term "aryl" or "aromatic ring" means a monocyclic or polycyclic aromatic ring comprising carbon atoms and hydrogen atoms. If indicated, such aromatic ring may include one or more heteroatoms, then also referred to as "heteroaryl" or "heteroaromatic ring", preferably, 1 to 3 heteroatoms, independently selected from nitrogen, oxygen, and sulfur, preferably nitrogen. As is well known to those skilled in the art, heteroaryl rings have less aromatic character than their all-carbon counter parts. Thus, for the purposes of the present invention, a heteroaryl group need only have some degree of aromatic character. Preferably, the ring component of aryl or heteroaryl groups comprises 5 or 6 members (i.e. atoms). Illustrative examples of aryl groups are optionally substituted phenyl. Illustrative examples of heteroaryl groups according to the invention include optionally substituted thiophene, oxazole, thiazole, thiadiazole, imidazole, pyrazole, pyrimidine, pyrazine, pyridine and pyridine N-oxide. Thus, examples of monocyclic aryl optionally containing one or more heteroatoms, for example one or two heteroatoms, are a 5- or 6-membered aryl or heteroaryl group such as, but not limited to, phenyl, pyridyl, pyrimidinyl, pyrazinyl, pyridazinyl, pyrrolyl, thienyl, thiazolyl, thiadiazolyl, pyrazolyl, imidazolyl, triazolyl, tetrazolyl, furyl, isoxazolyl, oxadiazolyl and oxazolyl. Examples of polycyclic aromatic ring, optionally containing one or more heteroatoms, for example one or two heteroatoms, are a 8-10 membered aryl or heteroaryl group such as, but not limited to, benzimidazolyl, benzofurandionyl, benzofuranyl, benzofurazanyl, benzopyrazolyl, benzotriazolyl, benzothienyl, benzoxazolyl, benzoxazolonyl, benzothiazolyl, benzothiadiazolyl, benzodioxolyl, benzoxadiazolyl, benzoisoxazolyl, benzoisothiazolyl, indolyl, indolinyl, indolizinyl, indazolyl, isobenzofuranyl, isoindolyl, isoindolinyl, isoquinolyl, quinazolinyl, quinolyl, quinoxalinyl, quinolizinyl, naphtyl, naphthyridinyl and phthalazinyl. Other examples of polycyclic heteroaromatic rings according to the invention are 2H-pyrazolo[3,4-b]pyridine, indazole, 2H-pyrazolo[3,4-c]pyridine, 6H-pyrrolo[3,4-b]pyridine, 6H-pyrrolo[3,4-b]pyrazine, 6H-pyrrolo[3,4-d]pyrimidine, 2H-pyrazolo [3 ,4-d]pyrimidine.

Polycyclic aromatic or heteroaromtic rings can also have a partially unsaturated structure and can thus be derived from the partially hydrogenated analogues of the before-listed aryl or heteroaryl groups but also from an aryl or heteroaryl ring fused with a cycloalkyl or heterocycloalkyl ring. Examples of said partially unsaturated polycyclic aryl or heteroaryl derivatives include 2,3-dihydro-1H-indene, 2,3-dihydro-1H-inden-1-one indoline, 1,2,3,4-tetrahydroquinoline, 1,2,3,4-tetrahydroisoquinoline, isoindoline, dihydroquinazoline, dihydroquinoxaline, 2,3-dihydrobenzofuran, benzo[d][1,3]dioxole, 1,3-dihydroisobenzofuran, 3,4-dihydro-2H-benzo[b][1,4]oxazine, 2,3,4,5-tetrahydrobenzo[f][1,4]oxazepine, quinazolin-4(3H)-one, 4,5,6,7-tetrahydro-1H-indazole, 4,5,6,7-tetrahydropyrazolo[1,5-a]pyrazine, 2,3,4,5-tetrahydro-1H-benzo[d]azepine, 6',7'-dihydrospiro[azetidine-3,5'-pyrrolo[1,2-a]imidazole], 2,3-dihydrobenzo[b][1,4]dioxine, benzo[d]oxazol-2(3H)-one, 2H-benzo[b][1,4]oxazin-3(4H)-one, indolin-2-one, 1,2,3,4-tetrahydro-1,5-naphthyridine, 3',4'-dihydro-2'H-spiro[azetidine-3,1'-pyrrolo[1,2-a]pyrazine] and the like. A preferred aryl according to the present invention is phenyl. A preferred heteroaryl according to the present invention is pyridyl.

As used herein, the term "aryloxy" represents an aryl group as defined above attached through an oxygen bridge. Aryloxy therefore encompasses the definitions of aryl above. Illustrative examples include phenoxy, naphtyloxy, pyridinyloxy and so on.

The expressions "optionally substituted aryl", "optionaly substituted heteroaryl" or "optionally substituted aryloxy" generically refer to aryl, heteroaryl or aryloxy groups wherein the aromatic or heteroaromatic ring may be substituted with one or more substituents. Examples of said substituents include alkyl, alkoxy, amino, trifluoromethyl, aryl, heteroaryl, hydroxyl, carboxyalkyl and the like.

The expressions "haloaryl" and "haloheteroaryl" respectively refer to an aryl and an heteroaryl ring substituted with one or more halogen atoms.

Heterocycle, heterocyclic compound or ring structure or heterocycloalkyl is a saturated monocyclic or polycyclic compound that has atoms of at least two different elements as members of its ring(s).

As used herein, the term "C₃₋₉heterocycloalkyl" is a saturated monocyclic or bicyclic ring system, of 3 to 9 members which contains one or more heteroatoms selected from N, O and S or contains a group selected from SO, SO₂ and C=O. In a particular embodiment of the invention, the C₃₋₉heterocycloalkyl group is restricted to a C₃₋₈heterocycloalkyl or to a C₅₋₇heterocycloalkyl group. Examples include, but are not limited to azetidinyl, piperazinyl, piperidinyl, morpholinyl, thiomorpholinyl, thiazolidinyl, pyrrolidinyl, azepanyl, diazepanyl, oxazepanyl, thiazepanyl, azocanyl, oxazocanyl, 8-oxabicyclo[3.2.1]octane, 2-oxabicyclo[2.1.1]hexane, hexahydrofuro[2,3-b]furane, 2-azaspiro[3.3]heptane, azepan-2-one, 3-azaspiro[5.5]undecane, 2-azaspiro[4.5]decane, 3-azabicyclo[3.3.1]nonane, 3-oxa-7-azabicyclo[3.3.1]nonane, 5-azaspiro[3.5]nonane, 1-thia-7-azaspiro[3.5]nonane 1,1-dioxide, 3-azabicyclo[3.2.0]heptane, 2-azabicyclo[2.1.1]hexane, 6-azabicyclo[3.2.1]octane, octahydroindole, octahydro-1H-isoindole, 5-oxa-2-azaspiro[3.4]octane, 1,2,3,4-tetrahydroquinoline, oxetanyl, tetrahydro-2H-pyranyl, tetrahydrofuranyl, thiolane 1,1-dioxide, 2-oxa-9-azaspiro[5.5]undecane, oxazocanyl 2-oxabicyclo[2.1.1]hexane, 7-azabicyclo[2.2.1]heptane, octahydro-4aH-cyclopenta[b]pyridine, octahydro-1H-cyclopenta[b]pyridine. Preferred are saturated cyclic hydrocarbons with 3, 4 or 5 carbon atoms and 1 oxygen or 1 nitrogen atom. Examples include oxetanyl, tetrahydrofuranyl, tetrahydro-2H-pyranyl, piperidinyl or pyrrolidinyl

As used herein, the term "C₅₋₇heterocycloalkoxy" represents a C₅₋₇heterocycloalkyl group as defined above attached through an oxygen bridge. Illustrative examples include the oxetan-3-yloxy, azetidin-3-yloxy, pyrrolidin-3-yloxy and so on.

A substituent on a saturated, partially saturated or unsaturated heterocycle can be attached at any substitutable position.

As used herein, the term "C₁₋₆ alkanediyl" as group or part of a group defines bivalent straight or branched chained saturated hydrocarbon radicals having from 1 to 6 carbon atoms. C₁₋₆ alkanediyl group, is preferably a C₁₋₄ alkanediyl group, a C₁₋₃ alkanediyl or more preferably a C₁₋₂ alkanediyl. Examples include, but are not limited to methanediyl, ethanediyl, propanediyl, butanenediyl, pentanediyl and hexanediyl. Preferred are methanediyl, ethanediyl and propanediyl.

As used herein, the term "C₂₋₇alkenediyl" as group or as part of a group defines bivalent straight or branched (carbon number limitation permitting) chained unsaturated hydrocarbon radicals having from 2 to 7 carbon atoms. C₂₋₇ alkenediyl group, is preferably a C₂₋₄ alkenediyl group. Non limiting examples of C₂₋₇alkenediyl are: -C=CH-, -CH=C(CH₃)CH₂-, -CH=CH-CH₂-.

As used herein, the term or "C₃₋₉cycloalkyl" means saturated cyclic hydrocarbon (cycloalkyl) with 3, 4, 5, 6, 7, 8 or 9 ring atoms and is generic for example to cyclopropyl, cyclobutyl, cyclopentyl, cyclohexyl, cycloheptyl, cyclooctyl or cyclononyl. As used herein, the term "C₃₋₈ cycloalkyl" means saturated cyclic hydrocarbon (cycloalkyl) with 3, 4, 5, 6, 7 or 8 carbon atoms and in a particular embodiment of the invention, the C₃₋₈cycloalkyl is restricted to a C₃₋₇ cycloalkyl, such as a 5 or 6 membered cycloalkyl. Depending on the dimension of the ring, it can be also of bicyclic structure, such as a bicycle[3.1.0]hexane, bicycle[4.1.0]heptane, octahydropentalene, bicyclo[1.1.1]pentane, and the like. In a particular embodiment of the invention, the 3-11 membered saturated ring" is restricted to a "3-9 membered saturated ring" or a "3-7 membered saturated ring".

As used herein, the expression "3-8 membered partially saturated ring" indicates a ring containing 3 to 8 carbon atoms and at least one double bond. Depending on the dimension of the ring, it can be of a cyclic or bicyclic structure. In a particular embodiment of the invention, the 3-8 membered partially saturated ring" is restricted to a "5-7 membered partially saturated ring". Each of the above rings may optionally contain one or more heteroatoms, such that at least one carbon is replaced by by a heteroatom selected from N, O and S, in particular from N and O. Examples include, but are not limited to cyclopentenyl, cyclohexenyl, cyclohexa-1,3-dienyl, cyclohexa-1,4-dienyl, cycloheptenyl, cyclohepta-1,4-dienyl, dihydrofuranyl, dihydropyrrole, dihydropyranyl, hexahydro-1H-cyclopenta[c]furanyl and the like.

It should be noted that different isomers of the various heterocycles may exist within the definitions as used throughout the specification. For example, pyrrolyl may be 1H-pyrrolyl or 2H-pyrrolyl.

It should also be noted that the radical positions on any molecular moiety used in the definitions may be anywhere on such moiety as long as it is chemically stable. For example, pyridyl includes 2-pyridyl, 3-pyridyl, 4-pyridyl.

As used herein, the term "halogen" refers to fluorine, chlorine, bromine and iodine, of which fluorine, chlorine and bromine are preferred.

The term "heteroatom" refers to an atom other than carbon or hydrogen in a ring structure or a saturated backbone as defined herein. Typical heteroatoms include N(H), O, S.

As used herein, "cycloalkoxy" represents a cycloalkyl group of the indicated number of carbons attached through an oxygen bridge. "Cycloalkoxy" therefore encompasses the definitions of cycloalkyl above and is preferably a C₁₋₆alkoxy as cyclopropyloxy, cyclobutyloxy, cyclopentyloxy, cyclohexyloxy.

The expression "cyano-C₃₋₇cycloalkyl" refers to one or more cyano groups appended to a C_{3- 7}cycloalkyl.

The term "C₁₋₆alkylaryl" as used herein indicates one or more aryl groups appended to a C₁₋₆alkyl radical. Preferably, the C₁₋₆alkylaryl is a "C₁₋₃alkylaryl", i.e. one or more aryl groups appended to a C₁₋₃alkyl radical. As used herein, the term "C₁₋₆alkylheteroaryl" indicates one or more heteroaryl groups appended to a C₁₋₆alkyl radical. Preferably, the C₁₋₆alkylheteroaryl is a "C_{1- 3}alkylheteroaryl", i.e. one or more heteroaryl groups appended to a C₁₋₃alkyl radical. As used herein, the term "C₁₋₆alkyl-C₃₋₈cycloalkyl" indicates one or more C₃₋₈cycloalkyl groups appended to a C₁₋₆alkyl radical.

The term "spiro-C₃₋₈cycloalkyl" indicates a C₃₋₈cycloalkyl forming a bicyclic organic compound with rings connected through just one atom. The rings can be different in nature or identical. The connecting atom is also called the spiroatom, most often a quaternary carbon ("spiro carbon"). Said spiro-C₃₋₈cycloalkyl ring may optionally contain a heteroatom and is also defined as a "spiro-C₃₋₈heterocycloalkyl".

Included in the instant invention is the free base of compounds of Formula (I) or Formula (II) as well as the pharmaceutically acceptable salts and stereoisomers thereof. Some of the specific compounds exemplified herein are the protonated salts of amine compounds. Compounds of Formula (I), (IA) or (IB) containing one or more N atoms may be protonated on any one, some or all of the N atoms. The term "free base" refers to the amine compounds in non-salt form. The encompassed pharmaceutically acceptable salts not only include the salts exemplified for the specific compounds described herein, but also all the typical pharmaceutically acceptable salts of the free form of compounds of Formula (I), (IA) or (IB). The free form of the specific salt compounds described may be isolated using techniques known in the art. For example, the free form may be regenerated by treating the salt with a suitable dilute aqueous base solution such as dilute aqueous NaOH, potassium carbonate, ammonia and sodium bicarbonate. The free forms may differ from their respective salt forms somewhat in certain physical properties, such as solubility in polar solvents, but the acid and base salts are otherwise pharmaceutically equivalent to their respective free forms for purposes of the invention.

The pharmaceutically acceptable salts of the instant compounds can be synthesized from the compounds of this invention which contain a basic or acidic moiety by conventional chemical methods. Generally, the salts of the basic compounds are prepared either by ion exchange chromatography or by reacting the free base with stoichiometric amounts or with an excess of the desired salt-forming inorganic or organic acid in a suitable solvent or various combinations of solvents. Similarly, the salts of the acidic compounds are formed by reactions with the appropriate inorganic or organic base. In a preferred embodiment, the compounds of the invention have at least one acidic proton and the corresponding sodium or potassium salt can be formed, for example, by reaction with the appropriate base.

Thus, pharmaceutically acceptable salts of the compounds of this invention include the conventional non-toxic salts of the compounds of this invention as formed by reacting a basic instant compound with an inorganic or organic acid or an acid compound with an inorganic or organic base. For example, conventional non-toxic salts include those derived from inorganic acids such as hydrochloric, hydrobromic, sulfuric, sulfamic, phosphoric, nitric and the like, as well as salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxy-benzoic, fumaric, toluenesulfonic, methanesulfonic, ethane disulfonic, oxalic, isethionic, trifluoroacetic and the like. Convential non-toxic salts further include those derived from an inorganic base, such as potassium, sodium hydroxide, magnesium or calcium hydroxide, as well as salts prepared from organic bases, such as ethylene diamine, lysine, tromethamine, meglumine and the like. Preferably, a pharmaceutically acceptable salt of this invention contains one equivalent of a compound of Formula (I), (IA) or (IB) and 1, 2 or 3 equivalent of an inorganic or organic acid or base. More particularly, pharmaceutically acceptable salts of this invention are the tartrate, trifluoroacetate or the chloride salts.

When the compound of the present invention is acidic, suitable "pharmaceutically acceptable salts" refers to salts prepared from pharmaceutically acceptable non-toxic bases including inorganic bases and organic bases. Salts derived from inorganic bases include aluminum, ammonium, calcium, copper, ferric, ferrous, lithium, magnesium, manganic salts, manganous, potassium, sodium, zinc and the like. Particularly preferred are the ammonium, calcium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins, such as arginine, betaine caffeine, choline, N,N¹-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine tripropylamine, tromethamine and the like.

The preparation of the pharmaceutically acceptable salts described above and other typical pharmaceutically acceptable salts is more fully described by Berg et al., "Pharmaceutical Salts," J. Pharm. Sci., 1977:66:1-19.

It will also be noted that the compounds of the present invention are potentially internal salts or zwitterions, since under physiological conditions a deprotonated acidic moiety in the compound, such as a carboxyl group, may be anionic, and this electronic charge might then be balanced off internally against the cationic charge of a protonated or alkylated basic moiety, such as a quaternary nitrogen atom.

Preferably, compounds of the present invention, including salts, tautomers, stereoisomers and solvates thereof, are SHP2 inhibitors, meaning that for example they can inhibit the activity or function of SHP2. Then, the present invention relates to compounds for use as inhibitors of at least one SHP2 function and to a method of inhibiting at least one SHP2 function comprising the step of contacting SHP2 with a compound as described herein.

"SHP2" means "Src Homology-2-phosphatase" and is also known as SH-PTP2, SH-PTP3, Syp, PTPID, PTP2C, SAP-2 or PTPN11.

The functions of SHP2 are varied. SHP2 regulates cancer cell survival and proliferation primarily by activating the RAS-ERK signaling pathway (T. Matozaki, Y. Murata, Y. Saito, H. Okazawa, H. Ohnishi, Cancer Sci, 100 (2009), pp. 1786-1793). Recently, Chen et al. (Y.N. Chen, M.J. LaMarche, H.M. Chan, P. Fekkes, J. Garcia-Fortanet, M.G. Acker, et al., Nature, 535 (2016), pp. 148-152) found that cancer cell lines sensitive to SHP2 depletion were also sensitive to EGFR depletion, which validated reports that RTK-driven cancer cells depend on SHP2 for survival. Furthermore, recent studies have shown that SHP2 is required for the growth of mutant KRAS-driven cancers while wild-type KRAS-amplified gastroesophageal cancer can be controlled through combined SHP2 and MEK inhibition (S. Mainardi, A. Mulero-Sanchez, A. Prahallad, G. Germano, A. Bosma, P. Krimpenfort, et al. Nat Med, 24 (2018), pp. 961-9; D.A. Ruess, G.J. Heynen, K.J. Ciecielski, J. Ai, A. Berninger, D. Kabacaoglu, et al. Nat Med, 24 (2018), pp. 954-960; G.S. Wong, J. Zhou, J.B. Liu, Z. Wu, X. Xu, T. Li, et al. Nat Med, 24 (2018), pp. 968-977). As a downstream target of several receptors, SHP2 is also involved in signaling in T-cells (M. Tajan, A. de Rocca Serra, P. Valet, T. Edouard, A. Yart, Eur J Med Genet, 58 (2015), pp. 509-525; R.J. Salmond, D.R. Alexander, Trends Immunol, 27 (2006), pp. 154-160). It is a downstream molecule in the PD-1 signaling pathway which not only suppresses T-cell activation but also causes T-cell anergy. SHP2-deficiency in T-cells triggered an anti-tumor immune response against colitis-associated cancer in mice (W. Liu, W. Guo, L. Shen, Z. Chen, Q. Luo, X. Luo, et al. Oncotarget, 8 (2017), pp. 7586-7597). Therefore, targeting SHP2 may restore or even enhance T-cell functions.

The cell phenotype, cell proliferation, activity of SHP2, change in biochemical output produced by active SHP2, expression of SHP2, or binding of SHP2 with a natural binding partner may be monitored as a measure of SHP2 inhibition. In particular, inhibition of SHP2 activity or function can be measured by the IC₅₀ (concentration of inhibitor which reduces the activity of SHP2 to half-maximal level), as described in the assays hereinbelow or in the biochemical assays for SHP2 inhibition reported for example by Chen et al., Nature (535) 2016 or by Bagdanoff et al., J. Med. Chem. 2019, 62, 1781-1792. Preferably, compounds of the invention exhibit an IC₅₀ towards SHP2 lower than or equal to 10 µM. Preferred compounds exhibit an enzymatic IC₅₀ towards SHP2, as defined hereinbelow, lower than or equal to 3 µM (preferably lower than or equal to 0.5 µM or between 0.5 µM and 3 µM) and/or a cellular IC₅₀ towards SHP2, as defined hereinbelow, lower than or equal to 5 µM (preferably lower than or equal to 1 µM or between 1 µM and 5 µM). Then, the compounds of the present invention, including salts, tautomers, stereoisomers and solvates thereof, may be for use in a method of inhibiting SHP2 activity. In other words, they may be for use in the prevention and/or treatment of any condition that would be ameliorated by SHP2 inhibition.

In a preferred embodiment, the compound or a pharmaceutically acceptable salt, tautomer, solvate, or stereoisomer thereof as defined above is for use in inhibiting SHP2 activity. Inhibition of SHP2 activity may be measured with respect to a proper control, such as a subject affected by a disease or disorder mediated by the activity of SHP2 or a subject throughout the course of a therapy for a disease or disorder mediated by the activity of SHP2. Preferably, compounds of the invention inhibit SHP2 activity by at least 10%, 20%, 30%, 40%, 50%, 60%, 70%, 80%, 90% in respect to a proper control. More preferably, compounds of the invention inhibit SHP2 activity by approximately 10%, 20%, 30%, 40%, 50%, 60%, 70%, 80%, 90% in respect to a proper control.

Yet more preferably, compounds of the invention inhibit SHP2 activity by more than 90%, for instance by approximately 92%, 94%, 95%, 98%, 99% or 100% in respect to a proper control.

Thereofore, the compounds of the invention can be used for the treatment of diseases and for carrying out biological assays, cellular assays, biochemical assays or the like.

It is an object of the invention a compound or a pharmaceutically acceptable salt, tautomer, solvate, or stereoisomer thereof as defined above for medical use.

Preferably, the compound or the pharmaceutically acceptable salt, tautomer, solvate, or stereoisomer thereof as defined above is for use in inhibiting SHP2 activity.

More preferably, the compound or the pharmaceutically acceptable salt, tautomer, solvate, or stereoisomer thereof as defined above is for use in the treatment and/or prevention of a disease or disorder mediated by the activity of SHP2.

Still preferably, the disease or disorder mediated by the activity of SHP2 is selected from the group consisting of: cancer, cardiovascular disease, immunological disorder, fibrosis, an ocular disorder, systemic lupus erythematosus, diabetes, neutropenia, and combinations thereof, preferably said cancer is a cancer metastasis.

Yet preferably, the disease or disorder mediated by the activity of SHP2 is selected from the group consisting of: Noonan Syndrome, Leopard Syndrome, juvenile myelomonocytic leukemias, neuroblastoma, melanoma, head and neck squamous-cell carcinoma, acute myeloid leukemia, breast cancer, esophageal tumor, lung cancer, colon cancer, head cancer, gastric carcinoma, lymphoma, glioblastoma, gastric cancer, pancreatic cancer and combinations thereof.

A disease or disorder mediated by the activity of SHP2 indicates a condition in a subject in which modulation, in particular inhibition, of SHP2 activity can prevent, inhibit, ameliorate, slow down or eradicate the condition and/or the symptomology thereof. Treatment of said disease or disorder might comprise administering to the subject in need thereof a therapeutically effective amount of a compound of Formula (I) according to the invention.

In diseases or disorders mediated by the activity of SHP2, mutations are often observed at the N-SH2/PTP interface (e.g. E76D/E76K), resulting in constitutively active protein and abnormal proliferation. Cancers harboring "PTPN11 mutations" include but are not limited to: N58Y; D61Y, V; E69K; A72V, T, D; E76G, Q, K (ALL); G60A; D61Y; E69V; F71K; A72V; T73I; E76G, K; R289G; G503V (AML); G60R, D61Y, V, N; Y62D; E69K; A72T, V; T73I; E76K, V, G, A, Q; E139D; G503A, R; Q506P (JMML); G60V; D61V; E69K; F71L; A72V; E76A (MDS); Y63C (CMML); Y62C; E69K; T507K (neuroblastoma); V46L; N58S; E76V (Lung cancer); R138Q (melanoma); E76G (colon cancer). The compounds of the invention can exhibit affinity at low concentrations for wild type SHP2 and can also be active against mutant forms of the protein.

Another aspect of the present invention relates to a compound of the invention, including any pharmaceutically acceptable salt, tautomer, solvate or stereoisomer thereof, as defined hereinabove for use in a method of preventing/treating an SHP2-mediated disorder.

Another aspect of the present invention relates to a method of preventing/treating an SHP2-mediated disorder comprising the step of administering to a patient in need thereof a therapeutically effective amount of a compound of the invention, including any pharmaceutically acceptable salt, tautomer, solvate or stereoisomer thereof, as defined hereinabove. In another aspect, the present invention relates to a method of preventing/treating an SHP2-mediated disorder, comprising the step of administering to a patient in need thereof a therapeutically effective amount of a chemotherapeutic agent, as further defined below, in combination with a therapeutically effective amount of a compound of the invention.

Another aspect of the present invention relates to the use of compounds of the invention, including any pharmaceutically acceptable salts tautomer, solvate or stereoisomer thereof, as defined hereinabove in preventing/treating an SHP2-mediated disorder.

In certain embodiments the present invention relates to the aforementioned use/method, wherein said disorder is selected from Noonan Syndrome (NS) and Leopard Syndrome (LS).

In further embodiments, the present invention relates to the aforementioned use/method, wherein said SHP2-mediated disorders are those due to dysregulated cellular proliferation, including cancer. The cancer may be hormone-dependent or hormone-resistant, such as in the case of breast cancers. Preferably, the cancer is RTK-driven or KRAS-driven, such as KRAS amplified gastroesophageal cancer. In certain embodiments, the cancer is a solid tumor. In other embodiments, the cancer is a lymphoma or leukemia or a glioma. In certain embodiments, the cancer is a drug resistant phenotype of a cancer disclosed herein or known in the art. The cancer may be primary or metastatic. Tumor invasion, tumor growth, tumor metastasis, and angiogenesis may also be treated using the compositions and methods disclosed herein. Precancerous neoplasias may also be treated using the compositions and methods disclosed herein.

Compounds of the invention can be used for the treatment of cancers selected from, but not limited to: Juvenile Myelomonocytic Leukemias (JMML); Acute Myeloid Leukemia (AML); Myelodysplastic Syndrome (MDS); B cell acute lymphoblastic leukemia (B-ALL); neuroblastoma; esophageal; breast cancer; lung cancer; colon cancer; gastric cancer, head and neck cancer; ovarian cancer; prostate cancer; cancers of the oral cavity and pharynx (lip, tongue, mouth, larynx, pharynx), stomach, small intestine, large intestine, colon, rectum, liver and biliary passages; pancreas, bone, connective tissue, skin, cervix, uterus, corpus endometrium, testis, bladder, kidney and other urinary tissues, including renal cell carcinoma (RCC); gastroesophageal cancer (preferably KRAS-amplified gastroesophageal cancer), cancers of the eye, brain, spinal cord, and other components of the central and peripheral nervous systems, as well as associated structures such as the meninges; thyroid and other endocrine glands, Hodgkin's disease, non-Hodgkin's lymphomas, multiple myeloma and hematopoietic malignancies including leukemias Chronic Lymphocytic Leukemia (CLL), Acute Lymphocytic Leukemia (ALL), Chronic Myelogenous Leukemia (CML), Acute Myelogenous Leukemia (AML), Chronic Myelomonocytic Leukemia (CMML), and lymphomas including lymphocytic, granulocytic and monocytic. Additional types of cancers which may be treated using the compounds and methods of the invention include, but are not limited to, adenocarcinoma, angiosarcoma, astrocytoma, acoustic neuroma, anaplastic astrocytoma, basal cell carcinoma, blastoglioma, chondrosarcoma, choriocarcinoma, chordoma, craniopharyngioma, cutaneous melanoma, cystadenocarcinoma, endotheliosarcoma, embryonal carcinoma, ependymoma, Ewing's tumor, epithelial carcinoma, fibrosarcoma, gastric cancer, genitourinary tract cancers, glioblastoma multiforme, hemangioblastoma, hepatocellular carcinoma, hepatoma, Kaposi's sarcoma, large cell carcinoma, leiomyosarcoma, leukemias, liposarcoma, lymphatic system cancer, lymphomas, lymphangiosarcoma, lymphangioendotheliosarcoma, medullary thyroid carcinoma, medulloblastoma, meningioma mesothelioma, myelomas, myxosarcoma neuroblastoma, neurofibrosarcoma, oligodendroglioma, osteogenic sarcoma, epithelial ovarian cancer, papillary carcinoma, papillary adenocarcinomas, paraganglioma, parathyroid tumours, pheochromocytoma, pinealoma, plasmacytomas, retinoblastoma, rhabdomyosarcoma, sebaceous gland carcinoma, seminoma, skin cancers, melanoma, small cell lung carcinoma, non-small cell lung carcinoma, squamous cell carcinoma, sweat gland carcinoma, synovioma, thyroid cancer, uveal melanoma, Wilm's tumor, anaplastic large-cell lymphoma, colitis associated cancer.

The compounds of the present invention may be useful in the treatment of any other disease or condition related to the aberrant activity of SHP2. Thus, as a further aspect, the invention relates to a method of treatment of a disorder selected from: NS; LS; JMML; AML; MDS; B-ALL; neuroblastoma; esophageal; breast cancer; lung cancer; colon cancer; gastric cancer; head and neck cancer.

A compound of the present invention, including any pharmaceutically acceptable salt, tautomer, solvate, or stereoisomer thereof, may be usefully combined with any another known therapy that is useful for the prevention/treatment of a disease or disorder mediated by the activity of SHP2. Such therapy may include radiotherapy. Such therapy may also comprise the administration of another pharmacologically active compound, or of two or more other pharmacologically active compounds, particularly compound(s) active in the prevention/treatment of cancer, also referred to as "anti-cancer drug(s)" or "chemotherapy agents". For example, a compound of the invention, including any pharmaceutically acceptable salt, tautomer, solvate, or stereoisomer thereof as defined above, may be administered simultaneously, sequentially or separately in combination with any one or more other pharmacologically active compound. For simultaneous administration, the compound of the present invention and the other one or more pharmacologically active compound may be formulated in the same composition.

Classes of anti-cancer drugs that may be combined with the compounds of the invention include, but are not limited to: alkylating agents, anti-metabolites, antimitotics, checkpoint inhibitors, plant alkaloids and terpenoids, topoisomerase inhibitors, cytotoxic antibiotics, aromatase inhibitors, angiogenesis inhibitors, anti-steroids and anti-androgens, mTOR inhibitors, tyrosine kinase inhibitors, and others. Chemotherapy agents include, for example, mitotic inhibitors such as a taxane, a vinca alkaloid, paclitaxel, docetaxel, vincristine, vinblastine, vinorelbine or vinflunine, and other anticancer agents, e.g. cisplatin, 5-fluorouracil or 5-fluoro-2-4(1H,3H)-pyrimidinedione (5FU), flutamide or gemcitabine. Such combinations may offer significant advantages, including synergistic activity, in therapy.

Alkylating agents are compounds that work by adding an alkyl group to the guanine base of the DNA molecule, preventing the strands of the double helix from linking as they should thus causing breakage of the DNA strands and affecting the ability of the cancer cells to multiply. Antimetabolites are drugs that interfere with one or more enzymes or their reactions that are necessary for DNA synthesis. An antimitotic agent is a type of drug that blocks cell growth by stopping mitosis. Checkpoint inhibitors are a type of immunotherapy which block proteins that stop the immune system from attacking the cancer cells. Topoisomerase inhibitors are chemical compounds that block the action of topoisomerase (topoisomerase I and II), which is a type of enzyme that controls the changes in DNA structure by catalyzing the breaking and rejoining of the phosphodiester backbone of DNA strands during the normal cell cycle. Aromatase inhibitors are a class of drugs that work by inhibiting the action of the enzyme aromatase, which converts androgens into estrogens by a process called aromatization. Angiogenesis inhibitors are substances that inhibit the growth of new blod vessels and are used to treat cancers and other diseases that involve a proliferation of blood vessels. mTOR inhibitors are a class of drugs that inhibit the mammalian target of rapamycin (mTOR), which is a serine/threonine-specific protein kinase that belongs to the family ofphosphatidylinositol-3 kinase (PI3K) related kinases (PIKKs). mTOR regulates cellular metabolism, growth, and proliferation by forming and signaling through two protein complexes, mTORC1 and mTORC2. The most established mTOR inhibitors are so-called rapalogs (rapamycin and its analogs), which have shown tumor responses in clinical trials against various tumor types.

It is thus a preferred object of the invention a compound as defined above or the pharmaceutically acceptable salt, tautomer, solvate, or stereoisomer thereof, for use in combination with at least one further therapeutic agent.

In any case, the multiple therapeutic agents (at least one of which is a compound disclosed herein) may be administered in any order or even simultaneously.

Preferably, said at least one further therapeutic agent is selected from the group consisting of:
(a) alkylating agents, including but not limited to carmustine, chlorambucil (LEUKERAN), cisplatin (PLATIN), carboplatin (PARAPLATIN), oxaliplatin (ELOXATIN), streptozocin (ZANOSAR), busulfan (MYLERAN), dacarbazine, ifosfamide, lomustine (CCNU), melphalan (ALKERAN), procarbazine (MATULAN), temozolomide(TEMODAR), thiotepa, and cyclophosphamide (ENDOXAN);
(b) anti-metabolites, including but not limited to cladribine (LEUSTATIN), mercaptopurine (PURINETHOL), thioguanine, pentostatin (NIPENT), cytosine arabinoside (cytarabine, ARA-C), gemcitabine (GEMZAR), fluorouracil (5-FU, CARAC), capecitabine (XELODA), leucovorin (FUSILEV), methotrexate (RHEUMATREX) and raltitrexed;
(c) antimitotics, which are often plant alkaloids and terpenoids, or derivatives thereof, including but not limited to taxanes such as docetaxel (TAXITERE) and paclitaxel (ABRAXANE, TAXOL); vinca alkaloids such as vincristine (ONCOVIN), vinblastine, vindesine, vinorelbine (NAVELBINE), and vinflunine;
(d) checkpoint inhibitors, such as anti- PD-1 or PD-L1 antibodies pembrolizumab (KEYTRUDA), nivolumab (OPDIVO), MEDI4736 and MPDL3280A; anti-CTLA-4 antibody ipilimumab (YERVOY); inhibitors that target LAG3 (lymphocyte activation gene 3 protein), KIR (killer cell immunoglobulin-like receptor), 4-1BB (tumour necrosis factor receptor superfamily member 9), TIM3 (T-cell immunoglobulin and mucin-domain containing-3) and/or OX40 (tumour necrosis factor receptor superfamily member 4);
(e) topoisomerase inhibitors, including but not limited to camptothecin (CTP), irinotecan (CAMPTOSAR), topotecan (HYCAMTIN), teniposide (VUMON) and etoposide (EPOSIN);
(f) cytotoxic antibiotics, including but not limited to actinomycin D (dactinomycin, COSMEGEN), bleomycin (BLENOXANE) doxorubicin (ADRIAMYCIN), daunorubicin (CERUBIDINE), epirubicin (ELLENCE), fludarabine (FLUDARA), idarubicin, mitomycin (MITOSOL), mitoxantrone (NOVANTRONE), plicamycin; (7) aromatase inhibitors, including but not limited to aminoglutethimide, anastrozole (ARIMIDEX), letrozole (FEMARA), vorozole (RIVIZOR) and exemestane (AROMASIN);
(g) angiogenesis inhibitors, including but not limited to genistein, sunitinib (SUTENT) and bevacizumab (AVASTIN);
(h) anti-steroids and anti-androgens such as aminoglutethimide (CYTADREN), bicalutamide (CASODEX), cyproterone, flutamide (EULEXIN) and nilutamide (NILANDRON);
(i) tyrosine kinase inhibitors, including but not limited to imatinib (GLEEVEC), erlotinib (TARCEVA), lapatininb (TYKERB), sorafenib (NEXAVAR), and axitinib (INLYTA);
(j) mTOR inhibitors such as everolimus, temsirolimus (TORISEL), and sirolimus; (12) monoclonal antibodies such as trastuzumab (HERCEPTIN) and rituximab (RITUXAN);
(k) other agents, such as amsacrine; Bacillus Calmette-Guérin (B-C-G) vaccine; buserelin (ETILAMIDE); chloroquine (ARALEN); clodronate, pamidronate, and other bisphosphonates; colchicine; demethoxyviridin; dichloroacetate; estramustine; filgrastim (NEUPOGEN); fludrocortisone (FLORINEF); goserelin (ZOLADEX); interferon; leucovorin; leuprolide (LUPRON); levamisole; lonidamine; mesna; metformin; mitotane (o,p'-DDD, LYSODREN); nocodazole; octreotide (SANDOSTATIN); perifosine; porfimer (particularly in combination with photo- and radiotherapy); suramin; tamoxifen; titanocene dichloride; tretinoin; anabolic steroids such as fluoxymesterone(HALOTESTIN); estrogens such as estradiol, diethylstilbestrol (DES), and dienestrol; progestins such as medroxyprogesterone acetate (MPA) and megestrol; testosterone; 5-fluoro-2-4(1H,3H)-pyrimidinedione and combinations thereof.

The invention further provides pharmaceutical preparations comprising the compounds of the invention. In particular, it is a further object of the invention a pharmaceutical composition comprising the compound or the pharmaceutically acceptable salt, tautomer, solvate, or stereoisomer thereof as defined above, alone or in combination with at least one further therapeutic agent, and at least one pharmaceutically acceptable excipient. Preferably, said at least one further therapeutic agent in the pharmaceutical composition is selected among those indicated above.

In a preferred embodiment, the pharmaceutical combination or the composition of the invention is for use in the treatment and/or prevention of a disease or disorder mediated by the activity of SHP2. Preferably, said disease or disorder mediated by the activity of SHP2 is selected among those indicated above.

The invention also provides pharmaceutical compositions comprising one or more compounds of this invention and a pharmaceutically acceptable carrier. The pharmaceutical compositions containing the active ingredient may be in a form suitable for oral use, for example, as tablets, troches, lozenges, aqueous or oily suspensions, dispersible powders or granules, emulsions, hard or soft capsules, or syrups or elixirs. Compositions of the invention may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, microcrystalline cellulose, sodium crosscarmellose, corn starch, or alginic acid; binding agents, for example starch, gelatin, polyvinyl-pyrrolidone or acacia, and lubricating agents, for example, magnesium stearate, stearic acid or talc. The tablets may be uncoated or they may be coated by known techniques to mask the unpleasant taste of the drug or delay disintegration and absorption in the gastrointestinal tract and thereby provide a sustained action over a longer period. For example, a water-soluble taste masking material such as hydroxypropyl-methylcellulose or hydroxypropylcellulose, or a time delay material such as ethyl cellulose, cellulose acetate butyrate may be employed.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water soluble carrier such as polyethyleneglycol or an oil medium, for example peanut oil, liquid paraffin, or olive oil.

Aqueous suspensions contain the active material in admixture with excipients suitable for the manufacture of aqueous suspensions. Such excipients are suspending agents, for example sodium carboxymethylcellulose, methylcellulose, hydroxypropylmethyl-cellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl p-hydroxybenzoate, one or more coloring agents, one or more flavoring agents, and one or more sweetening agents, such as sucrose, saccharin or aspartame.

Oily suspensions may be formulated by suspending the active ingredient in a vegetable oil, for example arachis oil, olive oil, sesame oil or coconut oil, or in mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti-oxidant such as butylated hydroxyanisol or alpha-tocopherol.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present. These compositions may be preserved by the addition of an anti-oxidant such as ascorbic acid.

The pharmaceutical compositions of the invention may also be in the form of an oil-in-water emulsions. The oily phase may be a vegetable oil, for example olive oil or arachis oil, or a mineral oil, for example liquid paraffin or mixtures of these. Suitable emulsifying agents may be naturally occurring phosphatides, for example soy bean lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening, flavoring agents, preservatives and antioxidants.

Syrups and elixirs may be formulated with sweetening agents, for example glycerol, propylene glycol, sorbitol or sucrose. Such formulations may also contain a demulcent, a preservative, flavoring and coloring agents and antioxidant.

The pharmaceutical compositions may be in the form of a sterile injectable aqueous solutions. Among the acceptable vehicles and solvents that may be employed are water, Ringer's solution and isotonic sodium chloride solution.

The sterile injectable preparation may also be a sterile injectable oil-in-water microemulsion where the active ingredient is dissolved in the oily phase. For example, the active ingredient may be first dissolved in a mixture of soybean oil and lecithin. The oil solution then introduced into a water and glycerol mixture and processed to form a microemulsion.

The injectable solutions or microemulsions may be introduced into a patient's blood stream by local bolus injection. Alternatively, it may be advantageous to administer the solution or microemulsion in such a way as to maintain a constant circulating concentration of the instant compound. In order to maintain such a constant concentration, a continuous intravenous delivery device may be utilized. An example of such a device is the Deltec CADD-PLUS™ model 5400 intravenous pump.

The pharmaceutical compositions may be in the form of a sterile injectable aqueous or oleagenous suspension for intramuscular and subcutaneous administration. This suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent, for example as a solution in 1,3-butanediol. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Compounds of the invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials include cocoa butter, glycerinated gelatin, hydrogenated vegetable oils, mixtures of polyethylene glycols of various molecular weights and fatty acid esters of polyethylene glycol.

For topical use, creams, ointments, jellies, solutions or suspensions, etc., containing the compound(s) of the invention are employed. For purposes of this application, topical application shall include mouth washes and gargles.

The compounds for the present invention can be administered in intranasal form via topical use of suitable intranasal vehicles and delivery devices, or via transdermal routes, using those forms of transdermal skin patches well known to those of ordinary skill in the art. To be administered in the form of a transdermal delivery system, the dosage administration will, of course, be continuous rather than intermittent throughout the dosage regimen. Compounds of the present invention may also be delivered as a suppository employing bases such as cocoa butter, glycerinated gelatin, hydrogenated vegetable oils, mixtures of polyethylene glycols of various molecular weights and fatty acid esters of polyethylene glycol.

The compounds of the invention may be presented in a liposome or other micro particulate or other nanoparticle designed to target the compound. Acceptable liposomes can be neutral, negatively, or positively charged, the charge being a function of the charge of the liposome components and pH of the liposome solution. Liposomes can be normally prepared using a mixture of Phospholipids and cholesterol. Suitable phospholipids include phosphatidylcholine, phosphatidylethanolamine, phosphatidic acid, phosphotidylglycerol, phosphatidylinositol. Polyethylene glycol can be added to improve the blood circulation time of liposomes. Acceptable nanoparticles include albumin nanoparticles and gold nanoparticles.

When a compound according to this invention is administered into a human subject, the daily dosage will normally be determined by the prescribing physician with the dosage generally varying according to the age, weight, sex and response of the individual patient, as well as the severity of the patient's symptoms. Generally, dosage levels on the order of from about 0.01 mg/kg to about 150 mg/kg of body weight are useful in the treatment of the above indicated conditions.

As used herein, the term "composition" is intended to encompass a product comprising the specified ingredients in the specified amounts, as well as any product which results, directly or indirectly, from combination of the specified ingredients in the specified amounts.

Another object of the present invention relates to an *in vitro* method of inhibiting SHP2 with the compound of the present invention. This may be useful, for instance, to evaluate whether any given compound is an inhibitor/activator of SHP2.

A further object of the present invention concerns a kit comprising at least one pharmaceutically acceptable vial or container of other type, containing one or more doses of a compound of the invention, including any pharmaceutically acceptable salt, tautomer, solvate or stereoisomer thereof, or of a pharmaceutical composition of the invention and optionally a) instructions for use thereof in mammals and/or b) an infusion bag or container containing a pharmaceutically acceptable diluent.

In certain embodiments, the compound or the composition of the invention is administered parenterally, intramuscularly, intravenously, subcutaneously, orally, pulmonary, intrathecally, topically, intranasally, or systemically.

In certain embodiments, the patient who is administered the compound or the composition of the invention is a mammal, preferably a primate, more preferably a human.

The compounds of this invention may be administered to mammals, preferably humans, either alone or in combination with pharmaceutically acceptable carriers, excipients or diluents, in a pharmaceutical composition, according to standard pharmaceutical practice. In one embodiment, the compounds of this invention may be administered to animals. The compounds can be administered orally or parenterally, including the intravenous, intramuscular, intraperitoneal, subcutaneous, rectal and topical routes of administration.

As used herein, the term "prevention" means no disorder or disease development if none had occurred, or no further disorder or disease development if there had already been development of the disorder or disease. Also considered is the ability of one to prevent some or all of the symptoms associated with the disorder or disease. As used herein, any reference to "treatment"/"treating" includes the amelioration of at least one symptom of the disease/disorder to be treated. Such amelioration is to be evaluated in comparison to the same symptom prior to administration of the compound or composition of the invention.

The term "therapeutically effective amount" as used herein means that amount of active compound or pharmaceutical agent that elicits the biological or medicinal response in a tissue, system, animal or human that is being sought by a researcher, veterinarian, medical doctor or other clinician. The present invention will be described by means of the following non-limiting examples and biological data.

### MATERIALS AND METHODS

### Chemistry

As used herein, the following abbreviations have the following meanings. If an abbreviation is not defined, it has its generally accepted meaning.

### Abbreviations

AcOH: Acetic acid; (Boc)₂O: Di-*tert*-butyl dicarbonate; CDI: 1,1'-Carbonyldiimidazole; DCM:Dichloromethane; DIPEA: *N,N*-Diisopropylethylamine; DMA: *N,N*-Dimethylacetamide; DMAP:4-(Dimethylamino)pyridine; DMSO: Dimethylsulfoxide; ES⁺: Electrospray Positive Ionisation; EtOAc: Ethyl acetate; EtOH: Ethanol; h: Hour; H₂O: Water; HPLC:High Performance Liquid Chromatography; LCMS: Liquid Chromatography Mass Spectrometry; *m*CPBA: *meta-*Chloroperoxybenzoic acid; MeCN: Acetonitrile; MeI: Iodomethane; MeOH: Methanol; min: Minute; MW: Microwave; Na₂CO₃: Sodium carbonate; NaHCO₃ Sodium bicarbonate; Pd₂(dba)₃: Tris(dibenzylideneacetone)dipalladium(O); POCl₃: Phosphoryl chloride; Pd(PPh₃)₄: Tetrakis(triphenylphosphine)palladium(0); RP: Reverse Phase; RT: Retention time; rt: Room temperature; SEM: trimethylsilylethoxymethyl; TCDI: 1-1'-Thiocarbonyldiimidazole; TEA: Triethylamine; TFA: Trifluoroacetic acid; THF: Tetrahydrofuran; Sat.: Saturated; Sol.: Solution; UPLC: Ultra High Performance Liquid Chromatography; Xantphos: 4,5-Bis(diphenylphosphino)-9,9-dimethylxanthene.

### General

Solvents and reagents were obtained from commercial suppliers and were used without further purification. Flash chromatography purifications were performed on prepacked cartridges on a Biotage system. Purity of final compounds was determined using MS and UPLC. UPLC-MS analyses were performed on a Waters Acquity UPLC™, equipped with a diode array and a ZQ mass spectrometer, using an X-Terra C18 column (5 µm, 4.6 x 50 mm) or a BEH C18 column (1.7 mm, 2.1 x 50 mm). Mobile phase comprised a linear gradient of binary mixtures of H₂O containing 0.1% formic acid (A), and MeCN containing 0.1% formic acid (B). The linear gradient used is: (A): 90% (0.1 min), 90%-0% (2.6 min), 0% (0.3 min), 0%-90% (0.1 min) with a 0.5 mL/min flow. Purity of final compounds was ≥95%. All ¹H spectra were recorded on Bruker AV400 spectrometer at 400 MHz except where indicated. Chemical shift (δ) are reported in parts per million relative to TMS using CDCl₃ as a solvent or relative to the residual solvent signal using DMSO-d₆. Coupling costants (*J*) are reported in Hertz (Hz). Multiplicities are reported as singlet (s), broad (br), doublet (d), doublet of doublet (dd), doublet of doublet of doublet (ddd), triplet (t), doublet of triplet (dt), doublet of doublet of triplet (ddt), triplet of triplet (tt), quartet (q), doublet of quartets (dq) or multiplet (m). Unless indicated, spectra were acquired at 300 K. Temperatures are expressed in degrees Celsius (°C) and are uncorrected.

### Processes for Making the Compounds of the Invention

The present invention also includes processes for the preparation of compounds of the invention. The following schemes are examples of synthetic routes that may be adopted to prepare compounds of the invention.

In the reaction schemes described below, it can be useful to protect reactive functional groups, for example amino, imino, hydroxyl, thio or carboxy groups, to avoid their unwanted participations to reactions. Conventional protecting groups can be used in accordance with standard practice, for example see T. Greene and P.G. M. Wuts in "Protective Groups in Organic Chemistry", John Wiley and Sons, 1991.

Those skilled in the art will readily appreciate that certain compounds of the invention can be converted into other compounds of the invention according to standard chemical methods (e.g. by salification/desalification).

Compounds of Formula (I) can be prepared according to the following Reaction Schemes A-D, wherein all substituents are as defined above.

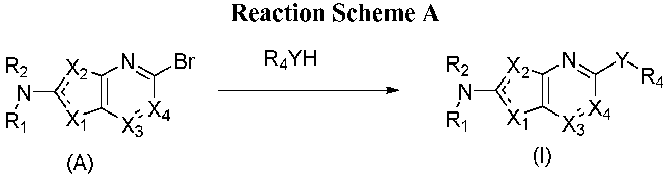

According to Reaction Scheme A, compounds of formula (I) are prepared by reacting the bromine derivative (A) with the appropriate R₄ nucleophilic derivative (including, for example, an aryl or heteroaryl thiol or the corresponding thiolate salt, or an amino derivative, or an hydroxyl-derivative) in the presence of the suitable catalyst (such as a palladium or copper catalyst) and a buffering base. In the absence of a catalyst, the reaction can also be carried out under photochemical conditions.

Compounds of Formula (I), wherein Y is S can also be prepared according to the following Reaction Scheme B.

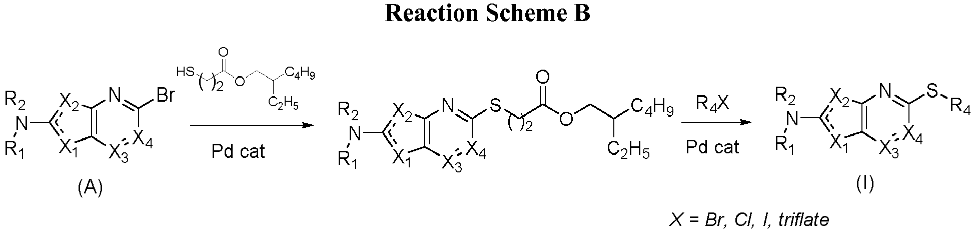

In reaction Scheme B, the bromine on compound (A) is first converted into a thioether derivative in the presence of Palladium catalyst and then further cross-coupled with an aryl derivative of formula R₄X still in the presence of a suitable Pd catalyst and a base.

Compounds of Formula (I) wherein Y is a bond can be prepared according to the Reaction Scheme C, wherein the bromine on compound (A) is coupled with the appropriate R₄-boronic acid (or boronate) derivative in the presence of a suitable palladium catalyst.

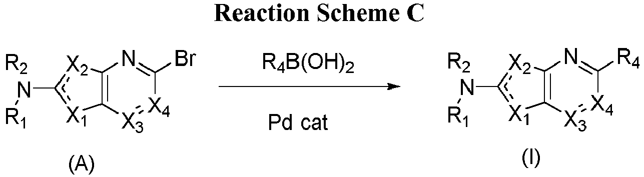

Compounds of Formula (I) can also be prepared according to the Reaction Scheme D:

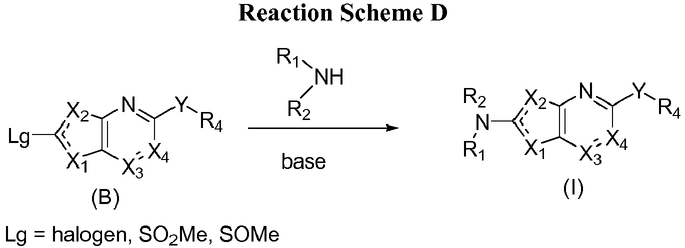

According to Reaction Scheme D, the compound of Formula (I) is obtained by displacing a leaving group, such as halogen, SO₂Me or SOMe, with an amine in the presence of a further suitable base. In the above reaction strategies, when X₁, or X₂ is NH the nitrogen can be protected, for instance as the corresponding trimethylsilylethoxymethyl (SEM) derivative. The reactions above proceed at a temperature ranging from room temperature to about 140 °C.

Compounds of Formula (I) can also be modified by appending the appropriate functionalities to enhance selective biological properties. For example, the synthesis of compounds of Formula (I) may include an alkylation step of a NH group, the methylation or the halogenation of the aromatic or heteroaromatic structure, or the preparation of the N-oxide of a pyridine derivative. Said modifications are included in the detailed examples of synthesis of compounds of Formula (I).

Detailed examples of the synthesis of compounds of Formula (I) and of Formula (II) can be found in the Schemes and in the Examples below.

Unless otherwise indicated, all starting materials reported in this experimental section have been previously described in literature or are commercially available. The indication of the commercial source of certain compounds in the description of the experimental procedure, when provided, is only for easy reference to skilled chemist and should not be interpreted as the indication to use only that particular commercial compound. (S)-1,3-dihydrospiro[indene-2,4'-piperidin]-1-amine, (S)-5,7-dihydrospiro[cyclopenta[b]pyridine-6,4'-piperidin]-5-amine and (R)-3H-spiro[benzofuran-2,4'-piperidin]-3-amine were prepared according to the procedures indicated in WO2018/172984.

The following examples were synthesized using according to the Schemes and the procedures described infra or modifications using the appropriate starting materials (Table 1).

**Table 1 - Compounds prepared according to the Examples and experimental data (MS).**

| **Ex** | **Structure** | **IUPAC Name** | **MS [M+H]⁺** |
|---|---|---|---|
| 1 | 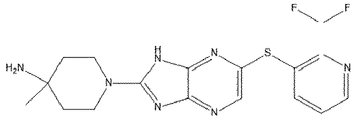 | 4-methyl-1-(6-((2-(trifluoromethyl)pyridin-3-yl)thio)-1H-imidazo[4,5-b]pyrazin-2-yl)piperidin-4-amine | 410 |
| 2 | 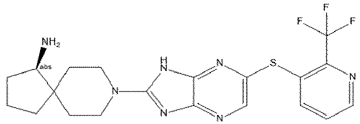 | (R)-8-(6-((2-(trifluoromethyl)pyridin-3-yl)thio)-1H-imidazo[4,5-b]pyrazin-2-yl)-8-azaspiro[4.5]decan-1-amine | 450 |
| 3 | 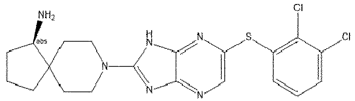 | (R)-8-(6-((2,3-dichlorophenyl)thio)-1H-imidazo[4,5-b]pyrazin-2-yl)-8-azaspiro[4.5]decan-1-amine | 449 |
| 4 | 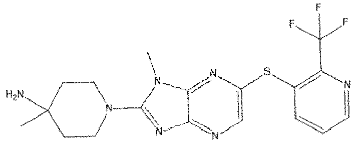 | 4-methyl-1-(1-methyl-6-((2-(trifluoromethyl)pyridin-3-yl)thio)-1H-imidazo[4,5-b]pyrazin-2-yl)piperidin-4-amine | 424 |
| 5 | 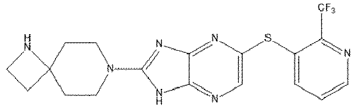 | 2-(1,7-diazaspiro[3.5]nonan-7-yl)-5-((2-(trifluoromethyl)pyridin-3-yl)thio)-1H-imidazo[4,5-b]pyrazine | 422 |
| 6 | 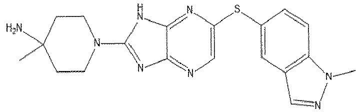 | 4-methyl-1-(6-((1-methyl-1H-indazol-5-yl)thio)-1H-imidazo[4,5-b]pyrazin-2-yl)piperidin-4-amine | 395 |
| 7 | 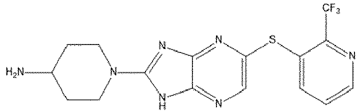 | 1-(5-((2-(trifluoromethyl)pyridin-3-yl)thio)-1H-imidazo[4,5-b]pyrazin-2-yl)piperidin-4-amine | 396 |
| 8 | 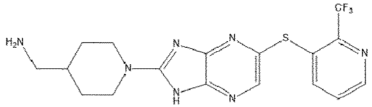 | (1-(5-((2-(trifluoromethyl)pyridin-3-yl)thio)-1H-imidazo[4,5-b]pyrazin-2-yl)piperidin-4-yl)methanamine | 410 |
| 9 | 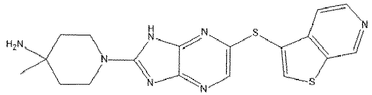 | 4-methyl-1-(6-(thieno[2,3-c]pyridin-3-ylthio)-1H-imidazo[4,5-b]pyrazin-2-yl)piperidin-4-amine | 398 |
| 10 | 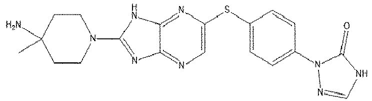 | 2-(4-((2-(4-amino-4-methylpiperidin-1-yl)-1H-imidazo[4,5-b]pyrazin-6-yl)thio)phenyl)-2,4-dihydro-3H-1,2,4-triazol-3-one | 424 |
| 11 | 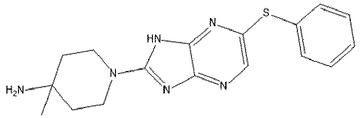 | 4-methyl-1-(6-(phenylthio)-1H-imidazo[4,5-b]pyrazin-2-yl)piperidin-4-amine | 341 |
| 12 | 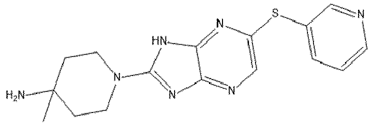 | 4-methyl-1-(6-(pyridin-3-ylthio)-1H-imidazo[4,5-b]pyrazin-2-yl)piperidin-4-amine | 342 |
| 13 | 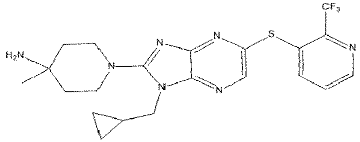 | 1-(1-(cyclopropylmethyl)-5-((2-(trifluoromethyl)pyridin-3-yl)thio)-1H-imidazo[4,5-b]pyrazin-2-yl)-4-methylpiperidin-4-amine | 464 |
| 14 | 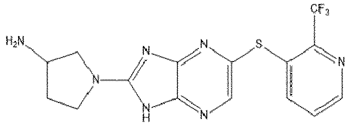 | 1-(5-((2-(trifluoromethyl)pyridin-3-yl)thio)-1H-imidazo[4,5-b]pyrazin-2-yl)pyrrolidin-3-amine | 382 |
| 15 | 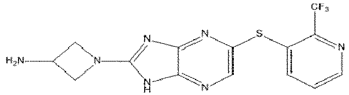 | 1-(5-((2-(trifluoromethyl)pyridin-3-yl)thio)-1H-imidazo[4,5-b]pyrazin-2-yl)azetidin-3-amine | 368 |
| 16 | 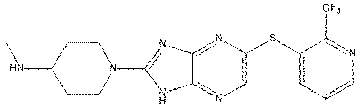 | N-methyl-1-(5-((2-(trifluoromethyl)pyridin-3-yl)thio)-1H-imidazo[4,5-b]pyrazin-2-yl)piperidin-4-amine | 410 |
| 17 | 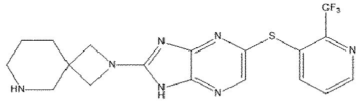 | 2-(2,6-diazaspiro[3.5]nonan-2-yl)-5-((2-(trifluoromethyl)pyridin-3-yl)thio)-1H-imidazo[4,5-b]pyrazine | 422 |
| 18 | 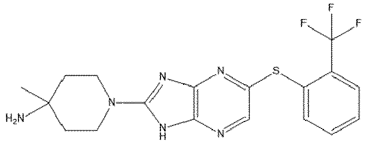 | 4-methyl-1-(5-((2-(trifluoromethyl)phenyl)thio)-1 H-imidazo[4,5-b]pyrazin-2-yl)piperidin-4-amine | 409 |
| 19 | 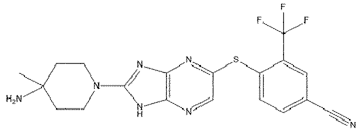 | 4-((2-(4-amino-4-methylpiperidin-1-yl)-1H-imidazo[4,5-b]pyrazin-5-yl)thio)-3-(trifluoromethyl)benzonitrile | 434 |
| 20 | 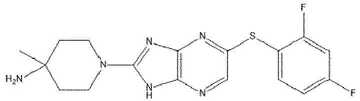 | 1-(5-((2,4-difluorophenyl)thio)-1H-imidazo[4,5-b]pyrazin-2-yl)-4-methylpiperidin-4-amine | 377 |
| 21 | 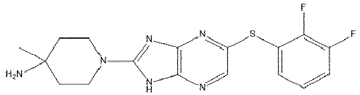 | 1-(5-((2,3-difluorophenyl)thio)-1H-imidazo[4,5-b]pyrazin-2-yl)-4-methylpiperidin-4-amine | 377 |
| 22 | 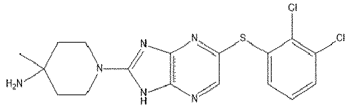 | 1-(5-((2,3-dichlorophenyl)thio)-1H-imidazo[4,5-b]pyrazin-2-yl)-4-methylpiperidin-4-amine | 409 |
| 23 | 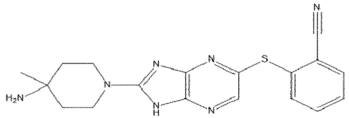 | 2-((2-(4-amino-4-methylpiperidin-1-yl)-1H-imidazo[4,5-b]pyrazin-5-yl)thio)benzonitrile | 366 |
| 24 | 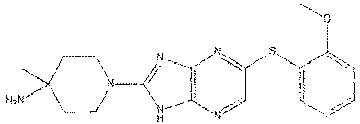 | 1-(5-((2-methoxyphenyl)thio)-1H-imidazo[4,5-b]pyrazin-2-yl)-4-methylpiperidin-4-amine | 371 |
| 25 | 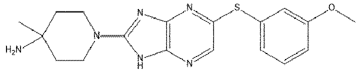 | 1-(5-((3-methoxyphenyl)thio)-1H-imidazo[4,5-b]pyrazin-2-yl)-4-methylpiperidin-4-amine | 371 |
| 26 | 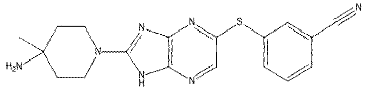 | 3-((2-(4-amino-4-methylpiperidin-1-yl)-1H-imidazo[4,5-b]pyrazin-5-yl)thio)benzonitrile | 366 |
| 27 | 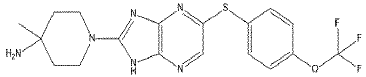 | 4-methyl-1-(5-((4-(trifluoromethoxy)phenyl)thio)-1H-imidazo[4,5-b]pyrazin-2-yl)piperidin-4-amine | 425 |
| 28 | 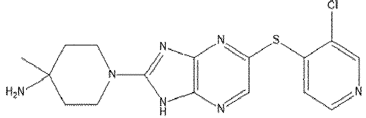 | 1-(5-((3-chloropyridin-4-yl)thio)-1H-imidazo[4,5-b]pyrazin-2-yl)-4-methylpiperidin-4-amine | 376 |
| 29 | 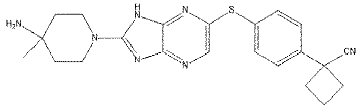 | 1-(4-((2-(4-amino-4-methylpiperidin-1-yl)-1H-imidazo[4,5-b]pyrazin-6-yl)thio)pheny)cyclobutane-1-carbonitrile | 420 |
| 30 | 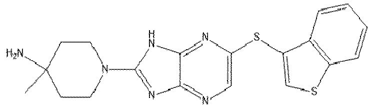 | 1-(6-(benzo[b]thiophen-3-ylthio)-1H-imidazo[4,5-b]pyrazin-2-yl)-4-methylpiperidin-4-amine | 397 |
| 31 | 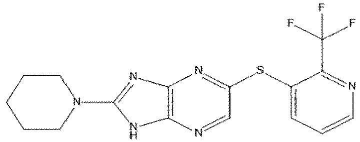 | 2-(piperidin-1-yl)-5-((2-(trifluoromethyl)pyridin-3-yl)thio)-1H-imidazo[4,5-b]pyrazine | 381 |
| 32 | 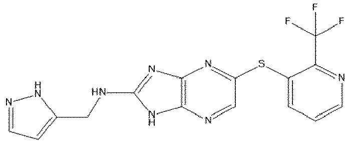 | N-((1H-pyrazol-5-yl)methyl)-5-((2-(trifluoromethyl)pyridin-3-yl)thio)-1H-imidazo[4,5-b]pyrazin-2-amine | 393 |
| 33 | 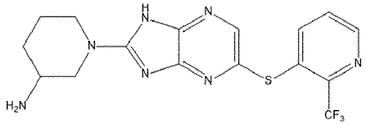 | 1-(5-((2-(trifluoromethyl)pyridin-3-yl)thio)-1H-imidazo[4,5-b]pyrazin-2-yl)piperidin-3-amine | 396 |
| 34 | 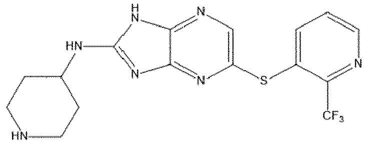 | N-(piperidin-4-yl)-5-((2-(trifluoromethyl)pyridin-3-yl)thio)-1H-imidazo[4,5-b]pyrazin-2-amine | 396 |
| 35 | 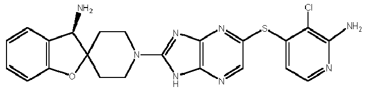 | (R)-1'-(5-((2-amino-3-chloropyridin-4-yl)thio)-1H-imidazo[4,5-b]pyrazin-2-yl)-3H-spiro[benzofuran-2,4'-piperidin]-3-amine | 481 |
| 36 | 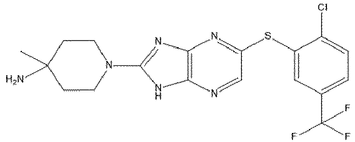 | 1-(5-((2-chloro-5-(trifluoromethyl)phenyl)thio)-1H-imidazo[4,5-b]pyrazin-2-yl)-4-methylpiperidin-4-amine | 443 |
| 37 | 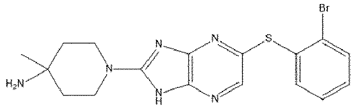 | 1-(5-((2-bromophenyl)thio)-1H-imidazo[4,5-b]pyrazin-2-yl)-4-methylpiperidin-4-amine | 419 |
| 38 | 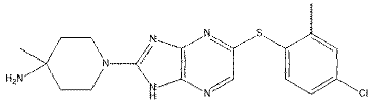 | 1-(5-((4-chloro-2-methylphenyl)thio)-1H-imidazo[4,5-b]pyrazin-2-yl)-4-methylpiperidin-4-amine | 389 |
| 39 | 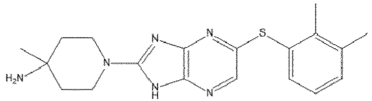 | 1-(5-((2,3-dimethylphenyl)thio)-1H-imidazo[4,5-b]pyrazin-2-yl)-4-methylpiperidin-4-amine | 369 |
| 40 | 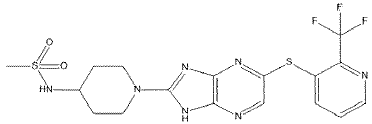 | N-(1-(5-((2-(trifluoromethyl)pyridin-3-yl)thio)-1H-imidazo[4,5-b]pyrazin-2-yl)piperidin-4-yl)methanesulfonamide | 474 |
| 41 | 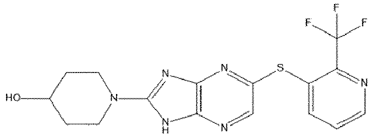 | 1-(5-((2-(trifluoromethyl)pyridin-3-yl)thio)-1H-imidazo[4,5-b]pyrazin-2-yl)piperidin-4-ol | 397 |
| 42 | 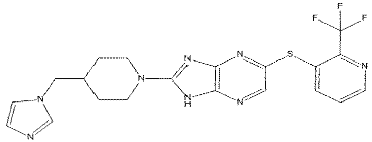 | 2-(4-((1H-imidazol-1-yl)methyl)piperidin-1-yl)-5-((2-(trifluoromethyl)pyridin-3-yl)thio)-1 H-imidazo [4,5-b]pyrazine | 461 |
| 43 | 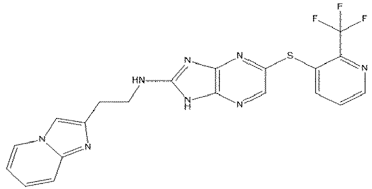 | N-(2-(imidazo[1,2-a]pyridin-2-yl)ethyl)-5-((2-(trifluoromethyl)pyridin-3-yl)thio)-1H-imidazo[4,5-b]pyrazin-2-amine | 457 |
| 44 | 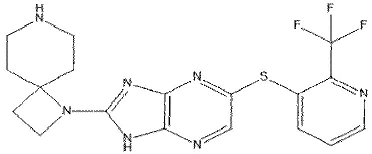 | 2-(1,7-diazaspiro[3.5]nonan-1-yl)-5-((2-(trifluoromethyl)pyridin-3-yl)thio)-1H-imidazo[4,5-b]pyrazine | 422 |
| 45 | 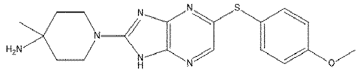 | 1-(5-((4-methoxyphenyl)thio)-1H-imidazo[4,5-b]pyrazin-2-yl)-4-methylpiperidin-4-amine | 371 |
| 46 | 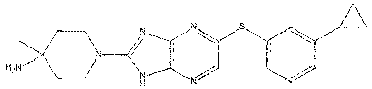 | 1-(5-((3-cyclopropylphenyl)thio)-1H-imidazo[4,5-b]pyrazin-2-yl)-4-methylpiperidin-4-amine | 381 |
| 47 | 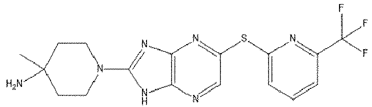 | 4-methyl-1-(5-((6-(trifluoromethyl)pyridin-2-yl)thio)-1H-imidazo[4,5-b]pyrazin-2-yl)piperidin-4-amine | 410 |
| 48 | 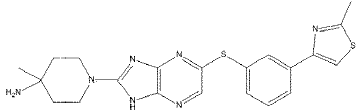 | 4-methyl-1-(5-((3-(2-methylthiazol-4-yl)phenyl)thio)-1H-imidazo[4,5-b]pyrazin-2-yl)piperidin-4-amine | 438 |
| 49 | 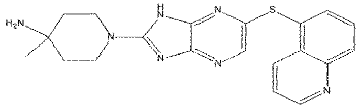 | 4-methyl-1-(6-(quinolin-5-ylthio)-1H-imidazo[4,5-b]pyrazin-2-yl)piperidin-4-amine | 392 |
| 50 | 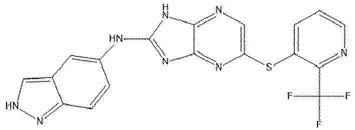 | N-(2H-indazol-5-yl)-5-((2-(trifluoromethyl)pyridin-3-yl)thio)-1H-imidazo[4,5-b]pyrazin-2-amine | 429 |
| 51 | 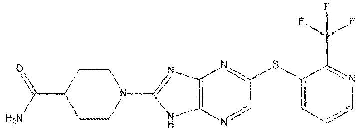 | 1-(5-((2-(trifluoromethyl)pyridin-3-yl)thio)-1H-imidazo[4,5-b]pyrazin-2-yl)piperidine-4-carboxamide | 424 |
| 52 | 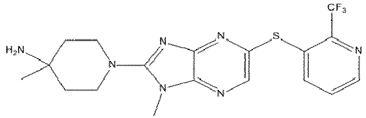 | 4-methyl-1-(1-methyl-5-((2-(trifluoromethyl)pyridin-3-yl)thio)-1H-imidazo[4,5-b]pyrazin-2-yl)piperidin-4-amine | 424 |
| 53 | 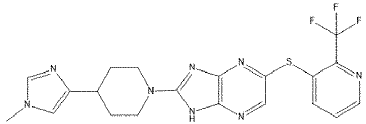 | 2-(4-(1-methyl-1 H-imidazol-4-yl)piperidin-1-yl)-5-((2-(trifluoromethyl)pyridin-3-yl)thio)-1 H-imidazo [4,5-b]pyrazine | 461 |
| 54 | 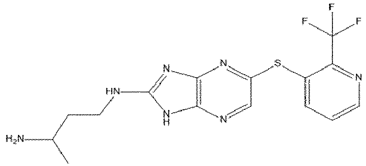 | N1-(5-((2-(trifluoromethyl)pyridin-3-yl)thio)-1H-imidazo[4,5-b]pyrazin-2-yl)butane-1,3-diamine | 384 |
| 55 | 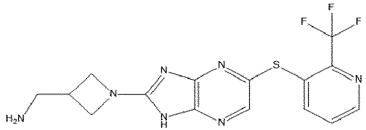 | (1-(5-((2-(trifluoromethyl)pyridin-3-yl)thio)-1H-imidazo[4,5-b]pyrazin-2-yl)azetidin-3-yl)methanamine | 382 |
| 56 | 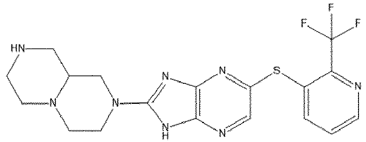 | 2-(5-((2-(trifluoromethyl)pyridin-3-yl)thio)-1H-imidazo[4,5-b]pyrazin-2-yl)octahydro-2H-pyrazino[1,2-a]pyrazine | 437 |
| 57 | 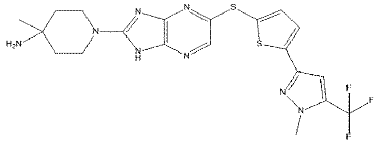 | 4-methyl-1-(5-((5-(1-methyl-5-(trifluoromethyl)-1H-pyrazol-3-yl)thiophen-2-yl)thio)-1H-imidazo[4,5-b]pyrazin-2-yl)piperidin-4-amine | 495 |
| 58 | 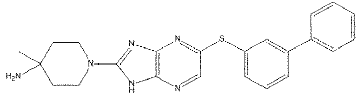 | 1-(5-([1,1'-biphenyl]-3-ylthio)-1H-imidazo[4,5-b]pyrazin-2-yl)-4-methylpiperidin-4-amine | 417 |
| 59 | 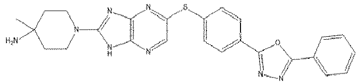 | 4-methyl-1-(5-((4-(5-phenyl-1,3,4-oxadiazol-2-yl)phenyl)thio)-1H-imidazo[4,5-b]pyrazin-2-yl)piperidin-4-amine | 485 |
| 60 | 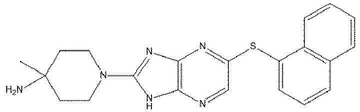 | 4-methyl-1-(5-(naphthalen-1-ylthio)-1H-imidazo[4,5-b]pyrazin-2-yl)piperidin-4-amine | 391 |
| 61 | 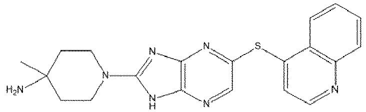 | 4-methyl-1-(5-(quinolin-4-ylthio)-1H-imidazo[4,5-b]pyrazin-2-yl)piperidin-4-amine | 392 |
| 62 | 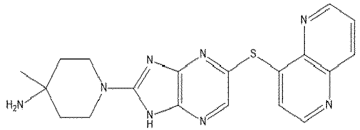 | 1-(5-((1,5-naphthyridin-4-yl)thio)-1H-imidazo[4,5-b]pyrazin-2-yl)-4-methylpiperidin-4-amine | 393 |
| 63 | 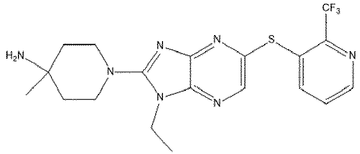 | 1-(1-ethyl-5-((2-(trifluoromethyl)pyridin-3-yl)thio)-1H-imidazo[4,5-b]pyrazin-2-yl)-4-methylpiperidin-4-amine | 438 |
| 64 | 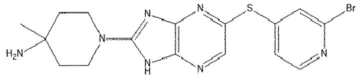 | 1-(5-((2-bromopyridin-4-yl)thio)-1H-imidazo[4,5-b]pyrazin-2-yl)-4-methylpiperidin-4-amine | 420 |
| 65 | 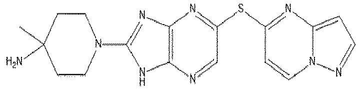 | 4-methyl-1-(5-(pyrazolo[1,5-a]pyrimidin-5-ylthio)-1H-imidazo[4,5-b]pyrazin-2-yl)piperidin-4-amine | 382 |
| 66 | 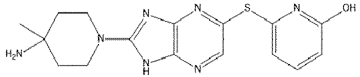 | 6-((2-(4-amino-4-methylpiperidin-1-yl)-1H-imidazo[4,5-b]pyrazin-5-yl)thio)pyridin-2-ol | 358 |
| 67 | 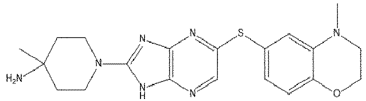 | 4-methyl-1-(5-((4-methyl-3,4-dihydro-2H-benzo[b][1,4]oxazin-6-yl)thio)-1H-imidazo [4,5-b]pyrazin-2-yl)piperidin-4-amine | 412 |
| 68 | 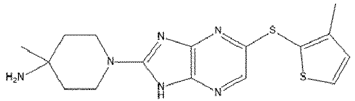 | 4-methyl-1-(5-((3-methylthiophen-2-yl)thio)-1H-imidazo[4,5-b]pyrazin-2-yl)piperidin-4-amine | 361 |
| 69 | 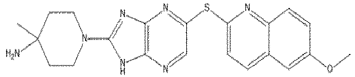 | 1-(5-((6-methoxyquinolin-2-yl)thio)-1H-imidazo[4,5-b]pyrazin-2-yl)-4-methylpiperidin-4-amine | 422 |
| 70 | 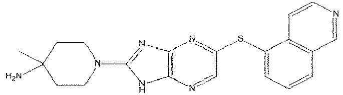 | 1-(5-(isoquinolin-5-ylthio)-1H-imidazo[4,5-b]pyrazin-2-yl)-4-methylpiperidin-4-amine | 392 |
| 71 | 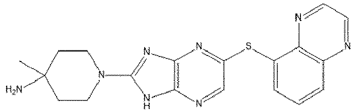 | 4-methyl-1-(5-(quinoxalin-5-ylthio)-1H-imidazo[4,5-b]pyrazin-2-yl)piperidin-4-amine | 393 |
| 72 | 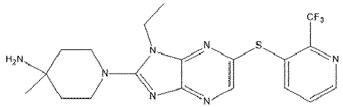 | 1-(1-ethyl-6-((2-(trifluoromethyl)pyridin-3-yl)thio)-1H-imidazo[4,5-b]pyrazin-2-yl)-4-methylpiperidin-4-amine | 438 |
| 73 | 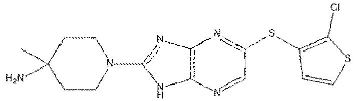 | 1-(5-((2-chlorothiophen-3-yl)thio)-1H-imidazo[4,5-b]pyrazin-2-yl)-4-methylpiperidin-4-amine | 381 |
| 74 | 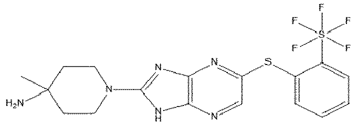 | 4-methyl-1-(5-((2-(pentafluoro-16-sulfaneyl)phenyl)thio)-1H-imidazo[4,5-b]pyrazin-2-yl)piperidin-4-amine | 467 |
| 75 | 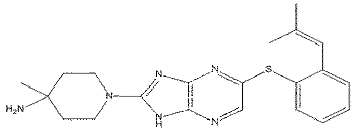 | 4-methyl-1-(5-((2-(2-methylprop-1-en-1-yl)phenyl)thio)-1H-imidazo[4,5-b]pyrazin-2-yl)piperidin-4-amine | 395 |
| 76 | 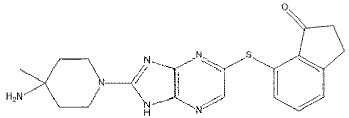 | 7-((2-(4-amino-4-methylpiperidin-1-yl)-1H-imidazo[4,5-b]pyrazin-5-yl)thio)-2,3-dihydro-1H-inden-1-one | 395 |
| 77 | 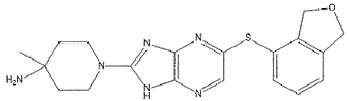 | 1-(5-((1,3-dihydroisobenzofuran-4-yl)thio)-1H-imidazo[4,5-b]pyrazin-2-yl)-4-methylpiperidin-4-amine | 383 |
| 78 | 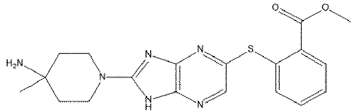 | methyl 2-((2-(4-amino-4-methylpiperidin-1-yl)-1H-imidazo[4,5-b]pyrazin-5-yl)thio)benzoate | 399 |
| 79 | 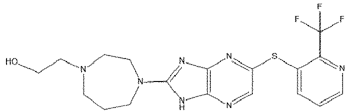 | 2-(4-(5-((2-(trifluoromethyl)pyridin-3-yl)thio)-1H-imidazo[4,5-b]pyrazin-2-yl)-1,4-diazepan-1-yl)ethan-1-ol | 440 |
| 80 | 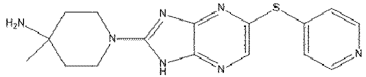 | 4-methyl-1-(5-(pyridin-4-ylthio)-1H-imidazo[4,5-b]pyrazin-2-yl)piperidin-4-amine | 342 |
| 81 | 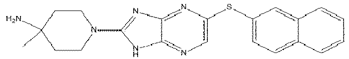 | 4-methyl-1-(5-(naphthalen-2-ylthio)-1H-imidazo[4,5-b]pyrazin-2-yl)piperidin-4-amine | 391 |
| 82 | 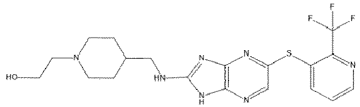 | 2-(4-(((5-((2-(trifluoromethyl)pyridin-3-yl)thio)-1H-imidazo[4,5-b]pyrazin-2-yl)amino)methyl)piperidin-1-yl)ethan-1-ol | 454 |
| 83 | 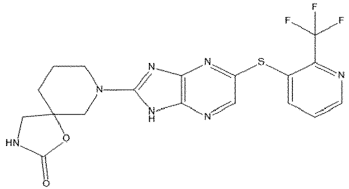 | 7-(5-((2-(trifluoromethyl)pyridin-3-yl)thio)-1H-imidazo[4,5-b]pyrazin-2-yl)-1-oxa-3,7-diazaspiro[4.5]decan-2-one | 452 |
| 84 | 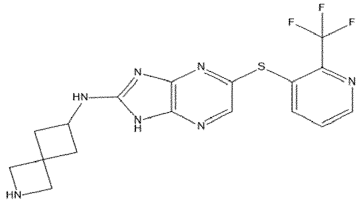 | N-(2-azaspiro[3.3]heptan-6-yl)-5-((2-(trifluoromethyl)pyridin-3-yl)thio)-1H-imidazo[4,5-b]pyrazin-2-amine | 408 |
| 85 | 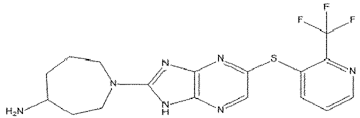 | 1-(5-((2-(trifluoromethyl)pyridin-3-yl)thio)-1H-imidazo[4,5-b]pyrazin-2-yl)azepan-4-amine | 410 |
| 86 | 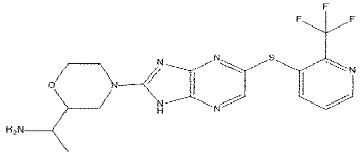 | 1-(4-(5-((2-(trifluoromethyl)pyridin-3-yl)thio)-1H-imidazo[4,5-b]pyrazin-2-yl)morpholin-2-yl)ethan-1-amine | 426 |
| 87 | 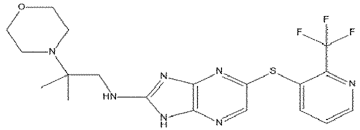 | N-(2-methyl-2-morpholinopropyl)-5-((2-(trifluoromethyl)pyridin-3-yl)thio)-1H-imidazo[4,5-b]pyrazin-2-amine | 454 |
| 88 | 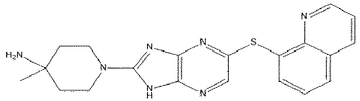 | 4-methyl-1-(5-(quinolin-8-ylthio)-1H-imidazo[4,5-b]pyrazin-2-yl)piperidin-4-amine | 392 |
| 89 | 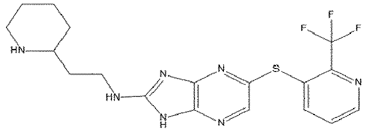 | N-(2-(piperidin-2-yl)ethyl)-5-((2-(trifluoromethyl)pyridin-3-yl)thio)-1H-imidazo[4,5-b]pyrazin-2-amine | 424 |
| 90 | 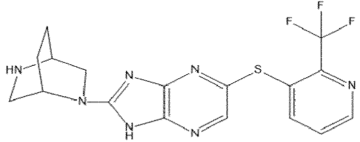 | (1S,4S)-2-(5-((2-(trifluoromethyl)pyridin-3-yl)thio)-1H-imidazo[4,5-b]pyrazin-2-yl)-2,5-diazabicyclo[2.2.2]octane | 408 |
| 91 | 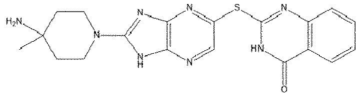 | 2-((2-(4-amino-4-methylpiperidin-1-yl)-1H-imidazo[4,5-b]pyrazin-5-yl)thio)quinazolin-4(3H)-one | 409 |
| 92 | 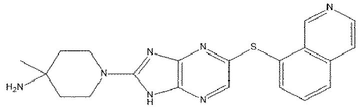 | 1-(5-(isoquinolin-8-ylthio)-1H-imidazo[4,5-b]pyrazin-2-yl)-4-methylpiperidin-4-amine | 392 |
| 93 | 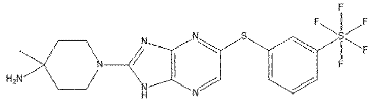 | 4-methyl-1-(5-((3-(pentafluoro-16-sulfaneyl)phenyl)thio)-1H-imidazo[4,5-b]pyrazin-2-yl)piperidin-4-amine | 467 |
| 94 | 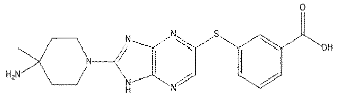 | 3-((2-(4-amino-4-methylpiperidin-1-yl)-1H-imidazo[4,5-b]pyrazin-5-yl)thio)benzoic acid | 385 |
| 95 | 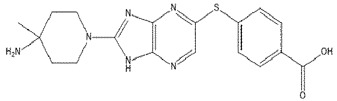 | 4-((2-(4-amino-4-methylpiperidin-1-yl)-1H-imidazo[4,5-b]pyrazin-5-yl)thio)benzoic acid | 385 |
| 96 | 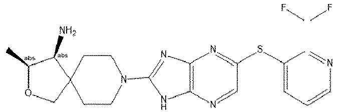 | (3S,4S)-3-methyl-8-(5-((2-(trifluoromethyl)pyridin-3-yl)thio)-1H-imidazo[4,5-b]pyrazin-2-yl)-2-oxa-8-azaspiro[4.5]decan-4-amine | 466 |
| 97 | 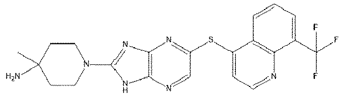 | 4-methyl-1-(5-((8-(trifluoromethyl)quinolin-4-yl)thio)-1H-imidazo[4,5-b]pyrazin-2-yl)piperidin-4-amine | 460 |
| 98 | 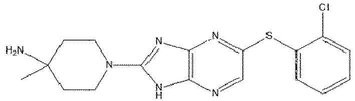 | 1-(5-((2-chlorophenyl)thio)-1H-imidazo[4,5-b]pyrazin-2-yl)-4-methylpiperidin-4-amine | 375 |
| 99 | 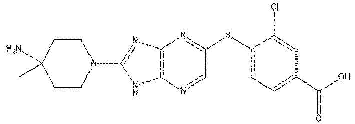 | 4-((2-(4-amino-4-methylpiperidin-1-yl)-1H-imidazo[4,5-b]pyrazin-5-yl)thio)-3-chlorobenzoic acid | 419 |
| 100 | 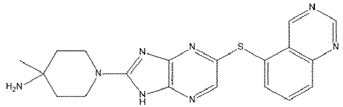 | 4-methyl-1-(5-(quinazolin-5-ylthio)-1H-imidazo[4,5-b]pyrazin-2-yl)piperidin-4-amine | 393 |
| 101 | 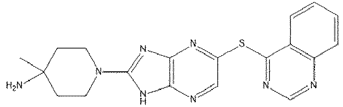 | 4-methyl-1-(5-(quinazolin-4-ylthio)-1H-imidazo[4,5-b]pyrazin-2-yl)piperidin-4-amine | 393 |
| 102 | 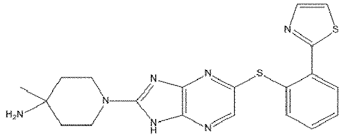 | 4-methyl-1-(5-((2-(thiazol-2-yl)phenyl)thio)-1H-imidazo[4,5-b]pyrazin-2-yl)piperidin-4-amine | 424 |
| 103 | 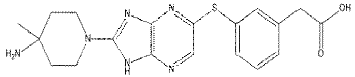 | 2-(3-((2-(4-amino-4-methylpiperidin-1-yl)-1H-imidazo[4,5-b]pyrazin-5-yl)thio)phenyl)acetic acid | 399 |
| 104 | 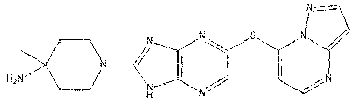 | 4-methyl-1-(5-(pyrazolo[1,5-a]pyrimidin-7-ylthio)-1H-imidazo[4,5-b]pyrazin-2-yl)piperidin-4-amine | 382 |
| 105 | 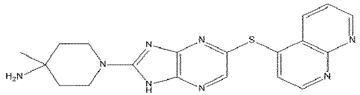 | 1-(5-((1,8-naphthyridin-4-yl)thio)-1H-imidazo[4,5-b]pyrazin-2-yl)-4-methylpiperidin-4-amine | 393 |
| 106 | 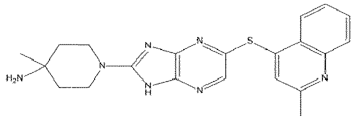 | 4-methyl-1-(5-((2-methylquinolin-4-yl)thio)-1H-imidazo[4,5-b]pyrazin-2-yl)piperidin-4-amine | 406 |
| 107 | 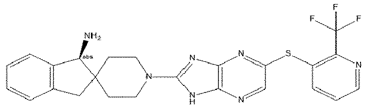 | (S)-1'-(5-((2-(trifluoromethyl)pyridin-3-yl)thio)-1H-imidazo[4,5-b]pyrazin-2-yl)-1,3-dihydrospiro[indene-2,4'-piperidin]-1-amine | 498 |
| 108 | 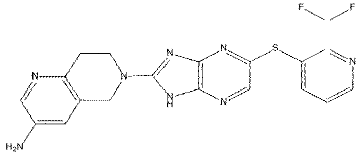 | 6-(5-((2-(trifluoromethyl)pyridin-3-yl)thio)-1H-imidazo[4,5-b]pyrazin-2-yl)-5,6,7,8-tetrahydro-1,6-naphthyridin-3 -amine | 445 |
| 109 | 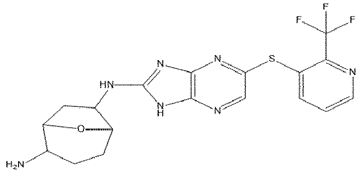 | N6-(5-((2-(trifluoromethyl)pyridin-3-yl)thio)-1H-imidazo[4,5-b]pyrazin-2-yl)-8-oxabicyclo[3.2.1]octane-2,6-diamine | 438 |
| 110 | 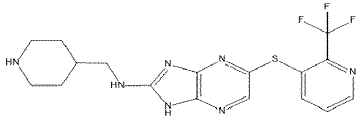 | N-(piperidin-4-ylmethyl)-5-((2-(trifluoromethyl)pyridin-3-yl)thio)-1H-imidazo[4,5-b]pyrazin-2-amine | 410 |
| 111 | 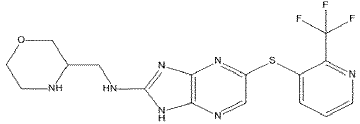 | N-(morpholin-3-ylmethyl)-5-((2-(trifluoromethyl)pyridin-3-yl)thio)-1H-imidazo[4,5-b]pyrazin-2-amine | 412 |
| 112 | 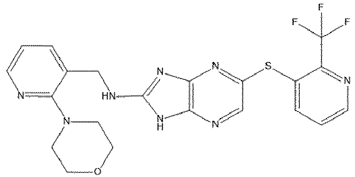 | N-((2-morpholinopyridin-3-yl)methyl)-5-((2-(trifluoromethyl)pyridin-3-yl)thio)-1 H-imidazo [4,5-b]pyrazin-2-amine | 489 |
| 113 | 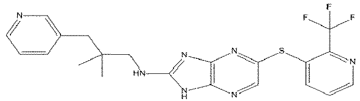 | N-(2,2-dimethyl-3-(pyridin-3-yl)propyl)-5-((2-(trifluoromethyl)pyridin-3-yl)thio)-1 H-imidazo [4,5-b]pyrazin-2-amine | 460 |
| 114 | 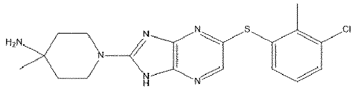 | 1-(5-((3-chloro-2-methylphenyl)thio)-1H-imidazo[4,5-b]pyrazin-2-yl)-4-methylpiperidin-4-amine | 389 |
| 115 | 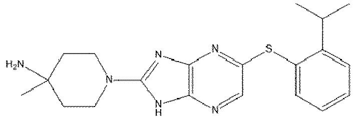 | 1-(5-((2-isopropylphenyl)thio)-1H-imidazo[4,5-b]pyrazin-2-yl)-4-methylpiperidin-4-amine | 383 |
| 116 | 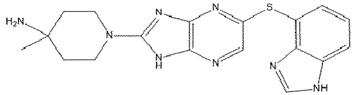 | 1-(5-((1H-benzo[d]imidazol-4-yl)thio)-1H-imidazo[4,5-b]pyrazin-2-yl)-4-methylpiperidin-4-amine | 381 |
| 117 | 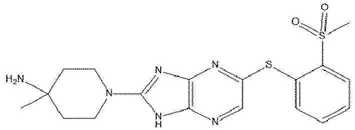 | 4-methyl-1-(5-((2-(methylsulfonyl)phenyl)thio)-1H-imidazo[4,5-b]pyrazin-2-yl)piperidin-4-amine | 419 |
| 118 | 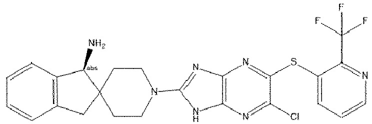 | (S)-1'-(6-chloro-5-((2-(trifluoromethyl)pyridin-3-yl)thio)-1H-imidazo[4,5-b]pyrazin-2-yl)-1,3-dihydrospiro[indene-2,4'-piperidin]-1-amine | 532 |
| 119 | 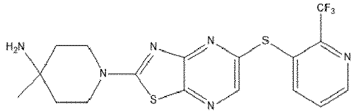 | 4-methyl-1-(5-((2-(trifluoromethyl)pyridin-3-yl)thio)thiazolo[4,5-b]pyrazin-2-yl)piperidin-4-amine | 427 |
| 120 | 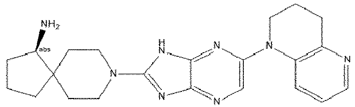 | (R)-8-(6-(3,4-dihydro-1,5-naphthyridin-1(2H)-yl)-1H-imidazo[4,5-b]pyrazin-2-yl)-8-azaspiro[4.5]decan-1-amine | 405 |
| 121 | 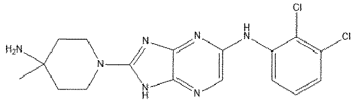 | 2-(4-amino-4-methylpiperidin-1-yl)-N-(2,3-dichlorophenyl)-1H-imidazo[4,5-b]pyrazin-5-amine | 392 |
| 122 | 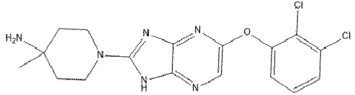 | 1-(5-(2,3-dichlorophenoxy)-1H-imidazo[4,5-b]pyrazin-2-yl)-4-methylpiperidin-4-amine | 393 |
| 123 | 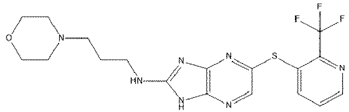 | N-(3-morpholinopropyl)-5-((2-(trifluoromethyl)pyridin-3-yl)thio)-1H-imidazo[4,5-b]pyrazin-2-amine | 440 |
| 124 | 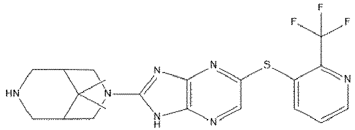 | 2-(9,9-dimethyl-3,7-diazabicyclo[3.3.1]nonan-3-yl)-5-((2-(trifluoromethyl)pyridin-3-yl)thio)-1H-imidazo [4,5-b]pyrazine | 450 |
| 125 | 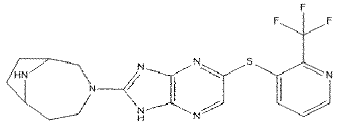 | 2-(3,9-diazabicyclo[4.2.1]nonan-3-yl)-5-((2-(trifluoromethyl)pyridin-3-yl)thio)-1H-imidazo[4,5-b]pyrazine | 422 |
| 126 | 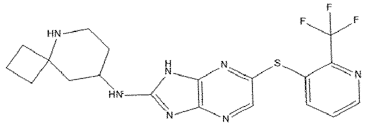 | N-(5-azaspiro[3.5]nonan-8-yl)-6-((2-(trifluoromethyl)pyridin-3-yl)thio)-1H-imidazo[4,5-b]pyrazin-2-amine | 436 |
| 127 | 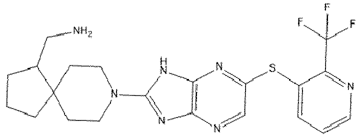 | (8-(6-((2-(trifluoromethyl)pyridin-3-yl)thio)-1H-imidazo[4,5-b]pyrazin-2-yl)-8-azaspiro[4.5]decan-1-yl)methanamine | 464 |
| 128 | 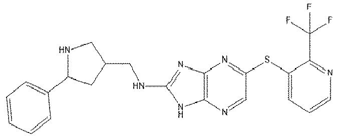 | N-((5-phenylpyrrolidin-3-yl)methyl)-5-((2-(trifluoromethyl)pyridin-3-yl)thio)-1H-imidazo[4,5-b]pyrazin-2-amine | 472 |
| 129 | 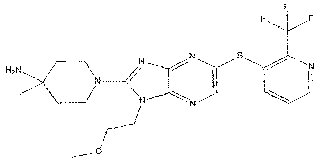 | 1-(1-(2-methoxyethyl)-5-((2-(trifluoromethyl)pyridin-3-yl)thio)-1H-imidazo[4,5-b]pyrazin-2-yl)-4-methylpiperidin-4-amine | 468 |
| 130 | 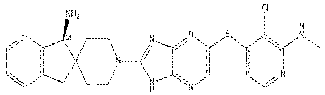 | (S)-1'-(6-((3-chloro-2-(methylamino)pyridin-4-yl)thio )-1H-imidazo[4,5-b]pyrazin-2-yl)-1,3-dihydrospiro[indene-2,4'-piperidin]-1-amine | 493 |
| 131 | 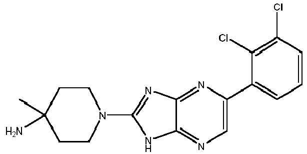 | 1-[5-(2,3-Dichlorophenyl)-1H-imidazo[4,5-b]pyrazin-2-yl]-4-methylpiperidin-4-amine | 377 |
| 132 | 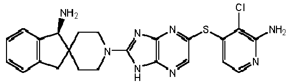 | (S)-1'-(5-((2-amino-3-chloropyridin-4-yl)thio)-1H-imidazo[4,5-b]pyrazin-2-yl)-1,3-dihydrospiro[indene-2,4'-piperidin]-1-amine | 479 |
| 133 | 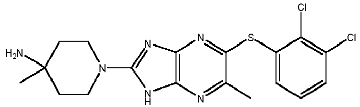 | 1-(5-((2,3-dichlorophenyl)thio)-6-methyl-1H-imidazo[4,5-b]pyrazin-2-yl)-4-methylpiperidin-4-amine | 423 |
| 134 | 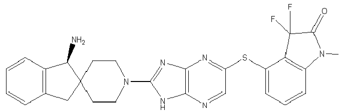 | (S)-4-((2-(1-amino-1,3-dihydrospiro[indene-2,4'-piperidin]-1'-yl)-1H-imidazo[4,5-b]pyrazin-5-yl)thio)-3,3-difluoro-1-methylindolin-2-one | 534 |
| 135 | 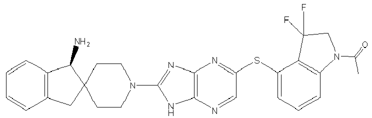 | (S)-1-(4-((2-(1-amino-1,3-dihydrospiro[indene-2,4'-piperidin]-1'-yl)-1H-imidazo[4,5-b]pyrazin-5-yl)thio)-3,3-difluoroindolin-1-yl)ethan-1-one | 548 |
| 136 | 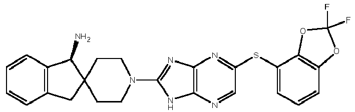 | (S)-1'-(5-((2,2-difluorobenzo[d][1,3]dioxol-4-yl)thio)-1H-imidazo[4,5-b]pyrazin-2-yl)-1,3-dihydrospiro[indene-2,4'-piperidin]-1-amine | 509 |
| 137 | 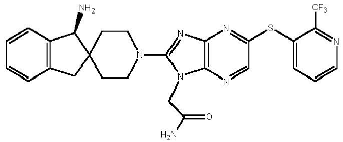 | (S)-2-(2-(1-amino-1,3-dihydrospiro[indene-2,4'-piperidin]-1'-yl)-5-((2-(trifluoromethyl)pyridin-3-yl)thio)-1H-imidazo[4,5-b]pyrazin-1-yl)acetamide | 555 |
| 138 | 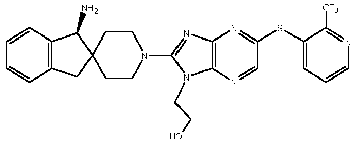 | (S)-2-(2-(1-amino-1,3-dihydrospiro[indene-2,4'-piperidin]-1'-yl)-5-((2-(trifluoromethyl)pyridin-3-yl)thio)-1H-imidazo[4,5-b]pyrazin-1-yl)ethan-1-ol | 454 |
| 139 | 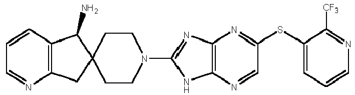 | (S)-1'-(5-((2-(trifluoromethyl)pyridin-3-yl)thio)-1H-imidazo[4,5-b]pyrazin-2-yl)-5,7-dihydrospiro[cyclopenta[b]pyridine-6,4'-piperidin]-5-amine | 499 |
| 140 | 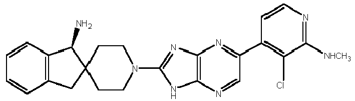 | (S)-1'-(5-(3-chloro-2-(methylamino )pyridin-4-yl)-1H-imidazo[4,5-b]pyrazin-2-yl)-1,3-dihydrospiro[indene-2,4'-piperidin]-1-amine | 461 |
| 141 | 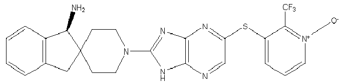 | (S)-3-((2-(1-amino-1,3-dihydrospiro[indene-2,4'-piperidin]-1'-yl)-1H-imidazo[4,5-b]pyrazin-5-yl)thio)-2-(trifluoromethyl)pyridine 1-oxide | 514 |
| 142 | 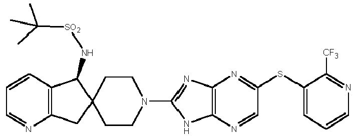 | (S)-2-methyl-N-(1'-(5-((2-(trifluoromethyl)pyridin-3-yl)thio)-1H-imidazo[4,5-b]pyrazin-2-yl)-5,7-dihydrospiro[cyclopenta[b]pyridine-6,4'-piperidin]-5-yl)propane-2-sulfonamide | 619 |
| 143 | 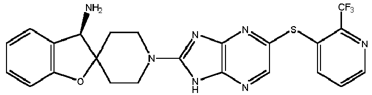 | (R)-1'-(5-((2-(trifluoromethyl)pyridin-3-yl)thio)-1H-imidazo[4,5-b]pyrazin-2-yl)-3H-spiro[benzofuran-2,4'-piperidin]-3-amine | 500 |
| 144 | 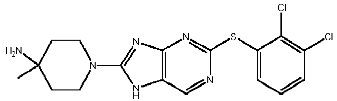 | 1-(2-((2,3-dichlorophenyl)thio)-7H-purin-8-yl)-4-methylpiperidin-4-amine | 409 |
| 145 | 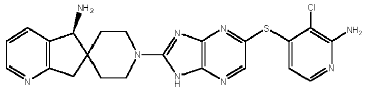 | (S)-1'-(5-((2-amino-3-chloropyridin-4-yl)thio)-1H-imidazo[4,5-b]pyrazin-2-yl)-5,7-dihydrospiro[cyclopenta[b]pyridine-6,4'-piperidin]-5-amine | 480 |
| 146 | 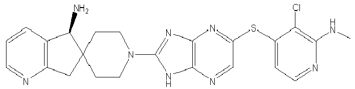 | (S)-1'-(5-((3-chloro-2-(methylamino)pyridin-4-yl)thio)-1H-imidazo[4,5-b]pyrazin-2-yl)-5,7-dihydrospiro[cyclopenta[b]pyridine-6,4'-piperidin]-5-amine | 494 |
| 147 | 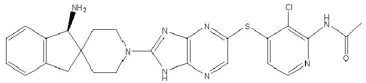 | (S)-N-(4-((2-(1-amino-1,3-dihydrospiro[indene-2,4'-piperidin]-1'-yl)-1H-imidazo[4,5-b]pyrazin-5-yl)thio)-3-chloropyridin-2-yl)acetamide | 521 |
| 148 | 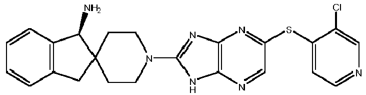 | (S)-1'-(5-((3-chloropyridin-4-yl)thio)-1H-imidazo[4,5-b]pyrazin-2-yl)-1,3-dihydrospiro[indene-2,4'-piperidin]-1-amine | 464 |
| 149 | 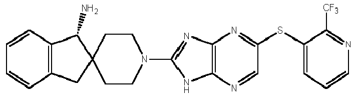 | (R)-1'-(5-((2-(trifluoromethyl)pyridin-3-yl)thio)-1H-imidazo[4,5-b]pyrazin-2-yl)-1,3-dihydrospiro[indene-2,4'-piperidin]-1-amine | 498 |
| 150 | 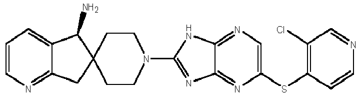 | (S)-1'-(5-((3-chloropyridin-4-yl)thio)-1H-imidazo[4,5-b]pyrazin-2-yl)-5,7-dihydrospiro[cyclopenta[b]pyridine-6,4'-piperidin]-5-amine | 465 |
| 151 | 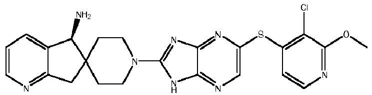 | (S)-1'-(5-((3-chloro-2-methoxypyridin-4-yl)thio)-1H-imidazo[4,5-b]pyrazin-2-yl)-5,7-dihydrospiro[cyclopenta[b]pyridine-6,4'-piperidin]-5-amine | 495 |
| 152 | 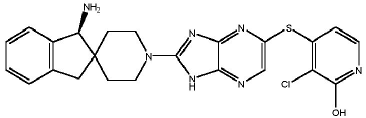 | (S)-4-((2-(1-amino-1,3-dihydrospiro[indene-2,4'-piperidin]-1'-yl)-1H-imidazo[4,5-b]pyrazin-5-yl)thio)-3-chloropyridin-2-ol | 480 |
| 153 | 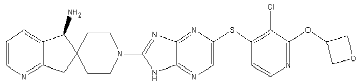 | (S)-1'-(5-((3-chloro-2-(oxetan-3-yloxy)pyridin-4-yl)thio)-1H-imidazo[4,5-b]pyrazin-2-yl)-5,7-dihydrospiro[cyclopenta[b]pyridine-6,4'-piperidin]-5-amine | 537 |
| 154 | 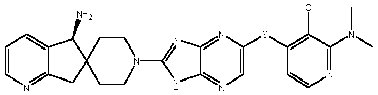 | (S)-1'-(5-((3-chloro-2-(dimethylamino)pyridin-4-yl)thio)-1H-imidazo[4,5-b]pyrazin-2-yl)-5,7-dihydrospiro[cyclopenta[b]pyridine-6,4'-piperidin]-5-amine | 508 |
| 155 | 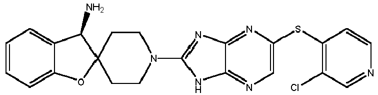 | (R)-1'-(5-((3-chloropyridin-4-yl)thio)-1H-imidazo[4,5-b]pyrazin-2-yl)-3H-spiro[benzofuran-2,4'-piperidin]-3-amine | 466 |
| 156 | 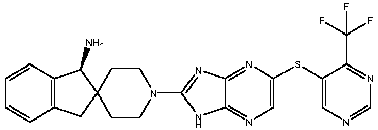 | (S)-1'-(5-((4-(trifluoromethyl)pyrimidin-5-yl)thio)-1H-imidazo[4,5-b]pyrazin-2-yl)-1,3-dihydrospiro[indene-2,4'-piperidin]-1-amine | 499 |
| 157 | 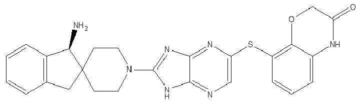 | (S)-8-((2-(1-amino-1,3-dihydrospiro[indene-2,4'-piperidin]-1'-yl)-1H-imidazo[4,5-b]pyrazin-5-yl)thio)-2H-benzo[b][1,4]oxazin-3(4H)-one | 500 |
| 158 | 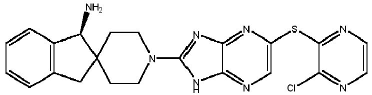 | (S)-1'-(5-((3-chloropyrazin-2-yl)thio)-1H-imidazo[4,5-b]pyrazin-2-yl)-1,3-dihydrospiro[indene-2,4'-piperidin]-1-amine | 465 |
| 159 | 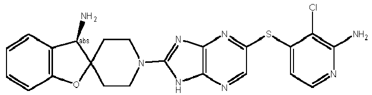 | (R)-1'-(5-((2-amino-3-chloropyridin-4-yl)thio)-1H-imidazo[4,5-b]pyrazin-2-yl)-3H-spiro[benzofuran-2,4'-piperidin]-3-amine | 481 |
| 160 | 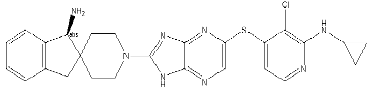 | (S)-1'-(5-((3-chloro-2-(cyclopropylamino)pyridin-4-yl)thio)-1H-imidazo[4,5-b]pyrazin-2-yl)-1,3-dihydrospiro[indene-2,4'-piperidin]-1-amine | 519 |
| 161 | 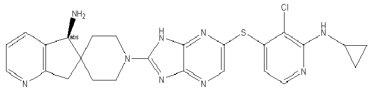 | (S)-1'-(6-((3-chloro-2-(cyclopropylamino)pyridin-4-yl)thio)-1H-imidazo[4,5-b]pyrazin-2-yl)-5,7-dihydrospiro[cyclopenta[b]pyridine-6,4'-piperidin]-5-amine | 520 |
| 162 | 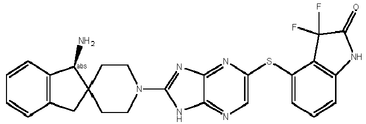 | (S)-4-((2-(1-amino-1,3-dihydrospiro[indene-2,4'-piperidin]-1'-yl)-1H-imidazo[4,5-b]pyrazin-5-yl)thio)-3,3-difluoroindolin-2-one | 520 |
| 163 | 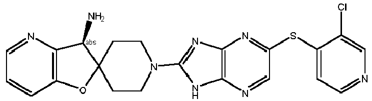 | (R)-1'-(5-((3-chloropyridin-4-yl)thio)-1H-imidazo[4,5-b]pyrazin-2-yl)-3H-spiro[furo[3,2-b]pyridine-2,4'-piperidin]-3-amine | 467 |
| 164 | 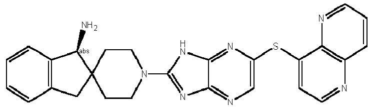 | (S)-1'-(6-((1,5-naphthyridin-4-yl)thio)-1H-imidazo[4,5-b]pyrazin-2-yl)-1,3-dihydrospiro[indene-2,4'-piperidin] -1 -amine | 481 |
| 165 | 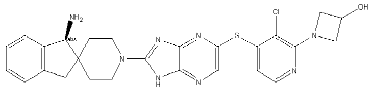 | (S)-1-(4-((2-(1-amino-1,3-dihydrospiro[indene-2,4'-piperidin]-1'-yl)-1H-imidazo[4,5-b]pyrazin-5-yl)thio)-3-chloropyridin-2-yl)azetidin-3-ol | 535 |
| 166 | 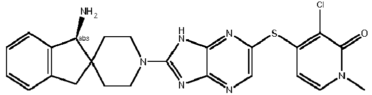 | (S)-4-((2-(1-amino-1,3-dihydrospiro[indene-2,4'-piperidin]-1'-yl)-1H-imidazo[4,5-b]pyrazin-6-yl)thio)-3-chloro-1-methylpyridin-2(1 H)-one | 494 |
| 167 | 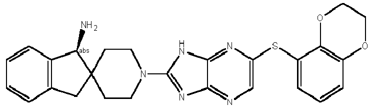 | (S)-1'-(6-((2,3-dihydrobenzo[b][1,4]dioxin-5-yl)thio)-1H-imidazo[4,5-b]pyrazin-2-yl)-1,3-dihydrospiro[indene-2,4'-piperidin] -1 -amine | 487 |
| 168 | 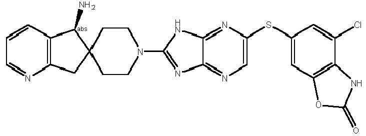 | (S)-6-((2-(5-amino-5,7-dihydrospiro[cyclopenta[b]pyridine-6,4'-piperidin]-1'-yl)-1H-imidazo[4,5-b]pyrazin-6-yl)thio)-4-chlorobenzo[d] oxazol-2(3H)-one | 521 |
| 169 | 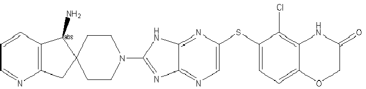 | (S)-6-((2-(5-amino-5,7-dihydrospiro[cyclopenta[b]pyridine-6,4'-piperidin]-1'-yl)-1H-imidazo[4,5-b]pyrazin-6-yl)thio)-5-chloro-2H-benzo[b][1,4]oxazin-3(4H)-one | 535 |

Exemplary and non-limiting processes for the synthesis of specific examples are reported hereinbelow.

### Example 22: 1-[5-((2,3-Dichlorophenyl)thio)-1H-imidazo[4,5-b]pyrazin-2-yl]-4-methylpiperidin-4-amine (trifluoroacetate salt)

The compound was prepared according to the general scheme indicated below (Scheme 1), wherein R is 2-ethylhexyl, Ar is 2,3-dichlorophenyl and X is bromine

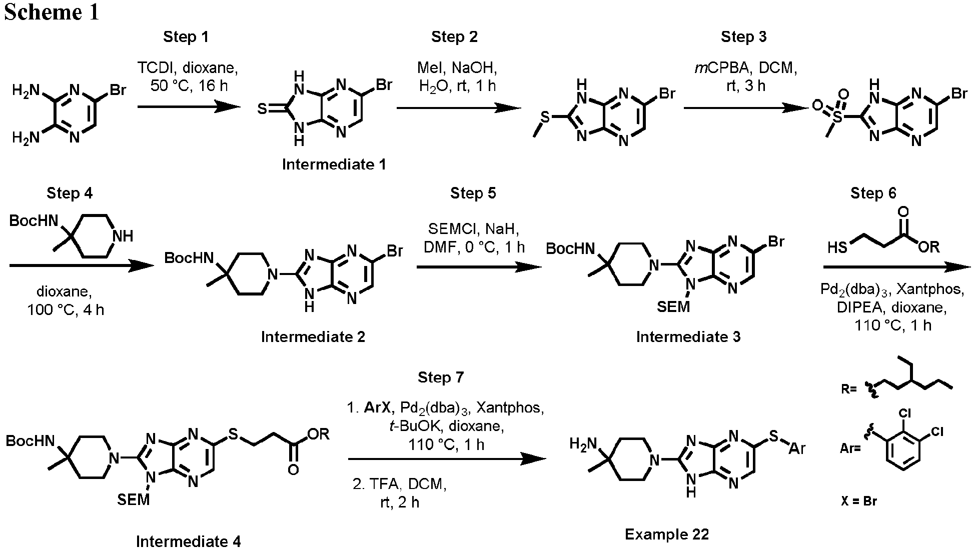

### Step 1: 5-Bromo-1,3-dihydro-2H-imidazo[4,5-b]pyrazine-2-thione (Intermediate 1)

A solution of 5-bromopyrazine-2,3-diamine (Sigma Aldrich, cat. No. 68573) (5.0 g, 26.5 mmol) in 1,4-dioxane (55 mL) was treated with TCDI (6.6 g, 37.0 mmol). The mixture was heated at 50 °C for 16 h. After cooling, the reaction mixture was concentrated *in vacuo* and purified on silica gel (eluting with 0-50% EtOAc/Petroleum ether) to give the title compound as a yellow solid (5.4 g, 79%). ¹H NMR (DMSO-*d*₆) δ 13.62 (br s, 2H), 8.22 (s, 1H). LCMS (ES⁺) *m*/*z* 231, 233 (M+H)⁺, RT 0.87 min.

### Step 2: 6-Bromo-2-(methylthio)-1H-imidazo[4,5-b]pyrazine

A solution of 5-bromo-1,3-dihydro-2*H*-imidazo[4,5-*b*]pyrazine-2-thione (**Intermediate 1**) (3.0 g, 13.0 mmol) in H₂O (85 mL) at rt was treated with NaOH (799 mg, 19.5 mmol) and stirred at this temperature until complete dissolution of the starting material. Then, iodomethane (1.2 mL, 19.5 mmol) was added and the reaction mixture was stirred at rt for 1 h. A solution of NaOH 2N was added until dissolution of the solid and the aqueous solution was washed with CHCl₃, concentrated and acidified to pH 6.5 with aqueous HCl 6 N. The resulting precipitate was filtered off and washed with H₂O to give the title compound as a pale yellow solid (1.8 g, 56%). ¹H NMR (DMSO-*d₆*) δ 7.76 (s, 1H), 2.55 (s, 3H). LCMS (ES⁺) *m*/*z* 245, 247 (M+H)⁺; RT 1.03 min.

### Step 3: 6-Bromo-2-(methylsulfonyl)-1H-imidazo[4,5-b]pyrazine

*m*-CPBA (4.2 g, 18.4 mmol) was added portion-wise to a solution of 6-bromo-2-(methylthio)-1*H-*imidazo[4,5-*b*]pyrazine (1.8 g, 7.3 mmol) in DCM (70 mL) at 0 °C, then the resulting reaction mixture was stirred at rt for 3 h. After addition of aqueous HCl 6 N (10 mL) the mixture was extracted with a solution of CHCl₃/EtOH 95:5 (3x150 mL). The organic phase was washed with H₂O and brine. The dried organics were concentrated *in vacuo* and the residue was purified on RP silica gel (eluting with 0-50% MeCN/H₂O (0.1 % TFA) to give the title compound as a white solid (1.5 g, 75%). ¹H NMR (DMSO-*d*₆) δ 8.79 (s, 1H), 3.56 (s, 3H). LCMS (ES⁺) *m*/*z* 277, 279 (M+H)⁺; RT 0.86 min.

### Step 4: tert-Butyl (1-[5-bromo-1H-imidazo[4,5-b]pyrazin-2-yl]-4-methylpiperidin-4-yl)carbamate (Intermediate 2)

*tert*-Butyl *N*-(4-methylpiperidin-4-yl)carbamate (1.4 g, 6.6 mmol) and 6-bromo-2-(methylsulfonyl)-1*H*-imidazo[4,5-*b*]pyrazine (1.5 g, 5.5 mmol) were dissolved in 1,4-dioxane (40 mL) and the solution was heated at 100 °C for 4 h. After cooling, the mixture was concentrated *in vacuo* and purified on silica gel (eluting with 0-55% EtOAc/Petroleum ether) to give the title compound as a pale yellow solid (1.1 g, 46%). ¹H NMR (CDCl₃) δ 7.96 (s, 1H), 4.48 (br s, 1H), 3.99 - 3.96 (m, 2H), 3.59 - 3.56 (m, 2H), 2.28 - 2.25 (m, 2H), 1.75 - 1.73 (m, 2H), 1.46 (s, 9H), 1.43 (s, 3H). LCMS (ES⁺) *m*/*z* 411, 413 (M+H)⁺, RT 1.51 min.

### Step 5: tert-Butyl (1-[5-bromo-1-((2-(trimethylsilyl)ethoxy)methyl]-1H-imidazo[4,5-b]pyrazin-2-yl)-4-methylpiperidin-4-yl)carbamate (Intermediate 3)

NaH (21.4 mg, 0.5 mmol, 60% in mineral oil) was added portionwise to a solution of *tert*-butyl (1-[5-bromo-1*H*-imidazo[4,5-*b*]pyrazin-2-yl]-4-methylpiperidin-4-yl)carbamate (**Intermediate 2;** 200 mg, 0.5 mmol) in DMF (4.5 mL) at 0 °C. The reaction mixture was stirred at rt for 1 h, then 2-(chloromethoxy)ethyl-trimethylsilane (125 µL, 0.6 mmol) was added dropwise and the mixture was stirred at rt for 30 min. After carefully addition of H₂O (2 mL) the mixture was extracted with DCM. The organic extracts were washed with H₂O and brine. The dried organics were concentrated *in vacuo* and the residue was purified on silica gel (eluting with 0-50% EtOAc/Petroleum ether) to give the title compound (1:1 mixture of regioisomers) as a white solid (160 mg, 61%). ¹H NMR (DMSO-*d*₆) δ 8.24 (s, 1H), 8.04 (s, 1H), 6.69 (br s, 2H), 5.38 (d, *J* = 12.3 Hz, 4H), 3.95 - 3.92 (m, 4H), 3.75 - 3.72 (m, 4H), 3.48 - 3.45 (m, 4H), 2.19 - 2.16 (m, 4H), 1.59 - 1.56 (m, 4H), 1.40 (s, 18H), 1.27 (s, 6H), 0.93 - 0.90 (m, 4H), -0.04 (s, 18H). LCMS (ES⁺) *m*/*z* 541, 543 (M+H)⁺, RT 2.61 and 2.63 min.

*Step 6: 2-Ethylhexyl 3-[(2-(4-((tert-butoxycarbonyl)amino)-4-methylpiperidin-1-yl]-1-[(2-(trimethylsilyl)ethoxy)methyl]-1H-imidazo[4,5-b]pyrazin-5-yl)thio)propanoate (**Intermediate 4**) tert*-Butyl (1-[5-bromo-1-((2-(trimethylsilyl)ethoxy)methyl)-1*H*-imidazo[4,5-*b*]pyrazin-2-yl]-4-methylpiperidin-4-yl)carbamate **(Intermediate 3;** 855 mg, 1.6 mmol), 2-ethylhexyl 3-mercaptopropanoate (718 µL, 3.2 mmol), DIPEA (550 µL, 3.12 mmol), Pd₂(dba)₃ (72 mg, 0.08 mmol) and Xantphos (91 mg, 0.16 mmol) were dissolved in 1,4-dioxane (16 mL) and the reaction mixture was heated at 110 °C for 1 h. After cooling, the mixture was filtered through a pad of Solka Floc and washed with EtOAc. The organic phase was concentrated *in vacuo* and the residue was purified on silica gel (eluting with 0-50% EtOAc/Petroleum ether) to give the title compound (1:1 mixture of regioisomers) as a yellow oil (1.06 g, 99%). ¹H NMR (DMSO-*d*₆) δ 8.07 (s, 1H), 7.85 (s, 1H), 6.68 (br s, 2H), 5.38 (d, *J* = 3.7 Hz, 4H), 3.96 - 3.84 (m, 8H), 3.75 - 3.72 (m, 4H), 3.45-3.42 (m, 4H), 3.33 - 3.31 (m, 4H), 2.74 - 2.71 (m, 4H), 2.18 - 2.14 (m, 4H), 1.60 - 1.51 (m, 6H), 1.40 (s, 18H), 1.30 - 1.23 (m, 12H), 0.94 - 0.81 (m, 16H), -0.04 (s, 18H). LCMS (ES⁺) *m*/*z* 679 (M+H)⁺, RT 2.65 and 2.70 min.

### Step 7: 1-[5-((2,3-Dichlorophenyl)thio)-1H-imidazo[4,5-b]pyrazin-2-yl]-4-methylpiperidin-4-amine(trifluoroacetate salt)

A solution of 1-bromo-2,3-dichlorobenzene (7.5 mg, 0.02 mmol), Pd₂(dba)₃ (1.0 mg, 0.001 mmol), Xantphos (1.3 mg, 0.002 mmol) and 2-ethylhexyl 3-[(2-(4-((tert-butoxycarbonyl)amino)-4-methylpiperidin-1-yl)-1-((2-(trimethylsilyl)ethoxy)methyl)-1H-imidazo[4,5-*b*]pyrazin-5-yl)thio]propanoate (**Intermediate 4;** 15 mg, 0.02 mmol) in toluene (0.6 mL) was degassed using a positive flow of N₂ for 5 min. Then *^{t}*BuOK (49 µL, 0.05 mmol, 1.0 M in THF) was added dropwise and the reaction mixture was heated at 110 °C for 1h. After cooling, the mixture was concentrated *in vacuo,* and diluted with DCM (1 mL) and NH₄Cl sat. sol. The resulting mixture was stirred vigorously for 10 min. The organic layer was separated and treated with TFA (68 µL, 0.88 mmol). The mixture was concentrated *in vacuo* and the residue was purified by RP-HPLC using H₂O (+ 0.1% TFA) and MeCN (+ 0.1% TFA) as eluents (C₁₈ column). Lyophilization of the appropriate fractions afforded the title compound as a white solid (2.9 mg, 25%). LCMS (ES⁺) *m*/*z* 409 (M+H)⁺; RT 1.2 min.

The following examples were synthesized using the procedure indicated in Scheme 1, with the appropriate starting materials: 1, 6, 9-12, 18-30, 36-39, 45-49, 57-62, 64-71, 73-77, 92-95, 97, 100-106, 116, 130, 132, 134-136, 145-147, 149, 151, 153-155.

### Examples 4 and 52: 4-methyl-1-(1-methyl-6-((2-(trifluoromethyl)pyridin-3-yl)thio)-1H-imidazo[4,5-b]pyrazin-2-yl)piperidin-4-amine (Example 4) and 4-methyl-1-(1-methyl-5-((2-(trifluoromethyl)pyridin-3-yl)thio)-1H-imidazo[4,5-b]pyrazin-2-yl)piperidin-4-amine (Example 52)

The compounds were prepared according to Scheme 2, following the procedures indicated below.

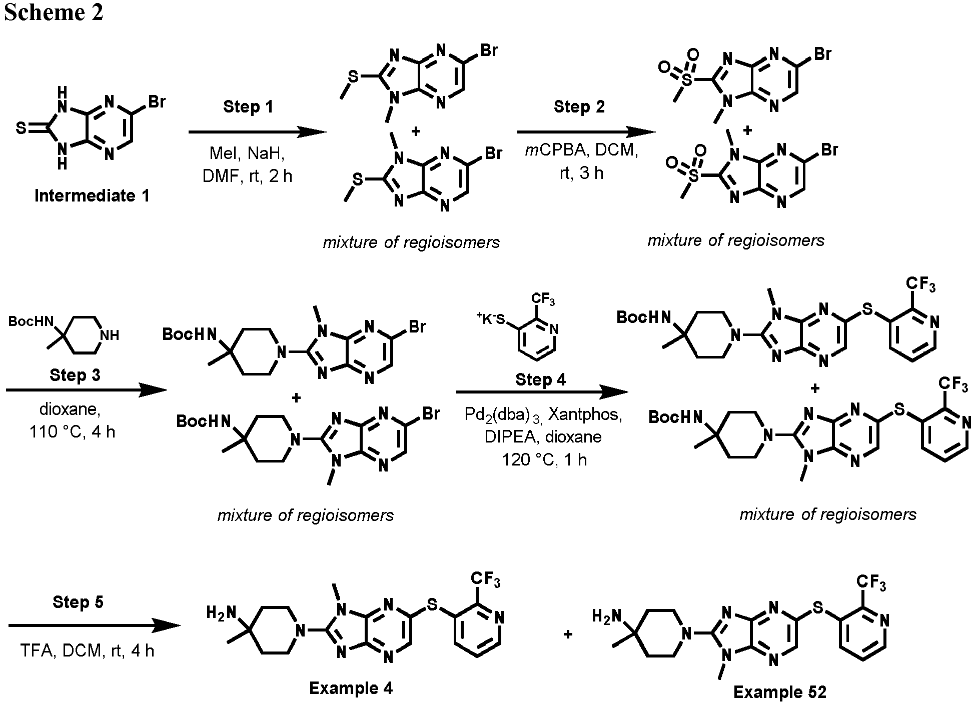

### Step 1: 6-bromo-1-methyl-2-(methylthio)-1H-imidazo[4,5-b]pyrazine and 5-bromo-1-methyl-2-(methylthio)-1H-imidazo[4,5-b]pyrazine

A solution of 5-bromo-1,3-dihydro-2H-imidazo[4,5-b]pyrazine-2-thione (**Intermediate 1**) (100 mg, 0.4 mmol, prepared as in Step 1 of Scheme 1) in DMF (2.5 mL) under N₂ atmosphere was treated at 0 °C with NaH (43 mg, 1.1 mmol, 60% in mineral oil). The resulting yellow mixture was stirred at rt for 1 h. To this solution was added in one portion iodomethane (55 uL, 0.9 mmol) and mixture stirred for 1 h at rt. H₂O (2 mL) was added dropwise and mixture extracted with DCM (3x15 mL). Organic phase was washed with H₂O, brine, dried over Na₂SO₄, filtered and concentrated in vacuo. Purification by flash chromatography (gradient elution 0-40% EtOAc in petroleum ether) gave the title compound (1:1 mixture of regioisomers by ¹H NMR) as a yellow viscous oil (87 mg, 77%). ¹H NMR (400 MHz, DMSO-*d₆*) δ 8.48 (s, 1 H), 8.39 (s, 1 H), 3.68 (s, 3 H), 3.66 (s, 3 H), 2.80 (s, 6 H). LCMS (ES⁺) *m*/*z* 259, 261 (M+H)⁺, RT 1.35 min.

### Step 2: 6-bromo-1-methyl-2-(methylsulfonyl)-1H-imidazo[4,5-b]pyrazine and 5-bromo-1-methyl-2-(methylsulfonyl)-1H-imidazo[4,5-b]pyrazine

A solution of 6-bromo-1-methyl-2-(methylthio)-1H-imidazo[4,5-b]pyrazine and 5-bromo-1-methyl-2-(methylthio)-1H-imidazo[4,5-b]pyrazine (1:1 mixture of regioisomers) (87 mg, 0.3 mmol) in DCM (3 mL) was treated at 0 °C with *m*-CPBA (209 mg, 0.9 mmol). Mixture was stirred at rt for 3 h. NaHCO₃ sat. sol. (3 mL) was added dropwise and mixture was extracted with DCM (3x20 mL). Then organic phase was washed with H₂O, brine, dried over Na₂SO₄, filtered and concentrated in vacuo. Purification by flash chromatography (gradient elution 0-40% EtOAc in petroleum ether) gave the title compounds (1:1 mixture of regioisomers by ¹H NMR) as a white solid (90 mg, 98%). ¹H NMR (400 MHz, DMSO-*d₆*) δ 8.90 (s, 1 H), 8.88 (s, 1 H), 4.10 (s, 3 H), 4.07 (s, 3 H), 3.66 (s, 6 H). LCMS (ES⁺) *m*/*z* 291, 293 (M+H)⁺, RT 1.20 min.

### Step 3: tert-butyl (1-(5-bromo-1-methyl-1H-imidazo[4,5-b]pyrazin-2-yl)-4-methylpiperidin-4-yl)carbamate and tert-butyl (1-(6-bromo-1-methyl-1H-imidazo[4,5-b]pyrazin-2-yl)-4-methylpiperidin-4-yl)carbamate

A solution of 6-bromo-1-methyl-2-(methylsulfonyl)-1H-imidazo[4,5-b]pyrazine and 5-bromo-1-methyl-2-(methylsulfonyl)-1H-imidazo[4,5-b]pyrazine (1:1 mixture of regioisomers) (90 mg, 0.3 mmol) and tert-butyl N-(4-methylpiperidin-4-yl)carbamate (79.5 mg, 0.4 mmol) in 1,4-dioxane (2.5 mL) was heated at 110 °C for 4 h. Mixture was concentrated under reduced pressure. Purification by flash chromatography (gradient elution 0-40% EtOAc in petroleum ether) gave the title compound (mixture of regioisomers by UPLC) as a white solid (40 mg, 30%). LCMS (ES⁺) *m*/*z* 425, 427 (M+H)⁺, RT 1.78 min.

### Step 4: tert-butyl (4-methyl-1-(1-methyl-5-((2-(trifluoromethyl)pyridin-3-yl)thio)-1H-imidazo[4,5-b]pyrazin-2-yl)piperidin-4-yl)carbamate and tert-butyl (4-methyl-1-(1-methyl-6-((2-(trifluoromethyl)pyridin-3-yl)thio)-1H-imidazo[4,5-b]pyrazin-2-yl)piperidin-4-yl)carbamate

A vial was charged with DIPEA (33 uL, 0.2 mmol), tert-butyl (1-(5-bromo-1-methyl-1H-imidazo[4,5-*b*]pyrazin-2-yl)-4-methylpiperidin-4-yl)carbamate and tert-butyl (1-(6-bromo-1-methyl-1H-imidazo[4,5-b]pyrazin-2-yl)-4-methylpiperidin-4-yl)carbamate (mixture of regioisomers) (40 mg, 0.09 mmol), potassium 2-(trifluoromethyl)pyridine-3-thiolate (82 mg, 0.19 mmol), Xantphos (5.4 mg, 0.01 mmol) and Pd₂(dba)₃ (4.3 mg, 0.005 mmol) in 1,4-dioxane (1 mL). The vial was sealed and heated at 120 °C for 1 h. Mixture was filtered through a pad of Solka Floc and concentrated under reduced pressure. Purification by flash chromatography (gradient elution 0-40% EtOAc in petroleum ether) gave the title compound (2:1 mixture of regioisomers by UPLC) as a white solid (33 mg, 67%). LCMS (ES⁺) *m*/*z* 524 (M+H)⁺, RT 1.97, 2.02 min.

### Step 5: Example 4 and Example 52: 4-methyl-1-(1-methyl-5-((2-(trifluoromethyl)pyridin-3-yl)thio)-1H-imidazo[4,5-b]pyrazin-2-yl)piperidin-4-amine and 4-methyl-1-(1-methyl-6-((2-(trifluoromethyl)pyridin-3-yl)thio)-1H-imidazo[4,5-b]pyrazin-2-yl)piperidin-4-amine

To a stirred solution of tert-butyl (4-methyl-1-(1-methyl-5-((2-(trifluoromethyl)pyridin-3-yl)thio)-1*H*-imidazo[4,5-*b*]pyrazin-2-yl)piperidin-4-yl)carbamate and tert-butyl (4-methyl-1-(1-methyl-6-((2-(trifluoromethyl)pyridin-3-yl)thio)-1*H*-imidazo[4,5-*b*]pyrazin-2-yl)piperidin-4-yl)carbamate (2:1 mixture of regioisomers) (33 mg, 0.06 mmol) in DCM (0.9 mL) at 0 °C was added TFA (97 uL, 1.3 mmol) and the reaction mixture was stirred at rt for 4 h. Volatiles were removed under reduced pressure and purification by reverse phase flash chromatography [gradient elution 0-100% MeCN (0.1% TFA) in H₂O (0.1% TFA)] gave the title compounds (2:1 mixture of regioisomers by ¹H NMR) as a white solid (22 mg, 82%).

Mixture was resolved by automated SFC using as stationary phase an IB™ CHIRALPAK®column and as mobile phase a linear gradient of MeOH in CO₂.

**Example 4: 4-methyl-1-(1-methyl-6-((2-(trifluoromethyl)pyridin-3-yl)thio)-1H-imidazo[4,5-b]pyrazin-2-yl)piperidin-4-amine.** ¹H NMR (500 MHz, DMSO-*d₆*) δ 8.55 (d, *J* = 4.5 Hz, 1 H), 8.31 (s, 1 H), 7.66 (d, *J* = 8.2 Hz, 1 H), 7.58 - 7.52 (m, 1 H), 3.78 - 3.59 (m, 6 H), 3.61 (s, 3 H), 1.64 - 1.55 (m, 3 H), 1.13 (s, 3 H); ¹⁹F NMR (400 MHz, DMSO-*d₆*) *δ* -63.55. LCMS (ES⁺) *m*/*z* 424 (M+H)⁺, RT 1.14 min.

**Example 52: 4-methyl-1-(1-methyl-5-((2-(trifluoromethyl)pyridin-3-yl)thio)-1H-imidazo[4,5-b]pyrazin-2-yl)piperidin-4-amine.** ¹H NMR (500 MHz, DMSO-*d₆*) δ 8.61 - 8.58 (m, 1 H), 8.16 (s, 1 H), 7.78 (d, *J* = 8.1 Hz, 1 H), 7.61 (dd, *J* = 8.2, 4.5 Hz, 1 H), 3.69 - 3.67 (m, 3 H), 3.73 - 3.56 (m, 4 H), 1.65 - 1.44 (m, 4 H), 1.12 (s, 3 H); ¹⁹F NMR (400 MHz, DMSO-*d₆*) δ - 63.26. LCMS (ES⁺) *m*/*z* 424 (M+H)⁺, RT 1.14 min.

### Example 63: 1-(1-Ethyl-5-[(2-(trifluoromethyl)pyridin-3-yl)thio]-1H-imidazo[4,5-b]pyrazin-2-yl)-4-methylpiperidin-4-amine (trifluoroacetate salt)

The compound was prepared according to Scheme 3, following the procedures reported below.

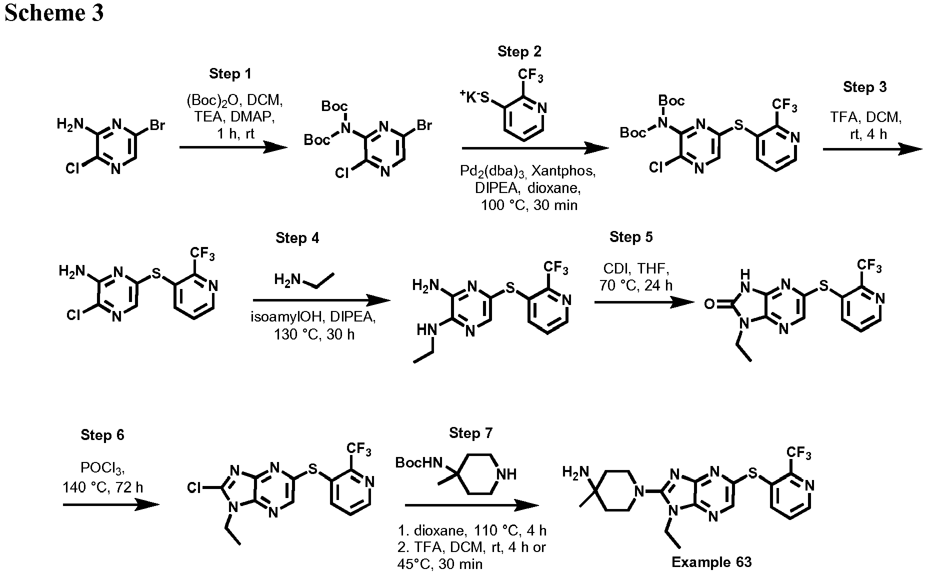

### Step 1: tert-butyl (6-bromo-3-chloropyrazin-2-yl)(tert-butoxycarbonyl)carbamate

A solution of 6-bromo-3-chloropyrazin-2-amine(500 mg, 2.4 mmol) in DCM (5 mL) cooled to 0 °C was treated with (Boc)₂O (1.15 g, 5.28 mmol), DMAP (29 mg, 0.24 mmol) and TEA.The reaction mixture was stirred at rt for 1 h then NaHCO₃ sat. sol. was added. The organic phase was separated and washed with HCl 1 N and brine. The dried organics were concentrated *in vacuo* to give the title compound as a pale yellow solid (927 mg, 94%) which was used in the next step without further purification. ¹H NMR (DMSO-*d*₆) δ 8.9 (s, 1H), 1.39 (s, 18H). LCMS (ES⁺) *m*/*z* 431 (M+Na)⁺; RT 2.48 min.

### Step 2: tert-butyl (tert-butoxycarbonyl)(3-chloro-6-((2-(trifluoromethyl)pyridin-3-yl)thio)pyrazin-2-yl)carbamate

A solution of tert-butyl (6-bromo-3-chloropyrazin-2-yl)(tert-butoxycarbonyl)carbamate (927 mg, 2.27 mmol) in 1,4-dioxane (18 mL) was treated with Pd₂(dba)₃ (128 mg, 0.14 mmol), Xantphos (163 mg, 0.28 mmol), [2-(trifluoromethyl)pyridin-3-yl]sulfanylpotassium (738 mg, 3.4 mmol) and DIPEA (0.97 mL, 5.6 mmol). The reaction mixture was heated at 100 °C for 30 min. After cooling, the mixture was filtered through a pad of Solka Floc and the organic solvent was concentrated *in vacuo* and the residue purified on silica gel (eluting with 0-60% EtOAc/Petroleum ether) to give the title compound as a yellow powder (970 mg, 84%). ¹H NMR (DMSO-*d*₆) δ 8.85 (dd, *J* = 4.4 and 1.3 Hz, 1H), 8.64 (s, 1H), 8.3 (d, *J* = 7.9 Hz, 1H), 7.84 (dd, *J* = 8.1 and 4.6 Hz, 1H), 1.9 (s, 18H). LCMS (ES⁺) *m*/*z* 507 (M+H)⁺, RT 2.57 min.

### Step 3: 3-Chloro-6-[(2-(trifluoromethyl)pyridin-3-yl)thio]pyrazin-2-amine

A solution of compound from Step 2 (970 mg, 1.91 mmol) in DCM (6 mL) was treated with TFA (2 mL). The reaction mixture was stirred at rt for 4 h, cooled to 0 °C and diluted H₂O, NaHCO₃ sat. sol. was added until pH 8. The organic phase was separated, dried and concentrated *in vacuo* to give the title compound as a yellow solid (550 mg, 94%) which was used in the next step without further purification. ¹H NMR (DMSO-*d*₆) δ 8.71 (dd, *J* = 4.4 and 1.3 Hz, 1H), 8.12 (d, *J* = 7.9 Hz, 1H), 7.73 (dd, *J* = 8.1 and 4.6 Hz, 1H), 7.49 (s, 1H), 7.1 (br s, 2H). LCMS (ES⁺) *m*/*z* 307 (M+H)⁺; RT 1.87 min.

### Step 4: N²-Ethyl-5-[(2-(trifluoromethyl)pyridin-3-yl)thio]pyrazine-2,3-diamine

A solution of 3-chloro-6-[(2-(trifluoromethyl)pyridin-3-yl)thio]pyrazin-2-amine (30 mg, 0.1 mmol), ethylamine (684 µL, 1.36 mmol, 2.0 M in THF) and DIPEA (51 µL, 0.29 mmol) in isoamyl alcohol (0.6 mL) was heated to 130 °C for 30 h. After cooling, the mixture was diluted with H₂O and EtOAc. The organic phase was separated, washed with brine, dried and concentrated *in vacuo* to give the title compound as an orange solid (30 mg, 97%) which was used in the next step without further purification. LCMS (ES⁺) *m*/*z* 316 (M+H)⁺; RT 1.68 min.

### Step 5: 1-Ethyl-5-((2-(trifluoromethyl)pyridin-3-yl)thio)-1,3-dihydro-2H-imidazo[4,5-b]pyrazin-2-one

*N*²-Ethyl-5-[2-(trifluoromethyl)pyridin-3-yl]sulfanyl-pyrazine-2,3-diamine (31 mg, 0.10 mmol) and CDI (79 mg, 0.49 mmol) were dissolved in THF (1.2 mL) and the obtained mixture was stirred at 70 °C for 24 h. The solvent was concentrated *in vacuo* and the residue was purified on silica gel (eluting with 0-100% EtOAc/Petroleum ether) to give the title compound as an orange solid (15 mg, 46%). LCMS (ES⁺) *m*/*z* 342 (M+H)⁺; RT 1.79 min.

### Step 6: 2-Chloro-1-ethyl-5-((2-(trifluoromethyl)pyridin-3-yl)thio)-1H-imidazo[4,5-b]pyrazine

A solution of 1-ethyl-5-((2-(trifluoromethyl)pyridin-3-yl)thio)-1,3-dihydro-2H-imidazo[4,5-b]pyrazin-2-one (15.5 mg, 0.045 mmol) in POCl₃ (0.42 mL, 4.54 mmol) was heated to 140 °C for 72 h. After cooling, the mixture was treated with NaHCO₃ sat. sol. and extracted with EtOAc. The organic extracts were washed with brine, dried and concentrated *in vacuo* to give the title compound as a yellow oil (11 mg) which was used in the next step without further purification. LCMS (ES⁺) *m*/*z* 360 (M+H)⁺; RT 1.85 min.

### Step 7: 1-(1-Ethyl-5-[(2-(trifluoromethyl)pyridin-3-yl)thio]-1H-imidazo[4,5-b]pyrazin-2-yl)-4-methylpiperidin-4-amine (trifluoroacetate salt)

A solution of 2-chloranyl-1-ethyl-5-[2-(trifluoromethyl)pyridin-3-yl]sulfanyl-imidazo[4,5-b]pyrazine (11.3 mg, 0.03 mmol) and *tert*-butyl *N*-(4-methylpiperidin-4-yl)carbamate (8.2 mg, 0.04 mmol) in 1,4-dioxane (0.6 mL) was heated to 110 °C for 4 h. After cooling, the mixture was concentrated *in vacuo* and the residue diluted with DCM (0.5 mL) and treated with TFA (0.5 mL, 6.5 mmol). The mixture was stirred at 45 °C for 30 min, then concentrated *in vacuo* and the residue purified by RP-HPLC using H₂O (+ 0.1% TFA) and MeCN (+ 0.1% TFA) as eluents (C₁₈ column). Lyophilization of the appropriate fractions afforded the title compound as an orange powder (8.1 mg, 56% over two steps). ¹H NMR (DMSO-*d*₆) δ 8.63 (d, *J* = 4.4 Hz, 1H), 8.23 (1 H, s), 7.99 (3 H, br s), 7.84 (1 H, d, *J* = 7.9 Hz, 1H), 7.64 (dd, *J* = 8.1 and 4.6 Hz, 1H), 4.21 - 4.16 (m, 2H), 3.85 - 3.81 (m, 2H), 3.49 - 3.44 (m, 2H), 1.93 - 1.80 (m, 4H), 1.40 (s, 3H), 1.40 (t, *J* = 6.6 Hz, 3H). LCMS (ES⁺) *m*/*z* 438 (M+H)⁺; RT 1.21 min.

Example 13 was synthesized according to the above procedures (Scheme 3), by using cyclopropylmethylamine instead of ethyl amine in Step 4.

Example 129 was synthesized according to the above procedures (Scheme 3), by using 2-methoxyethylamine instead of ethyl amine in Step 4.

### Example 72: 1-(1-Ethyl-6-[(2-(trifluoromethyl)pyridin-3-yl)thio]-1H-imidazo[4,5-b]pyrazin-2-yl)-4-methylpiperidin-4-amine (trifluoroacetate salt)

The compound was prepared according to Scheme 4, following the procedures reported below.

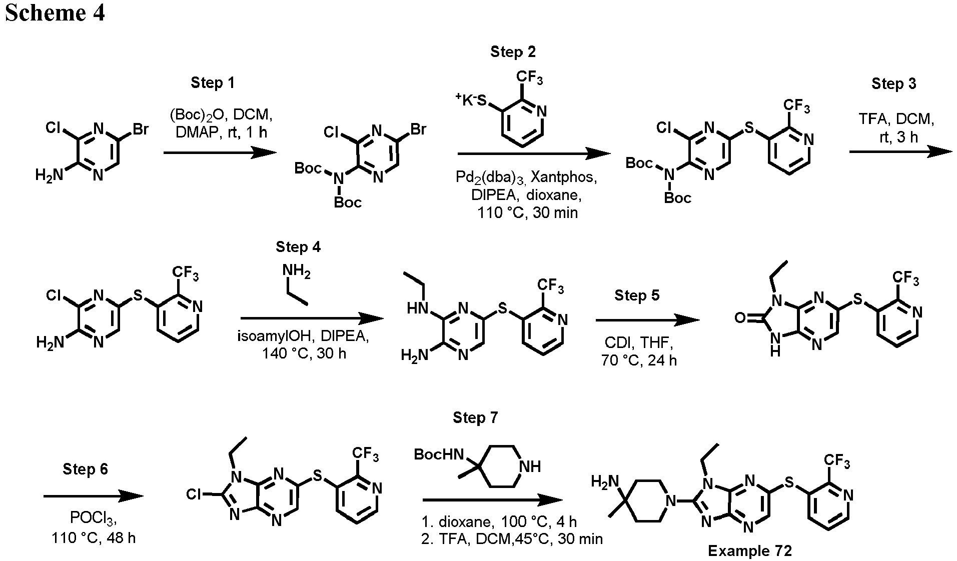

### Step 1: tert-butyl (5-bromo-3-chloropyrazin-2-yl)(tert-butoxycarbonyl)carbamate

A solution of 5-bromo-3-chloropyrazin-2-amine (800 mg, 3.83 mmol) in DCM (8 mL) at 0 °C was treated with (Boc)₂O (1.84 g, 8.44 mmol) and DMAP (117 mg, 0.96 mmol). The mixture was stirred at rt for 1 h, then NaHCO₃ sat. sol. was added. The organic phase was separated and washed with HCl 1 N, brine, dried and concentrated *in vacuo* to give the title compound as an orange solid (1.52 g, 97%). ¹H NMR (DMSO-*d*₆) δ 8.95 (s, 1H), 1.38 (s, 18H). LCMS (ES⁺) *m*/*z* 431 (M+Na)⁺; RT 2.54 min.

### Step 2: tert-butyl (tert-butoxycarbonyl)(3-chloro-5-((2-(trifluoromethyl)pyridin-3-yl)thio)pyrazin-2-yl)carbamate

A solution of tert-butyl (5-bromo-3-chloropyrazin-2-yl)(tert-butoxycarbonyl)carbamate (1.55 g, 3.72 mmol) in 1,4-dioxane (30 mL) at rt was treated with Pd₂(dba)₃ (213 mg, 0.23 mmol), Xantphos (269 mg, 0.46 mmol), [2-(trifluoromethyl)pyridin-3-yl]sulfanylpotassium (1.21 g, 5.57 mmol) and DIPEA (1.62 mL, 9.3 mmol). The reaction mixture was heated at 110 °C for 30 min. After cooling, the mixture was filtered through a pad of Solka Floc and the filtrates concentrated *in vacuo.* The residue was purified on silica gel (eluting with 0-60% EtOAc/Petroleum ether) to give the title compound as an orange solid (1.54 g, 82%). ¹H NMR (DMSO-*d*₆) δ 8.89 (dd, *J* = 4.8, 1.3 Hz, 1H), 8.64 (s, 1H), 8.4 (d, *J* = 7.4 Hz, 1H), 7.89 (dd, *J* = 7.9 and 4.8 Hz, 1H), 1.9 (s, 18H). LCMS (ES⁺) *m*/*z* 507 (M+H)⁺; RT 2.61 min.

### Step 3: 3-Chloro-5-[(2-(trifluoromethyl)pyridin-3-yl)thio]pyrazin-2-amine

A solution of *tert*-butyl *N*-[3-chloranyl-5-[2-(trifluoromethyl)pyridin-3-yl]sulfanyl-pyrazin-2-yl]-*N*-[(2-methylpropan-2-yl)oxycarbonyl]carbamate (1.54 g, 3.03 mmol) in DCM (10 mL) was treated with TFA (10 mL, 130 mmol) and stirred at rt for 3 h. Then, the mixture was cooled to 0 °C, diluted with H₂O and treated with NaHCO₃ sat. sol. until pH 8. The organic phase was separated, dried and concentrated *in vacuo* to give the title compound as a yellow solid (930 mg, 99%) which was used in the next step without further purification. LCMS (ES⁺) *m*/*z* 307 (M+H)⁺; RT 1.82 min.

### Step 4: N²-Ethyl-6-[(2-(trifluoromethyl)pyridin-3-yl)thio]pyrazine-2,3-diamine

A solution of 3-chloro-5-[(2-(trifluoromethyl)pyridin-3-yl)thio]pyrazin-2-amine (30 mg, 0.1 mmol), ethylamine (1 mL, 2.02 mmol, 2.0 M in THF) and DIPEA (51 µL, 0.29 mmol) in isoamyl alcohol (0.60 mL) was heated at 140 °C for 24 h. After cooling, the mixture was diluted with H₂O and EtOAc. The organic phase was washed with brine, dried and concentrated *in vacuo* to give the title compound as an orange solid (30 mg, 97%) which was used in the next step without further purification. LCMS (ES⁺) *m*/*z* 316 (M+H)⁺; RT 1.48 min.

### Step 5: 1-Ethyl-6-[(2-(trifluoromethyl)pyridin-3-yl)thio]-1,3-dihydro-2H-imidazo[4,5-b]pyrazin-2-one

A solution of *N*²-ethyl-6-[(2-(trifluoromethyl)pyridin-3-yl)thio]pyrazine-2,3-diamine (31 mg, 0.10 mmol) in THF (1.2 mL) at rt was treated with CDI (79 mg, 0.49 mmol). The reaction mixture was heated to 70 °C for 24 h. After cooling, the solvent was concentrated *in vacuo* and the residue purified on silica gel (eluting with 0-100% EtOAc/Petroleum ether) to give the title compound as an orange solid (15.5 mg, 46%). LCMS (ES⁺) *m*/*z* 342 (M+H)⁺; RT 1.76 min.

### Step 6: 2-Chloro-1-ethyl-6-[(2-(trifluoromethyl)pyridin-3-yl)thio]-1H-imidazo[4,5-b]pyrazine

A solution of 1-ethyl-6-[(2-(trifluoromethyl)pyridin-3-yl)thio]-1,3-dihydro-2*H*-imidazo[4,5-*b*]pyrazin-2-one (15 mg, 0.045 mmol) in POCl₃ (0.42 mL, 4.54 mmol) was heated to 110 °C for 48 h. After cooling, the mixture was diluted with NaHCO₃ sat. sol. and extracted with EtOAc. The organic phase was washed with brine, dried and concentrated *in vacuo* to give the title compound as a yellow oil (16 mg, 24%) which was used in the next step without further purification. LCMS (ES⁺) *m*/*z* 360 (M+H)⁺; RT 1.79 min.

### Step 7: 1-(1-Ethyl-6-[(2-(trifluoromethyl)pyridin-3-yl)thio]-1H-imidazo[4,5-b]pyrazin-2yl)-4-methylpiperidin-4-amine (trifluoroacetate salt)

A solution of 2-chloro-1-ethyl-6-[(2-(trifluoromethyl)pyridin-3-yl)thio]-1*H*-imidazo[4,5-*b*]pyrazine (16 mg, 0.044 mmol) and *tert*-butyl *N*-(4-methylpiperidin-4-yl)carbamate (11 mg, 0.05 mmol) in 1,4-dioxane (0.6 mL) was heated to 100 °C for 4 h. The mixture was concentrated *in vacuo* and the residue was diluted with DCM (0.5 mL) and treated with TFA (0.5 mL, 6.5 mmol). The mixture was stirred at 45 °C for 30 min. After cooling, the mixture was concentrated *in vacuo* and the residue was purified by RP chromatography (gradient elution 0-30% MeCN (+ 0.1% TFA)/H₂O (+ 0.1% TFA)) to give the title compound as an orange solid (3.2 mg, 16% over two steps). ¹H NMR (DMSO-*d*₆) δ 8.60 (d, *J* = 4.4 Hz, 1H), 8.36 (s, 1H), 7.96 (br s, 3H), 7.77 (d, *J* = 7.4 Hz, 1H), 7.59 (dd, *J* = 7.9 and 4.4 Hz, 1H), 4.12 - 4.06 (m, 2H), 3.86 - 3.83 (m, 2H), 3.52 - 3.46 (m, 2H), 1.95-1.81 (m, 4H), 1.40 (s, 3H), 1.30 (t, *J* = 7.5 Hz, 3H). LCMS (ES⁺) *m*/*z* 438 (M+H)⁺; RT 1.2 min.

### Example 88: 4-methyl-1-(5-(quinolin-8-ylthio)-1H-imidazo[4,5-b]pyrazin-2-yl)piperidin-4-amine (trifluoroacetate salt)

The compound was prepared according to Scheme 5, following the procedure indicated below.

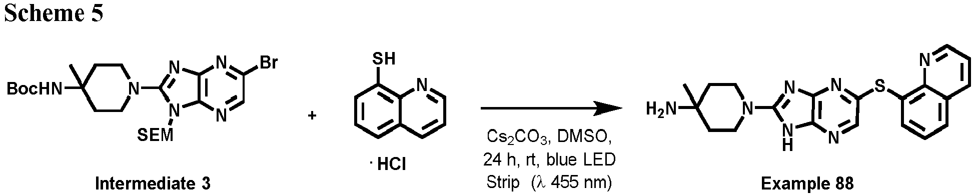

A 4 mL vial was charged with tert-butyl (1-(5-bromo-1-((2-(trimethylsilyl)ethoxy)methyl)-1H-imidazo[4,5-b]pyrazin-2-yl)-4-methylpiperidin-4-yl)carbamate (**Intermediate 3;** 15 mg, 0.03 mmol, prepared as in Step 5 of Scheme 1), 8-mercaptoquinoline hydrochloride (8.2 mg, 0.04 mmol) and Cs₂CO₃ (13.7 mg, 0.07 mmol) in DMSO (0.27 mL) and degassed with N₂ for 30 seconds. The reaction mixture was then irradiated with a blue LED Strip (λ 455 nm) for 24 h at rt then was filtered on a SiO₂ cartridge (2 g) eluting with a DCM/EtOAc 1:1 (6 mL). The organic solvent was evaporated and DCM:TFA (1:1; 0.5 mL) were added. The mixture was stirred at 45°C for 30 min then concentrated *in vacuo* to give a residue that was purified by preparative HPLC to give the title compound as a yellow powder (0.4 mg, 4%). LCMS (ES⁺) *m*/*z* 392 (M+H)⁺; RT 0.86 min.

The following examples were synthesized using the above procedure (Scheme 5), by reaction of Intermediate 3 with the appropriate aryl- or heteroaryl mercaptane: 78, 80, 81, 91, 98, 99, 114, 115, 117.

### Example 107: (S)-1'-(5-[(2-(Trifluoromethyl)pyridin-3-yl)thio]-1H-imidazo[4,5-b]pyrazin-2-yl)-1,3-dihydrospiro[indene-2,4'-piperidin]-1-amine

The compound was prepared according to Scheme 6, following the procedures reported below.

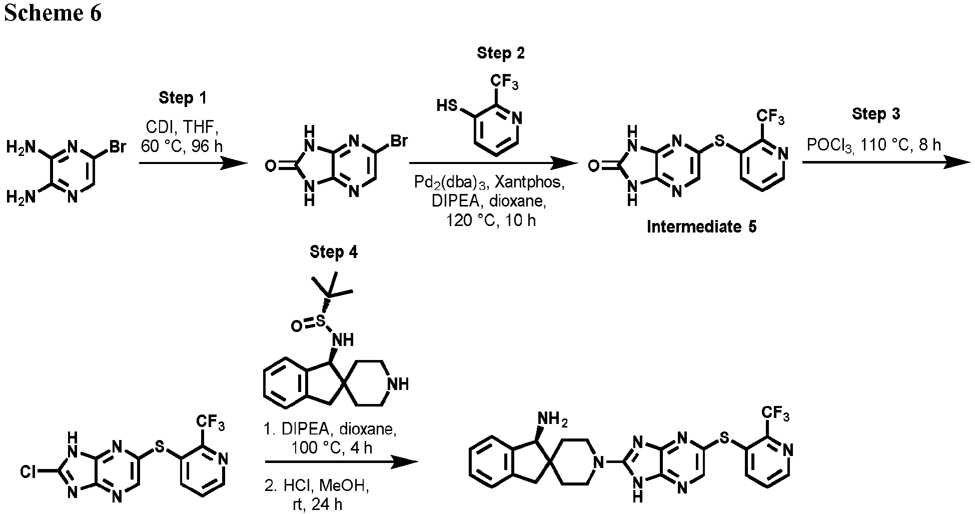

### Step 1: 5-Bromo-1,3-dihydro-2H-imidazo[4,5-b]pyrazin-2-one

A solution of 5-bromo-2,3-pyrazindiamine (4.1 g, 21.7 mmol) and CDI (7.0 g, 43.4 mmol) in THF (120 mL) was heated to 60 °C for 72 h. Then, a second portion of CDI (7.0 g, 43.4 mmol) was added and heating to 60 °C was continued for 24 h. After cooling, the mixture was concentrated *in vacuo* and the residue was purified by RP chromatography (gradient elution 0-50% MeCN (+ 0.1% TFA)/H₂O (+ 0.1% TFA)). Evaporation of the fractions containing the desired product furnished a compound which was triturated with MeCN and filtered off to give the title compound as a beige solid (3.04 g, 65%). ¹H NMR (DMSO-*d*₆) δ 11.98 (br s, 1H), 11.91 (br s, 1H), 7.98 (s, 1H). LCMS (ES⁺) *m*/*z* 215, 217 (M+H)⁺; RT 0.76 min.

### Step 2: 5-[(2-(Trifluoromethyl)pyridin-3-yl)thio]-1,3-dihydro-2H-imidazo[4,5-b]pyrazin-2-one (Intermediate 5)

A solution of 5-bromanyl-1,3-dihydroimidazo[4,5-*b*]pyrazin-2-one (1.0 g, 4.65 mmol) in 1,4-dioxane (47 mL) was degassed with N₂ for 5 min, then Pd₂(dba)₃ (0.21 g, 0.23 mmol), Xantphos (0.27 g, 0.47 mmol), 2-(trifluoromethyl)pyridine-3-thiol (1.19 g, 5.12 mmol) and DIPEA (1.62 mL, 9.3 mmol) were added sequentially. The reaction mixture was degassed with N₂ for further 5 min and heated to 100 °C for 2 h. After cooling, the mixture was concentrated *in vacuo* and the residue was treated with MeCN affording a precipitate which was filtered off to give the title compound as a beige solid (1.33 g, 91%). ¹H NMR (DMSO-*d*₆) δ 11.96 (br s, 2H), 8.56 (d, *J* = 3.7 Hz, 1H), 8.10 (s, 1H), 7.75 (d, *J* = 8.3 Hz, 1H), 7.58 (dd, *J* = 8.2 and 4.5 Hz, 1H). LCMS (ES⁺) *m*/*z* 314 (M+H)⁺; RT 1.24 min.

### Step 3: 2-Chloro-6-[(2-(trifluoromethyl)pyridin-3-yl)thio]-1H-imidazo[4,5-b]pyrazine

A solution of 5-[2-(trifluoromethyl)pyridin-3-yl]sulfanyl-1,3-dihydroimidazo[4,5-*b*]pyrazin-2-one (**Intermediate 5;** 1.3 g, 4.15 mmol) in POCl₃ (20 mL, 0.21 mol) was heated to 120 °C in a sealed vial for 8 h. After cooling, the mixture was concentrated *in vacuo* to give a residue that was purified by RP chromatography (gradient elution 0-100% MeCN (+ 0.1% TFA)/H₂O (+ 0.1% TFA)) to furnish the title compound as a pale yellow solid (0.26 g, 19%). ¹H NMR (DMSO-*d*₆) δ 8.71 (d, *J* = 3.9 Hz, 1H), 8.55 (s, 1H), 8.05 (d, *J* = 7.9 Hz, 1H), 7.70 (dd, *J* = 8.1 and 4.6 Hz, 1H). LCMS (ES⁺) *m*/*z* 332 (M+H)⁺; RT 1.4 min.

### Step 4: (S)-1'-(5-[(2-(Trifluoromethyl)pyridin-3-yl)thio]-1H-imidazo[4,5-b]pyrazin-2-yl)-1,3-dihydrospiro[indene-2,4'-piperidin]-1-amine (trifluoroacetate salt)

A solution of 2-chloro-6-[(2-(trifluoromethyl)pyridin-3-yl)thio]-1*H*-imidazo[4,5-*b*]pyrazine (50 mg, 0.15 mmol), 2-methyl-*N*-[(1*S*)-spiro[1,3-dihydroindene-2,4'-piperidine]-1-yl]propane-2-sulfinamide (prepared according to the procedure described in WO2018172984) (132 mg, 0.30 mmol) and DIPEA (0.11 mL, 0.60 mmol) in 1,4-dioxane (1.5 mL) was heated in a sealed vial to 100 °C for 4 h. After cooling, the mixture was concentrated *in vacuo* to give a residue that was dissolved with MeOH (8.5 mL) and treated with HCl (5 mL, 4.0 mmol, 1.25 M in MeOH). The mixture was stirred at rt for 24 h, then concentrated *in vacuo* to give a residue that was purified by RP-HPLC using H₂O (+ 0.1% TFA) and MeCN (+ 0.1% TFA) as eluents (C₁₈ column). Lyophilization of the appropriate fractions afforded the title compound as a white powder (28 mg, 30%). ¹H NMR (DMSO-*d*₆) δ 8.57 (d, *J* = 4.4 Hz, 1H), 8.24 (br s, 3H), 8.14 (br s, 1H), 7.74 (d, *J* = 8.1 Hz, 1H), 7.59 (dd, *J* = 8.2 and 4.5 Hz, 1H), 7.50 (d, *J* = 7.2 Hz, 1H), 7.40 - 7.30 (m, 3H), 4.42 (d, *J* = 4.6 Hz, 1H), 4.17 (dd, *J* = 33.1 and 14.0 Hz, 2H), 3.41 (t, *J* = 12.2 Hz, 2H), 3.19 (d, J = 16.4 Hz, 1H), 3.01 (d, *J* = 16.2 Hz, 1H), 1.86 - 1.68 (m, 2H), 1.55 (t, *J* = 12.3 Hz, 2H). LCMS (ES⁺) *m*/*z* 498 (M+H)⁺; RT 1.18 min.

The following examples were synthesized using the above procedure (Scheme 6), with the corresponding starting materials: 2, 3, 5, 7, 8, 14-17, 31-35, 40-44, 50, 51, 53-56, 79, 82-87, 89, 90, 96, 108-113, 123-128, 139, 142, 143, 150.

### Example 118: (S)-1'-(6-Chloro-5-[(2-(trifluoromethyl)pyridin-3-yl)thio]-1H-imidazo[4,5-b]pyrazin-2-yl)-1,3-dihydrospiro[indene-2,4'-piperidin]-1-amine

The compound was prepared according to Scheme 7, following the procedures reported below.

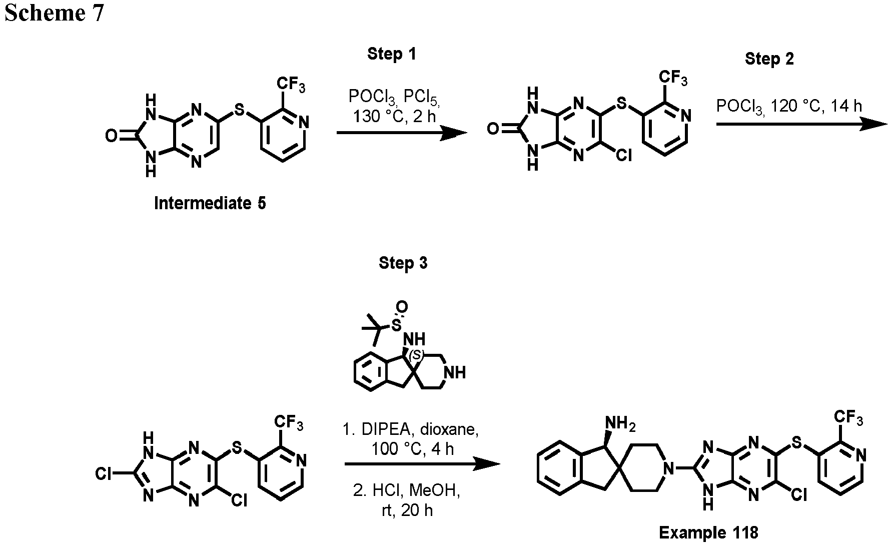

### Step 1: 5-Chloro-6-[(2-(trifluoromethyl)pyridin-3-yl)thio]-1,3-dihydro-2H-imidazo[4,5-b]pyrazin-2-one

A microwave vial was charged with POCl₃ (1.79 mL, 19.15 mmol), PCl₅ (425 mg, 2.04 mmol) and 5-[2-(trifluoromethyl)pyridin-3-yl]sulfanyl-1,3-dihydroimidazo[4,5-*b*]pyrazin-2-one (**Intermediate 5;** 400 mg, 1.28 mmol, prepared as in Step 2 of Scheme 6). The vial was sealed and heated to 130 °C for 2 h. After cooling, the mixture was diluted with MeCN and the precipitate was filtered off to give the title compound as a yellow solid (247 mg, 50%). ¹H NMR (DMSO-*d*₆) δ 12.15 (s, 1H), 11.96 (s, 1H), 8.67 (d, *J* = 4.7 Hz, 1H), 7.96 (d, *J* = 7.9 Hz, 1H), 7.68 (dd, *J* = 8.1 and 4.6 Hz, 1H). LCMS (ES⁺) *m*/*z* 348, 350 (M+H)⁺; RT 1.48 min.

### Step 2: 2,5-Dichloro-6-[(2-(trifluoromethyl)pyridin-3-yl)thio]-1H-imidazo[4,5-b]pyrazine

A solution of 5-chloro-6-[(2-(trifluoromethyl)pyridin-3-yl)thio]-1,3-dihydro-2*H*-imidazo[4,5-*b*]pyrazin-2-one (83 mg, 0.22 mmol) in POCl₃ (1 mL, 10.74 mmol) was heated at 120 °C in a sealed vial for 14 h. After cooling, the mixture was concentrated *in vacuo* to give a residue that was purified by RP chromatography (gradient elution 0-100% MeCN (+ 0.1% TFA)/H₂O (+ 0.1% TFA)) to furnish the title compound as a pale yellow solid (21.3 mg, 27%). ¹H NMR (DMSO-*d*₆) δ 8.84 (d, *J* = 4.4 Hz, 1H), 8.27 (d, *J* = 7.9 Hz, 1H), 7.84 (dd, *J* = 7.9 and 4.6 Hz, 1H). LCMS (ES⁺) *m*/*z* 366, 368 (M+H)⁺; RT 1.69 min.

### Step 3: (S)-1'-(6-Chloro-5-[(2-(trifluoromethyl)pyridin-3-yl)thio]-1H-imidazo[4,5-b]pyrazin-2-yl)-1,3-dihydrospiro[indene-2,4'-piperidin]-1-amine (trifluoroacetate salt)

A solution of 2,5-dichloro-6-((2-(trifluoromethyl)pyridin-3-yl)thio)-1*H*-imidazo[4,5-*b*]pyrazine (10 mg, 0.03 mmol), 2-methyl-*N*-[(1*S*)-spiro[1,3-dihydroindene-2,4'-piperidine]-1-yl]propane-2-sulfinamide (17 mg, 0.05 mmol) and DIPEA (0.019 mL, 0.11 mmol) in 1,4-dioxane (0.3 mL) was heated in a sealed vial to 100 °C for 4 h. After cooling, the mixture was concentrated *in vacuo* and the residue was dissolved in MeOH (1.6 mL) and treated with HCl (1 mL, 0.5 mmol, 1.25 M in MeOH) at rt for 20 h. The mixture was concentrated *in vacuo* to give a residue that was purified by RP-HPLC using H₂O (+ 0.1% TFA) and MeCN (+ 0.1% TFA) as eluents (C₁₈ column). Lyophilization of the appropriate fractions afforded the title compound as a white powder (8.3 mg, 40%). ¹H NMR (DMSO-*d*₆) δ 8.71 (d, *J* = 4.4 Hz, 1H), 8.23 (br s, 3H), 7.99 (d, *J* = 7.9 Hz, 1H), 7.71 (dd, *J* = 7.9 and 4.6 Hz, 1H), 7.49 (d, *J* = 7.4 Hz, 1H), 7.40 - 7.28 (m, 3H), 4.45 - 4.37 (m, 1H), 4.17 - 4.05 (m, 2H), 4.08 (d, *J* = 12.7 Hz, 1H), 3.39 (t, *J* = 12.5 Hz, 2H), 3.18 (d, *J* = 16.4 Hz, 1H), 3.00 (d, *J* = 16.4 Hz, 1H), 1.84 - 1.67 (m, 2H), 1.54 (t, *J* = 11.3 Hz, 2H). LCMS (ES⁺) *m*/*z* 532, 534 (M+H)⁺; RT 1.35 min.

### Example 119: 4-Methyl-1-(5-[(2-(trifluoromethyl)pyridin-3-yl)thio]thiazolo[4,5-b]pyrazin-2-yl)piperidin-4-amine (trifluoroacetate salt)

The compound was prepared according to Scheme 8, following the procedures reported below.

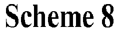

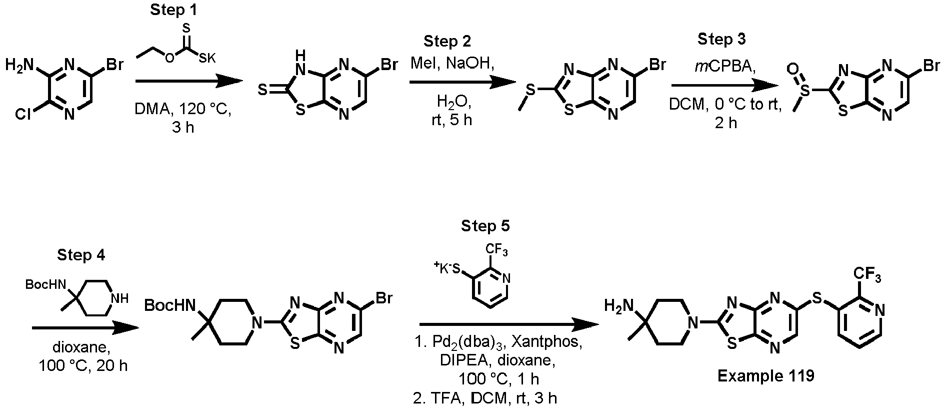

### Step 1: 5-Bromothiazolo[4,5-b]pyrazine-2(3H)-thione

A solution of 6-bromo-3-chloropyrazin-2-amine (1.0 g, 4.80 mmol) and ethoxycarbothioylsulfanylpotassium (1.15 g, 7.20 mmol) in DMA (8.6 mL) was heated to 120 °C for 3 h. After cooling, the mixture was treated with AcOH (2 mL) and H₂O (50 mL) affording a precipitate which was filtered off to give the title compound as a yellow solid (0.64 g, 54%). ¹H NMR (DMSO-*d*₆) δ 8.58 (1 H, s). LCMS (ES⁻) *m*/*z* 246, 248 (M-H)⁻; RT 1.40 min.

### Step 2: 5-Bromo-2-(methylthio)thiazolo[4,5-b]pyrazine

A solution of 5-bromothiazolo[4,5-b]pyrazine-2(3H)-thione (320 mg, 1.29 mmol) in NaOH (79 mg, 1.93 mmol)/H₂O (9.1 mL) was treated with iodomethane (0.12 mL, 1.93 mmol). The reaction mixture was stirred at rt for 5 h, observing the formation of precipitate, which was filtered off and washed with H₂O and co-evaporated *in vacuo* with MeCN to give the title compound as a yellow solid (299 mg, 88%) which was used in the next step without further purification. ¹H NMR (DMSO-*d*₆) δ 8.73 (s, 1H), 2.86 (s, 3H). LCMS (ES⁺) *m*/*z* 262, 264 (M+H)⁺; RT 1.66 min.

### Step 3: 5-Bromo-2-(methylsulfinyl)thiazolo[4,5-b]pyrazine

A solution of 5-bromo-2-(methylthio)thiazolo[4,5-b]pyrazine (290 mg, 1.11 mmol) in DCM (11 mL) at 0 °C was treated with mCPBA (600 mg, 2.43 mmol). The reaction mixture was stirred at rt for 2 h, then diluted with DCM and the organic phase was washed with NaHCO₃ sat. sol., brine, dried and concentrated *in vacuo* to give the title compound as a yellow solid (299 mg, 75%) which was used in the next step without further purification. ¹H NMR (DMSO-*d*₆) δ 9.17 (s, 1H), 3.67 (s, 3H). LCMS (ES⁺) *m*/*z* 278, 280 (M+H)⁺; RT 1.29 min.

### Step 4: tert-Butyl [1-(5-bromothiazolo[4,5-b]pyrazin-2-yl)-4-methylpiperidin-4-yl]carbamate

A solution of 5-bromo-2-(methylsulfinyl)thiazolo[4,5-b]pyrazine (116 mg, 0.42 mmol) and *tert-*butyl *N*-(4-methylpiperidin-4-yl)carbamate (107 mg, 0.5 mmol) in 1,4-dioxane (2.3 mL) was heated to 100 °C for 20 h. After cooling, the mixture was concentrated *in vacuo* and the residue was purified on silica gel (eluting with 0-50% EtOAc/Petroleum ether) to give the title compound as a yellow solid (170 mg, 95%). ¹H NMR (DMSO-*d*₆) δ 8.23 (s, 1H), 3.55-3.42 (m, 4H), 2.28 - 2.12 (m, 2H), 1.60 - 1.46 (m, 2H), 1.40 (s, 9H), 1.27 (s, 3H). LCMS (ES⁺) *m*/*z* 428, 430 (M+H)⁺; RT 2.06 min.

### Step 5: 4-Methyl-1-(5-[(2-(trifluoromethyl)pyridin-3-yl)thio]thiazolo[4,5-b]pyrazin-2-yl)piperidin-4-amine (trifluoroacetate salt)

A microwave vial was charged with a solution of *tert*-butyl [1-(5-bromothiazolo[4,5-b]pyrazin-2-yl)-4-methylpiperidin-4-yl]carbamate (30 mg, 0.06 mmol) in 1,4-dioxane (1 mL) and degassed with N₂ for 5 min. Then Pd₂(dba)₃ (2.8 mg, 0.003 mmol), Xantphos (3.6 mg, 0.006 mmol), [potassium 2-(trifluoromethyl)pyridine-3-thiolate (15 mg, 0.07 mmol) and DIPEA (20 µL, 0.12 mmol) were added sequentially. The reaction mixture was degassed with N₂ for further 5 min, then the vial was sealed and heated at 100 °C for 1 h. After cooling, the mixture was diluted with EtOAc and the organic phase was washed with H₂O, NaHCO₃ sat. sol. and brine. Aqueous phases were back extracted with EtOAc and the combined organic phases were dried and concentrated *in vacuo* to give a residue that was dissolved with DCM (0.9 mL) cooled to 0 °C and treated with TFA (0.1 mL, 1.3 mmol). The reaction mixture was stirred at rt for 3 h and concentrated *in vacuo* to give a residue that was purified by RP-HPLC using H₂O (+ 0.1% TFA) and MeCN (+ 0.1% TFA) as eluents (C₁₈ column). Lyophilization of the appropriate fractions afforded the title compound as a white powder (23 mg, 69%). ¹H NMR (DMSO-*d*₆) δ 8.76 (d, *J* = 3.7 Hz, 1H), 8.21 (s, 1H), 8.15 (d, *J* = 7.9 Hz, 1H), 8.03 (br s, 3H), 7.76 (dd, *J* = 8.1 and 4.6 Hz, 1H), 4.09-3.81 (m, 2H), 3.67 - 3.46 (m, 4H), 1.88 - 1.75 (m, 4H), 1.39 (s, 3H). LCMS (ES⁺) *m*/*z* 427 (M+H)⁺; RT 1.25 min.

### Example 120: (R)-8-(5-(3,4-dihydro-1,5-naphthyridin-1(2H)-yl)-1H-imidazo[4,5-b]pyrazin-2-yl)-8-azaspiro[4.5]decan-1-amine

The compound was prepared according to Scheme 9, following the procedures reported below.

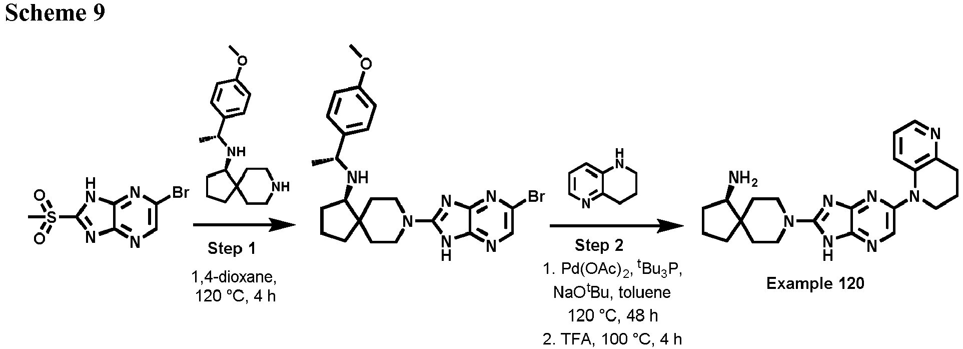

### Step 1: (R)-8-(5-bromo-1H-imidazo[4,5-b]pyrazin-2-yl)-N-((R)-1-(4-methoxyphenyl)ethyl)-8-azaspiro[4.5]decan-1-amine

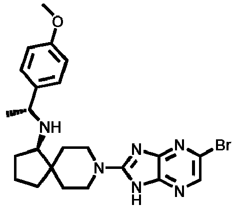

A solution of 5-bromo-2-(methylthio)-1*H*-imidazo[4,5-b]pyrazine (300 mg, 1.08 mmol) (prepared as described in Example 22, Scheme 1, Step 3) and (4*R*)-*N*-[(1*R*)-1-(4-methoxyphenyl)ethyl]-8-azaspiro[4.5]decan-4-amine (prepared according to the procedure described in WO2017/216706; 374 mg, 1.3 mmol) in 1,4-Dioxane (6.0 mL, 0.18 M) was heated at 120 °C for 4 h, then concentrated under reduced pressure and purified by flash chromatography [gradient elution 0-50% MeCN (+ 0.1% TFA) in water (+ 0.1% TFA)]. The title compound was obtained as yellow solid (330 mg, 63%). ¹H NMR (400 MHz, DMSO-*d₆*) δ 8.62 (br s, 1H), 8.02 (br s, 1H), 7.93 (s, 1H), 7.46 (d, *J* = 8.8 Hz, 2H), 7.00 (d, *J* = 8.8 Hz, 2H), 4.42 - 4.32 (m, 1H), 4.24 (br t, *J* = 12.9 Hz, 2H), 3.77 (s, 3H), 3.26 - 3.08 (m, 3H), 2.08 - 1.89 (m, 2H), 1.77 - 1.66 (m, 2H), 1.64 - 1.47 (m, 8H), 1.38 (br d, *J* = 12.7 Hz, 1H). LCMS (ES⁺) *m*/*z* 485 (M+H)⁺; RT 1.2 min;

### Step 2: (R)-8-(5-(3,4-dihydro-1,5-naphthyridin-1(2H)-yl)-1H-imidazo[4,5-b]pyrazin-2-yl)-8-azaspiro[4.5]decan-1-amine

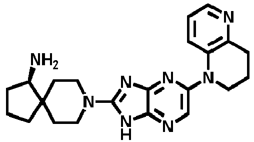

A 0.5 - 2 mL MW vial was charged with a solution of (R)-8-(5-bromo-1H-imidazo[4,5-b]pyrazin-2-yl)-N-((R)-1-(4-methoxyphenyl)ethyl)-8-azaspiro[4.5]decan-1-amine (30 mg, 0.06 mmol) in toluene (1 mL, 0.06 M). The reaction mixture was degassed with N₂ for 5 min, then Pd(OAc)₂ (1.4 mg, 0.01 mmol) and tri-*tert*-butylphosphine (1.2 mg, 0.010 mmol) followed by 1,2,3,4-tetrahydro-1,5-naphthyridine (16.6 mg, 0.12 mmol) and sodium tert-butoxide (20.8 mg, 0.22 mmol) were added. The reaction mixture was degassed with N₂ for further 5 min and heated at 120 °C for 24 h. All reagents were added again, the reaction mixture was degassed with N₂ and heated for further 24 h. Then it was cooled to rt, diluted with EtOAc and filtered through a pad of Solka Floc (washing with MeOH). The filtrate was concentrated to dryness. The crude intermediate was dissolved with TFA and heated at 100 °C for 4 h. TFA was removed under reduced pressure and the crude product was purified by preparative HPLC to give the title compound as a yellow solid (4 mg, 10%). ¹H NMR (400 MHz, DMSO-*d₆*) δ 8.24 (d, *J* = 4.8 Hz, 1H), 8.19 (s, 1H), 8.01 (d, *J* = 8.8 Hz, 1H), 7.86 (br s, 3H), 7.60 (dd, *J* = 8.8, 5.5 Hz, 1H), 4.11 - 3.97 (m, 2H), 3.89 - 3.78 (m, 2H), 3.54 - 3.42 (m, 2H), 3.27 - 3.21 (m, 1H), 3.18 (br t, *J* = 6.4 Hz, 2H), 2.17 - 2.05 (m, 3H), 1.87 - 1.74 (m, 3H), 1.74 - 1.61 (m, 4H), 1.60 - 1.46 (m, 2H). LCMS (ES⁺) *m*/*z* 405 (M+H)⁺; RT 0.4 min.

### Example 121: tert-Butyl (1-[5-((2,3-dichlorophenyl)amino)-1H-imidazo[4,5-b]pyrazin-2-yl)-4-methylpiperidin-4-yl]carbamate (trifluoroacetate salt)

The compound was prepared according to the Scheme 10, following the procedures below.

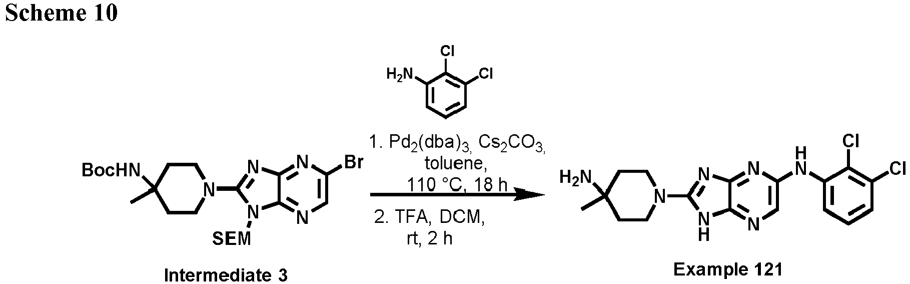

A solution of 2,3-dichloroaniline (7.0 mg, 0.04 mmol) in toluene (0.4 mL) at rt was treated with Pd₂(dba)₃ (1.3 mg, 0.001 mmol), BINAP (1.8 mg, 0.003 mmol), Cs₂CO₃ (19.5 mg, 0.06 mmol) and tert-butyl (1-(5-bromo-1-((2-(trimethylsilyl)ethoxy)methyl)-1*H*-imidazo[4,5-*b*]pyrazin-2-yl)-4-methylpiperidin-4-yl)carbamate (**Intermediate 3;** 16 mg, 0.03 mmol, prepared as in Step 5 of Scheme 1). The reaction mixture was degassed with N₂ for 5 min and heated to 110 °C for 18 h. After cooling, the mixture was concentrated *in vacuo,* diluted with DCM (1 mL) and NH₄Cl sat. sol. (0.5 mL) and stirred vigorously for 10 min. The organic phase was separated and treated with TFA (226 µL, 2.95 mmol) at rt for 2 h. The mixture was concentrated *in vacuo* to give a residue that was purified by RP-HPLC using H₂O (+ 0.1% TFA) and MeCN (+ 0.1% TFA) as eluents (C₁₈ column). Lyophilization of the appropriate fractions afforded the title compound as a white solid (4.5 mg, 49%). ¹H NMR (DMSO-*d*₆) δ 8.42 (s, 1H), 7.94 (br s, 3H), 7.86 - 7.84 (m, 2H), 7.27 - 7.24 (m, 1H), 7.18 - 7.16 (m, 1H), 3.98 - 3.95 (m, 2H), 3.46 - 3.39 (m, 2H), 1.76 - 1.74 (m, 4H), 1.38 (s, 3H). LCMS (ES⁺) *m*/*z* 392 (M+H)⁺, 394 (M+Na)⁺, RT 1.01 min.

### Example 122: tert-Butyl [1-(5-(2,3-dichlorophenoxy)-1H-imidazo[4,5-b]pyrazin-2-yl)-4-methylpiperidin-4-yl]carbamate (trifluoroacetate salt)

The compound was prepared according to Scheme 11, following the procedure below.

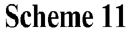

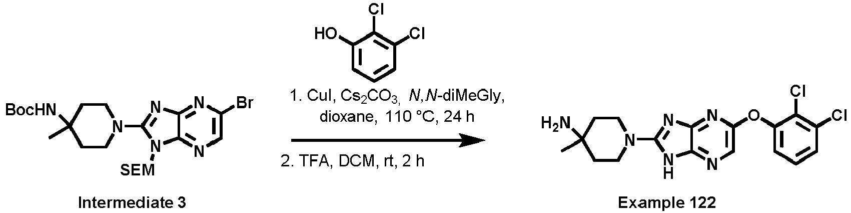

A solution of 2,3-dichlorophenol (6.8 mg, 0.04 mmol) in 1,4-dioxane (0.4 mL) was treated with N,N-dimethylglycine (0.5 mg, 0.003 mmol), CuI (0.5 mg, 0.003 mmol), *tert*-butyl [1-(5-bromo-1-((2-(trimethylsilyl)ethoxy)methyl)-1*H*-imidazo[4,5-*b*]pyrazin-2-yl]-4-methylpiperidin-4-yl)carbamate (**Intermediate 3;** 5 mg, 0.03 mmol, prepared as in Step 5 of Scheme 1) and Cs₂CO₃ (18 mg, 0.06 mmol). The reaction mixture was heated at 1 10 °C for 24 h. After cooling, the mixture was concentrated *in vacuo,* diluted with DCM (1 mL) and NH₄Cl sat. sol. (0.5 mL) and stirred vigorously for 10 minutes. The organic phase was separated, treated with TFA (226 µL, 2.95 mmol) and stirred for 2 h at rt. Then, the mixture was concentrated *in vacuo* to give a residue that was purified by RP-HPLC using H₂O (+ 0.1% TFA) and MeCN (+ 0.1% TFA) as eluents (C₁₈ column). Lyophilization of the appropriate fractions afforded the title compound as a yellow solid (6.5 mg, 60%). ¹H NMR (DMSO-*d*₆) δ 7.92 (br s, 3H), 7.83 (br s, 1H), 7.53 (dd, *J* = 8.1 Hz, and *J* = 1.3 Hz, 1H), 7.41 (t, *J* = 8.2 Hz, 1H), 7.22 (d, *J* = 7.0 Hz, 1H), 3.98 - 3.96 (m, 2H), 3.44 - 3.41 (m, 2H), 1.79 - 1.75 (m, 4H), 1.38 (s, 3H). LCMS (ES⁺) *m*/*z* 393 (M+H)⁺, RT 1.22 min.

### Example 131: 1-[5-(2,3-Dichlorophenyl)-1H-imidazo[4,5-b]pyrazin-2-yl]-4-methylpiperidin-4-amine (trifluoroacetate salt)

The compound was prepared according to Scheme 12, following the procedure below.

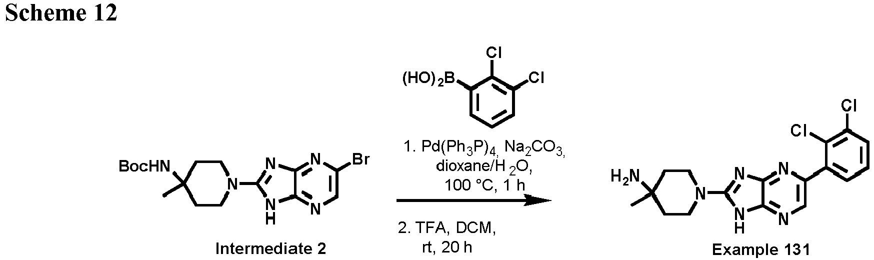

A microwave vial was charged with a solution of *tert*-butyl *N*-[1-(5-bromanyl-3*H*-imidazo[4,5-*b*]pyrazin-2-yl)-4-methyl-piperidin-4-yl]carbamate (**Intermediate 2,** prepared as in Step 4 of Scheme 1; 50 mg, 0.12 mmol), (2,3-dichlorophenyl)boronic acid (25 mg, 0.13 mmol) and Na₂CO₃ (39 mg, 0.36 mmol) in 1,4-dioxane/water (5:1; 1.2 mL) and degassed with N₂ for 5 min. Pd(PPh₃)₄ (42 mg, 0.04 mmol) was added and the reaction mixture was degassed with N₂ for further 5 min, then the vial was sealed and heated at 100 °C for 1 h. After cooling, the mixture was diluted with EtOAc and concentrated *in vacuo* to give a residue that was purified on silica gel (eluting with 10-100% EtOAc/Petroleum ether) to give the desired intermediate as a yellow solid (30 mg, 26%). This residue was dissolved with DCM (2 mL) at 0 °C and treated with TFA (0.2 mL, 2.64 mmol). The reaction mixture was stirred at rt for 20 h, then concentrated *in vacuo* to give a residue that was purified by preparative HPLC to give the title compound as a white powder (1.4 mg, 2%). ¹H NMR (DMSO-*d*₆) δ 12.57 (br s, 1H), 8.13 (br s, 1H), 7.94 (br s, 3H), 7.71 (dd, *J* = 7.9 and 1.7 Hz, 1H), 7.56 - 7.44 (m, 2H), 4.12 - 3.97 (m, 2H), 3.63 - 3.45 (m, 2H), 1.84 - 1.74 (m, 4H), 1.40 (s, 3H). LCMS (ES⁺) *m*/*z* 375, 376, 377 (M+H)⁺; RT 1.08 min.

The procedure according to Scheme 12 was used for the synthesis of Example 140, by using (3-chloro-2-(methylamino)pyridin-4-yl)boronic acid instead of (2,3-dichlorophenyl)boronic acid.

### Example 133: 1-(5-((2,3-dichlorophenyl)thio)-6-methyl-1H-imidazo[4,5-b]pyrazin-2-yl)-4-methylpiperidin-4-amine

The compound was prepared according to the Scheme 13, following the procedure below.

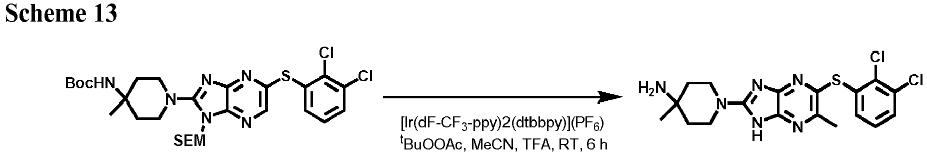

To a 4 mL glass vial equipped with a pressure release septa was added *tert*-butyl (1-(5-((2,3-dichlorophenyl)thio)-1-((2-(trimethylsilyl)ethoxy)methyl)-1H-imidazo[4,5-b]pyrazin-2-yl)-4-methylpiperidin-4-yl)carbamate (25 mg, 0.040 mmol) (prepared as described in Scheme 1, Example 22 Step 7, excluding the TFA treatment to avoid removal of the SEM and Boc protecting groups), Ir catalyst (0.9 mg, 0.001 mmol) in MeCN / TFA (1/1 v/v, 0.4 mL, 0.1 M) followed by *tert*-butyl ethaneperoxoate (0.07 mL, 0.120 mmol). The reaction mixture was degassed with N₂ for 15 min then irradiated with a 36W Kessil blue LED lamp in presence of a fan for 6 h at rt. The reaction mixture was diluted with DMSO and filtered before purification by preparative HPLC. The title compound was obtained as yellow solid (0.6 mg, 3%). ¹H NMR (400 MHz, DMSO-*d₆*) δ 8.07 (br s, 3H), 7.60 (d, *J* = 7.9 Hz, 1H), 7.30 (t, *J* = 7.9 Hz, 1H), 7.19 (d, *J* = 8.1 Hz, 1H), 4.03 - 3.90 (m, 2 H), 3.63 - 3.51 (m, 2 H), 2.58 (s, 3H), 1.87 - 1.79 (m, 4H), 1.38 (s, 3H). LCMS (ES⁺) *m*/*z* 423 (M+H)⁺; RT 1.29 min.

### Example 137: (S)-2-(2-(1-amino-1,3-dihydrospiro[indene-2,4'-piperidin]-1'-yl)-5-((2-(trifluoromethyl)pyridin-3-yl)thio)-1H-imidazo[4,5-b]pyrazin-1-yl)acetamide (trifluoroacetate salt)

The compound was prepared according to Scheme 14, following the procedures below.

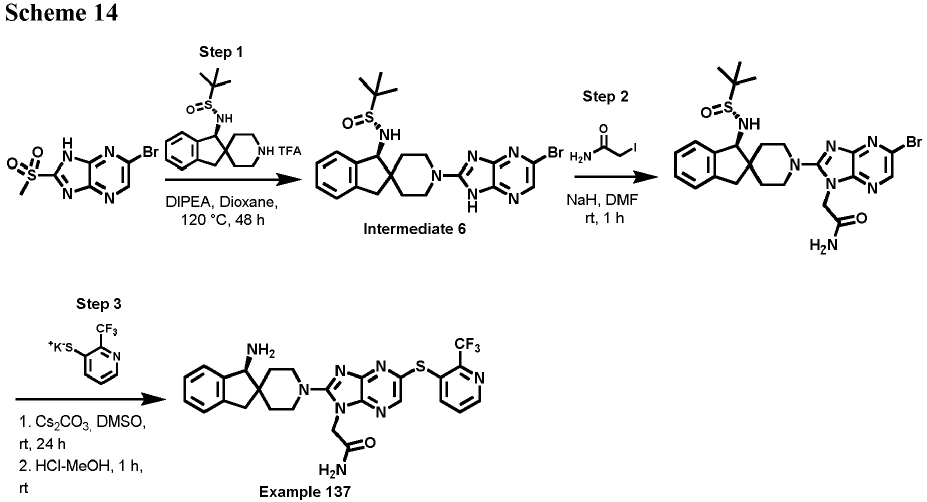

### Step 1: (R)-N((S)-1'(5-bromo-1H-imidazo[4,5-b]pyrazin-2-yl)-1,3-dihydrospiro[indene-2,4'-piperidin]-1-yl)-2-methylpropane-2-sulfinamide (Intermediate 6)

A solution of 2-methyl-N-[(1S)-spiro[1,3-dihydroindene-2,4'-piperidine]-1-yl]propane-2-sulfinamide; 2,2,2-tris(fluoranyl)ethanoic acid (650 mg, 1.08 mmol), DIPEA (0.79 mL, 4.51 mmol) and 6-bromo-2-(methylsulfonyl)-1H-imidazo[4,5-b]pyrazine (250 mg, 0.90 mmol, prepared as in Step 3 of Scheme 1) in 1,4-dioxane (9 mL) was heated at 120 °C for 48h. The mixture was concentrated under reduced pressure and the crude material was purified on silica gel (eluting with 10-100% EtOAc/Petroleum ether) to give the desired intermediate as a yellow solid (190 mg, 41%). ¹H NMR (DMSO-*d*₆) δ 12.43 (br s, 1H), 7.95 (s, 1H), 7.29 - 7.19 (m, 4H), 5.69 (d, *J* = 10.5 Hz, 1H), 4.45 (d, *J* = 10.5 Hz, 1H), 4.19 (br s, 2H), 3.38 - 3.15 (m, 4H), 2.08 (td, *J* = 12.8, 4.3 Hz, 1H), 1.80 - 1.59 (m, 2H), 1.38 - 1.33 (m, 1H), 1.20 (s, 9H). LCMS (ES⁺) *m*/*z* 503 (M+H)⁺; RT 1.62 min.

### Step 2: 2-(5-bromo-2-((S)-1-(((R)-tert-butylsulfinyl)amino)-1,3-dihydrospiro[indene-2,4'-piperidin]-1'-yl)-1H-imidazo[4,5-b]pyrazin-1-yl)acetamide

NaH (60% in mineral oil; 14 mg, 0.36 mmol) was put in a flask and DMF (1.8 mL, 0.023 mol) was added, then a solution of (R)-N-((S)-1'-(5-bromo-1H-imidazo[4,5-b]pyrazin-2-yl)-1,3-dihydrospiro[indene-2,4'-piperidin]-1-yl)-2-methylpropane-2-sulfinamide (90 mg, 0.180 mmol) in DMF (0.5 mL) was added dropwise at 0°C. The mixture was stirred for 15 min at rt. Then a solution of 2-iodoacetamide (66 mg, 0.360 mmol) in DMF (0.2 mL) was added dropwise at 0°C. After 1 h at rt the mixture was diluted with EtOAc and washed with H₂O. Organic phases were dried over Na₂SO₄, filtered and concentrated to dryness. The residue crude was purified on silica gel (eluting with 10-100% EtOAc + 5% MeOH/Petroleum ether) to give the desired intermediate as a yellow powder (57 mg, 56%). ¹H NMR (DMSO-*d*₆) δ 7.79 (br d, J = 7.0 Hz, 1H), 7.60 (s, 1H), 7.44 (br d, J = 7.0 Hz, 1H), 7.26 - 7.21 (m, 4H), 5.65 (br dd, J = 15.2, 10.4 Hz, 1H), 4.95 - 4.88 (m, 2H), 4.57 - 4.43 (m, 3H), 3.28 - 3.17 (m, 2H), 2.07 - 2.02 (m, 1H), 1.66 (br s, 2H), 1.34 - 1.36 (m, 2H), 1.27 - 1.12 (m, 10H). LCMS (ES⁺) *m*/*z* 561 (M+H)⁺; RT 1.29 min.

### Step 3: (S)-2-(2-(1-amino-1,3-dihydrospiro[indene-2,4'-piperidin]-1'-yl)-5-((2-(trifluoromethyl)pyridin-3-yl)thio)-1H-imidazo[4,5-b]pyrazin-1-yl)acetamide (trifluoroacetate salt)

A 4 mL vial was charged with 2-(5-bromo-2-((S)-1-(((R)-tert-butylsulfinyl)amino)-1,3-dihydrospiro[indene-2,4'-piperidin]-1'-yl)-1H-imidazo[4,5-b]pyrazin-1-yl)acetamide (57 mg, 0.100 mmol), potassium 2-(trifluoromethyl)pyridine-3-thiolate (44 mg, 0.20 mmol), Cs₂CO₃ (66 mg, 0.20 mmol) in DMSO (0.8 mL) and degassed with N₂ for 30 seconds. The reaction mixture was then irradiated with a blue LED Strip (λ 455 nm) for 24 h at rt then filtered on a SiO₂ cartridge (12 g) eluting with a mixture of DCM/EtOAc (1:9). The organic solvent was evaporated and the residue was dissolved in MeOH (0.5 mL) and a solution of HCl in MeOH (0.5 mL, 0.5 M) was added. The mixture was stirred at rt for 1 h then concentrated in vacuo to give a residue that was purified by prep HPLC to give the *title* compound as a yellow powder (0.6 mg, 3%). LCMS (ES⁺) *m*/*z* 555 (M+H)⁺; RT 0.95 min.

### Example 138: 2-(2-(4-amino-4-methylpiperidin-1-yl)-5-((2-(trifluoromethyl)pyridin-3-yl)thio)-1H-imidazo[4,5-b]pyrazin-1-yl)ethan-1-ol

The compound was prepared according to Scheme 15, following the procedures indicated below.

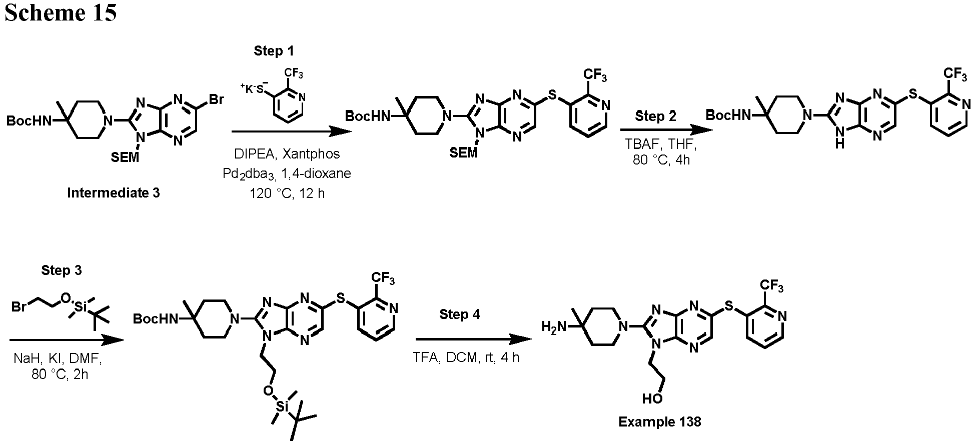

### Step 1: tert-Butyl (4-methyl-1-(5-((2-(trifluoromethyl)pyridin-3-yl)thio)-1-((2-(trimethylsilyl)ethoxy)methyl)-1H-imidazo[4,5-b]pyrazin-2-yl)piperidin-4-yl)carbamate

A microwave vial was charged with a solution of tert-butyl (1-(5-bromo-1-((2-(trimethylsilyl)ethoxy)methyl)-1H-imidazo[4,5-b]pyrazin-2-yl)-4-methylpiperidin-4-yl)carbamate (**Intermediate 3;** 180 mg, 0.32 mmol, prepared as in Step 5 of Scheme 1), Pd₂(dba)₃ (10 mg, 0.02 mmol), Xantphos (20 mg, 0.03 mmol), potassium 2-(trifluoromethyl)pyridine-3-thiolate (80 mg, 0.36 mmol) and DIPEA (0.11 mL, 0.65 mmol) in 1,4-Dioxane (3.7 mL). The mixture was heated at 120 °C for 12 h. After cooling, the mixture was concentrated to dryness. The residue was purified on silica gel (eluting with 10-70% EtOAc/petroleum ether) to give the desired intermediate as a red solid (150 mg, 72%). ¹H NMR (DMSO-*d*₆) δ 8.73 - 8.72 (m, 0.5H), 8.62 - 8.61 (m, 0.5H), 8.56 - 8.55 (m, 0.5H), 8.34 (s, 0.5H), 8.12 (s, 0.5H), 7.86 - 7.83 (m, 1H), 7.83 - 7.70 (m, 1H), 7.64 - 7.61 (m, 1H), 7.55 - 7.52 (m, 1H), 6.70 (bs, 1H), 5.41 (s, 1H), 5.30 (s, 1H), 4.04 - 3.96 (m, 2H), 3.75 (t, *J* = 7.89 Hz, 1H), 3.58 (t, *J* = 7.89 Hz, 1H), 3.52 - 3.44 (m, 2H), 2.18 - 2.15 (m, 2H), 1.59 - 1.53 (m, 2H), 1.40 (s, 8.5), 1.26 (d, *J* = 3.29 Hz, 3H) 1.11 - 1.07 (m, 1H), 0.96 (t, *J* = 7.89 Hz, 1H), 0.84 (t, *J* = 7.89 Hz, 1H), -0.04 - 0.04 (m, 4.5H), -0.10 (s, 4H). LCMS (ES⁺) *m*/*z* 640 (M+H)⁺; RT 2.62, 2.73 min (mixture of regioisomers).

### Step 2: tert-butyl (4-methyl-1-(5-((2-(trifluoromethyl)pyridin-3-yl)thio)-1H-imidazo[4,5-b]pyrazin-2-yl)piperidin-4-yl)carbamate

tert-butyl (4-methyl-1-(5-((2-(trifluoromethyl)pyridin-3-yl)thio)-1-((2-(trimethylsilyl)ethoxy)methyl)-1H-imidazo[4,5-b]pyrazin-2-yl)piperidin-4-yl)carbamate (150 mg, 0.230 mmol) was dissolved in THF (1.2 mL) and treated with TBAF (0.47 mL, 0.470 mmol) at reflux for 4 h. After cooling, the mixture was diluted in DCM and washed with H₂O. Organic phases were dried over Na₂SO₄, filtered and concentrated to dryness. The residue crude was purified on silica gel (eluting with 10-100% EtOAc/Petroleum ether) to give the desired intermediate as a yellow oil (57 mg, 48%). ¹H NMR (DMSO-*d*₆) δ 12.48 (br s, 1H), 8.55 (d, *J* = 4.4 Hz, 1H), 8.10 (br s, 1H), 7.69 (br d, *J* = 8.1 Hz, 1H), 7.58 - 7.55 (m, 1H), 6.67 (br s, 1H), 3.89 - 3.86 (m, 2H), 3.40 - 3.37 (m, 2H), 2.13 (br d, *J* = 12.9 Hz, 2H), 1.51 - 1.47 (m, 2H), 1.39 (s, 9H), 1.25 (s, 3H). LCMS (ES⁺) *m*/*z* 510 (M+H)⁺; RT 1.75 min.

### Step 3: tert-butyl (1-(1-(2-((tert-butyldimethylsilyl)oxy)ethyl)-5-((2-(trifluoromethyl)pyridin-3-yl)thio)-1H-imidazo[4,5-b]pyrazin-2-yl)-4-methylpiperidin-4-yl)carbamate

tert-butyl N-[4-methyl-1-[5-[2-(trifluoromethyl)pyridin-3-yl]sulfanyl-1H-imidazo[4,5-b]pyrazin-2-yl]piperidin-4-yl]carbamate (57 mg, 0.110 mmol) was dissolved in DMF (1.1 mL) and NaH (60% in mineral oil; 5.4 mg, 0.130 mmol) was added at 0°C. The mixture was stirred for 15 min at rt and then 2-bromoethoxy-tert-butyl-dimethyl-silane (37 mg, 0.160 mmol), potassium iodide (18.57mg, 0.110 mmol) were added. The reaction mixture was heated at 80 °C for 2 h. After cooling, the mixture was diluted in DCM and washed with H₂O. Organic phases were dried over Na₂SO₄, filtered and concentrated to dryness. The residue crude was purified on silica gel (eluting with 10-100% EtOAc/Petroleum ether) to give the desired intermediate as a yellow solid (60 mg, 80%). LCMS (ES⁺) *m*/*z* 668 (M+H)⁺; RT 2.36 min.

### Step 4: 2-(2-(4-amino-4-methylpiperidin-1-yl)-5-((2-(trifluoromethyl)pyridin-3-yl)thio)-1H-imidazo[4,5-b]pyrazin-1-yl)ethan-1-ol (trifluoroacetate salt)

tert-butyl N-[1-[1-[2-[tert-butyl(dimethyl)silyl]oxyethyl]-5-[2-(trifluoromethyl)pyridin-3-yl]sulfanyl-imidazo[4,5-b]pyrazin-2-yl]-4-methyl-piperidin-4-yl]carbamate (60 mg, 0.090 mmol) was dissolved in DCM (0.5 mL) and treated with TFA (0.1 mL, 0.09 mmol) for 4 h at rt. The mixture was concentrated to dryness and the residue crude was purified by preparative HPLC to give the title compound as a yellow powder (11 mg, 27%). ¹H NMR (DMSO-*d*₆) δ 8.62 (d, *J* = 4.6 Hz, 1H), 8.44 (s, 1H), 8.15 (br s, 3H), 7.90 (d, *J* = 8.1 Hz, 1H), 7.63 (dd, *J* = 8.1, 4.6 Hz, 1H), 4.47 (br t, *J* = 5.0 Hz, 2H), 4.31 (br d, *J* = 13.4 Hz, 1H), 4.00 (br d, *J* = 13.2 Hz, 1H), 3.94 - 3.90 (m, 1H), 3.73 (br d, *J* = 11.8 Hz, 2H), 1.93 - 1.81 (m, 4H), 1.76 (s, 1H), 1.41 (s, 3H). LCMS (ES⁺) *m*/*z* 454 (M+H)⁺; RT 0.82 min.

### Example 144: 1-(2-((2,3-Dichlorophenyl)thio)-7H-purin-8-yl)-4-methylpiperidin-4-amine (trifluoroacetate salt)

The compound was prepared according to Scheme 16, following the procedures reported below.

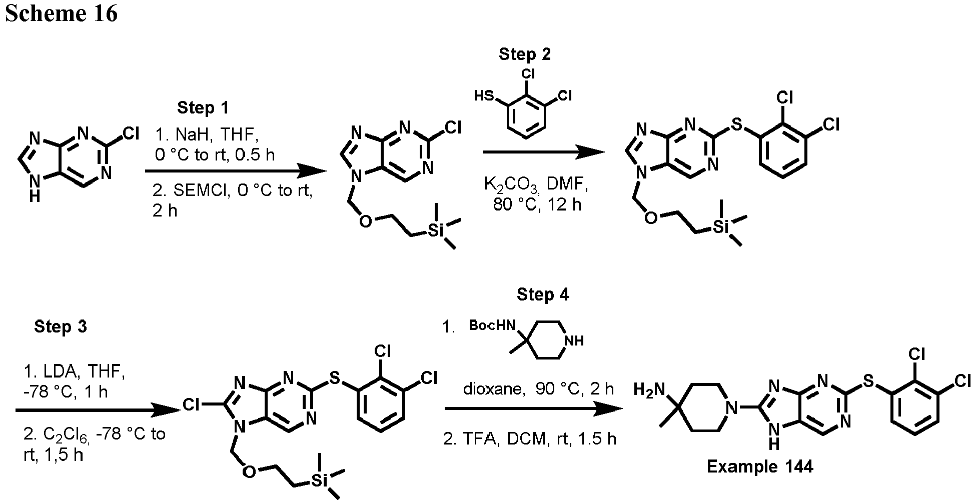

### Step 1: 2-Chloro-7-((2-(trimethylsilyl)ethoxy)methyl)-7H-purine

To a suspension of 2-chloro-*7H*-purine (1.0 g, 6.47 mmol) in THF was added 60% NaH (388 mg, 9.7 mmol) in mineral oil portion-wise (14 mL, 0.46 M) at 0 °C. The reaction mixture was stirred at rt for 30 min, then cooled to 0 °C and treated with 2-(chloromethoxy)ethyltrimethylsilane (1.53 mL, 7.76 mmol). The reaction mixture was stirred at rt for 2 h. After careful addition of NH₄Cl sat. sol. the mixture was extracted with EtOAc. The dried organics were concentrated *in vacuo* to give the title compound (mixture of regioisomers) as a pale orange oil (1.93 g, 99%) which was used in the next step without further purification. LCMS (ES⁺) *m*/*z* 285 (M+H)⁺; RT 1.73, 1.88 min.

### Step 2: 2-((2,3-Dichlorophenyl)thio)-7-((2-(trimethylsilyl)ethoxy)methyl)-7H-purine

A solution of 2-chloro-7-((2-(trimethylsilyl)ethoxy)methyl)-7*H*-purine (200 mg, 0.70 mmol) in DMF (1 mL, 0.70 M) was treated with potassium carbonate (107 mg, 0.77 mmol) and 2,3-dichlorobenzenethiol (140 mg, 0.77 mmol). The reaction mixture was stirred at 80 °C for 12 h. After cooling, NH₄Cl sat. sol. was added and the mixture extracted with EtOAc. The dried organics were concentrated *in vacuo.* The residue was purified on silica gel (eluting with 0-50% EtOAc/Petroleum ether) to give the title compound (mixture of regioisomers) as a yellow oil (145 mg, 48%). LCMS (ES⁺) *m*/*z* 427, 429 (M+H)⁺; RT 2.03, 2.15 min.

### Step 3: 8-Chloro-2-((2,3-dichlorophenyl)thio)-7-((2-(trimethylsilyl)ethoxy)methyl)-7H-purine

A solution of 2-((2,3-dichlorophenyl)thio)-7-((2-(trimethylsilyl)ethoxy)methyl)-7*H*-purine (30 mg, 0.07 mmol) in THF (1 mL, 0.07 M) was treated with (diisopropylamino)lithium (0.18 mL, 0.35 mmol) at -78 °C and stirred at this temperature for 1 h. 1,1,1,2,2,2-hexachloroethane (83 mg, 0.35 mmol) was added and the reaction mixture was stirred at -78 °C for 1 h, then at rt for 30 min. After addition of NH₄Cl sat. sol. the mixture was diluted with EtOAc. The organic phase was separated and washed with brine, dried and concentrated *in vacuo.* The residue was purified on silica gel (eluting with 0-50% EtOAc/Petroleum ether) to give the title compound as a yellow foam (21 mg, 65%). LCMS (ES⁺) *m*/*z* 461 (M+H)⁺; RT 2.32 min.

### Step 4: 1-(2-((2,3-Dichlorophenyl)thio)-7H-purin-8-yl)-4-methylpiperidin-4-amine (trifluoroacetate salt)

A solution of 8-chloro-2-((2,3-dichlorophenyl)thio)-7-((2-(trimethylsilyl)ethoxy)methyl)-7*H-*purine (21 mg, 0.05 mmol), *tert-butyl N*-(4-methylpiperidin-4-yl)carbamate (12 mg, 0.05 mmol) and DIPEA (0.02 mL, 0.14 mmol) in 1,4-dioxane (0.5 mL, 0.1 M) was heated at 90 °C for 2 h. After cooling, the mixture was concentrated *in vacuo* and the residue diluted with DCM (0.5 mL, 0.08 M) and TFA (0.1 mL). The mixture was stirred at room temperature for 90 min, then concentrated *in vacuo* and the residue purified by RP-HPLC using H₂O (+ 0.1% TFA) and MeCN (+ 0.1% TFA) as eluents (C₁₈ column). Lyophilization of the appropriate fractions afforded the title compound as a creamy powder (13 mg, 52% over two steps). ¹H NMR (DMSO-*d*₆) δ 8.28 (s, 1H), 7.95 (br s, 3H), 7.74 (d, *J* = 8.0 Hz, 2H), 7.43 (t, *J* = 8 Hz, 1H), 3.96-3.93 (m, 2H), 3.46-3.41 (m, 2H), 1.76-1.74 (m, 4H), 1.37 (s, 3H). LCMS (ES⁺) *m*/*z* 408 (M+H)⁺; RT 1.19 min.

### Example 148: (S)-1'-(5-((2-chloropyridin-3-yl)thio)-1H-imidazo[4,5-b]pyrazin-2-yl)-1,3-dihydrospiro[indene-2,4'-piperidin]-1-amine (trifluoroacetate salt)

The compound was prepared according to Scheme 17, following the procedures reported below.

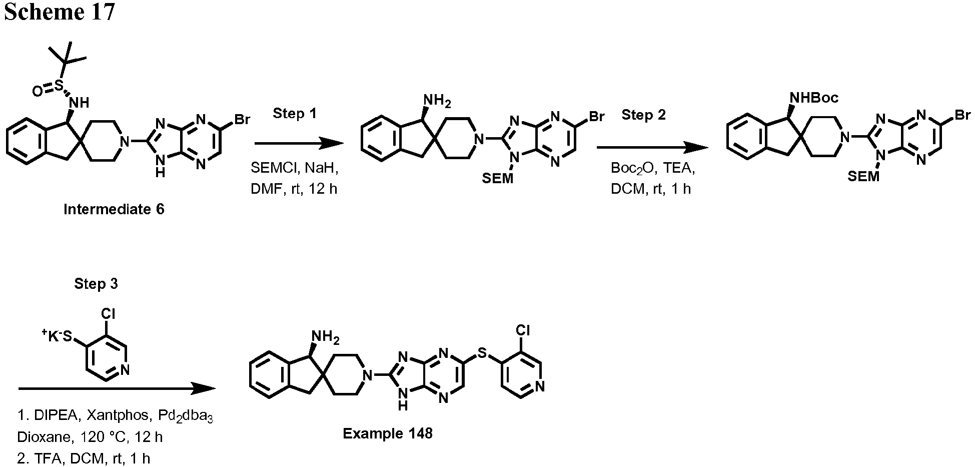

### Step 1: (S)-1'-(5-bromo-1-((2-(tert-butyldimethylsilyl)ethoxy)methyl)-1H-imidazo[4,5-b]pyrazin-2-yl)-1,3-dihydrospiro[indene-2,4'-piperidin]-1-amine

A solution of (R)-*N*-((S)-1'-(5-bromo-*1H*-imidazo[4,5-b]pyrazin-2-yl)-1,3-dihydrospiro[indene-2,4'-piperidin]-1-yl)-2-methylpropane-2-sulfinamide (**Intermediate 6;** 640 mg, 1.27 mmol, prepared as in Step 1 of Scheme 14) in DMF (12 mL) was treated with NaH (60% in mineral oil; 56 mg, 1.4 mmol) at 0°C. The mixture was stirred for 1h at rt and 2-(chloromethoxy)ethyl-trimethyl-silane (0.326 mL, 1.65 mmol) was added dropwise and then stirred for 12 h at rt. During this time, the sulfinimide protecting group of target molecule has been removed. The mixture was quenched with H₂O and diluted in DCM. Organic phases were dried over Na₂SO₄, filtered and evaporated under reduced pressure and the crude residue was purified on silica gel (eluting with 10-100% EtOAc/Petroleum ether) to give the desired intermediate as a yellow solid (240 mg, 35%). LCMS (ES⁺) *m*/*z* 529/531 (M+H)⁺; RT 1.79 min.

### Step 2: tert-butyl (S)-(1'-(5-bromo-1-((2-(tert-butyldimethylsilyl)ethoxy)methyl)-1H-imidazo[4,5-b]pyrazin-2-yl)-1,3-dihydrospiro[indene-2,4'piperidin]-1-yl)carbamate

A solution of (S)-1'-(5-bromo-1-((2-(*tert*-butyldimethylsilyl)ethoxy)methyl)-1H-imidazo[4,5-b]pyrazin-2-yl)-1,3-dihydrospiro[indene-2,4'-piperidin]-1-amine (240 mg, 0.450 mmol) in DCM (2.3 mL) was treated with TEA (0.08 mL, 0.54 mmol) and tert-butoxycarbonyl tert-butyl carbonate (118 mg, 0.54 mmol) at rt for 1h. Mixture was evaporated under reduced pressure and the residue crude was purified on silica gel (eluting with 10-50% EtOAc/Petroleum ether) to give the desired intermediate as a yellow powder (150 mg, 52%). ¹H NMR (DMSO-*d*₆) δ 8.24 (s, 0.25H), 8.04 (s, 0.65H), 7.24-7.18 (m, 5H), 5.40 (s, 1.4H), 5.37 (s, 0.6H), 4.83 (d, *J* = 9.4 Hz, 0.8H), 4.09-4.06 (m, 1.5H), 3.75-3.70 (m, 2H), 3.56-3.52 (m, 2H), 3.26 (d, *J* = 16.2 Hz, 1.2H), 2.79 (d, *J* =15.8 Hz, 1H), 1.76-1.65 (m, 3H), 1.39 (s, 10H), 0.95-0.88 (m, 2H), -0.04 - -0.07 (m, 9H). LCMS (ES⁺) *m*/*z* 629/631 (M+H)⁺; RT 2.76, 2.74 min (mixture of regioisomers).

### Step 3: (S)-1'-(5-((2-chloropyridin-3-yl)thio)-1H-imidazo[4,5-b]pyrazin-2-yl)-1,3-dihydrospiro[indene-2,4'-piperidin]-1-amine (trifluoroacetate salt)

In a microwave vial was charged a solution of tert-butyl (S)-(1'-(5-bromo-1-((2-(tertbutyldimethylsilyl)ethoxy)methyl)-1H-imidazo[4,5-b]pyrazin-2-yl)-1,3-dihydrospiro[indene-2,4'-piperidin]-1-yl)carbamate (21 mg, 0.030 mmol), potassium 3-chloropyridine-4-thiolate (13 mg, 0.04 mmol), DIPEA (0.01mL, 0.07 mmol), Pd₂(dba)₃ (1.5 mg, 0.05 mmol) and Xantphos (1.9 mg, 0.1 mmol) in 1,4-dioxane (0.37 mL). The mixture was stirred at 120°C for 12 h. The mixture was evaporated and the residue was dissolved in TFA (0.2 mL) and DCM (1 mL). After stirring at rt for 1h, the mixture was concentrated under reduced pressure and the resultant residue was purified by prep HPLC to give the title compound as a white powder (4.6 mg, 29%). ¹H NMR (DMSO-*d*₆) δ 8.56 (s, 1H), 8.25 (br d, *J* = 5.5 Hz, 5H), 7.51 (d, *J* = 7.5 Hz, 1H), 7.41 - 7.31 (m, 4H), 6.80 (d, *J* = 5.5 Hz, 1H), 4.44 (br d, *J* = 5.0 Hz, 1H), 4.27 - 4.15 (br d, *J* = 10.7 Hz, 3H), 3.21 (br d, *J* = 16.2 Hz, 1H), 3.03 (br d, *J* = 16.0 Hz, 1H), 1.87 - 1.71 (m, 2H), 1.67 - 1.52 (m, 2H). LCMS (ES⁺) *m*/*z* 464 (M+H)⁺; RT 1.03 min.

Example 141 was synthesized using the above procedure, by using potassium 3-sulfido-2-(trifluoromethyl)pyridine 1-oxide instead of potassium 3-chloropyridine-4-thiolate in Step 3.

### Example 152: (S)-4-((2-(1-amino-1,3-dihydrospiro[indene-2,4'-piperidin]-1'-yl)-1H-imidazo[4,5-b]pyrazin-5-yl)thio)pyridin-2(1H)-one

The compound was prepared according to Scheme 18, following the procedures indicated below.

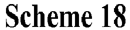

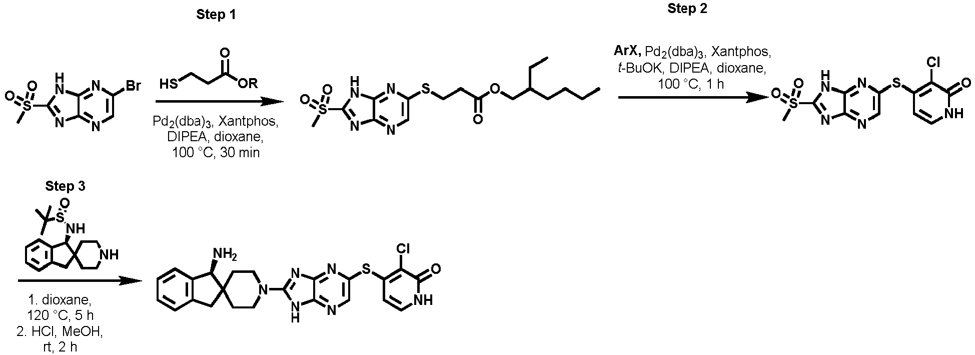

### Step 1: 2-ethylhexyl 3-((2-(methylsulfonyl)-1H-imidazo[4,5-b]pyrazin-6-yl)thio)propanoate

A pressure tube was charged with 5-bromanyl-2-methylsulfonyl-3H-imidazo[4,5-b]pyrazine (800 mg, 2.9 mmol) (prepared as described in Example 22, Scheme 1, Step 3), 2-ethylhexyl 3-mercaptopropanoate (722 uL, 3.2 mmol), DIPEA (1.0 mL, 5.8 mmol), Xantphos (83 mg, 0.14 mmol) and Pd₂(dba)₃ (66 mg, 0.07 mmol) in 1,4-dioxane (15 mL). The mixture was degassed with nitrogen for 1 min, capped and heated at 100 °C for 30 min. After cooling, the mixture was concentrated *in vacuo* and purified on silica gel (eluting with 10-100% EtOAc + 10% MeOH/Petroleum ether) to give the title compound as a pale yellow solid (993 mg, 75%). LCMS (ES⁺) *m*/*z* 415 (M+H)⁺, RT 2.18 min.

### Step 2: 3-chloro-4-((2-(methylsulfonyl)-1H-imidazo[4,5-b]pyrazin-6-yl)thio)pyridin-2(1H)-one

A solution of 2-ethylhexyl 3-[(2-methylsulfonyl-3*H*-imidazo[4,5-b]pyrazin-5-yl)sulfanyl]propanoate (35 mg, 0.08 mmol), 4-bromanyl-3-chloranyl-pyridin-2-ol (21 mg, 0.10 mmol), Xantphos (2.4 mg, 0.044 mmol), Pd₂(dba)₃ (1.93mg, 0.002 mmol) were dissolved in 1,4-dioxane (0.5 mL) under nitrogen flux, then potassium 2-methylpropan-2-olate (126 uL, 0.126 mmol, 1 M in THF) and DIPEA (0.03 mL, 0.17 mmol) were added and stirred at 100 °C for 1 h. After cooling, the mixture was concentrated *in vacuo* and purified on silica gel (eluting with 10-100% EtOAc + 20% MeOH/petroleum ether) to give the title compound as a yellow solid (8 mg, 26%). LCMS (ES⁺) *m*/*z* 358 (M+H)⁺, RT 0.84 min.

### Step 3: (S)-4-((2-1,3-amino-1,3-dihydrospiro[indene-2,4'-piperidin]-1'-yl)-1H-imidazo[4,5-b]pyrazin-5-yl)thio)-3-chloropyridin-2(1H)-one

A solution of 2-methyl-*N*-[(1S)-spiro[1,3-dihydroindene-2,4'-piperidine]-1-yl]propane-2-sulfinamide; 2,2,2-tris(fluoranyl)ethanoic acid (12 mg, 0.03 mmol) and 3-chloro-4-((2-(methylsulfonyl)-1H-imidazo[4,5-b]pyrazin-6-yl)thio)pyridin-2(1H)-one (8 mg, 0.02 mmol) in 1,4-dioxane (0.2 mL) was treated with DIPEA (15 uL, 0.09 mmol) and stirred for 5 h at 120 °C. The organic solvent was evaporated and the residue was dissolved in MeOH (0.3 mL) and a solution of HCl in MeOH (0.3 mL, 0.5 M) was added. The mixture was stirred at rt for 2 h then concentrated in vacuo to give a residue that was purified by prep HPLC to give the title compound as a yellow powder (0.4 mg, 4%). 1H NMR (DMSO-*d6*) δ 11.97 (br d, J = 5.5 Hz, 1H), 8.23 (br s, 4H), 7.50 (d, J = 7.2 Hz, 1H), 7.40 - 7.31 (m, 3H), 7.21 - 7.18 (m, 1H), 5.53 (d, J = 7.0 Hz, 1H), 4.42 (br d, J = 4.2, 1H), 4.26 - 4.14 (m, 2H), 3.39 (t, *J* = 12.5 Hz, 2H), 3.18 (d, *J* = 16.4 Hz, 1H), 3.00 (d, *J* = 16.4 Hz, 1H), 1.83 - 1.73 (m, 2H), 1.61 - 1.55 (m, 2H). LCMS (ES⁺) *m*/*z* 480 (M+H)⁺, RT 0.92 min.

The following examples were synthesized using the above procedure (Scheme 18) with the corresponding starting materials 152, 155, 156-169.

### Example 165: (S)-1-(4-((2-(1-amino-1,3-dihydrospiro[indene-2,4'-piperidin]-1'-yl)-1H-imidazo[4,5-b]pyrazin-5-yl)thio)-3-chloropyridin-2-yl)azetidin-3-ol

The compound was prepared according to Scheme 19, following the procedures indicated below.

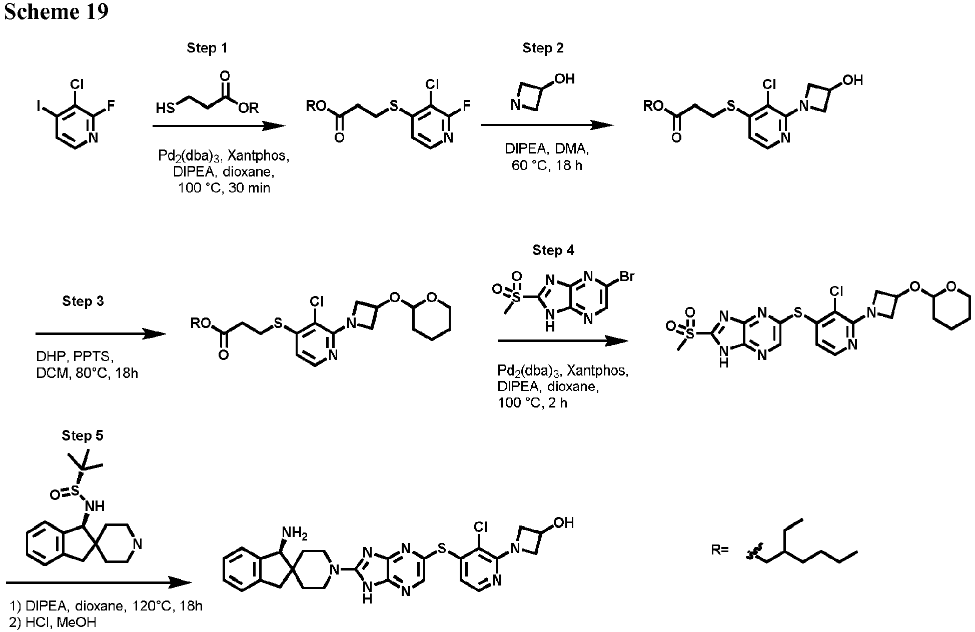

### Step 1: 2-ethylhexyl 3-((3-chloro-2-fluoropyridin-4-yl)thio)propanoate

3-chloro-2-fluoro-4-iodopyridine (500 mg, 1.9 mmol), 2-ethylhexyl 3-mercaptopropanoate (424 mg, 1.9 mmol), Pd₂(dba)₃ (89 mg, 0.1 mmol), xantphos (112 mg, 0.2 mmol) and DIPEA (0.68 mL, 3.9 mmol) were dissolved in 1,4-dioxane (13 mL) and the obtained mixture was degassed using a positive flow of N₂ and heated at 100 °C for 30 min. After cooling, the solvent was concentrated *in vacuo* and the residue was purified on silica gel (eluting with 0-30% Petroleum ether/EtOAc) to give the title compound as a yellow oil (617 mg, 91%). LCMS (ES⁺) m/z 348 (M+H)⁺, RT 2.66.

### Step 2: 2-ethylhexyl 3-((3-chloro-2-(3-hydroxyazetidin-1-yl)pyridin-4-yl)thio)propanoate

2-ethylhexyl 3-((3-chloro-2-fluoropyridin-4-yl)thio)propanoate (50 mg, 0.14 mmol), 3-hydroxyazetidin-1-ium 2,2,2-trifluoroacetate (86.6 mg, 0.29 mmol) and DIPEA (0.1 mL, 0.57 mmol) were dissolved in DMA (1 mL) and the obtained mixture was stirred at 60 °C for 18 h. The solvent was concentrated and EtOAc was added and washed with water, brine, dried over Na₂SO₄, filtered and concentrated in vacuo. The title compound was obtained as a yellow oil (57 mg, 99%). LCMS (ES⁺) *m*/*z* 401 (M+H)⁺, RT 2.1 min.

### Step 3: 2-ethylhexyl 3-((3-chloro-2-(3-((tetrahydro-2H-pyran-2-yl)oxy)azetidin-1-yl)pyridin-4-yl)thio)propanoate

2-ethylhexyl 3-((3-chloro-2-(3-hydroxyazetidin-1-yl)pyridin-4-yl)thio)propanoate (126 mg, 0.3 mmol), was dissolved in DCM (2.0 mL) then pyridinium p-toluenesulfonate (PPTS, 39.4 mg, 0.16 mmol) and 3,4-dihydro-2H-pyran (DHP, 132 mg, 1.6 mmol) were sequentially added. The mixture was stirred at 20° C for 18 h then the solvent was concentrated. Purification by flash chromatography (gradient elution 0-100% EtOAc in petroleum ether) gave the title compound as a yellow oil (127 mg, 83%). LCMS (ES⁺) *m*/*z* 485 (M+H)⁺, RT 2.8 min.

### Step 4: 5-((3-chloro-2-(3-((tetrahydro-2H-pyran-2-yl)oxy)azetidin-1-yl)pyridin-4-yl)thio)-2-(methylsulfonyl)-1H-imidazo[4,5-b]pyrazine

The reaction was made following the procedure reported in Scheme 18, Step 2. The reaction was performed using 1 eq of *^{t}*BuOK (126 µL, 0.13 mmol, 1.0 M in THF).

### Step 5: (S)-1-(4-((2-(1-amino-1,3-dihydrospiro[indene-2,4'-piperidin]-1'-yl)-1H-imidazo[4,5-b]pyrazin-5-yl)thio)-3-chloropyridin-2-yl)azetidin-3-ol

The reaction was made following the procedure reported in the Scheme 18, Step 3 to obtain the title compound (7.8 mg, 14%). ¹H NMR (DMSO-*d*₆) δ 8.3 (br s, 3H), 8.26 (s, 1H), 7.73 (d, *J* = 6.58 Hz, 1H), 7.52 (d, *J* = 6.58 Hz, 1H), 7.39 - 7.30 (m, 3H), 6.15 (d, *J* = 6.14 Hz, 1H), 4.54 - 4.51 (m, 3H), 4.47 - 4.42 (m, 1H), 4.26 - 4.14 (m, 2H), 4.06 - 4.05 (m, 2H), 3.49 (t, 2H), 3.22 (d, *J* = 16.6 Hz, 1H), 3.03 (d, *J* = 15.8 Hz, 1H), 1.86 - 1.78 (m, 2H), 1.63 - 1.59 (m, 2H). LCMS (ES⁺) *m*/*z* 535 (M+H)⁺; RT 1.22 min.

### Intermediate 7: (S)-N-(5,7-dihydrospiro[cyclopenta[b]pyridine-6,4'-piperidin]-5-yl)-2-methylpropane-2-sulfonamide

The compound was prepared according to Scheme 20, following the procedures reported below.

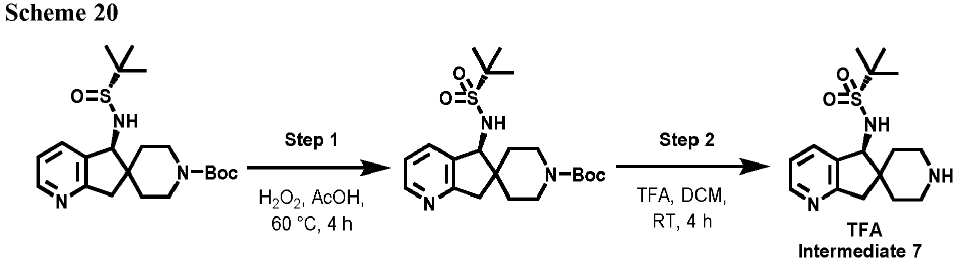

### Step 1: tert-butyl (S)-5-((1,1-dimethylethyl)sulfonamido)-5,7-dihydrospiro[cyclopenta[b]pyridine-6,4-piperidine]-1'-carboxylate

A solution of *tert*-butyl (5*S*)-5-[[(*R*)-*tert*-butylsulfinyl]amino]spiro[5,7-dihydrocyclopenta[b]pyridine-6,4'-piperidine]-1'-carboxylate (50 mg, 0.120 mmol) (prepared as described in WO2018/172984) in acetic acid (0.3 mL, 0.4 M) cooled to 0 °C was treated with hydrogen peroxide (30 wt. % in H₂O, 0.010 mL, 0.130 mmol), then heated at 60 °C for 4 h. The reaction mixture was concentrated to dryness and purified by flash chromatography (gradient elution 0-20% MeOH in DCM. The title compound was obtained as white solid (53 mg, 100%). ¹H NMR (400 MHz, CDCl₃) δ 8.39 (d, *J* = 5.0 Hz, 1H), 7.90 (d, *J* = 7.5 Hz, 1H), 7.22 - 7.14 (m, 1H), 4.60 (br d, *J* = 11.0 Hz, 1H), 4.16 (br d, *J* = 10.0 Hz, 1H), 4.11 - 3.85 (m, 2H), 3.17 (d, *J* = 16.9 Hz, 1H), 2.97 - 2.75 (m, 3H), 1.82 (td, *J* = 12.9, 4.6 Hz, 1H), 1.59 (td, *J* = 11.4, 4.4 Hz, 1H), 1.53 - 1.46 (m, 1H), 1.43 (s, 9H), 1.40 (s, 9H), 1.33 - 1.23 (m, 1H). LCMS (ES⁺) *m*/*z* 424 (M+H)⁺, RT 1.44 min.

### Step 2: (S)-N-(5,7-dihydrospiro[cyclopenta[b]pyridine-6,4'-piperidin]-5-yl)-2-methylpropane-2-sulfonamide (Intermediate 7)

A stirred solution of *tert*-butyl (*S*)-5-((1,1-dimethylethyl)sulfonamido)-5,7-dihydrospiro[cyclopenta[b]pyridine-6,4'-piperidine]-1'-carboxylate (53 mg, 0.13 mmol) in DCM (1 mL, 0.12 M) cooled to 0°C was treated with TFA (0.1mL, 1.31 mmol). The reaction mixture was stirred at rt for 4 h, then concentrated under reduced pressure. The title product was obtained as white solid (55 mg, 100%) and used in the next step without further purification. ¹H NMR (400 MHz, DMSO-*d₆*) δ 8.49 (br s, 1H), 8.37 (d, *J* = 4.8 Hz, 1H), 8.24 (br s, 1H), 7.72 (d, *J* = 7.4 Hz, 1H), 7.41 (d, *J* = 9.6 Hz, 1H), 7.31 - 7.22 (m, 1H), 4.56 (br d, *J* = 9.6 Hz, 1H), 3.35 - 3.27 (m, 1H), 3.25 - 3.15 (m, 2H), 3.09 (d, *J* = 16.7 Hz, 1H), 3.05 - 2.90 (m, 1H), 2.86 (d, *J* = 16.7 Hz, 1H), 2.02 - 1.87 (m, 1H), 1.82 - 1.70 (m, 1H), 1.66 - 1.55 (m, 1H), 1.32 (s, 9H), 1.22 - 1.13 (m, 1H). LCMS (ES⁺) *m*/*z* 324 (M+H)⁺, RT 0.59 min.
Intermediate 7 was used for the synthesis of Example 142 and Example 150.

### Intermediate 8: 4-chloro-6-iodo-3λ²-benzo[d]oxazol-2-one

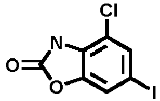

### Step 1: 4-chloro-3λ²-benzo[d]oxazol-2-one

2-amino-3-chlorophenol (500 mg, 3.5 mmol) was dissolved in THF (20 mL) then CDI (903 mg, 5.6 mmol) was added. The mixture was stirred at 65 °C for 2 h then the reaction was cooled and concentrated. The residue was dissolved in EtOAc and washed with water, 2M HCl, water, brine and dried over Na₂SO₄. The title compound was obtained as yellow solid (590 mg, 99%). LCMS (ES⁺) *m*/*z* 170 (M+H)⁺, RT 1.2 min.

### Step 2: 4-chloro-6-iodo-3λ²-benzo[d]oxazol-2-one

I₂ (150 mg, 0.59 mmol) and Ag₂SO₄ (184 mg, 0.59 mmol) were dissolved in EtOH (6 mL) then 4-chloranyl-3*H*-1,3-benzoxazol-2-one (100 mg, 0.59 mmol) was added and the obtained mixture was stirred for 18 h at 20 °C. The resulting suspension was filtered through a pad of solka floc and the solvent was concentrated. EtOAc and NaHCO₃ sat. sol. were added and the organic phase was washed with water, brine, dried over Na₂SO₄, filtered and concentrated in vacuo. Purification by flash chromatography (gradient elution 0-40% EtOAc in petroleum ether) gave the title compounds as a white solid (120 mg, 48%). LCMS (ES⁺) *m*/*z* 295 (M+H)⁺, RT 1.6 min.

The intermediate 8 was used to synthesize compound 168

### Intermediate 9: 5-chloro-6-iodo-4λ²-benzo[b][1,4]oxazin-3(2H)-one

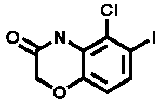

### Step 1: 5-chloro-4λ²-benzo[b][1,4]oxazin-3(2H)-one

2-amino-3-chlorophenol (200 mg, 1.4 mmol), 2-chloroacetyl chloride (189 mg, 1.7 mmol) and K₂CO₃ (577 mg, 4.2 mmol) were dissolved in DMF (35 mL) and the mixture was stirred at 50° C for 15 h. The resulting suspension was filtered through a pad of solka floc and the solvent was concentrated. EtOAc and NaHCO₃ sat. sol. were added and the organic phase was washed with water, brine, dried over Na₂SO₄, filtered and concentrated in vacuo. The title compound was obtained as a yellow solid (240 mg, 94%). LCMS (ES⁺) *m*/*z* 184 (M+H)⁺, RT 1.2 min.

### Step 2: 5-chloro-6-iodo-4λ²-benzo[b][1,4]oxazin-3(2H)-one

I₂ (498 mg, 2.0 mmol) and Ag₂SO₄ (611 mg, 2 mmol) were dissolved in EtOH (13 mL) then 5-chloro-4l2-benzo[b][1,4]oxazin-3(2H)-one (240 mg, 1.3 mmol) was added and the obtained mixture was stirred for 18 h at 20 °C. The resulting suspension was filtered through a pad of solka floc and the solvent was concentrated. EtOAc and NaHCO₃ sat. sol. were added and the organic phase was washed with water, brine, dried over Na₂SO₄, filtered and concentrated in vacuo. Purification by flash chromatography (gradient elution 0-40% EtOAc in petroleum ether) gave the title compounds as a white solid (77 mg, 20 %, 75% pure). LCMS (ES⁺) *m*/*z* 310 (M+H)⁺, RT 1.26 min.

The intermediate 9 was used to synthesize compound 169.

### Intermediate 10: 3-chloro-4-iodo-N-methylpyridin-2-amine

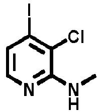

A pressure tube was loaded with methylamine (25.0 mL, 50.1 mmol) (2M in THF), 3-chloranyl-2-fluoranyl-4-iodanyl-pyridine (4.3 g, 16.7 mmol) and DMSO (30 mL). Mixture was heated at 70 °C for 2 h. After cooling, water (10 mL) was added and mixture extracted with EtOAc (2x50 mL). The organic layer was washed with water, brine, dried over Na₂SO₄, filtered and concentrated in vacuo. The residue was purified on silica gel (eluting with 0-20% EtOAc/petroleum ether) to give the title compound as a white solid (4.3 g, 96%). ¹H NMR (DMSO-*d*₆) δ 7.64 (m, 1H), 7.03 (m, 1H), 6.71 (bs, 1H), 2.82 (d, *J* = 7.9 Hz, 3H). LCMS (ES⁺) *m*/*z* 269 (M+H)⁺; RT 1.37 min.

The intermediate 10 was used to synthesize compound 130 and the same procedure was used to synthesize the aminopyridines to obtain 154, 160, 161.

### Biology

### SHP2 inhibition enzymatic assay

The assay was performed as described in YP Chen et al, Nature (535)2016. Assay volume of 20µL/well was assembled in 384 well black polystyrene low-binding microplates (Greiner), using the following buffer: 60 mM HEPES pH 7.2, 75 mM NaCl, 75 mM KCl, 1 mM EDTA pH 8, 0.05% tween-20, 5 mM DTT. The SHP-2 enzyme (synthetized by Origene, Met1 - Leu525, cat#TP750155) was used at a final concentration of 0.5 nM. The enzyme was activated by 500 nM IRS1 peptide (sequence: H2N-LN(pY)IDLDLV(dPEG8)LST(pY)ASINFQK-amide SEQ ID No. 1) and incubated with 75 µM DiFMUP (Sigma) as substrate.

Briefly, DMSO serially diluted testing compounds were transferred to the bottom of the assay plate. SHP2 was then added together with the IRS1 peptide. 30 minutes post incubation, the DiFMUP substrate was added to the reaction and incubated 30 min at room temperature. Finally 5µL of 160 µM bpV (Potassium bisperoxo[1,10-phenanthroline]oxovanadate [V], Sigma) were added to stop and quench the reaction. The fluorescence was detected by a microplate reader (Envision, PerkinElmer) according to the DiFMUP excitation and emission wavelength. The lower the fluorescence the higher the SHP2 inhibition.

The activity of each compound dilution was calculated as percentage of inhibition between vehicle (DMSO, 0% inhibition) and no enzyme (100% inhibition). The percentage inhibition is fitted against the compound dilutions with a four-paramenter logistic regression. The inflection point (i.e. the concetration at which half-maximal inhibiton is achieved) is the IC50.

The IC₅₀ results of the compounds of the invention in the SHP2 inhibition enzymatic assay are shown in Table 2. Legend: A indicates IC₅₀ less or equal to 0.5 µM; B indicates IC₅₀ greater than 0.5 µM and lower or equal to 3 µM; C indicates IC₅₀ higher than 3 µM.

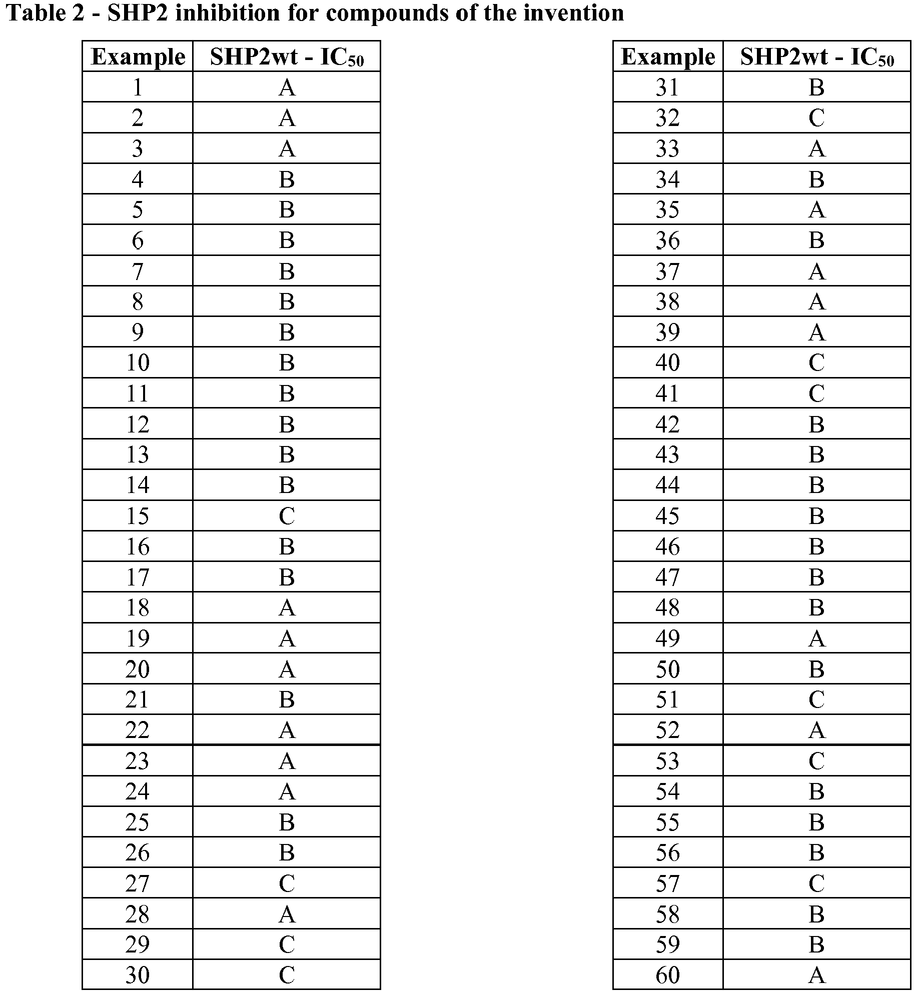

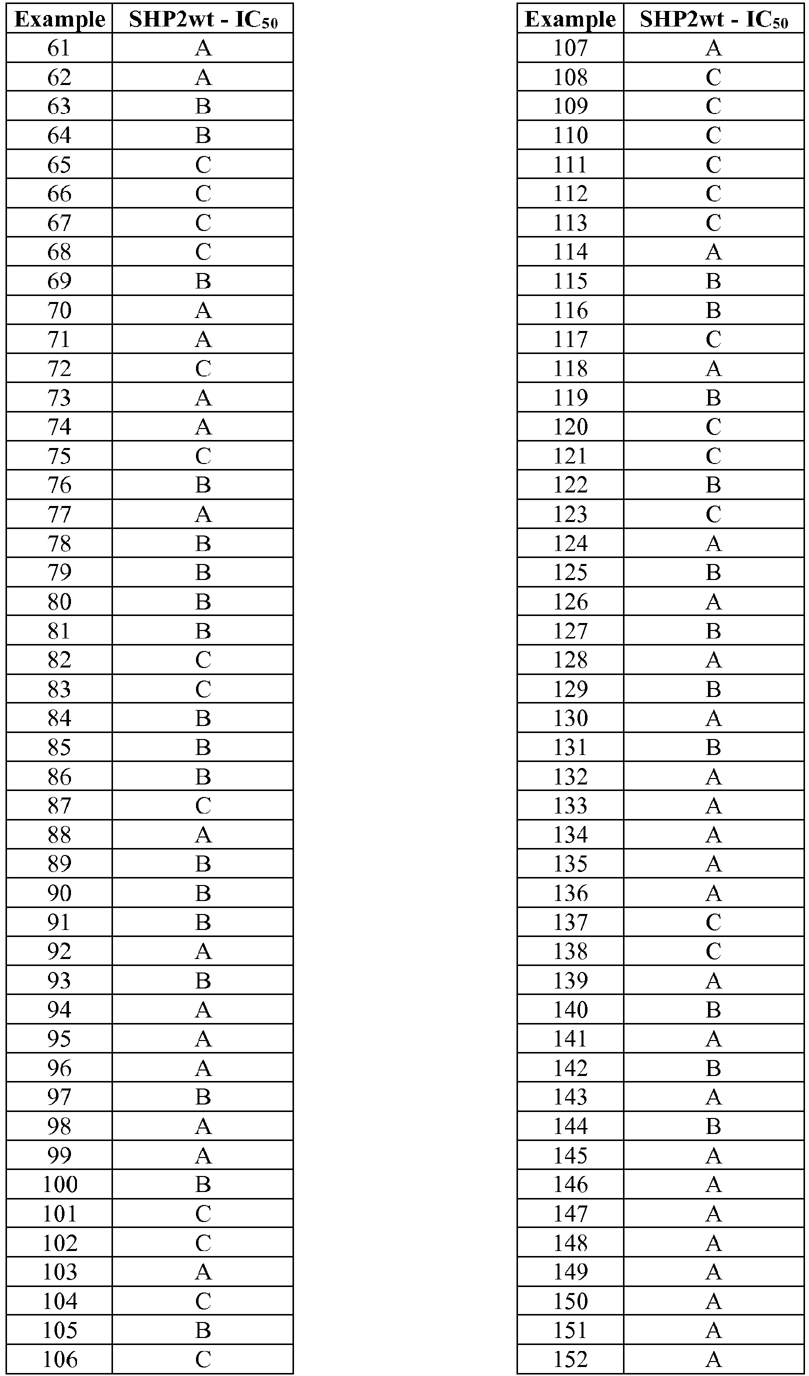

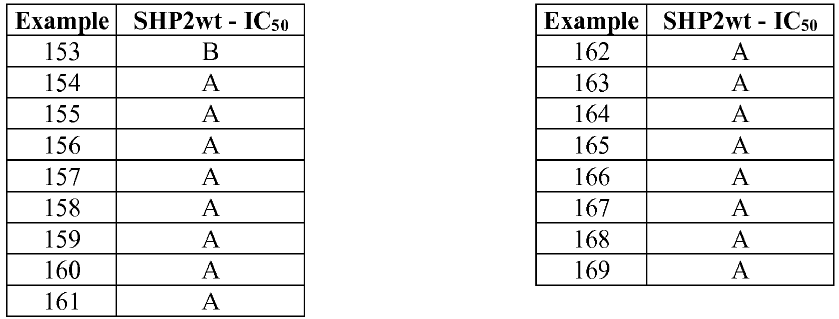

### Phospho-ERK cellular assay

ERK phosphorylation was detected using the "Advanced phospho-ERK1/2 (Thr202/Tyr204)" TR-FRET kit (Cisbio, Cat #64AERPEG/H), following the manufacturer reagents and instructions.

Briefly, 20.000/well KYSE-520 cells (DSMZ ACC 371) were plated in 6 µL RPMI-1640 (Invitrogen) growth medium, into 384 white low-volume high base TC microplates (Greiner). After an overnight incubation, cells were treated with DMSO serial diluted compounds and incubated for 2 h at 37 °C. After incubation, 2 µL/well of 4X Lysis Buffer (Cisbio 64KL1FDF), were added and incubated with cells for 30 min on gentle shaking. Finally, lysates were added with 2 µL/well of Eu cryptate (donor) and D2 (acceptor) conjugated antibodies (as provided by the Cisbio kit #64AERPEG/H) diluted 1:100 in Detection Buffer (as provided by the Cisbio kit #64AERPEG/H). Plates were then sealed and incubated at room temperature in the dark. After an overnight incubation, the TR-FRET signal was detected on a suitable reader (Envision, PerkinElmer). The lower the TR-FRET signal the higher the higher the SHP2 inhibition in cells.

The activity of each compound dilution was calculated as percentage between vehicle DMSO treated cells and no cells, 0% inhibition and 100% inhibition respectively. The percentage activity is fitted against the compound dilutions with a four-paramenter logistic regression. The inflection point (i.e. the concetration at which half-maximal inhibiton is achieved) is the IC₅₀.

The IC₅₀ results of the compounds of the invention in the phospho-ERK cellular assay are shown in Table 3. Legend: "+" indicates IC₅₀ equal or higher than 5µM; "++" indicates IC₅₀ less than 5 µM and higher or equal to 1 µM; "+++" indicates IC₅₀ less than 1µM.

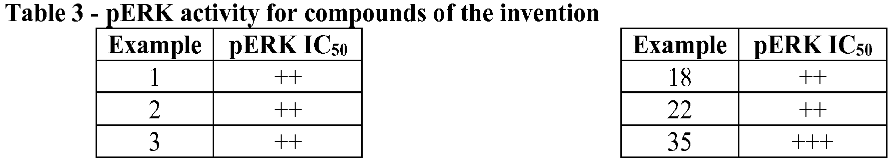

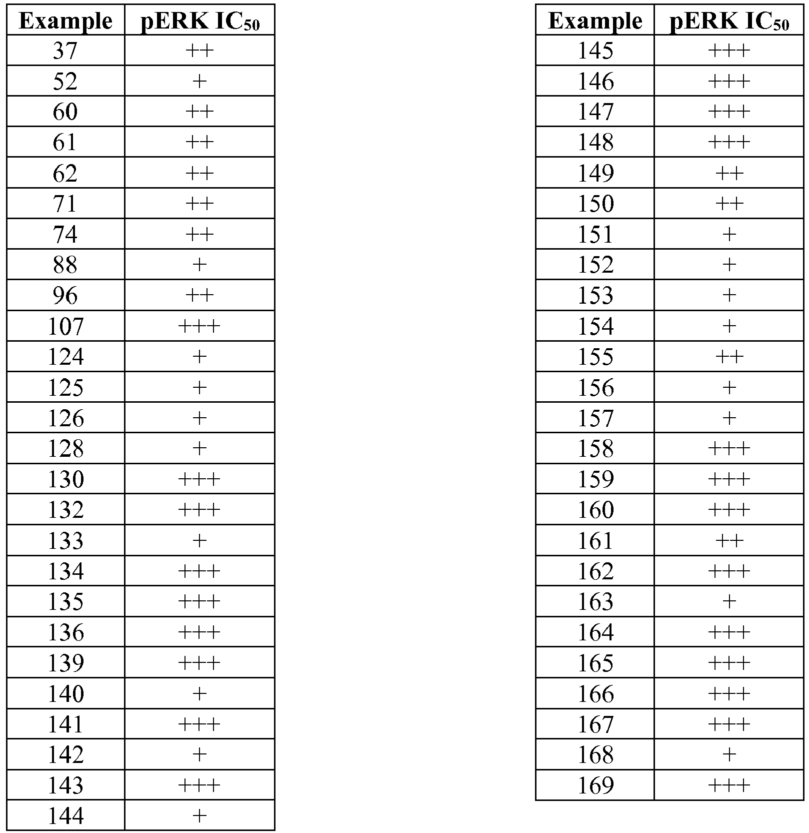

## Claims

1. A compound of Formula (I): 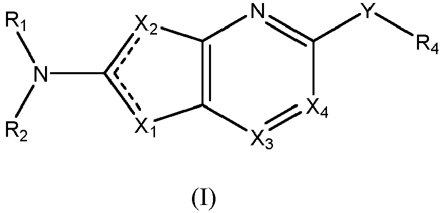 Wherein:
-̅-̅-̅-̅-̅ represents a single bond or a double bond;
X₁ is N, S, O or NR₃;
X₂ is N or NR₃;
if X₁ is N then X₂ is NR₃;
if X₁ is S, O or NR₃ then X₂ is N;
X₃ is N or CRX₃ and RX₃ is H, halogen or C₁₋₃alkyl;
X₄ is N or CR₅;
Y is S, O, NR₆, CH₂, CHF, CF₂, CHOH, C(O), SO, SO₂ or a single bond;
R₁ and R₂ are each independently selected from:
- hydrogen;
- linear or branched C₁₋₁₂alkyl optionally substituted with one or more substituents independently selected from the group consisting of: OH, halogen, N(R₇)₂, aryl, heteroaryl, partially unsaturated heteroaryl, C₃₋₉cycloalkyl, C₃₋₉heterocycloalkyl and spiro-C₃₋₈cycloalkyl ring optionally containing one heteroatom selected from the group consisting of O, N and S, wherein each of said aryl, heteroaryl, partially unsaturated heteroaryl, C₃₋₉cycloalkyl, C₃₋₉heterocycloalkyl and spiro-C₃₋₈cycloalkyl ring is optionally further substituted with one or more groups independently selected from the group consisting of: C(O)CH₃, C(O)OCH₃, heteroaryl, aryl, OH, halogen, NH₂, C₁₋₆alkyl optionally substituted with N(R₇)₂, C₁₋₃alkylaryl, C₁₋₃alkylheteroaryl, haloC₁₋₆alkyl, hydroxyC₁₋₆alkyl, CN, haloC₁₋₆alkoxy, C₁₋₆alkoxy, C₅₋₇heterocycloalkoxy and a cyclic amine selected from the group consisting of: pyrrolidine, piperidine, morpholine, thiomorpholine, piperazine, N-methylpyperazine and pyrrolidin-3-yloxy; and
- a cyclic structure selected from the group consisting of: C₃₋₇cycloalkyl, C₃₋₉heterocycloalkyl, aryl, heteroaryl and partially unsaturated heteroaryl, each of said cyclic structure being optionally substituted with one ore more substituents independently selected from the group consisting of: halogen, C₁₋₃alkyl optionally substituted with N(R₇)₂, C₁₋₃alkylaryl, C₁₋₃alkylheteroaryl, C(O)OC₁₋₃alkyl, C(O)C₁₋₃alkyl, N(R₇)₂, aryl and heteroaryl, each of said aryl or heteroaryl being optionally substituted with one or more substituents independently selected from the group consisting of: halogen, hydroxyl, cyano, C₁₋₃alkoxy and C₁₋₃haloalkyl;
or R₁ and R₂ form together with the nitrogen atom to which they are attached a cyclic amine of formula (II) 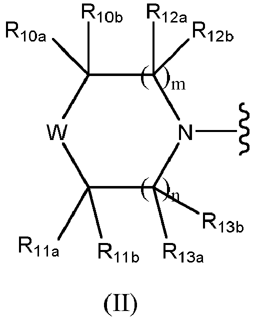 Wherein:
m and n are each independently selected from 0, 1 and 2;
W is absent, O, CR₈R₉, NR₁₀, S, SO or SO₂;
R₁₀ₐ, R_{10b}, R₁₁ₐ, R_{11b}, R₁₂ₐ, R_{12b}, R₁₃ₐ, R_{13b} are each independently selected from the group consisting of: H, C₁₋₆alkyl, aminoC₁₋₆alkyl, C₁₋₆alkoxy, halogen, NH₂, CN, OH, C(O)NH₂, heteroaryl, optionally substituted aryl, hydroxy-C₁₋₆alkyl, halo-C₁₋₆alkyl, C₁₋₆alkoxy and C₃₋₉heterocycloalkyl;
or any two of R₁₀ₐ, R_{10b}, R₁₁ₐ, R_{11b}, R₁₂ₐ, R_{12b}, R₁₃ₐ, R_{13b} which are not germinal groups, taken together represent a single bond, a C₁₋₄alkanediyl or a C₂₋₄alkenediyl, each of said C₁₋₄alkanediyl or C₂₋₄alkenediyl being independently optionally substituted with one or more of C₁₋₄alkyl and/or halogen; said single bond or said optionally substituted C₁₋₄alkanediyl or C₂₋₄alkenediyl forming together with the bridging atoms to which they are respectively linked a 4-10 membered saturated or partially unsaturated ring;
or any of R₁₀ₐ and R_{10b}, R₁₁ₐ and R_{11b}, R₁₂ₐ and R_{12b} or R₁₃ₐ and R_{13b} taken together with the carbon atom to which they are attached form a 3-7 membered saturated ring optionally containing one or more of O, S, N and/or C(O), the 3-7 membered saturated ring optionally being substituted with one or more substituents each independently selected from the group consisting of halogen, OH, haloC₁₋₆alkyl, CN, C₁₋₆alkyl, C₁₋₆alkoxy, haloC₁₋₆alkoxy, C(O)OC₁₋₃alkyl and C(O)C₁₋₃alkyl;
R₈ and R₉ are each independently selected from the group consisting of: H, OH, NH₂, aryl, heteroaryl, C₁-₃alkylheteroaryl, C(O)NH₂, C₁-₆alkyl, aminoC₁-₆alkyl, NHCH₃ and NHSO₂CH₃;
or R₈ and R₉ taken together with the carbon atom to which they are bound form a spiro-C₃₋₈cycloalkyl ring of formula (III) as indicated below: 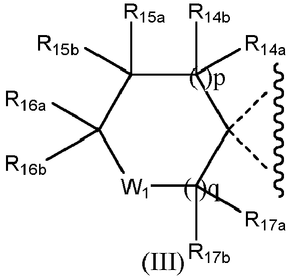
wherein:
p and q are each independently selected from 0, 1 and 2;
W₁ is absent, O, S, SO₂, CHF, CF₂ or NR_{w} and R_{w} is H, C(O)C₁₋₆alkyl, C(O)OC₁₋₆alkyl or C₁₋₆alkyl optionally substituted with one or more substituents each independently selected from the group consisting of: aryl, heteroaryl, OH, C₁₋₃alkoxy and halogen;
R₁₄ₐ, R_{14b}, R₁₅ₐ, R_{15b}, R₁₆ₐ, R_{16b}, R₁₇ₐ, R_{17b} are each independently selected from the group consisting of: H, NH₂, NHSOC₁₋₆alkyl, NHSO₂C₁₋₆alkyl, NHC₁₋₆alkyl, N(C₁₋₆alkyl)₂, NHC(O)C₁₋₆alkyl, NHC(O)OC₁₋₆alkyl, halogen, OH, CN, C₁₋₃alkoxy, C₁₋₆alkyl optionally substituted with NH₂;
or R₁₅ₐ and R_{15b} taken together with the carbon atom to which they are bound form a spiro-C₃₋₆cycloalkyl ring optionally containing an heteroatom selected from the group consisting of: N, O and S;
or R₁₅ₐ and R₁₆ₐ are absent and R_{15b} and R_{16b} are joined together to form an aryl or heteroaryl ring, each being optionally substituted with one or more substituents each independently selected from the group consisting of: halogen, NH₂, NHC₁₋₆alkyl, N(C₁₋₆alkyl)₂, NHC(O)C₁₋₆alkyl, NHC(O)OC₁₋₆alkyl, NHSOC₁₋₆alkyl, NHSO₂C₁₋₆alkyl, CN, OH, C₁₋₆alkyl, C₁₋₆alkoxy, haloC₁₋₆alkyl and haloC₁₋₆alkoxy;
or R_{15b} and R_{16b} are joined together to form a C₅-₇cycloalkyl or C₅-₇heterocycloalkyl ring, each being independently optionally substituted with one or more substituents each independently selected from the group consisting of: halogen, NH₂, NHC₁₋₆alkyl, N(C₁₋₆alkyl)₂, NHC(O)C₁₋₆alkyl, NHC(O)OC₁₋₆alkyl, NHSOC₁₋₆alkyl, NHSO₂C₁₋₆alkyl, CN, OH, =O, C₁₋₆alkyl, C₁₋₆alkoxy, haloC₁₋₆alkyl, haloC₁₋₆alkoxy and SO₂CH₃;
R₁₀ is H, SO₂C₁₋₆alkyl, C(O)C₁₋₆alkyl, C(O)OC₁₋₆alkyl or C₁₋₆alkyl optionally substituted with one or more groups each independently selected from the group consisting of: OH, NH₂, NHC(O)C₁₋₆alkyl, C₁₋₆alkoxy, halogen, cyano, aryl and heteroaryl;
or R₁₀ and R₁₀ₐ are joined together to form a C₃₋₇heterocycloalkyl ring optionally containing another heteroatom selected from the group consisting of: N, S and O and optionally substituted with one or more groups each independently selected from the group consisting of: C₁₋₆alkyl, halogen, OH and CN;
or R₁ and R₂ form together with the nitrogen atom to which they are attached a monocyclic or polycyclic heteroaryl or partially unsaturated heteroaryl ring, each of said ring is optionally substituted with one or more groups each independently selected from the group consisting of: halogen, C₁-₆alkyl, C₁-₆alkoxy, haloC₁₋₆alkyl, haloC₁₋₆alkoxy, OH, CN, SO₂C₁-₆alkyl, NH₂ and C(O)NH₂;
R₃ is H, C₁-₆alkyl, C₁-₆alkyl-cycloalkyl or C₁-₆alkyl-heterocycloalkyl, each of said groups being optionally substituted with one or more groups independently selected from the group consisting of: C₁-₆alkoxy, hydroxyl, cyano, C(O)OC₁-₆alkyl, C(O)NH₂, C(O)NHC₁-₆alkyl; C(O)N(C₁-₆alkyl)₂, SO₂C₁-₆alkyl, SOC₁-₆alkyl, SO₂NHC₁-₆alkyl and SO₂N(C₁-₆alkyl)₂;
R₄ is a ring selected from the group consisting of: aryl, heteroaryl, partially unsaturated aryl, partially unsaturated heteroaryl, cycloalkyl, heterocycloalkyl, C₁-₆alkylaryl, C₁-₆alkylheteroaryl and C₁-₆alkylC₃-₇cycloalkyl, each of said ring being optionally substituted with one or more groups each independently selected from the group consisting of: halogen, hydroxy, cyano, C₁-₆alkyl, C₁-₇alkenyl, C₁-₆alkoxy, haloC₁-₆alkyl, haloC₁-₆alkoxy, C₃-₇cycloalkyl, cyano-C₃-₇cycloalkyl, -SF₅, C₅₋₇heterocycloalkoxy, optionally substituted aryl or heteroaryl, partially unsaturated heteroaryl, optionally substituted aryloxy, C(O)OH, C(O)OC₁-₆alkyl, C(O)C₁-₆alkyl, C₁-₆alkylCOOH, SO₂C₁-₆alkyl and N(R₇)₂;
R₅ is H, halogen, C₁-₆alkyl, C₁-₆alkoxy, haloC₁-₆alkyl, haloC₁-₆alkoxy, OH, C₃-₆cycloalkyl, C₃-₆cycloalkoxy, C₃₋₆heterocycloalkyl or NRₓ₁Rₓ₂ wherein Rₓ₁ and Rₓ₂ are each independently H or C₁-₆alkyl, or Rₓ₁ and Rₓ₂ taken together with the nitrogen atom to which they are attached form a 3-7 membered saturated ring optionally containing one or more heteroatoms each independently selected from the group consisting of O, S and N;
R₆ is H or C₁-₆alkyl;
or R₄ and R₆ form together with the nitrogen atom to which they are attached a C₃₋₉heterocycloalkyl, a heteroaryl or a partially unsaturated heteroaryl ring, each being optionally substituted with one or more groups independently selected from the group consisting of: OH, halogen, CN, C₁-₆alkyl, C₁-₆alkoxy, haloC₁-₆alkyl, haloC₁-₆alkoxy, C₃-₇cycloalkyl, C₃-₇cycloalkoxy, aryl and heteroaryl;
each R₇ is independently selected from the group consisting of: H, C₁-₆alkyl, SO₂C₁-₆alkyl, SOC₁-₆alkyl, C(O)OC₁-₆alkyl, C(O)C₁-₆alkyl, C₃-₇cycloalkyl, aryl, heteroaryl, C₁-₆alkylaryl and C₁-₆alkylheteroaryl;
or two R₇ taken together with the nitrogen atom to which they are bound form a 3 to 7 membered cyclic amine optionally containing one additional heteroatom selected from the group consisting of S, N and O, said 3 to 7 membered cyclic amine being optionally substituted with one or more groups each independently selected from the group consisting of: OH, halogen, CN, C₁-₆alkyl, C₁-₆alkoxy, haloC₁-₆alkyl, haloC₁-₆alkoxy, C₃-₇cycloalkyl and C₃-₇cycloalkoxy;
or a pharmaceutically acceptable salt, tautomer, solvate, or stereoisomer thereof.

2. The compound according to claim 1, wherein:
- X₁ is N and X₂ is NR₃; or
- X₁ is NR₃ and X₂ is N; or a pharmaceutically acceptable salt, tautomer, solvate, or stereoisomer thereof.

3. The compound according to claim 1 or 2, wherein R₁ and R₂ are each independently selected from:
- hydrogen;
- linear or branched C₁₋₁₂alkyl optionally substituted with one or more substituents independently selected from the group consisting of: OH, halogen, N(R₇)₂, aryl, heteroaryl, partially unsaturated heteroaryl, C₃₋₉cycloalkyl, C₃₋₉heterocycloalkyl and spiro-C₃₋₈cycloalkyl ring optionally containing one heteroatom selected from the group consisting of O, N and S, wherein each of said aryl, heteroaryl, partially unsaturated heteroaryl, C₃₋₉cycloalkyl, C₃₋₉heterocycloalkyl and spiro-C₃₋₈cycloalkyl ring is optionally further substituted with one or more groups independently selected from the group consisting of: C(O)CH₃, C(O)OCH₃, heteroaryl, aryl, OH, halogen, NH₂, C₁₋₆alkyl optionally substituted with N(R₇)₂, C₁₋₃alkylaryl, C₁₋₃alkylheteroaryl, haloC₁₋₆alkyl, hydroxyC₁₋₆alkyl, CN, haloC₁₋₆alkoxy, C₁₋₆alkoxy, C₅₋₇heterocycloalkoxy and a cyclic amine selected from the group consisting of: pyrrolidine, piperidine, morpholine, thiomorpholine, piperazine, N-methylpyperazine and pyrrolidin-3-yloxy; and
- a C₃₋₇cycloalkyl, C₃₋₉heterocycloalkyl or a partially unsaturated heteroaryl selected from bicyclo[1.1.1]pentane, pirrolidine, piperidine, morpholine, piperazine, 2-azaspiro[3.3]heptane, azepan-2-one, 3-azaspiro[5.5]undecane, 2-azaspiro[4.5]decane, 3-azabicyclo[3.3.1]nonane, 3-oxa-7-azabicyclo[3.3.1]nonane, 6',7'-dihydrospiro[azetidine-3,5'-pyrrolo[1,2-a]imidazole], 5-azaspiro[3.5]nonane, 1-thia-7-azaspiro[3.5]nonane 1,1-dioxide, 3-azabicyclo[3.2.0]heptane, 2-azabicyclo[2.1.1]hexane, 6-azabicyclo[3.2.1]octane, octahydroindole, octahydro-1H-isoindole, 5-oxa-2-azaspiro[3.4]octane and 1,2,3,4-tetrahydroquinoline, each of said groups being optionally substituted with one ore more substituents independently selected from the group consisting of: halogen, C₁₋₃alkyl optionally substituted with N(R₇)₂, C₁₋₃alkylaryl, C₁₋₃alkylheteroaryl, C(O)OC₁₋₃alkyl, C(O)C₁₋₃alkyl, N(R₇)₂, aryl and heteroaryl, each of said aryl or heteroaryl being optionally substituted with one or more substituents independently selected from the group consisting of: halogen, hydroxyl, cyano, C₁₋₃alkoxy and C₁₋₃haloalkyl;
or R₁ and R₂ form together with the nitrogen atom to which they are attached a cyclic amine of formula (II) 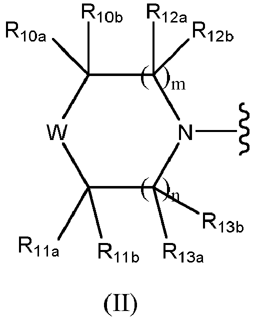 Wherein:
m and n are each independently selected from 0, 1 and 2;
W is absent, O, CR₈R₉, NR₁₀, S, SO or SO₂;
R₁₀ₐ, R_{10b}, R₁₁ₐ, R_{11b}, R₁₂ₐ, R_{12b}, R₁₃ₐ, R_{13b} are each independently selected from the group consisting of: H, C₁₋₆alkyl, aminoC₁₋₆alkyl, C₁₋₆alkoxy, halogen, NH₂, CN, OH, C(O)NH₂, heteroaryl, optionally substituted aryl, hydroxy-C₁₋₆alkyl, halo-C₁₋₆alkyl, C₁₋₆alkoxy and C₃₋₉heterocycloalkyl;
or any two of R₁₀ₐ, R_{10b}, R₁₁ₐ, R_{11b}, R₁₂ₐ, R_{12b}, R₁₃ₐ, R_{13b} which are not germinal groups, taken together represent a single bond, a C₁₋₄alkanediyl or a C₂₋₄alkenediyl, each of said C₁₋₄alkanediyl or C₂₋₄alkenediyl being independently optionally substituted with one or more of C₁₋₄alkyl and/or halogen; said single bond or said optionally substituted C₁₋₄alkanediyl or C₂₋₄alkenediyl forming together with the bridging atoms to which they are respectively linked a 4-10 membered saturated or partially unsaturated ring;
or any of R₁₀ₐ and R_{10b}, R₁₁ₐ and R_{11b}, R₁₂ₐ and R_{12b} or R₁₃ₐ and R_{13b} taken together with the carbon atom to which they are attached form a 3-7 membered saturated ring optionally containing one or more of O, S, N and/or C(O), the 3-7 membered saturated ring optionally being substituted with one or more substituents each independently selected from the group consisting of halogen, OH, haloC₁₋₆alkyl, CN, C₁₋₆alkyl, C₁₋₆alkoxy, haloC₁₋₆alkoxy, C(O)OC₁₋₃alkyl and C(O)C₁₋₃alkyl;
R₈ and R₉ are each independently selected from the group consisting of: H, OH, NH₂, aryl, heteroaryl, C₁-₃alkylheteroaryl, C(O)NH₂, C₁-₆alkyl, aminoC₁-₆alkyl, NHCH₃ and NHSO₂CH₃;
or R₈ and R₉ taken together with the carbon atom to which they are bound form a spiro-C₃₋₈cycloalkyl ring of formula (III) as indicated below: 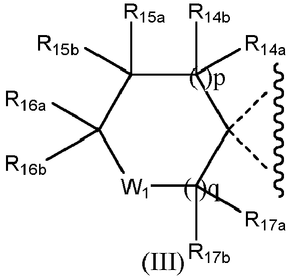 wherein:
p and q are each independently selected from 0, 1 and 2;
W₁ is absent, O, S, SO₂, CHF, CF₂ or NR_{w} and R_{w} is H, C(O)C₁₋₆alkyl, C(O)OC₁₋₆alkyl or C₁₋₆alkyl optionally substituted with one or more substituents each independently selected from the group consisting of: aryl, heteroaryl, OH, C₁₋₃alkoxy and halogen;
R₁₄ₐ, R_{14b}, R₁₅ₐ, R_{15b}, R₁₆ₐ, R_{16b}, R₁₇ₐ, R_{17b} are each independently selected from the group consisting of: H, NH₂, NHSOC₁₋₆alkyl, NHSO₂C₁₋₆alkyl, NHC₁₋₆alkyl, N(C₁₋₆alkyl)₂, NHC(O)C₁₋₆alkyl, NHC(O)OC₁₋₆alkyl, halogen, OH, CN, C₁₋₃alkoxy, C₁₋₆alkyl optionally substituted with NH₂;
or R₁₅ₐ and R_{15b} taken together with the carbon atom to which they are bound form a spiro-C₃₋₆cycloalkyl ring optionally containing an heteroatom selected from the group consisting of: N, O and S;
or R₁₅ₐ and R₁₆ₐ are absent and R_{15b} and R_{16b} are joined together to form an aryl or heteroaryl ring, each being optionally substituted with one or more substituents each independently selected from the group consisting of: halogen, NH₂, NHSOC₁₋₆alkyl, NHSO₂C₁₋₆alkyl, NHC₁₋₆alkyl, N(C₁₋₆alkyl)₂, NHC(O)C₁₋₆alkyl, NHC(O)OC₁₋₆alkyl, CN, OH, C₁₋₆alkyl, C₁₋₆alkoxy, haloC₁₋₆alkyl and haloC₁₋₆alkoxy;
or R_{15b} and R_{16b} are joined together to form a C₅-₇cycloalkyl or C₅-₇heterocycloalkyl ring, each being independently optionally substituted with one or more substituents each independently selected from the group consisting of: halogen, NH₂, NHSOC₁₋₆alkyl, NHSO₂C₁₋₆alkyl, NHC₁₋₆alkyl, N(C₁₋₆alkyl)₂, NHC(O)C₁₋₆alkyl, NHC(O)OC₁₋₆alkyl, CN, OH, =O, C₁₋₆alkyl, C₁₋₆alkoxy, haloC₁₋₆alkyl, haloC₁₋₆alkoxy, SO₂CH₃;
R₁₀ is H, SO₂C₁₋₆alkyl, C(O)C₁₋₆alkyl, C(O)OC₁₋₆alkyl or C₁₋₆alkyl optionally substituted with one or more groups each independently selected from the group consisting of: OH, NH₂, NHC(O)C₁₋₆alkyl, C₁₋₆alkoxy, halogen, cyano, aryl and heteroaryl;
or R₁₀ and R₁₀ₐ are joined together to form a C₃₋₇heterocycloalkyl ring optionally containing another heteroatom selected from the group consisting of: N, S and O and optionally substituted with one or more groups each independently selected from the group consisting of: C₁₋₆alkyl, halogen, OH and CN;
or R₁ and R₂ form together with the nitrogen atom to which they are attached a monocyclic or polycyclic heteroaryl or partially unsaturated heteroaryl ring selected from the group consisting of: pyrrole, pyrazole, indole, benzimidazole, 2H-pyrazolo[3,4-b]pyridine, indazole, 2H-pyrazolo[3,4-c]pyridine, 6H-pyrrolo[3,4-b]pyridine, 6H-pyrrolo[3,4-b]pyrazine, 6H-pyrrolo[3,4-d]pyrimidine, 2H-pyrazolo[3,4-d]pyrimidine and 1,2,3,4-tetrahydroquinoline, and each of said ring is optionally substituted with one or more groups each independently selected from the group consisting of: halogen, C₁-₆alkyl, C₁-₆alkoxy, haloC₁₋₆alkyl, haloC₁₋₆alkoxy, OH, CN, SO₂C₁-₆alkyl, NH₂ and C(O)NH₂;
R₄ is a ring selected from the group consisting of: phenyl, pyridine, pyrimidine, pyrazine, pyridazine, 1,2,4-triazine, quinoline, indazole, benzothiophene, isoquinoline, thiophene, imidazopyridine, naphthyridine, quinazoline, benzimidazole, indoline, isoindoline, 1,3-dihydroisobenzofuran, 1,2,3,4-tetrahydroquinoline, 3,4-dihydro-2H-benzo[b][1,4]oxazine, 2,3,4,5-tetrahydrobenzo[f][1,4]oxazepine, quinazolin-4(3H)-one, indolin-2-one and 2,3-dihydro-1H-inden-1-one, each of said ring being optionally substituted with one or more groups each independently selected from the group consisting of: halogen, hydroxy, cyano, C₁-₆alkyl, C₁-₇alkenyl, C₁-₆alkoxy, haloC₁-₆alkyl, haloC₁-₆alkoxy, C₃-₇cycloalkyl, cyano-C₃-₇cycloalkyl, -SF₅, C₅₋₇heterocycloalkoxy, optionally substituted aryl or heteroaryl, partially unsaturated heteroaryl, optionally substituted aryloxy, C(O)OH, C(O)OC₁-₆alkyl, C(O)C₁-₆alkyl, C₁-₆alkylCOOH, SO₂C₁-₆alkyl and N(R₇)₂;
or a pharmaceutically acceptable salt, tautomer, solvate, or stereoisomer thereof.

4. The compound according to any one of previous claims having general Formula (IA) or (IB): 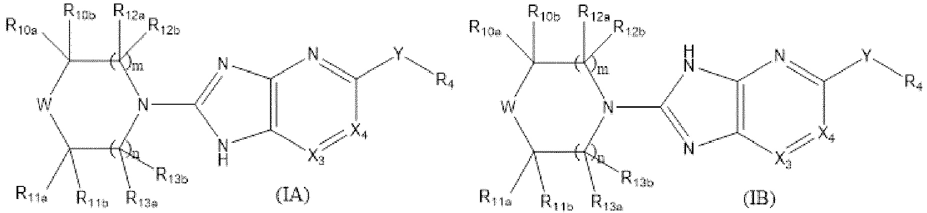 wherein: X₃, X₄, Y, R₄, m, n, W, R₁₀ₐ, R_{10b}, R₁₁ₐ, R_{11b}, R₁₂ₐ, R_{12b}, R₁₃ₐ and R_{13b} are as defined in any one of previous claims, or a pharmaceutically acceptable salt, tautomer, solvate, or stereoisomer thereof.

5. The compound according to any one of previous claims, wherein R₁ and R₂ together with the nitrogen atom to which they are attached form a a cyclic amine selected from the group consisting of: aziridine, azetidine, pyrrolidine, piperidine, azepane, morpholine, thiomorpholine, piperazine, 1,4-diazepane, 1,5-diazocane, 8-azaspiro[4.5]decane, 1,7-diazaspiro[3.5]nonane, 2,6-diazaspiro[3.5]nonane, 4,5,6,7-tetrahydro-1H-pyrazolo[4,3-c]pyridine, 5,6,7,8-tetrahydro-1,7-naphthyridine, 4,5,6,7-tetrahydro-1H-imidazo[4,5-c]pyridine, 1,7-diazaspiro[3.5]nonane, 1-oxa-3,7-diazaspiro[4.5]decan-2-one, (1S,4S)-2,5-diazabicyclo[2.2.2]octane, 1-oxa-8-azaspiro[4.5]decane, 2-oxa-8-azaspiro[4.5]decane, 1,3-dihydrospiro[indene-2,4'-piperidine], 5,7-dihydrospiro[cyclopenta[b]pyridine-6,4'-piperidine], 5,7-dihydrospiro[cyclopenta[c]pyridine-6,4'-piperidine], 5,6,7,8-tetrahydro-1,6-naphthyridine, octahydropyrrolo[3,2-b]pyrrole, octahydropyrrolo[3,4-b]pyrrole, 3,9-diazabicyclo[4.2.1]nonane, 3,8-diazabicyclo[3.2.1]octane, , 3-azabicyclo[3.1.0]hexane, 2,6-diazaspiro[3.4]octane, 3-azabicyclo[3.2.1]octane, 6-azabicyclo[3.2.1]octane, 5-oxa-2,8-diazaspiro[3.5]nonane, 3,9-diazabicyclo[3.3.1]nonane, 1,2,3,4-tetrahydroisoquinoline, 1-oxa-4,8-diazaspiro[5.5]undecane, hexahydro-1H-thieno[3,4-c]pyrrole 2,2-dioxide, 2-azaspiro[3.4]octane, 5-azaspiro[3.4]octane, 2,7-diazaspiro[4.6]undecane, 4,5,6,7-tetrahydrothiazolo[4,5-c]pyridine, 1-oxa-9-azaspiro[5.5]undecane, 6,8-diazaspiro[3.5]nonane, 6-azaspiro[3.5]nonane, tetrahydro-1H,4H-3a,6a-(methanoiminomethano)cyclopenta[c]pyrrole, 8-oxa-2-azaspiro[4.5]decane, 6-oxa-2,9-diazaspiro[4.5]decane, 1-oxa-4-azaspiro[5.5]undecane, 8-thia-2-azaspiro[4.5]decane 8,8-dioxide, 3',4'-dihydro-2'H-spiro[azetidine-3,r-pyrrolo[1,2-a]pyrazine], 2,6-diazabicyclo[3.2.2]nonane, 2,7-diazaspiro[4.4]nonane, 3H-spiro[benzofuran-2,4'-piperidine];
each of said cyclic amine being optionally substituted with one or more substituents each independently selected from the group consisting of:, C₁₋₆alkyl, aminoC₁₋₆alkyl, C₁₋₆alkoxy, halogen, NH₂, CN, OH, C(O)NH₂, heteroaryl, optionally substituted aryl, hydroxy-C₁₋₆alkyl, halo-C₁₋₆alkyl, C₁₋₆alkoxy, C₃₋₉heterocycloalkyl; or a pharmaceutically acceptable salt, tautomer, solvate, or stereoisomer thereof.

6. The compound according to any one of previous claims, wherein Y is S or a pharmaceutically acceptable salt, tautomer, solvate, or stereoisomer thereof.

7. The compound according to any one of previous claims, wherein R₄ is an aryl or heteroaryl ring selected from the group consisting of: phenyl, pyridine, pyrimidine, pyrazine, pyridazine, 1,2,4-triazine, quinoline, isoquinoline, indolin-2-one, indoline and isoindoline and each of said aryl or heteroaryl ring is optionally independently substituted with one or more groups each independently selected from the group consisting of: halogen, cyano, NH₂, CF₃, NHCH₃, NHCOCH₃, cyclopropanamine, cyclobutanamine, azetidine and pyrrolidine, each of said cyclopropanamine, cyclobutanamine, azetidine or pyrrolidine being optionally substituted with one or more groups independently selected from the group consisting of: OH, halogen, cyano and methyl; or a pharmaceutically acceptable salt, tautomer, solvate, or stereoisomer thereof.

8. A compound according to claim 1 selected from:
- 4-methyl-1-(6-((2-(trifluoromethyl)pyridin-3-yl)thio)-1H-imidazo[4,5-b]pyrazin-2-yl)piperidin-4-amine
- (8-(6-((2-(trifluoromethyl)pyridin-3-yl)thio)-1H-imidazo[4,5-b]pyrazin-2-yl)-8-azaspiro[4.5]decan-1-yl)methanamine
- (R)-8-(6-((2,3-dichlorophenyl)thio)-1H-imidazo[4,5-b]pyrazin-2-yl)-8-azaspiro[4.5]decan-1-amine
- 2-(1,7-diazaspiro[3.5]nonan-7-yl)-5-((2-(trifluoromethyl)pyridin-3-yl)thio)-1H-imidazo[4,5-b]pyrazine
- 1-(5-((2-(trifluoromethyl)pyridin-3-yl)thio)-1H-imidazo[4,5-b]pyrazin-2-yl)piperidin-4-amine
- 2-(4-((2-(4-amino-4-methylpiperid-1-yl)-1H-imidazo[4,5-b]pyrazin-6-yl)thio)phenyl)-2,4-dihydro-3H-1,2,4-triazol-3-one
- 4-methyl-1-(6-(phenylthio)-1H-imidazo[4,5-b]pyrazin-2-yl)piperidin-4-amine
- 4-methyl-1-(5-((2-(trifluoromethyl)phenyl)thio)-1H-imidazo[4,5-b]pyrazin-2-yl)piperidin-4-amine
- 4-((2-(4-amino-4-methylpiperid-1-yl)-1H-imidazo[4,5-b]pyrazin-5-yl)thio)-3-(trifluoromethyl)benzonitrile
- 1-(5-((2,4-difluorophenyl)thio)-1H-imidazo[4,5-b]pyrazin-2-yl)-4-methylpiperidin-4-amine
- 1-(5-((2,3-difluorophenyl)thio)-1H-imidazo[4,5-b]pyrazin-2-yl)-4-methylpiperidin-4-amine
- 1-(5-((2,3-dichlorophenyl)thio)-1H-imidazo[4,5-b]pyrazin-2-yl)-4-methylpiperidin-4-amine
- 2-((2-(4-amino-4-methylpiperid-1-yl)-1H-imidazo[4,5-b]pyrazin-5-yl)thio)benzonitrile
- 1-(5-((2-methoxyphenyl)thio)-)-1H-imidazo[4,5-b]pyrazin-2-yl)-4-methylpiperidin-4-amine
- 1-(5-((3-chloropyridin-4-yl)thio)-1H-imidazo[4,5-b]pyrazin-2-yl)-4-methylpiperidin-4-amine
- 1-(5-((2-(trifluoromethyl)pyridin-3-yl)thio)-1H-imidazo[4,5-b]pyrazin-2-yl)piperidin-3-amine
- N-(piperidin-4-yl)-5-((2-(trifluoromethyl)pyridin-3-yl)thio)-1H-imidazo[4,5-b]pyrazin-2-amine
- 1-(5-((2-bromophenyl)thio)-1H-imidazo[4,5-b]pyrazin-2-yl)-4-methylpiperidin-4-amine
- 1-(5-((4-chloro-2-methylphenyl)thio)-1H-imidazo[4,5-b]pyrazin-2-yl)-4-methylpiperidin-4-amine
- 1-(5-((2,3-dimethylphenyl)thio)-1H-imidazo[4,5-b]pyrazin-2-yl)-4-methylpiperidin-4-amine
- 2-(1,7-diazaspiro[3.5]nonan-1-yl)-5-((2-(trifluoromethyl)pyridin-3-yl)thio)-1H-imidazo[4,5-b]pyrazine
- 4-methyl-1-(5-((6-(trifluoromethyl)pyridin-2-yl)thio)-1H-imidazo[4,5-b]pyrazin-2-yl)piperidin-4-amine
- 4-methyl-1-(5-((3-(2-methylthiazol-4-yl)phenyl)thio)-1H-imidazo[4,5-b]pyrazin-2-yl)piperidin-4-amine
- 4-methyl-1-(6-(quinolin-5-ylthio)-1H-imidazo[4,5-b]pyrazin-2-yl)piperidin-4-amine
- 4-methyl-1-(1-methyl-5-((2-(trifluoromethyl)pyridin-3-yl)thio)-1H-imidazo[4,5-b]pyrazin-2-yl)piperidin-4-amine
- 1-(5-([1,1'-biphenyl]-3-ylthio)-)-1H-imidazo[4,5-b]pyrazin-2-yl)-4-methylpiperidin-4-amine
- 4-methyl-1-(5-(naphthalen-1-ylthio)-1H-imidazo[4,5-b]pyrazin-2-yl)piperidin-4-amine
- 4-methyl-1-(5-(quinolin-4-ylthio)-1H-imidazo[4,5-b]pyrazin-2-yl)piperidin-4-amine
- 1-(5-((1,5-naphthyridin-4-yl)thio)-1H-imidazo[4,5-b]pyrazin-2-yl)-4-methylpiperidin-4-amine
- 1-(5-((2-bromopyridin-4-yl)thio)-1H-imidazo[4,5-b]pyrazin-2-yl)-4-methylpiperidin-4-amine
- 1-(5-(isoquinolin-5-ylthio)-1H-imidazo[4,5-b]pyrazin-2-yl)-4-methylpiperidin-4-amine
- 4-methyl-1-(5-(quinoxalin-5-ylthio)-1H-imidazo[4,5-b]pyrazin-2-yl)piperidin-4-amine
- 1-(5-((2-chlorothiophen-3-yl)thio)-1H-imidazo[4,5-b]pyrazm-2-yl)-4-methylpiperidm-4-amine
- 4-methyl-l-(5-((2-(pentafluoro-16-sulfaneyl)phenyl)thio)-1H-imidazo[4,5-b]pyrazin-2-yl)piperidin-4-amine
- 1-(5-((1,3-dihydroisobenzofuran-4-yl)thio)-1H-imidazo[4,5-b]pyrazin-2-yl)-4-methylpiperidin-4-amine
- 4-methyl-1-(5-(naphthalen-2-ylthio)-1H-imidazo[4,5-b]pyrazin-2-yl)piperidin-4-amine
- 4-methyl-1-(5-(quinolin-8-ylthio)-1H-imidazo[4,5-b]pyrazin-2-yl)piperidin-4-amine
- 1-(5-(isoquinolin-8-ylthio)-1H-imidazo[4,5-b]pyrazin-2-yl)-4-methylpiperidin-4-amine
- 4-methyl-1-(5-((3-(pentafluoro-16-sulfaneyl)phenyl)thio)-1H-imidazo[4,5-b]pyrazin-2-yl)piperidin-4-amine
- 3-((2-(4-amino-4-methylpiperidin-1-yl)-1H-imidazo[4,5-b]pyrazin-5-yl)thio)benzoic acid
- 4-((2-(4-amino-4-methylpiperidin-1-yl)-1H-imidazo[4,5-b]pyrazin-5-yl)thio)benzoic acid
- (3S,4S)-3-methyl-8-(5-((2-(trifluoromethyl)pyridin-3-yl)thio)-1H-imidazo[4,5-b]pyrazin-2-yl)-2-oxa-8-azaspiro[4.5]decan-4-amine
- 4-methyl-1-(5-((8-(trifluoromethyl)quinolin-4-yl)thio)-1H-imidazo[4,5-b]pyrazin-2-yl)piperidin-4-amine
- 1-(5-((2-chlorophenyl)thio)-1H-imidazo[4,5-b]pyrazin-2-yl)-4-methylpiperidin-4-amine
- 4-((2-(4-amino-4-methylpiperidin-1-yl)-1H-imidazo[4,5-b]pyrazin-5-yl)thio)-3-chlorobenzoic acid
- 2-(3-((2-(4-amino-4-methylpiperidin-1-yl)-1H-imidazo[4,5-b]pyrazin-5-yl)thio)phenyl)acetic acid
- 1-(5-((1,8-naphthyridin-4-yl)thio)-1H-imidazo[4,5-b]pyrazin-2-yl)-4-methylpiperidin-4-amine
- (S)-1'-(5-((2-(trifluoromethyl)pyridin-3-yl)thio)-1H-imidazo[4,5-b]pyrazin-2-yl)-1 ,3-dihydrospiro[indene-2,4'-piperidin]-1-amine
- 1-(5-((3-chloro-2-methylphenyl)thio)-1H-imidazo[4,5-b]pyrazin-2-yl)-4-methylpiperidin-4-amine
- 1-(5-((2-isopropylphenyl)thio)-1H-imidazo[4,5-b]pyrazin-2-yl)-4-methylpiperidin-4-amine
- (S)-1'-(6-chloro-5-((2-(trifluoromethyl)pyridin-3-yl)thio)-1H-imidazo[4,5-b]pyrazin-2-yl)-1 ,3-dihydrospiro[indene-2,4'-piperidin]-]-1-amine
- 2-(9,9-dimethyl-3,7-diazabicyclo[3.3.1]nonan-3-yl)-5-((2-(trifluoromethyl)pyridin-3-yl)thio)-1H-imidazo[4,5-b]pyrazine
- 2-(3,9-diazabicyclo[4.2.1]nonan-3-yl)-5-((2-(trifluoromethyl)pyridin-3-yl)thio)-1H-imidazo [4,5 -b]pyrazine
- N-(5-azaspiro[3.5]nonan-8-yl)-6-((2-(trifluoromethyl)pyridin-3-yl)thio)-1H-imidazo[4,5-b]pyrazin-2-amine
- (8-(6-((2-(trifluoromethyl)pyridin-3-yl)thio)-1H-imidazo[4,5-b]pyrazin-2-yl)-8-azaspiro[4.5]decan-1-yl)methanamine
- N-((5-phenylpyrrolidin-3-yl)methyl)-5-((2-(trifluoromethyl)pyridin-3-yl)thio)-1H-imidazo[4,5-b]pyrazin-2-amine
- (S)-1'-(6-((3-chloro-2-(methylamino)pyridin-4-yl)thio)-1H-imidazo[4,5-b]pyrazin-2-yl)-1,3-dihydrospiro[indene-2,4'-piperidin]-1-amine
- (S)-1'-(5-((2-amino-3-chloropyridin-4-yl)thio)-1H-imidazo[4,5-b]pyrazin-2-yl)-1,3-dihydrospiro[indene-2,4'-piperidin]-1-amine
- (S)-1'-(5-((2,3-dichlorophenyl)thio)-1H-imidazo[4,5-b]pyrazin-2-yl)-1,3-dihydrospiro[indene-2,4'-piperidin]-1-amine
- (S)-4-((2-(1-amino-1,3-dihydrospiro[indene-2,4'-piperidin]-1'-yl)-1H-imidazo[4,5-b]pyrazin-5-yl)thio)-3,3-difluoro-1-methylindolin-2-one
- (S)-1-(4-((2-(1-amino-1,3-dihydrospiro[indene-2,4'-piperidin]-1'-yl)-1H-imidazo[4,5-b]pyrazin-5-yl)thio)-3,3-difluoroindolin-1-yl)ethan-1-one
- (S)-1'-(5-((2,2-difluorobenzo[d][1,3]dioxol-4-yl)thio)-1H-imidazo[4,5-b]pyrazin-2-yl)-1,3-dihydrospiro[indene-2,4'-piperidin]-1-amine
- (S)-1'-(5-((2-(trifluoromethyl)pyridin-3-yl)thio)-1H-imidazo[4,5-b]pyrazin-2-yl)-5,7-dihydrospiro[cyclopenta[b]pyridine-6,4'-piperidin]-5-amine
- (S)-3-((2-(1-amino-1,3-dihydrospiro[indene-2,4'-piperidin]-1'-yl)-1H-imidazo[4,5-b]pyrazin-5-yl)thio)-2-(trifluoromethyl)pyridine 1-oxide
- (R)-1'-(5-((2-(trifluoromethyl)pyridin-3-yl)thio)-1H-imidazo[4,5-b]pyrazin-2-yl)-3H-spiro[benzofuran-2,4'-piperidin]-3-amine
- 1-(2-((2,3-dichlorophenyl)thio)-7H-purin-8-yl)-4-methylpiperidin-4-amine
- (S)-1'-(5-((2-amino-3-chloropyridin-4-yl)thio)-1H-imidazo[4,5-b]pyrazin-2-yl)-5,7-dihydrospiro[cyclopenta[b]pyridine-6,4'-piperidin]-5-amine
- (S)-1'-(5-((3-chloro-2-(methylamino)pyridin-4-yl)thio)-1H-imidazo[4,5-b]pyrazin-2-yl)-5,7-dihydrospiro[cyclopenta[b]pyridine-6,4'-piperidin]-5-amine
- (S)-N-(4-((2-(1-amino-1,3-dihydrospiro[indene-2,4'-piperidin]-1'-yl)-1H-imidazo[4,5-b]pyrazin-5-yl)thio)-3-chloropyridin-2-yl)acetamide
- (S)-1'-(5-((3-chloropyridin-4-yl)thio)-1H-imidazo[4,5-b]pyrazin-2-yl)-1,3-dihydrospiro[indene-2,4'-piperidin]-1-amine
- (R)-1'-(5-((2-(trifluoromethyl)pyridin-3-yl)thio)-1H-imidazo[4,5-b]pyrazin-2-yl)-1,3-dihydrospiro[indene-2,4'-piperidin]-1-amine
- (R)-1'-(5-((2-amino-3-chloropyridin-4-yl)thio)-1H-imidazo[4,5-b]pyrazin-2-yl)-3H-spiro[benzofuran-2,4'-piperidin]-3-amine
- (S)-1'-(5-((3-chloropyridin-4-yl)thio)-1H-imidazo[4,5-b]pyrazin-2-yl)-5,7-dihydrospiro[cyclopenta[b]pyridine-6,4'-piperidin]-5-amine
- (S)-1'-(5-((3-chloro-2-methoxypyridin-4-yl)thio)-1H-imidazo[4,5-b]pyrazin-2-yl)-5,7-dihydrospiro[cyclopenta[b]pyridine-6,4'-piperidin]-5-amine
- (S)-4-((2-(1-amino-1,3-dihydrospiro[indene-2,4'-piperidin]-1'-yl)-1H-imidazo[4,5-b]pyrazin-5-yl)thio)-3-chloropyridin-2-ol
- (S)-1'-(5-((3-chloro-2-(dimethylamino)pyridin-4-yl)thio)-1H-imidazo[4,5-b]pyrazin-2-yl)-5,7-dihydrospiro[cyclopenta[b]pyridine-6,4'-piperidin]-5-amine
- (R)-1'-(5-((3-chloropyridin-4-yl)thio)-1H-imidazo[4,5-b]pyrazin-2-yl)-3H-spiro[benzofuran-2,4'-piperidin]-3-amine
- (S)-1'-(5-((4-(trifluoromethyl)pyrimidin-5-yl)thio)-1H-imidazo[4,5-b]pyrazin-2-yl)-1 ,3-dihydrospiro[indene-2,4'-piperidin]-1-amine
- (S)-8-((2-(1-amino-1,3-dihydrospiro[indene-2,4'-piperidin]-1'-yl)-1H-imidazo[4,5-b]pyrazin-5-yl)thio)-2H-benzo[b][1,4]oxazin-3(4H)-one
- (S)-1'-(5-((3-chloropyrazin-2-yl)thio)-1H-imidazo[4,5-b]pyrazin-2-yl)-1,3-dihydrospiro[indene-2,4'-piperidin]-1-amine
- (R)-1'-(5-((2-amino-3-chloropyridin-4-yl)thio)-1H-imidazo[4,5-b]pyrazin-2-yl)-3H-spiro[benzofuran-2,4'-piperidin]-3-amine
- (S)-1'-(5-((3-chloro-2-(cyclopropylamino)pyridin-4-yl)thio)-1H-imidazo[4,5-b]pyrazin-2-yl)-1,3-dihydrospiro[indene-2,4'-piperidin]-1-amine
- (S)-1'-(6-((3-chloro-2-(cyclopropylamino)pyridin-4-yl)thio)-1H-imidazo[4,5-b]pyrazin-2-yl)-5,7-dihydrospiro[cyclopenta[b]pyridine-6,4'-piperidin]-5-amine
- (S)-4-((2-(1-amino-1,3-dihydrospiro[indene-2,4'-piperidin]-1'-yl)-1H-imidazo[4,5-b]pyrazin-5-yl)thio)-3,3-difluoroindolin-2-one
- (R)-1'-(5-((3-chloropyridin-4-yl)thio)-1H-imidazo[4,5-b]pyrazin-2-yl)-3H-spiro[furo[3,2-b]pyridine-2,4'-piperidin]-3-amine
- (S)-1'-(6-((1,5-naphthyridin-4-yl)thio)-1H-imidazo[4,5-b]pyrazin-2-yl)-1,3-dihydrospiro[indene-2,4'-piperidin]-1-amine
- (S)-1-(4-((2-(1-amino-1,3-dihydrospiro[indene-2,4'-piperidin]-1'-yl)-1H-imidazo[4,5-b]pyrazin-5-yl)thio)-3-chloropyridin-2-yl)azetidin-3-ol
- (S)-4-((2-(1-amino-1,3-dihydrospiro[indene-2,4'-piperidin]-1'-yl)-1H-imidazo[4,5-b]pyrazin-6-yl)thio)-3-chloro-1-methylpyridin-2(1H)-one
- (S)-1'-(6-((2,3-dihydrobenzo[b][1,4]dioxin-5-yl)thio)-1H-imidazo[4,5-b]pyrazin-2-yl)-1,3-dihydrospiro[indene-2,4'-piperidin]-1-amine
- (S)-6-((2-(5-amino-5,7-dihydrospiro[cyclopenta[b]pyridine-6,4'-piperidin]-1'-yl)-1H-imidazo[4,5-b]pyrazin-6-yl)thio)-4-chlorobenzo[d]oxazol-2(3H)-one
- (S)-6-((2-(5-amino-5,7-dihydrospiro[cyclopenta[b]pyridine-6,4'-piperidin]-1'-yl)-1H-imidazo[4,5-b]pyrazin-6-yl)thio)-5-chloro-2H-benzo[b][1,4]oxazin-3(4H)-one
or a pharmaceutically acceptable salt, tautomer, solvate, or stereoisomer thereof.

9. A compound or a pharmaceutically acceptable salt, tautomer, solvate, or stereoisomer thereof as defined in any one of previous claims for medical use.

10. The compound or the pharmaceutically acceptable salt, tautomer, solvate, or stereoisomer thereof as defined in claim 10 for use in inhibiting SHP2 activity.

11. The compound or the pharmaceutically acceptable salt, tautomer, solvate, or stereoisomer thereof as defined in claim 9 or 10 for use in the treatment and/or prevention of a disease or disorder mediated by the activity of SHP2, wherein the disease or disorder mediated by the activity of SHP2 is preferably selected from the group consisting of: cancer, cardiovascular disease, immunological disorder, fibrosis, an ocular disorder, systemic lupus erythematosus, diabetes, neutropenia, and combinations thereof, preferably said cancer is a cancer metastasis, wherein more preferably the disease or disorder mediated by the activity of SHP2 is selected from the group consisting of: Noonan Syndrome, Leopard Syndrome, juvenile myelomonocytic leukemias, neuroblastoma, melanoma, head and neck squamous-cell carcinoma, acute myeloid leukemia, breast cancer, esophageal tumor, lung cancer, colon cancer, head cancer, gastric carcinoma, lymphoma, glioblastoma, gastric cancer, pancreatic cancer and combinations thereof.

12. The compound or the pharmaceutically acceptable salt, tautomer, solvate, or stereoisomer thereof according to any one of claims 9-11, for use in combination with radiotherapy or with at least one further therapeutic agent, preferably said at least one further therapeutic agent is selected from the group consisting of:
(a) alkylating agents, including but not limited to carmustine, chlorambucil (LEUKERAN), cisplatin (PLATIN), carboplatin (PARAPLATIN), oxaliplatin (ELOXATIN), streptozocin (ZANOSAR), busulfan (MYLERAN), dacarbazine, ifosfamide, lomustine (CCNU), melphalan (ALKERAN), procarbazine (MATULAN), temozolomide(TEMODAR), thiotepa, and cyclophosphamide (ENDOXAN);
(b) anti-metabolites, including but not limited to cladribine (LEUSTATIN), mercaptopurine (PURINETHOL), thioguanine, pentostatin (NIPENT), cytosine arabinoside (cytarabine, ARA-C), gemcitabine (GEMZAR), fluorouracil (5-FU, CARAC), capecitabine (XELODA), leucovorin (FUSILEV), methotrexate (RHEUMATREX) and raltitrexed;
(c) antimitotics, which are often plant alkaloids and terpenoids, or derivatives thereof, including but not limited to taxanes such as docetaxel (TAXITERE) and paclitaxel (ABRAXANE, TAXOL); vinca alkaloids such as vincristine (ONCOVIN), vinblastine, vindesine, vinorelbine (NAVELBINE), and vinflunine;
(d) checkpoint inhibitors, such as anti- PD-1 or PD-L1 antibodies pembrolizumab (KEYTRUDA), nivolumab (OPDIVO), MEDI4736 and MPDL3280A; anti-CTLA-4 antibody ipilimumab (YERVOY); inhibitors that target LAG3 (lymphocyte activation gene 3 protein), KIR (killer cell immunoglobulin-like receptor), 4-1BB (tumour necrosis factor receptor superfamily member 9), TIM3 (T-cell immunoglobulin and mucin-domain containing-3) and/or OX40 (tumour necrosis factor receptor superfamily member 4);
(e) topoisomerase inhibitors, including but not limited to camptothecin (CTP), irinotecan (CAMPTOSAR), topotecan (HYCAMTIN), teniposide (VUMON) and etoposide (EPOSIN);
(f) cytotoxic antibiotics, including but not limited to actinomycin D (dactinomycin, COSMEGEN), bleomycin (BLENOXANE) doxorubicin (ADRIAMYCIN), daunorubicin (CERUBIDINE), epirubicin (ELLENCE), fludarabine (FLUDARA), idarubicin, mitomycin (MITOSOL), mitoxantrone (NOVANTRONE), plicamycin; (7) aromatase inhibitors, including but not limited to aminoglutethimide, anastrozole (ARIMIDEX), letrozole (FEMARA), vorozole (RIVIZOR) and exemestane (AROMASIN);
(g) angiogenesis inhibitors, including but not limited to genistein, sunitinib (SUTENT) and bevacizumab (AVASTIN);
(h) anti-steroids and anti-androgens such as aminoglutethimide (CYTADREN), bicalutamide (CASODEX), cyproterone, flutamide (EULEXIN) and nilutamide (NILANDRON);
(i) tyrosine kinase inhibitors, including but not limited to imatinib (GLEEVEC), erlotinib (TARCEVA), lapatininb (TYKERB), sorafenib (NEXAVAR), and axitinib (INLYTA);
(j) mTOR inhibitors such as everolimus, temsirolimus (TORISEL), and sirolimus; (12) monoclonal antibodies such as trastuzumab (HERCEPTIN) and rituximab (RITUXAN);
(k) other agents, such as amsacrine; Bacillus Calmette-Guerin (B-C-G) vaccine; buserelin (ETILAMIDE); chloroquine (ARALEN); clodronate, pamidronate, and other bisphosphonates; colchicine; demethoxyviridin; dichloroacetate; estramustine; filgrastim (NEUPOGEN); fludrocortisone (FLORINEF); goserelin (ZOLADEX); interferon; leucovorin; leuprolide (LUPRON); levamisole; lonidamine; mesna; metformin; mitotane (o,p'-DDD, LYSODREN); nocodazole; octreotide (SANDOSTATIN); perifosine; porfimer (particularly in combination with photo- and radiotherapy); suramin; tamoxifen; titanocene dichloride; tretinoin; anabolic steroids such as fluoxymesterone(HALOTESTIN); estrogens such as estradiol, diethylstilbestrol (DES), and dienestrol; progestins such as medroxyprogesterone acetate (MPA) and megestrol; testosterone; 5-fluoro-2-4(1H,3H)-pyrimidinedione and combinations thereof.

13. A pharmaceutical composition comprising the compound or the pharmaceutically acceptable salt, tautomer, solvate, or stereoisomer thereof as defined in anyone of claims 1-8, alone or in combination with at least one further therapeutic agent, and at least one pharmaceutically acceptable excipient, preferably said at least one further therapeutic agent is selected from the group consisting of:
(a) alkylating agents, including but not limited to carmustine, chlorambucil (LEUKERAN), cisplatin (PLATIN), carboplatin (PARAPLATIN), oxaliplatin (ELOXATIN), streptozocin (ZANOSAR), busulfan (MYLERAN), dacarbazine, ifosfamide, lomustine (CCNU), melphalan (ALKERAN), procarbazine (MATULAN), temozolomide(TEMODAR), thiotepa, and cyclophosphamide (ENDOXAN);
(b) anti-metabolites, including but not limited to cladribine (LEUSTATIN), mercaptopurine (PURINETHOL), thioguanine, pentostatin (NIPENT), cytosine arabinoside (cytarabine, ARA-C), gemcitabine (GEMZAR), fluorouracil (5-FU, CARAC), capecitabine (XELODA), leucovorin (FUSILEV), methotrexate (RHEUMATREX) and raltitrexed;
(c) antimitotics, which are often plant alkaloids and terpenoids, or derivatives thereof, including but not limited to taxanes such as docetaxel (TAXITERE) and paclitaxel (ABRAXANE, TAXOL); vinca alkaloids such as vincristine (ONCOVIN), vinblastine, vindesine, vinorelbine (NAVELBINE), and vinflunine;
(d) checkpoint inhibitors, such as anti- PD-1 or PD-L1 antibodies pembrolizumab (KEYTRUDA), nivolumab (OPDIVO), MEDI4736 and MPDL3280A; anti-CTLA-4 antibody ipilimumab (YERVOY); inhibitors that target LAG3 (lymphocyte activation gene 3 protein), KIR (killer cell immunoglobulin-like receptor), 4-1BB (tumour necrosis factor receptor superfamily member 9), TIM3 (T-cell immunoglobulin and mucin-domain containing-3) and/or OX40 (tumour necrosis factor receptor superfamily member 4);
(e) topoisomerase inhibitors, including but not limited to camptothecin (CTP), irinotecan (CAMPTOSAR), topotecan (HYCAMTIN), teniposide (VUMON) and etoposide (EPOSIN);
(f) cytotoxic antibiotics, including but not limited to actinomycin D (dactinomycin, COSMEGEN), bleomycin (BLENOXANE) doxorubicin (ADRIAMYCIN), daunorubicin (CERUBIDINE), epirubicin (ELLENCE), fludarabine (FLUDARA), idarubicin, mitomycin (MITOSOL), mitoxantrone (NOVANTRONE), plicamycin; (7) aromatase inhibitors, including but not limited to aminoglutethimide, anastrozole (ARIMIDEX), letrozole (FEMARA), vorozole (RIVIZOR) and exemestane (AROMASIN);
(g) angiogenesis inhibitors, including but not limited to genistein, sunitinib (SUTENT) and bevacizumab (AVASTIN);
(h) anti-steroids and anti-androgens such as aminoglutethimide (CYTADREN), bicalutamide (CASODEX), cyproterone, flutamide (EULEXIN) and nilutamide (NILANDRON);
(i) tyrosine kinase inhibitors, including but not limited to imatinib (GLEEVEC), erlotinib (TARCEVA), lapatininb (TYKERB), sorafenib (NEXAVAR), and axitinib (INLYTA);
(j) mTOR inhibitors such as everolimus, temsirolimus (TORISEL), and sirolimus; (12) monoclonal antibodies such as trastuzumab (HERCEPTIN) and rituximab (RITUXAN);
(k) other agents, such as amsacrine; Bacillus Calmette-Guérin (B-C-G) vaccine; buserelin (ETILAMIDE); chloroquine (ARALEN); clodronate, pamidronate, and other bisphosphonates; colchicine; demethoxyviridin; dichloroacetate; estramustine; filgrastim (NEUPOGEN); fludrocortisone (FLORINEF); goserelin (ZOLADEX); interferon; leucovorin; leuprolide (LUPRON); levamisole; lonidamine; mesna; metformin; mitotane (o,p'-DDD, LYSODREN); nocodazole; octreotide (SANDOSTATIN); perifosine; porfimer (particularly in combination with photo- and radiotherapy); suramin; tamoxifen; titanocene dichloride; tretinoin; anabolic steroids such as fluoxymesterone(HALOTESTIN); estrogens such as estradiol, diethylstilbestrol (DES), and dienestrol; progestins such as medroxyprogesterone acetate (MPA) and megestrol; testosterone; 5-fluoro-2-4(1H,3H)-pyrimidinedione and combinations thereof.

14. The pharmaceutical composition according to claim 13 for use in the treatment and/or prevention of a disease or disorder mediated by the activity of SHP2, wherein the disease or disorder mediated by the activity of SHP2 is preferably selected from the group consisting of: cancer, cardiovascular disease, immunological disorder, fibrosis, an ocular disorder, systemic lupus erythematosus, diabetes, neutropenia, and combinations thereof, preferably said cancer is a cancer metastasis, wherein more preferably the disease or disorder mediated by the activity of SHP2 is selected from the group consisting of: Noonan Syndrome, Leopard Syndrome, juvenile myelomonocytic leukemias, neuroblastoma, melanoma, head and neck squamous-cell carcinoma, acute myeloid leukemia, breast cancer, esophageal tumor, lung cancer, colon cancer, head cancer, gastric carcinoma, lymphoma, glioblastoma, gastric cancer, pancreatic cancer and combinations thereof.

15. A process for the synthesis of the compound of Formula (I) or the pharmaceutically acceptable salt, tautomer, solvate or stereoisomer thereof as defined in any one of claims 1 to 8, said process comprising at least one of the following steps:
a) reacting a compound of formula (A) with a compound of formula R₄YH in the presence of a transition metal catalyst or under photochemical conditions, wherein said transition metal catalyst is preferably a palladium or copper catalyst, such as Pd₂(dba)₃, Pd(PPh₃)₄ and CuI: 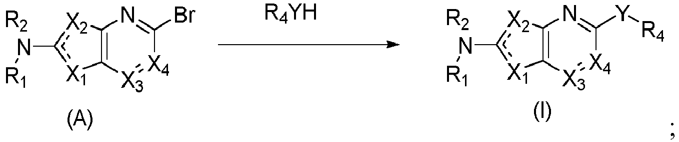 or
b) when in said compound of Formula (I) Y is S, reacting in a first step a compound of formula (A) with 2-ethylhexyl 3-mercaptopropanoate in the presence of a palladium catalyst, and further reacting in a second step the product from the first step with a compound of formula R₄X, wherein X is bromide, chloride, iodide or triflate, in the presence of a palladium catalyst, wherein said first and second steps are carried out in the presence of a tertiary amine, preferably DIPEA or TEA, and wherein the palladium catalyst in said first and/or second step is preferably Pd₂(dba)₃ or Pd(PPh₃)₄: 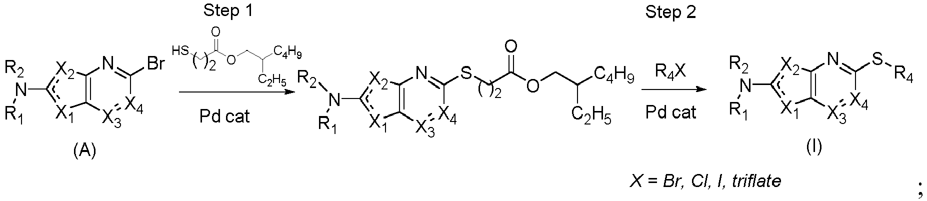 or
c) when in said compound of Formula (I) Y is a bond, reacting a compound of formula (A) with R₄-boronic acid in the presence of a palladium catalyst, preferably Pd(PPh₃)₄, and a base: 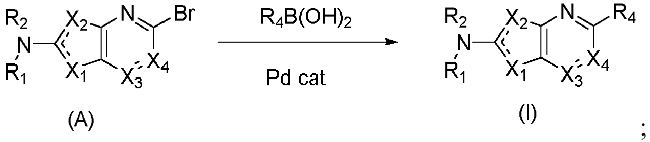 or
d) reacting a compound of formula (B), wherein Lg is a leaving group selected from the group consisting of halogen, SO₂Me and SOMe, with an amine of formula R₁R₂NH at temperature range of about 100°C to about 120°C: 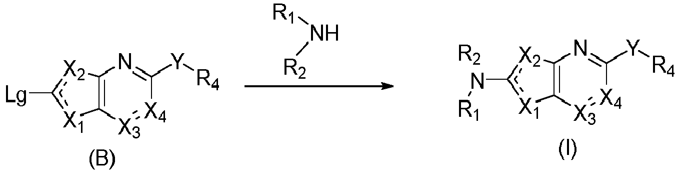 wherein in each of said a), b) c) or d) steps, if X₁ or X₂ is NH, it can be optionally protected for example as a trimethylsilylethoxymethyl (SEM) derivative.


SEQUENCE LISTING

<110>  IRBM S.P.A.
       C.N.C.C.S. S.C.A.R.L. Collezione Nazionale dei Composti
       Chimici e Centro Screening

<120>  SHP2 INHIBITORS

<130>  BE 140897

<160>  1

<170>  PatentIn version 3.5

<210>  1
<211>  18
<212>  PRT
<213>  Artificial Sequence

<220>
<223>  synthetic

<400>  1

Leu Asn Ile Asp Leu Asp Leu Val Leu Ser Thr Ala Ser Ile Asn Phe
1               5                   10                  15


Gln Lys